# EUROPÄISCHE PATENTANMELDUNG

(11) **EP 4 417 222 A2**
(43) Veröffentlichungstag der Anmeldung: **21.08.2024**
(21) Anmeldenummer: 24180881.5
(22) Anmeldetag: 22.03.2019
(51) Int. Cl.: A61L 2/14

(54) **PLASMAAPPLIKATOR**

(30) Priorität: 23.03.2018 DE 102018107049; 10.09.2018 DE 102018121978; 12.09.2018 DE 102018122309
(62) Teilanmeldung aus: 19713755.7
(71) Anmelder: Coldplasmatech GmbH, 17489 Greifswald (DE)
(72) Erfinder: Polak, Martin, 17498 Hinrichshagen (DE); Banaschik, Robert, 18184 Broderstorf (DE); Kühle, Axel, 17489 Greifswald (DE); Güra, Tobias, 17367 Eggesin (DE); Mahrenholz, Carsten, 17509 Lubmin (DE)
(74) Vertreter: Eisenführ Speiser

(57) **Zusammenfassung**

Die Erfindung betrifft einen elektrotechnischen Kern zum Erzeugen eines kalten Atmosphärendruck- oder Niederdruckplasmas für die Behandlung von menschlichen und/oder tierischen und/oder technischen Oberflächen. Der elektrotechnische Kern weist eine der zu behandelnden Oberfläche zugewandten Seite und eine der zu behandelnden Oberfläche abgewandten Seite auf und umfasst von der der zu behandelnden Oberfläche zugewandten Seite beginnend die folgenden übereinander angeordneten Schichten:
- eine erste Isolationsschicht,
- eine erste Elektrodenstruktur, die mit einer ersten Kontaktierung zum Herstellen eines elektrischen Kontakts zwischen der ersten Elektrodenstruktur und einer Energieversorgungseinheit verbunden ist, und im Betrieb geerdet ist,
- eine zweite Isolationsschicht, die ausgebildet ist, die erste Elektrodenstruktur und eine zweite Elektrodenstruktur galvanisch voneinander zu trennen,
- eine zweite Elektrodenstruktur, die mit einer zweiten Kontaktierung zum Herstellen eines elektrischen Kontakts zwischen der zweiten Elektrodenstruktur und einer Energieversorgungseinheit verbunden ist, und im Betrieb durch ein von einer Energieversorgungseinheit bereitgestellten und zum Zünden eines Plasmas ausreichendem Spannungssignal getrieben wird,
- eine dritte Isolationsschicht, die ausgebildet ist, die zweite Elektrodenstruktur und eine dritte Elektrodenstruktur galvanisch voneinander zu trennen,
- eine dritte Elektrodenstruktur, die mit einer dritten Kontaktierung versehen ist, um die dritte Elektrodenstruktur im Betrieb zu erden.

## Beschreibung

Die Erfindung betrifft einen Plasmaapplikator zum Erzeugen eines kalten Plasmas für die Behandlung von menschlichen oder tierischen oder technischen Oberflächen. Die Erfindung betrifft weiterhin einen elektrotechnischen Kern für einen Plasmaapplikator.

Typischerweise wird ein Plasmaapplikator zum Betrieb über ein Kabel an eine Energieversorgungseinheit, z.B. an einen Hochspannungsgenerator, angeschlossen, die ein Spannungssignal bereitstellt, das zum Zünden eines physikalischen Plasmas ausreicht. Die Energieversorgungseinheit kann ein Steuergerät umfassen, das beispielsweise Strom, Spannung oder Behandlungszeit steuert.

Ein Plasmaapplikator weist typischerweise einen elektrotechnischen Kern auf. Ein elektrotechnischer Kern umfasst ein Mehrschichtsystem, welches in Schichtdickenrichtung aufeinanderfolgend die folgenden Schichten umfasst: eine erste Isolationsschicht, eine erste Elektrodenstruktur, eine zweite Isolationsschicht, insbesondere einer Dielektrikumsschicht, eine zweiten Elektrodenstruktur. Wenn ein Plasmaapplikator mit einem solchen elektrotechnischen Kern auf einer zu behandelnden Oberfläche aufgelegt wird, ist die erste Isolationsschicht als unterste Schicht einerzu behandelnden Oberfläche zugeordnet. Die zweite Elektrodenstruktur wird typischerweise aktiv mit einem Spannungssignal getrieben und ist oftmals als eine Flächenelektrode ausgebildet. Die erste Elektrodenstruktur bildet in der Regel eine Gegenelektrode und liegt auf Masse.

Zwischen der zweiten Elektrodenstruktur und einer Gegenelektrode, die auch von einer zu behandelnden Oberfläche gebildet sein kann, wird im Betrieb eine Spannung angelegt, die moduliert sein kann und es typischerweise auch ist.

Das Potential der Gegenelektrode kann ein Massepotential oder ein davon abweichendes Potential sein. Als Spannungssignal wird hier die angelegte Spannung bezeichnet, die beispielsweise sinusförmig, dreieckförmig oder rechteckförmig moduliert sein kann. Weiterhin kann das Spannungssignal auch aus einzelnen Pulsen zusammengesetzt sein.

Der Einfachheit halber wird im Rahmen dieser Beschreibung immer wieder von einer "getriebenen" Elektrodenstruktur gesprochen. Die Spannung - also das Spannungssignal - wird immer zwischen der getriebenen Elektrodenstruktur und der Gegenelektrode angelegt. Da die Gegenelektrode typischerweise - aber nicht notwendigerweise - Massepotential hat, wird hier die vereinfachte Ausdrucksweise "das Spannungssignal wird an die getriebene Elektrodenstruktur angelegt" verwendet, um zu beschreiben, dass die angelegte Spannung zwischen getriebener Elektrodenstruktur und Gegenelektrode wirkt.

Ein elektrotechnischer Kern umfasst in der Regel einen Masseanschluss, der mit der ersten Elektrodenstruktur elektrisch leitend verbunden ist, sowie ein Hochspannungsanschluss, der mit der zweiten Elektrodenstruktur elektrisch leitend verbunden ist. An dem Masseanschluss und dem Hochspannungsanschluss werden die erste und zweite Elektrodenstruktur typischerweise über ein Kabel mit einer Energieversorgungseinheit verbunden, die im Betrieb ein zum Zünden eines Plasmas ausreichendes Spannungssignal bereitstellt, welches an die zweite Elektrodenstruktur übertragen wird.

Die zweite Isolationsschicht ist typischerweise zwischen der ersten und der zweiten Elektrodenstruktur angeordnet und isoliert die erste und die zweite Elektrodenstruktur elektrisch voneinander. Somit verhindert die zweite Isolationsschicht durch eine galvanische Trennung einen Kurzschluss zwischen der ersten und zweiten Elektrodenstruktur. Mittels einer in Form eines Spannungssignals an die zweite Elektrodenstruktur angelegten Spannung wird in dem abgeschlossenen Gasraum, der zwischen dem Plasmaapplikator und der zu behandelnden Oberfläche gebildet wird, ein zugeführtes Gas oder Gasgemisch wie z.B. Luft ionisiert und in einen reaktiven Zustand überführt. Es wird also ein physikalisches Plasma erzeugt. Das Plasma verteilt sich gleichmäßig im abgeschlossenen Gasraum und wechselwirkt mit der zu behandelnden Oberfläche.

Es sind auch elektrotechnische Kerne bekannt, die lediglich die zweite Elektrodenstruktur und die zweite Isolationsschicht aufweisen. Die zweite Isolationsschicht befindet sich dann im Betrieb auf der der zu behandelnden Oberfläche zugewandten Seite. Insbesondere die erste Elektrodenstruktur ist in solchen elektrotechnischen Kernen nicht vorgesehen, sondern die Funktion der Masseelektrode ist im Betrieb durch den menschlichen oder tierischen Körper bzw. die zu behandelnde Oberfläche selbst realisiert. Ein solcher elektrotechnischer Kern wird beispielsweise in DE 10 2017 100 161 A1 beschrieben. In diesem Fall bildet die zu behandelnde Oberfläche die Gegenelektrode.

Neben den drei Aggregatzuständen fest, flüssig oder gasförmig, wird Plasma als vierter Aggregatzustand bezeichnet. Wird einem Gas oder Gasgemisch hinreichend viel Energie, beispielsweise in Form von elektrischer Energie, zugefügt, so werden einige Atome des Gases ionisiert, d.h. Elektronen werden aus der Atomhülle entfernt und bewegen sich als freie Teilchen, sodass ein positiv geladenes Atom zurückbleibt. Wenn ein Gas aus einem ausreichend hohen Anteil an freien Ionen und Elektronen besteht, so bezeichnet man den Aggregatzustand als physikalisches Plasma. Physikalisches Plasma ist also Materie, dessen Bestandteile teilweise geladene Komponenten, Ionen und Elektronen sind, die sich als freie Ladungsträger bewegen.

Durch Stoßprozesse werden einige Atome des ionisierten Gases bzw. Gasgemisches in einen angeregten Zustand überführt. Beim Abregen geben diese Atome ihre Energie in Form von elektromagnetischer Strahlung ab, deren Spektrum vom UV-Bereich über den sichtbaren Spektralbereich bis hin zum IR-Bereich reicht. Die angeregten Atome und die Ionen können zusätzlich chemisch miteinander wechselwirken und sich zu neuen Molekülen binden.

Zum Erzeugen eines Plasmas sind z. B. Plasmadüsen bzw. Plasmajets, Torches, Koronaentladungen und die dielektrisch behinderte Entladung (DBE) bekannt. In dielektrisch behinderten Entladungen (DBE) werden Plasmen typischerweise im Atmosphärendruck weit ab vom thermischen Gleichgewicht erzeugt. Durch eine angelegte Wechselspannung bilden sich mit jeder Periode winzige Entladungskanäle aus. Da die Entladungskanäle nur einen Bruchteil des gesamten Entladungsvolumens ausmachen und die Zeitdauer der Entladung durch die kapazitive Kopplung stark begrenzt ist, bleibt die mittlere Gastemperatur in der Entladung in der Nähe der Raumtemperatur. Somit lässt sich durch die dielektrisch behinderte Entladung (DBE) ein kaltes Plasma im Atmosphären- oder Niederdruck erzeugen. Als Atmosphärendruckplasma bezeichnet man den Sonderfall eines physikalischen Plasmas, bei welchem der Gasdruck im Plasma ungefähr dem der umgebenden Atmosphäre - dem sogenannten Normaldruck - entspricht. Ist der Gasdruck im Plasma geringer als der Atmosphärendruck, so spricht man von einem Niederdruckplasma. Kaltes Plasma, mit einer Temperatur von unter 40°C, wird derzeit für Anwendungen in der Plasmamedizin untersucht und hat bereits erste Anwendungen gefunden.

Ein Hauptziel der Plasmamedizin ist die therapeutische Plasmaanwendung, d. h. physikalisches Plasma soll direkt auf den menschlichen oder tierischen Körper gebracht werden. Hierbei werden primär die Plasma-unterstützte Modifikation von biorelevanten Oberflächen, eine Plasma-basierte Biodekontamination/Sterilisation und eine direkte therapeutische Anwendung zur Förderung der Wundheilung verfolgt.

Mögliche Anwendungen liegen zum einen im Bereich der antimikrobiellen Wirkungen und zum anderen darin, Säugetierzellen und Gewebe gezielt und kontrollierbar zu modifizieren. Medizinische Plasmaanwendungen werden insbesondere im Zusammenhang mit der Unterstützung von Heilungsprozessen mit besonderem Schwerpunkt auf der Behandlung chronischer Wunden, der Behandlung infektiöser Hauterkrankungen sowie der Behandlung von entzündlichen Hauterkrankungen (Dermatitiden) diskutiert.

Nach gegenwärtigem Stand der Forschung sind wesentliche Wirkkomponenten von kalten Plasmen reaktive Stickstoff- und Sauerstoffspezies, UV-Strahlung, geladene Teilchen und elektrische Felder. Reaktive Stickstoff- und Sauerstoffspezies werden durch Einkoppeln von elektrischer Energie in an sich nicht biologisch-wirksame Gase (Argon, Helium, Stickstoff, Sauerstoff, Luft sowie Gemischen daraus) sowie durch eine anschließende Wechselwirkung mit angrenzenden Medien (atmosphärische Luft, Flüssigkeiten, Oberflächen) kurzzeitig und lokal gebildet. Elektrische Felder und durch sie hervorgerufene Signale spielen eine wichtige Rolle bei der Regulation von körpereigenen Heilungsvorgängen. Bei Verletzungen gehören diese Signale zu den ersten Signalen, die der Körper über eine Verletzung empfängt. So ist beispielweise die Elektrostimulation von Wunden eine anerkannte Behandlungsmethode, die gezielt körpereigene und physiologische Reparaturvorgänge reaktivieren und aufrechterhalten kann.

Von besonderem medizinischen Interesse sind hierbei flächige Plasmaanwendungen, die es erlauben, menschliche und/oder tierische Oberflächen, insbesondere Wunden, vollständig und homogen mit einem kalten Atomsphärendruckplasma zu behandeln.

In DE 10 2014 220 488 A1 wird eine Vorrichtung zum Erzeugen eines kalten Atmosphärendruckplasmas für die Behandlung von menschlichen und/oder tierischen Oberflächen beschrieben. Die Vorrichtung umfasst ein flexibles, flächiges Mehrschichtsystem mit einer der zu behandelnden Oberfläche zugewandten Seite und einer der zu behandelnden Oberfläche abgewandten Seite. Das Mehrschichtsystem, welches einen elektrotechnischen Kern bildet, umfasst die folgenden Schichten:
- eine erste Elektrodenschicht auf der abgewandten Seite des Mehrschichtsystems,
- eine zweite Elektrodenschicht auf der zugewandten Seite des Mehrschichtsystems, wobei die Elektrodenschicht eine Vielzahl von Aussparungen aufweist oder gitterartig oder mäanderförmig ausgebildet ist,
- eine Dielektrikumsschicht, die zwischen der ersten Elektrodenschicht und der zweiten Elektrodenschicht angeordnet ist, und
- eine Abstandshalterschicht, die benachbart zur zweiten Elektrodenschicht auf der zugewandten Seite des Mehrschichtsystems angeordnet ist und im Betrieb einen Abstand zwischen der zu behandelnden Oberfläche und der zuunterst angeordneten zweiten Elektrodenschicht sicherstellt.

Mit der beschriebenen Vorrichtung soll eine großflächige Wundbehandlung ermöglicht werden, insbesondere von Wunden größer als 200 cm².

Zum Erzeugen des kalten Atmosphärendruckplasmas wird eine wie in DE 10 2014 220 488 A1 beschriebene Vorrichtung typischerweise über ein Kabel an einen Hochspannungsgenerator angeschlossen, um eine Hochspannung an die erste Elektrodenschicht zu übertragen. Typischerweise kann das Kabel zur Übertragung der Hochspannung an eine Elektrodenschicht einer wie in DE 10 2014 220 488 A1 beschriebenen Vorrichtung über eine hochspannungsgeeignete Steckvorrichtung angeschlossen werden.

DE 10 2015 101 391 B4 beschreibt eine Plasmaerzeugungseinrichtung, die einen Elektrodenträger und eine erste Elektrode sowie eine zweite Elektrode umfasst. Die erste Elektrode ist am oder im Elektrodenträger angeordnet. Weiterhin umfasst sie eine hochspannungsgeeignete Steckkontaktverbindung zur elektrischen Kontaktierung wenigstens einer der Elektroden. Die Steckkontaktverbindung weist einen Stecker sowie eine Einschubbuchse zur Aufnahme des Steckers auf. Die Einschubbuchse ist fest am oder im Elektrodenträger angeordnet und mit einer der Elektroden elektrisch leitfähig verbunden.

Es ist eine Aufgabe der vorliegenden Erfindung einen verbesserten Plasmaapplikator zu ermöglichen.

Gemäß einem ersten Aspekt wird die Aufgabe durch einen elektrotechnischen Kern zum Erzeugen eines kalten Atmosphärendruck- oder Niederdruckplasmas für die Behandlung von menschlichen und/oder tierischen und/oder technischen Oberflächen gelöst.

Der elektrotechnische Kern weist eine der zu behandelnden Oberfläche zugewandten Seite und eine der zu behandelnden Oberfläche abgewandten Seite auf und umfasst von der der zu behandelnden Oberfläche zugewandten Seite beginnend die folgenden übereinander angeordneten Schichten:
- eine erste Isolationsschicht,
- eine erste Elektrodenstruktur, die mit einer ersten Kontaktierung zum Herstellen eines elektrischen Kontakts zwischen der ersten Elektrodenstruktur und einer Energieversorgungseinheit versehen ist, und im Betrieb geerdet ist,
- eine zweite Isolationsschicht, die ausgebildet ist, die erste Elektrodenstruktur und eine zweite Elektrodenstruktur galvanisch voneinander zu trennen,
- eine zweite Elektrodenstruktur, die mit einer zweiten Kontaktierung zum Herstellen eines elektrischen Kontakts zwischen der zweiten Elektrodenstruktur und einer Energieversorgungseinheit versehen ist, und im Betrieb durch ein von einer Energieversorgungseinheit bereitgestellten und zum Zünden eines Plasmas ausreichendem Spannungssignal getrieben wird,
- eine dritte Isolationsschicht, die ausgebildet ist, die zweite Elektrodenstruktur und eine dritte Elektrodenstruktur galvanisch voneinander zu trennen,
- eine dritte Elektrodenstruktur, die mit einer dritten Kontaktierung versehen ist, um die dritte Elektrodenstruktur im Betrieb zu erden.

Der Erfindung liegt die Erkenntnis zugrunde, dass sich die Fertigungstiefe eines herkömmlichen Plasmaapplikators reduzieren lässt, wenn ein elektrotechnischer Kern bereits an sich berührungssicher gestaltet wird. Wenn ein elektrotechnischer Kern selbst bereits einen Berührungsschutz gewährleistet kann, auf eine in der Fertigung aufwendige berührungssichere Umschließung verzichtet und eine alternative Umschließung verwendet werden, die lediglich eine einzige Spritzgussschicht aufweist und selbst keinen Berührungsschutz gewährleistet.

Die Erfinder haben erkannt, dass ein für sich allein bereits berührungssicherer elektrotechnischer Kern als eigenständiges Modul eingesetzt werden kann. Das heißt, ein solcher elektrotechnischer Kern kann in unterschiedliche Umschließungen eingeschoben oder in diesen angeordnet werden, ohne dass an eine Umschließung besondere Ansprüche bzgl. eines Berührungsschutzes gestellt werden müssen.

Die Erfinder haben weiterhin erkannt, dass wenn ein elektrotechnischer Kern auf der der zu behandelnden Oberfläche abgewandten Seite eine dritte Elektrodenstruktur umfasst, die die Funktion einer Masseelektrode erfüllt, besonders einfach ein Berührungsschutz und eine EMV-Abschirmung gewährleistet werden kann. Die dritte Isolationsschicht ist dann zwischen der zweiten und der dritten Elektrodenstruktur angeordnet und isoliert die zweite und die dritte Elektrodenstruktur elektrisch voneinander. Somit verhindert die dritte Isolationsschicht durch eine galvanische Trennung einen Kurzschluss zwischen der im Betrieb mit Spannung beaufschlagten zweiten Elektrodenstruktur und der abschirmenden dritten Elektrodenstruktur.

### Bevorzugte Ausführungsformen des elektrotechnischen Kerns gemäß dem ersten Aspekt

Die dritte Elektrodenstruktur ist vorzugsweise derart ausgebildet, dass sie abschirmend wirkt. Die dritte Elektrodenstruktur ist bevorzugt als Flächenelektrode ohne größere Lücken ausgebildet. Im Gegensatz dazu weist die erste Elektrodenstruktur vorzugsweise eine spezielle Geometrie auf und ist derart ausgebildet, dass sich an ihren Elektrodenabschnitten geeignet hohe elektrische Feldstärken bevorzugt auf der der zweiten Elektrodenstruktur abgewandten Seite ausbilden, sodass sich ein erzeugtes Plasma als Oberflächenplasma auf der körperzugewandten Seite der ersten Elektrodenstruktur verteilt. Dies kann dadurch erzielt werden, dass die erste Elektrodenstruktur entsprechend große Lücken aufweist, die z.B. durch entsprechende Abstände zwischen den Elektrodenabschnitten der ersten Elektrodenstruktur gebildet sein können. Die erste Elektrodenstruktur ist diejenige Elektrodenstruktur, die sich im Anwendungsfall am dichtesten an der zu behandelnden Oberfläche befindet. Direkt an der ersten Elektrodenstruktur, also zwischen Körper und der ersten Elektrodenstruktur, bildet sich im Betrieb das Plasma aus. Dies funktioniert, wenn die erste Elektrodenstruktur Aussparungen hat, durch die - wenn man das Konzept "Feldlinien" betrachtet - Feldlinien aus dem elektrotechnischen Kern austreten können.

Die erste Isolationsschicht ist vorzugsweise von einem biokompatiblen Material gebildet.

Die zweite Elektrodenstruktur kann ebenfalls eine spezielle Geometrie aufweisen und insbesondere dann in einer definierten Überlappung mit der ersten Elektrodenstruktur angeordnet sein.

Der elektrotechnische Kern kann derart flexibel ausgebildet sein, dass dieser für eine Plasmabehandlung in seiner Form an eine Form einer zu behandelnden Oberfläche angepasst werden kann.

Der elektrotechnische Kern kann auch starr in einer vorgegebenen Form ausgebildet sein, die beispielweise bereits besonders für eine Plasmabehandlung eines bestimmten Körperteils oder einer bestimmten technischen Oberfläche geeignet sein kann.

Die Grundform des elektrotechnischen Kerns kann beispielsweise eckig, rund oder eine beliebige Polygone Form sein.

In einer Ausführungsform weist der elektrotechnische Kern eine Steckvorrichtung auf, wobei die erste und zweite Kontaktierung der ersten bzw. zweiten Elektrodenstruktur entsprechend eine erste und zweite Leiterbahn der Steckvorrichtung bilden. Die Leiterbahnen stehen vorzugsweise jeweils an derselben Längsseite des elektrotechnischen Kerns jeweils von der entsprechenden Elektrodenstruktur von deren Längsseite ab. Die Steckvorrichtung weist bevorzugt weiterhin eine Lasche auf, die entsprechend mit der zweiten Isolationsschicht verbunden ist. Vorzugsweise sind die erste und die zweite Leiterbahn durch die Lasche galvanisch voneinander getrennt.

In einer Ausführungsform weist der elektrotechnische Kern weiterhin eine Distanzstruktur auf, die auf der der zu behandelnden Oberfläche zugewandten Seite des elektrotechnischen Kerns an die erste Isolationsschicht angrenzend angeordnet ist, sodass sich die Distanzstruktur während einer Plasmabehandlung zwischen einer zu behandelnden Oberfläche und der ersten Isolationsschicht befindet.

Die Distanzstruktur kann von einem biokompatiblen Material gebildet sein. Die Distanzstruktur kann beispielsweise eine Wabenform, oder eine X-, O-, Z-, M-, E-, oder W-Form haben.

Eine Kontaktierung oder eine Elektrodenstruktur des elektrotechnischen Kerns kann auch wenigstens ein Merkmal aufweisen, welches sich infolge eines erstmaligen Gebrauchs derart ändert, dass kein zum Zünden eines Plasmas ausreichendes Spannungssignal mehr an die zweite Elektrodenstruktur übertragen werden kann. Dadurch kann eine Einmalverwendung des elektrotechnischen Kerns sichergestellt werden. Ein solches Merkmal kann beispielsweise eine Verjüngung eines Elektrodenabschnitts einer Elektrodenstruktur sein, die kurz vor einem Ende des erstmaligen Gebrauchs durch einen dann bereitzustellenden hohen Strompuls zerstört wird. Im Detail wir auf den Aspekt der Einmalverwendbarkeit weiter unten eingegangen.

### Weitere Aspekte

Im Folgenden werden weitere Aspekte beschrieben, die jeweils für sich genommen und unabhängig von den anderen weiteren Aspekten zu einem verbesserten Plasmaapplikator beitragen können.

Ein weiterer Aspekt betrifft ein Merkmal, welches sich infolge eines Gebrauchs derart ändert, dass kein zum Zünden eines Plasmas ausreichendes Spannungssignal mehr an einen elektrotechnischen Kern übertragen werden kann. Dieses Merkmal kann an verschiedenen Plasmaapplikatoren und insbesondere an verschiedenen elektrotechnischen Kernen oder Steckvorrichtungen eines Plasmaapplikators in Kombination mit den oder auch unabhängig von den übrigen, hier beschriebenen Aspekten verwirklicht werden.

Dieser Aspekt, der für sich genommen zu einem verbesserten Plasmaapplikator beitragen kann, besteht darin, das eine Kontaktierung oder eine Elektrodenstruktur wenigstens ein Merkmal aufweist, welches sich infolge eines Gebrauchs derart ändert, dass kein zum Zünden eines Plasmas ausreichendes Spannungssignal mehr an einen elektrotechnischen Kern übertragen werden kann. Ein Merkmal, welches sich infolge eines Gebrauchs derart ändert, dass kein zum Zünden eines Plasmas ausreichendes Spannungssignal mehr an einen elektrotechnischen Kern übertragen werden kann, dient dem Sicherstellen einer Einmalverwendung eines Plasmaapplikators.

Dieser Aspekt kann vorzugsweise an einem elektrotechnischen Kern der hier beschriebenen Art, insbesondere an einem elektrotechnischen Kern gemäß dem ersten Aspekt, verwirklicht sein, aber auch bei anderen Plasmaapplikatoren. Insbesondere kann ein anderer Plasmaapplikator einen elektrotechnischen Kern mit lediglich einer zweiten Elektrodenstruktur und einer zweiten Isolationsschicht aufweisen, sodass eine Gegenelektrode im Anwendungsfall durch eine zu behandelnde Oberfläche realisiert ist. Ein anderer Plasmaapplikator kann auch einen elektrotechnischen Kern mit einer ersten Isolationsschicht, einer ersten Elektrodenstruktur, einer zweiten Isolationsschicht und einer zweiten Elektrodenstruktur aufweisen. In einem elektrotechnischen Kern ist das Merkmal, welches eine Einmalverwendung sicherstellt, vorzugsweise als Bestandteil einer Elektrodenstruktur realisiert, die im Betrieb mit einem Spannungssignal beaufschlagt wird.

In einer Ausführungsvariante dieses Aspekts ist das Merkmal von einer Verjüngung einer Kontaktierung oder eines Elektrodenabschnitts einer Elektrodenstruktur gebildet, die kurz vor einem Ende des erstmaligen Gebrauchs durch einen dann bereitgestellten hohen Strompuls zerstört wird.

Alternativ oder zusätzlich kann beispielsweise ein Speicher z.B. in Form eines Speicherbausteins vorgesehen sein, dessen Inhalt bei erstmaliger Benutzung geändert und der vor jeder Anwendung ausgelesen wird.

Ein Plasmaapplikator, der ein Merkmal aufweist, welches eine Einmalverwendung sicherstellt, kann auch einen elektrotechnischen Kern aufweisen, der von einem Drahtgitter, Drahtgewebe oder Drahtgeflecht gebildet ist.

Optional kann ein Plasmaapplikator, der ein Merkmal aufweist, welches eine Einmalverwendung sicherstellt, eine Umschließung und/oder eine Distanzstruktur und/oder einen Zugangsanschluss zum Zu- oder Abführen eines fluiden Mediums in oder aus einem Gasraum aufweisen. Optional kann ein Plasmaapplikator, der ein Merkmal aufweist, welches eine Einmalverwendung sicherstellt, auch eine intergierte Energieversorgungseinheit aufweisen.

Ein Plasmaapplikator mit wenigstens einem Merkmal, welches dem Sicherstellen einer Einmalverwendbarkeit des Plasmaapplikators dient, kann auch wenigstens einen Sensor aufweisen der ausgebildet ist, im Betrieb eine für eine Plasmabehandlung und/oder eine Wundheilung relevante Messgrößen, insbesondere physiologische und/oder physikalische Messgrößen eines im Anwendungsfall von dem Plasmaapplikator bedeckten Körperabschnitts, zu erfassen und auszugeben.

Ein weiterer Aspekt der eigenständig und unabhängig von den übrigen hierin beschriebenen Aspekten oder in Kombination mit diesen zu einem verbesserten Plasmaapplikator beitragen kann, betrifft eine Umschließung mit einer Tasche, in der ein elektrotechnischer Kern herausnehmbar angeordnet werden kann. Eine Umschließung mit Tasche kann als Bestandteil unterschiedlicher Plasmaapplikatoren verwirklicht und unter anderem auch mit dem eine Einmalverwendbarkeit betreffenden Aspekt kombiniert werden.

Gemäß diesem Aspekt wird die Aufgabe durch einen Plasmaapplikator mit einem elektrotechnischen Kern und einer Umschließung mit Tasche gelöst. Die Tasche ist bevorzugt derart ausgebildet, dass ein elektrotechnischer Kern in die Tasche eingeschoben werden kann und dann wenigstens teilweise von der Umschließung umschlossen ist. Ein elektrotechnischer Kern, der in die Tasche eingeschoben werden kann, ist vorzugsweise gemäß wenigstens einer der beschriebenen Ausführungsformen des ersten Aspekts ausgebildet. Ein elektrotechnischer Kern, der in die Tasche eingeschoben werden kann, kann aber auch ein von dem ersten Aspekt abweichend gestalteter elektrotechnischer Kern sein. Ein solcher elektrotechnischer Kern kann beispielsweise ein elektrotechnischer Kern sein, welcher lediglich eine zweite Elektrodenstruktur und eine zweite Isolationsschicht umfasst. Es ist auch denkbar, dass ein elektrotechnischer Kern, der in eine Tasche eingeschoben werden kann, lediglich eine erste Isolationsschicht, eine erste Elektrodenstruktur, eine zweite Isolationsschicht und eine zweite Elektrodenstruktur aufweist. Ein Plasmaapplikator der eine Umschließung mit einer Tasche aufweist, kann auch einen elektrotechnischen Kern aufweisen, der von einem Drahtgitter, Drahtgewebe oder Drahtgeflecht gebildet und in die Tasche eingeschoben ist. Optional kann ein Plasmaapplikator, der eine Umschließung mit einer Tasche aufweist, eine Distanzstruktur und/oder einen Zugangsanschluss für ein fluides Medium aufweisen. Optional kann ein Plasmaapplikator, der eine Umschließung mit einer Tasche umfasst, auch eine intergierte Energieversorgungseinheit aufweisen. Ein Plasmaapplikator, der eine Umschließung mit einer Tasche aufweist, kann auch eine Kontaktierung oder eine Elektrodenstruktur mit wenigstens einem Merkmal aufweisen, welches sich infolge eines Gebrauchs derart ändert, dass kein zum Zünden eines Plasmas ausreichendes Spannungssignal mehr an die zweite Elektrodenstruktur übertragen werden kann. Die Umschließung ist vorzugsweise von einem biokompatiblen Material gebildet. Insbesondere dann, wenn der elektrotechnische Kern selbst einen Berührungsschutz gewährleistet, wie z.B. der elektrotechnische Kern gemäß dem ersten Aspekt, kann die Umschlie-βung einlagig und selbst nicht berührungssicher ausgebildet sein. Die Umschließung kann Silikon und/oder Lacke und/oder eine Parylenbeschichtung aufweisen. Die Umschließung kann auch einen mit Widerhaken besetzten Bereich aufweisen, in dem der Plasmaapplikator an einem Textil, beispielsweise einem Verband, befestigt werden kann. Der Plasmaapplikator kann auch eine separate Schicht aufweisen, die auf einer Seite ein Adhäsionsschicht aufweist und auf der anderen Seite mit Widerhaken besetzt ist. Mit derjenigen Seite, auf der sich die Adhäsionsschicht befindet, kann diese Schicht an dem Plasmaapplikator und der Plasmaapplikator dann mit der mit Widerhaken besetzten Seite der Schicht an einem Textil befestigt werden. Grundsätzlich eignet sich eine solche Schicht mit einer Adhäsionsschicht auf der einen Seite und einer mit Widerhaken besetzten anderen Seite auch für die Verwendung bei einem beliebigen anderen der hier beschriebenen Plasmaapplikatoren, die lösbar an einem Textil befestigt werden sollen.

Ein Plasmaapplikator mit einem elektrotechnischen Kern und einer Umschließung mit Tasche kann auch wenigstens einen Sensor aufweisen der ausgebildet ist, im Betrieb eine für eine Plasmabehandlung und/oder eine Wundheilung relevante Messgrößen, insbesondere physiologische und/oder physikalische Messgrößen eines im Anwendungsfall von dem Plasmaapplikator bedeckten Körperabschnitts, zu erfassen und auszugeben.

Ein weiterer Aspekt der sowohl eigenständig und unabhängig von den übrigen hierin beschriebenen Aspekten als auch in Kombination mit einem oder mehrerer dieser Aspekte zu einem verbesserten Plasmaapplikator beitragen kann, betrifft einen an einem Beutel angeordneten Plasmaapplikator. Der Plasmaapplikator kann in unterschiedlichen Varianten verwirklicht sein und kann beispielsweise auch eine Umschließung mit Tasche und/oder ein Merkmal, welches eine Einmalverwendbarkeit sicherstellt, aufweisen. Zusätzlich oder alternativ kann der Plasmaapplikator auch einen elektrotechnischen Kern gemäß dem ersten Aspekt aufweisen.

Dieser Aspekt betrifft also einen Plasmaapplikator mit einem elektrotechnischen Kern und einer Umschließung, der an einem Beutel fixiert ist, der dafür vorgesehen ist, ein bestimmtes Körperteil, beispielsweise einen Fuß, zu umschließen und so einen abgeschlossenen Gasraum zu bilden. Der Beutel kann von einer dünnen Folie gebildet sein. Für eine Plasmabehandlung kann ein zu behandelnder Fuß in den Beutel geschoben werden. Der Beutel kann dann beispielsweise oberhalb des Knöchels z.B. mittels eines Gummis oder eines Bandes fixiert werden, um einen abgeschlossenen Gasraum zu bilden. Der Beutel kann wenigstens ein Loch aufweisen, welches vorzugsweise einen Durchmesser hat, der zwischen 1 cm und 8 cm beträgt. Der Plasmaapplikator ist so angeordnet, dass dieser das Loch abdeckt. Der Plasmaapplikator ist vorzugsweise derart über dem Loch angeordnet, dass der elektrotechnische Kern an das Loch angrenzt. Auf der dem Loch zugewandten Seite kann der Plasmaapplikator am äußeren Rand des Plasmaapplikators das wenigstens eine Loch umschließend an den Beutel fixiert sein. Im Anwendungsfall kann ein gezündetes Plasma durch das Loch in den Beutel eintreten und mit der zu behandelnden Oberfläche wechselwirken. Somit wird eine großflächige Behandlung beispielsweise eines Fußes oder wenigstens einer Fußunterseite ermöglicht.

Ein weiterer Aspekt der entweder eigenständig und unabhängig von den übrigen hierein beschriebenen Aspekten oder in Kombination mit einzelnen oder mehreren dieser Aspekte zu einem verbesserten Plasmaapplikator beitragen kann, betrifft einen Zugangsanschluss. Ein Zugangsanschluss kann als Bestandteil verschiedener Plasmaapplikatoren verwirklicht werden. Zum Beispiel kann ein Plasmaapplikator, der einen Zugangsanschluss gemäß diesem Aspekt aufweist, einen gemäß dem ersten Aspekt ausgebildeten elektrotechnischen Kern und/oder eine Umschließung mit Tasche und/oder ein Merkmal zum Sicherstellen einer Einmalverwendbarkeit aufweisen.

Gemäß diesem Aspekt weist ein Plasmaapplikator also einen Zugangsanschluss auf, wobei der Zugangsanschluss derart angeordnet und ausgebildet ist, dass ein fluides Medium vor, während und/oder nach einer Plasmabehandlung einem zwischen einem elektrotechnischen Kern und einer zu behandelnden Oberfläche durch die Umschließung gebildeten abgeschlossenen Gasraum zu- oder aus dem Gasraum abgeführt werden kann. Ein fluides Medium ist ein gasförmiges und/oder flüssiges Medium und/oder ein flüssiges Medium mit zugefügten festen Bestandteilen in Form von z.B. löslichen und/oder nichtlöslichen Mikropartikeln. Ein Plasmaapplikator mit einem Zugangsanschluss kann unterschiedlich gestaltet sein und insbesondere mit verschiedenen elektrotechnischen Kernen verwirklicht werden. Ein elektrotechnischer Kern dieses Plasmaapplikators mit Zugangsanschluss ist vorzugsweise flexibel ausgebildet und kann mit verschiedenen Grundformen, beispielsweise rund oder viereckig, verwirklicht werden. Eine Elektrodenstruktur eines elektrotechnischen Kerns eines Plasmaapplikators mit einem Zugangsanschluss kann eine spezielle Geometrie aufweisen. Wenn der elektrotechnische Kern wenigstens zwei Elektrodenstrukturen mit einer speziellen Geometrie aufweist, können die Elektrodenabschnitte der beiden Elektrodenstrukturen beispielsweise in einer definierten Überlappung zueinander angeordnet sein. Insbesondere kann ein elektrotechnischer Kern eines Plasmaapplikators mit Zugangsanschluss lediglich eine zweite Elektrodenstruktur und eine zweite Isolationsschicht oder lediglich eine erste Isolationsschicht, eine erste Elektrodenstruktur, eine zweite Isolationsschicht und eine zweite Elektrodenstruktur aufweisen oder gemäß dem oben beschriebenen ersten Aspekt ausgebildet sein. Ein elektrotechnischer Kern eines Plasmaapplikators mit Zugangsanschluss kann auch von einem Drahtgitter, Drahtgewebe oder Drahtgeflecht gebildet sein. Ein geeigneter elektrotechnischer Kern kann beispielsweise hergestellt werden, indem ein Elektrodenträger, der eine Isolationsschicht darstellt, auf einer oder auf beiden Seiten mit jeweils einer Elektrodenstruktur bedruckt wird. Dies kann im Rollensiebdruckverfahren durchgeführt werden. Ein Zugangsanschluss kann weiterhin ein Ventil und/oder eine Muffe oder ein Gegengewinde für eine Muffe aufweisen.

Ein Plasmaapplikator mit einem Zugangsanschluss kann auch eine Kontaktierung oder eine Elektrodenstruktur mit wenigstens einem Merkmal aufweisen, welches sich infolge eines Gebrauchs derart ändert, dass kein zum Zünden eines Plasmas ausreichendes Spannungssignal mehr an die zweite Elektrodenstruktur übertragen werden kann.

Bevorzugt umschließt die Umschließung eines Plasmaapplikators mit Zugangsanschluss den elektrotechnischen Kern wenigstens teilweise. Ein Plasmaapplikator mit einem Zugangsanschluss kann auch eine Umschließung mit einer Tasche umfassen, in die ein elektrotechnischer Kern eingeschoben werden kann. Vorzugsweise ist eine Umschließung eines Plasmaapplikators mit Zugangsanschluss von einem biokompatiblen Material gebildet. Eine Umschließung kann beispielweise Silikon, Lacke, Textil und/oder eine Parylenbeschichtung aufweisen. Die Umschließung kann auch eine V.A.C.-Folie aufweisen. Wenn der elektrotechnische Kern selbst bereits berührungssicher ausgebildet ist, kann die Umschließung einlagig ausgebildet und so gestaltet sein, dass diese selbst keinen Berührungsschutz gewährleistet. Die Umschließung kann auch einen Zugangsanschluss aufweisen, mit der ein fluides Medium aus dem abgeschlossenen Gasraum abtransportiert werden kann. Ein Plasmaapplikator mit einem Zugangsanschluss kann auch eine integrierte Energieversorgungseinheit umfassen. Ein Plasmaapplikator mit einem Zugangsanschluss kann auch eine Distanzstruktur umfassen. Die Distanzstruktur ist vorzugsweise von einem biokompatiblen Material gebildet. Die Distanzstruktur kann beispielsweise von einem V.A.C.-Schaum gebildet sein. Ein Plasmaapplikator mit einem Zugangsanschluss kann auch eine Adhäsionsschicht aufweisen, die vorzugsweise als letzte Schicht auf der einer zu behandelnden Oberfläche zugewandten Seite des Plasmaapplikators angeordnet und ausgebildet ist, den Plasmaapplikator auf einer zu behandelnden Oberfläche zu fixieren. Ein Plasmaapplikator mit einem Zugangsanschluss kann auch eine Steckvorrichtung aufweisen, die fest mit dem elektrotechnischen Kern verbunden sein kann. Die Steckvorrichtung ist vorzugsweise laschenförmig ausgebildet. Ein Plasmaapplikator mit einem Zugangsanschluss kann auch wenigstens einen Sensor aufweisen der ausgebildet ist, im Betrieb eine für eine Plasmabehandlung und/oder eine Wundheilung relevante Messgrößen, insbesondere physiologische und/oder physikalische Messgrößen eines im Anwendungsfall von dem Plasmaapplikator bedeckten Körperabschnitts, zu erfassen und auszugeben.

Ein Plasmaapplikator mit einem Zugangsanschluss kann auch ausgebildet sein, für eine Behandlung über eine längere Zeitdauer auf einer zu behandelnden Oberfläche zu verbleiben, insbesondere über die Zeitdauer einer Wundheilung. Ein solcher Plasmaapplikator mit einem Zugangsanschluss kann ausgebildet sein, eine zu behandelnde Oberfläche zu versiegeln. Bei einem solchen für eine Versiegelung vorgesehenen Plasmaapplikator mit einem Zugangsanschluss ist die Adhäsionsschicht vorzugsweise von einem Silikon- oder einem PU-Klebstoff und die Umschließung von einem luftundurchlässigen Material gebildet. Insbesondere kann die Adhäsionsschicht einen Klebstoff aufweisen, der durch Bestrahlung mit UV-Licht oder durch Kontakt mit Alkohol seine Hafteigenschaften verliert.

Ein weiterer Aspekt, der eigenständig und unabhängig von den übrigen hierein beschriebenen Aspekten oder in Kombination mit einzelnen oder mehreren dieser Aspekte zu einem verbesserten Plasmaapplikator beitragen kann, betrifft eine integrierte Energieversorgungseinheit. Eine Energieversorgungseinheit gemäß diesem Aspekt kann in verschiedene Plasmaapplikatoren intergiert werden. Beispielweise kann die Energieversorgungseinheit in einen Plasmaapplikator intergiert werden, der einen Zugangsanschluss und/oder einen gemäß dem ersten Aspekt ausgebildeten elektrotechnischen Kern und/oder eine Umschließung mit Tasche und/oder ein Merkmal zum Sicherstellen einer Einmalverwendbarkeit aufweist.

Dieser Aspekt kann durcheinen Plasmaapplikator mit einer integrierten Energieversorgungseinheit verwirklicht werden, die einen Energiespeicher umfasst, der elektrisch mit einem elektrotechnischen Kern des Plasmaapplikators verbunden und ausgebildet ist, im Betrieb ein zum Zünden eines Plasmas ausreichendes Spannungssignal an eine Elektrodenstruktur des elektrotechnischen Kerns zu übertragen. Der Energiespeicher kann beispielsweise ein Akkumulator, eine Batterie oder ein Kondensator sein. Eine in einen Plasmaapplikator integrierte Energieversorgungseinheit dieser Art kann in Kombination mit verschiedenen Arten von Plasmaapplikatoren verwirklicht sein. Insbesondere kann ein elektrotechnischer Kern eines Plasmaapplikators dieses Aspekts lediglich eine zweite Elektrodenstruktur und eine zweite Isolationsschicht oder lediglich eine erste Isolationsschicht, eine erste Elektrodenstruktur, eine zweite Isolationsschicht und eine zweite Elektrodenstruktur aufweisen oder gemäß dem oben beschriebenen ersten Aspekt ausgebildet sein. Ein geeigneter elektrotechnischer Kern kann im Rollensiebdruckverfahren hergestellt worden sein, bei dem auf eine oder auf beide Seiten eines Elektrodenträgers jeweils eine Elektrodenstruktur gedruckt wird. Die Isolationsschicht zwischen den beiden Elektrodenstrukturen ist dann durch den Elektrodenträger realisiert. Ein elektrotechnischer Kern eines Plasmaapplikators mit integrierter Energieversorgungseinheit kann auch von einem Drahtgitter, Drahtgewebe oder Drahtgeflecht gebildet sein. Eine Isolationsschicht, die auf der einer zu behandelnden Oberfläche zugewandten Seite des Plasmaapplikators angeordnet ist, ist vorzugsweise von einem biokompatiblen Material gebildet. Vorzugsweise ist der elektrotechnische Kern des Plasmaapplikators mit integrierter Energieversorgungseinheit flexibel ausgebildet, sodass der Plasmaapplikator in seiner Form an die Form einer zu behandelnden Oberfläche angepasst werden kann. Der elektrotechnische Kern des Plasmaapplikators mit einer integrierten Energieversorgungseinheit kann wenigstens eine Elektrodenstruktur mit einer speziellen Geometrie aufweisen. Wenn mehrere Elektrodenstrukturen des elektrotechnischen Kerns eine spezielle Geometrie aufweisen, können diese so zueinander angeordnet sein, dass die Elektrodenabschnitte der jeweiligen Elektrodenstrukturen zueinander eine definierte Überlappung haben.

Ein Plasmaapplikator mit einer integrierten Energieversorgungseinheit kann auch eine Kontaktierung oder eine Elektrodenstruktur mit wenigstens einem Merkmal aufweisen, welches sich infolge eines Gebrauchs derart ändert, dass kein zum Zünden eines Plasmas ausreichendes Spannungssignal mehr an die zweite Elektrodenstruktur übertragen werden kann. Ein Plasmaapplikator mit einer integrierten Energieversorgungseinheit kann auch eine Umschließung mit einer Tasche umfassen, in die ein elektrotechnischer Kern eingeschoben werden kann.

Vorzugsweise weist ein Plasmaapplikator mit integrierter Energieversorgungseinheit eine Umschließung auf, die den elektrotechnischen Kern wenigstens teilweise oder sogar vollständig umschließt. Insbesondere ist ein Plasmaapplikator mit integrierter Energieversorgungseinheit, bei dem der elektrotechnischen Kern vollständig von einer Umschließung umschlossen ist, dazu geeignet, für eine Plasmabehandlung in einen menschlichen oder tierischen Körper implantiert zu werden. Eine Umschließung eines Plasmaapplikators mit integrierter Energieversorgungseinheit ist vorzugweise von einem biokompatiblen Material gebildet. Eine Umschließung eines Plasmaapplikator mit integrierter Energieversorgungseinheit kann Silikon, Lacken Textilien und/oder eine Parylenbeschichtung aufweisen. Wenn der elektrotechnische Kern eines Plasmaapplikator mit integrierter Energieversorgungseinheit bereits selbst berührungssicher ausgebildet ist, kann die Umschließung einlagig ausgebildet sein und selbst keinen Berührungsschutz gewährleisten. Ein Plasmaapplikator mit einer integrierten Energieversorgungseinheit kann auch eine Distanzstruktur aufweisen, die vorzugsweise von einem biokompatiblen Material gebildet und dafür vorgesehen ist, zwischen dem Plasmaapplikator und einer zu behandelnden Oberfläche einen definierten Abstand zu schaffen, in dem ein Plasma zünden kann.

Ein Plasmaapplikator mit einer integrierten Energieversorgungseinheit kann auch einen Zugangsanschluss aufweisen, durch den ein fluides Medium in einen vor, während oder nach einer Plasmabehandlung gebildeten abgeschlossenen Gasraum hinzugeführt oder aus diesem abgeführt werden kann.

Ein Plasmaapplikator mit integrierter Energieversorgungseinheit kann eine elektrische Schaltung aufweisen, die vorzugsweise zwischen die intergierte Energieversorgungseinheit und den elektrotechnischen Kern geschaltet und ausgebildet ist, im Betrieb ein von der Energieversorgungseinheit bereitgestelltes Spannungssignal in ein zum Zünden eines Plasmas ausreichendes Spannungssignal zu wandeln und das gewandelte Spannungssignal an den elektrotechnischen Kern weiterzuleiten. Die elektrische Schaltung ist dann als Komponente des Plasmaapplikators in den Plasmaapplikator intergiert. Die elektrische Schaltung kann auch als Bestandteil der Energieversorgungseinheit realisiert sein, sodass die Energieversorgungseinheit ausgebildet ist, im Betrieb ein zum Zünden eines Plasmas ausreichendes Spannungssignals bereitzustellen. Die integrierte Energieversorgungseinheit kann auch eine mit dem Energiespeicher elektrisch verbundene Empfangs-Spulenanordnungen aufweisen und derart ausgebildet sein, dass der Energiespeicher geladen werden kann, indem von einer Sende-Spulenanordnung elektrische Energie von der Sende-Spulenanordnung auf die Empfangs-Spulenanordnung im Plasmaapplikator induktiv übertragen wird. Eine Energieversorgungseinheit mit Empfangs-Spulenanordnungen ist besonders geeignet für einen Plasmaapplikator, der dafür vorgesehen ist, implantiert zu werden.

Insbesondere dann, wenn ein Plasmaapplikator mit integrierter Energieversorgungseinheit nicht dafür vorgesehen ist, in einen Körper implantiert zu werden, kann dieser eine Steckvorrichtung aufweisen, die mit der intergierten Energieversorgungseinheit elektrisch leitfähig verbunden und dafür vorgesehen ist, mit einer externen Energieversorgungseinheit verbunden zu werden, um den Energiespeicherder intergierten Energieversorgungseinheit zu laden.

Ein Plasmaapplikator mit integrierter Energieversorgungseinheit kann auch wenigstens einen Sensor aufweisen, der ausgebildet ist, im Betrieb eine für eine Plasmabehandlung und/oder eine Wundheilung relevante Messgrößen, insbesondere physiologische und/oder physikalische Messgrößen eines im Anwendungsfall von dem Plasmaapplikator bedeckten Körperabschnitts, zu erfassen und auszugeben. Insbesondere dann, wenn ein Plasmaapplikator mit integrierter Energieversorgungseinheit dafür vorgesehen ist, in einen menschlichen oder tierischen Körper implantiert zu werden, kann es vorteilhaft sein, wenn dieser Plasmaapplikator wenigstens einen Sensor aufweist. Der Plasmaapplikator kann dann über einen längeren Zeitraum, der insbesondere die Zeitdauer einer Wundheilung umfasst, aber auch Monate oder sogar Jahre umfassen kann, in dem Körper implantiert bleiben. Der wenigstens eine Sensor des Plasmaapplikators kann während dieser Zeitdauer für eine Plasmabehandlung und/oder eine Wundheilung relevante Messgrößen erfassen und ausgegeben. Der Zustand einer Wunde und/oder der Fortschritt einer Wundheilung können dann anhand dieser ausgegebenen Messgrößen beurteilt werden. Insbesondere dann, wenn ein Plasmaapplikator dafür vorgesehen ist, implantiert zu werden, ist es bevorzugt, wenn von einem Sensor erfasste Messgrößen drahtlos übertragen und/oder ausgelesen werden können. Beispielweise kann der Plasmaapplikator einen RFID-Transponder aufweisen, der auf in einem Speicherbaustein gespeicherte, die erfassten Messgrößen repräsentierende Daten zugreifen und diese an ein Lesegerät übermitteln kann, wenn eine entsprechende Anfrage von einem Lesegerät an den Transponder gestellt wird.

Auch wenn der Plasmaapplikator mit integrierter Energieversorgungseinheit nicht dafür vorgesehen ist, implantiert zu werden, sondern im Anwendungsfall von außen auf eine zu behandelnde Oberfläche aufgelegt oder auf dieser fixiert wird, kann es vorteilhaft sein, wenn dieser nach einer Plasmabehandlung über einen längeren Zeitraum, insbesondre über die Zeitdauer einer Wundheilung, z.B. auch über Wochen oder Monate auf einer zu behandelnden Wunde verbleibt. Vorzugsweise ist der Plasmaapplikator mit integrierter Energieversorgungseinheit dann derart ausgebildet, dass eine zu behandelnde Oberfläche mit dem Plasmaapplikator versiegelt werden kann. Bei einem solchen für eine Versiegelung vorgesehenen Plasmaapplikator mit integrierter Energieversorgungseinheit ist die Adhäsionsschicht vorzugsweise von einem Silikon- oder einem PU-Klebstoff und die Umschließung von einem luftundurchlässigen Material gebildet. Insbesondere kann die Adhäsionsschicht einen Klebstoff aufweisen, der durch Bestrahlung mit UV-Licht oder durch Kontakt mit Alkohol seine Hafteigenschaften verliert. Wenn ein für eine Versiegelung vorgesehenen Plasmaapplikator mit integrierter Energieversorgungseinheit wenigstens einen Sensor aufweist, können mit diesem Sensor im Anwendungsfall für eine Plasmabehandlung und/oder eine Wundheilung relevante Messgrößen erfasst und ausgegeben werden, während eine zu behandelnde Oberfläche durch den Plasmaapplikator versiegelt ist.

Ein weiterer Aspekt, der eigenständig und unabhängig von den übrigen hierein beschriebenen Aspekten oder in Kombination mit diesen zu einem verbesserten Plasmaapplikator beitragen kann, betrifft eine autarke, mobile Energieversorgungseinheit. Die Energieversorgungseinheit gemäß diesem Aspekt kann zum Bereitstellen eines Spannungssignals an unterschiedliche Plasmaapplikatoren verwendet werden. Solche unterschiedlichen Plasmaapplikatoren können beispielsweise eine integrierte Energieversorgungseinheit und/oder einen Zugangsanschluss und/oder einen gemäß dem ersten Aspekt ausgebildeten elektrotechnischen Kern und/oder eine Umschließung mit Tasche und/oder ein Merkmal zum Sicherstellen einer Einmalverwendbarkeit aufweisen.

Gemäß diesem Aspekt wird die eingangs genannte Aufgabe gelöst durch eine autarke, mobile Energieversorgungseinheit mit einer Einschubvorrichtung und einem Energiespeicher zum Verbinden mit einer Steckvorrichtung eines Plasmaapplikators und zum Bereitstellen eines zum Zünden eines Plasmas ausreichenden Spannungssignals an eine mit der Einschubvorrichtung zusammengesteckte Steckvorrichtung. Die Energieversorgungseinheit weist vorzugsweise eine elektrische Schaltung auf, die ausgebildet ist, eine von dem Energiespeicher bereitgestellte Spannung in ein zum Zünden eines Plasmas ausreichendes Spannungssignal zu wandeln und dieses an die Einschubvorrichtung zu übertragen. Eine derartige Energieversorgungseinheit ist vorzugsweise als nur wenige Kubikzentimeter große, autarke Einheit ausgebildet, die mit einem Plasmaapplikator mechanisch und elektrisch zu verbinden ist und mit dem Plasmaapplikator zusammen z.B. an einem Patienten befestigt werden kann. Im Unterschied zu einer in einen Plasmaapplikator integrierten Energieversorgungseinheit ist die autarke, mobile Energieversorgungseinheit gemäß diesem Aspekt austauschbar mit dem Plasmaapplikator verbunden. Eine Einschubvorrichtung gemäß diesem Aspekt kann in Kombination mit verschiedenen Plasmaapplikatoren verwirklicht werden und zum Beispiel mit einem Plasmaapplikator verbunden werden, der einen elektrotechnischen Kern mit lediglich einer zweiten Elektrodenstruktur und einer zweiten Isolationsschicht oder mit lediglich einer ersten Isolationsschicht, einer ersten Elektrodenstruktur, einer zweiten Isolationsschicht und einer zweiten Elektrodenstruktur aufweist oder der gemäß dem oben beschriebenen ersten Aspekt ausgebildet ist. Eine Einschubvorrichtung gemäß diesem Aspekt kann mit einem Plasmaapplikator verbunden werden, der einen elektrotechnischen Kern aufweist, der von einem Drahtgitter, Drahtgewebe oder Drahtgeflecht gebildet ist. Eine Einschubvorrichtung gemäß diesem Aspekt kann mit einem Plasmaapplikator verbunden werden, der wenigstens ein Merkmal aufweist, welches sich infolge eines Gebrauchs derart ändert, dass kein zum Zünden eines Plasmas ausreichendes Spannungssignal mehr an den Plasmaapplikator übertragen werden kann. Eine Einschubvorrichtung gemäß diesem Aspekt kann auch einen Schlauch aufweisen, der mit einem Zugangsanschluss einer Steckvorrichtung oder eines Plasmaapplikators verbunden oder zusammengesteckt werden kann.

Ein weiterer Aspekt, der eigenständig und unabhängig von den übrigen hierin beschriebenen Aspekten oder in Kombination mit einem oder mehrerer dieser Aspekte zu einem verbesserten Plasmaapplikator beitragen kann, betrifft einen elektrotechnischen Kern, derwenigstens einen isolierten elektrisch leitenden Draht umfasst. Der elektrotechnische Kern gemäß diesem Aspekt kann als Bestandteil verschiedener Plasmaapplikatoren verwirklicht werden. Zum Beispiel bei Plasmaapplikatoren, die eine integrierte Energieversorgungseinheit und/oder einen Zugangsanschluss und/oder eine Umschließung mit Tasche und/oder ein Merkmal zum Sicherstellen einer Einmalverwendbarkeit aufweisen.

Dieser Aspekt betrifft also einen elektrotechnischen Kern, der wenigstens einen isolierten elektrisch leitenden Draht umfasst, der im Betrieb durch ein Spannungssignal getrieben wird, wobei die Gegenelektrode dann vorzugsweise durch einen weiteren isolierten elektrisch leitenden Draht oder durch eine zu behandelnde Oberfläche selbst realisiert ist. Vorzugsweise ist eine Umschließung vorgesehen, um den wenigstens einen isolierten elektrisch leitenden Draht derart auf einer zu behandelnden Oberfläche zu umschließen, dass ein möglichst abgeschlossener Gasraum gebildet wird, in dem ein Plasma während einer Plasmabehandlung zünden kann. Eine Umschließung kann auch eine Tasche aufweisen, in die der von dem elektrisch leitenden Draht gebildete elektrotechnische Kern eingeschoben werden kann. Ein solcher elektrotechnischer Kern kann beispielweise ein Drahtgitter, ein Drahtgewebe, oder ein Drahtgeflecht sein, dass von einem oder mehreren elektrische leitenden, isolierten Drähten gebildet ist. Insbesondere können eine im Anwendungsfall getriebene, von einem oder mehreren isolierten, elektrisch leitenden Drähten gebildete Elektrodenstruktur und eine weitere, ebenfalls von einem oder mehreren isolierten, elektrisch leitenden Drähten gebildete Elektrodenstruktur, die im Betrieb eine Gegenelektrode realisiert, gemeinsam ein Drahtgitter, ein Drahtgewebe, oder ein Drahtgeflecht bilden, indem entsprechende isolierte Drähte beider Elektrodenstrukturen z.B. miteinander verwoben werden. Es ist auch denkbar, dass ein im Anwendungsfall die getriebene Elektrodenstruktur bildender elektrisch leitender Draht und ein weiterer Draht, der im Betrieb eine Gegenelektrode realisiert, gemeinsam in einer Kabelummantelung angeordnet, jedoch galvanisch voneinander getrennt sind. Es ist auch denkbar, dass ein einfacher elektrisch leitender, isolierter Draht eine einfache Elektrodenstruktur bildet, ohne dass der Draht in einer vergleichsweise komplizierten Struktur wie einem Gewebe oder einem Gitter angeordnet ist. Im Anwendungsfall wird dann eine Gegenelektrode vorzugsweise durch eine zu behandelnde Oberfläche realisiert. Ein Plasmaapplikator mit einem elektrotechnischen Kern in Form eines isolierten Drahts kann auch mit einer Steckvorrichtung der hier beschriebenen Art versehen sein. Eine solche Steckvorrichtung kann auch ein Merkmal aufweisen, welches eine Einmalverwendung des Plasmaapplikators sicherstellt. Ein elektrischer Leiter kann auch mit einer mobilen Energieversorgungseinheit verbunden sein, die im Betrieb durch Schalten eines Schaltkontakts ein zum Zünden eines Plasmas ausreichendes Spannungssignal an den elektrisch leitenden, isolierten Draht überträgt. Ein Plasmaapplikator mit einem elektrotechnischen Kern, der wenigstens einen isolierten elektrisch leitenden Draht umfasst, kann auch wenigstens einen Sensor aufweisen der ausgebildet ist, im Betrieb eine für eine Plasmabehandlung und/oder eine Wundheilung relevante Messgrößen, insbesondere physiologische und/oder physikalische Messgrößen eines im Anwendungsfall von dem Plasmaapplikator bedeckten Körperabschnitts, zu erfassen und auszugeben.

Ein weiterer Aspekt, der ebenfalls eigenständig und unabhängig von den übrigen hierein beschriebenen Aspekten oder in Kombination mit einem oder mehrerer dieser Aspekte zu einem verbesserten Plasmaapplikator beitragen kann, betrifft einen Plasmaapplikator mit einem elektrotechnischen Kern, einer Umschließung, einer Distanzstruktur, einer Adhäsionsschicht und einer Steckvorrichtung. Der Plasmaapplikator gemäß diesem Aspekt kann auch eine integrierte Energieversorgungseinheit und/oder einen Zugangsanschluss und/oder eine Umschließung mit Tasche und/oder ein Merkmal zum Sicherstellen einer Einmalverwendbarkeit und/oder einen elektrotechnischen Kern, der wenigstens einen isolierten elektrisch leitenden Draht umfasst oder einen gemäß dem ersten Aspekt ausgebildeten elektrotechnischen Kern aufweisen. Zum Zünden eines Plasmas kann der Plasmaapplikator gemäß diesem Aspekt auch mit einer mobilen Energieversorgungseinheit verbunden werden.

Gemäß diesem Aspekt umfasst ein Plasmaapplikator einen elektrotechnischen Kern, eine Umschließung, eine Distanzstruktur, eine Adhäsionsschicht und eine Steckvorrichtung. Der elektrotechnische Kern ist vorzugsweise flexibel ausgebildet. Der elektrotechnische Kern kann mit verschiedenen Grundformen verwirklicht werden, beispielweise einer runden, oder einer vieleckigen und insbesondere einer viereckigen Grundform. Bevorzugt ist der elektrotechnischen Kern ein elektrotechnischer Kern gemäß dem ersten Aspekt, also sechslagig ausgebildet und im Betrieb selbst berührungssicher. Der Plasmaapplikator gemäß diesem Aspekt kann aber auch mit verschiedenen anderen elektrotechnischen Kernen verwirklicht werden. Wenigstens eine Elektrodenstruktur des elektrotechnischen Kerns kann eine spezielle Geometrie aufweisen. Wenn wenigstens zwei Elektrodenstrukturen des elektrotechnischen Kerns eine spezielle Geometrie aufweisen, sind die Elektrodenabschnitte dieser Elektrodenstrukturen vorzugsweise mit einer definierten Überlappung zueinander angeordnet. Wenn der elektrotechnische Kern mehrere Elektrodenstrukturen aufweist, sind zwischen jeweils zwei der Elektrodenstrukturen vorzugsweise eine Isolationsschicht angeordnet, um die entsprechenden Elektrodenstrukturen galvanisch voneinander zu trennen. Vorzugsweise ist wenigstens eine weitere Isolationsschicht auf der der zu behandelnden Oberfläche zugewandten Seite des Plasmaapplikators angeordnet und bevorzugt von einem biokompatiblen Material gebildet. Vorzugsweise umschließt die Umschlie-βung den elektrotechnischen Kern wenigstens teilweise. Die Umschließung kann derart ausgebildet sein, dass sie im Betrieb zwischen dem elektrotechnischen Kern und einer zu behandelnden Oberfläche einen abgeschlossenen Gasraum bildet. Vorzugsweise ist die Umschließung von einem biokompatiblen Material gebildet. Insbesondere dann, wenn der elektrotechnische Kern bereits selbst berührungssicher ausgebildet ist, kann die Umschlie-βung einlagig ausgebildet sein und selbst keinen Berührungsschutz gewährleisten. Die Umschließung kann beispielweise Silikon, Lacke oder eine Parylenbeschichtung aufweisen. Die Distanzstruktur ist vorzugsweise von einem biokompatiblen Material gebildet. Die Distanzstruktur ist vorzugsweise auf der der zu behandelnden Oberfläche zugewandten Seite des Plasmaapplikators und benachbart zu der der zu behandelnden Oberfläche zugewandten Elektrodenstruktur des Plasmaapplikators angeordnet und ausgebildet, zwischen einem elektrotechnischen Kern und einer zu behandelnden Oberfläche einen definierten Abstand und somit ein definiertes Gasvolumen zu schaffen, in dem sich ein erzeugtes Plasma verteilen kann. Die Distanzstruktur kann beispielweise eine Wabenform oder eine X-, O-, Z-, M-, E-, oder eine W-Form haben. Die Adhäsionsschicht ist ausgebildet, den Plasmaapplikator auf einer zu behandelnden Oberfläche zu fixieren und ist vorzugsweise als letzte Schicht auf der der zu behandelnden Oberfläche zugewandten Seite des Plasmaapplikators angeordnet. Die Adhäsionsschicht kann beispielsweise an der der zu behandelnden Oberfläche zugewandten Seite des Plasmaapplikators wie ein Rahmen entlang des Umfangs des Plasmaapplikators angeordnet sein. Es ist aber auch möglich, dass die Adhäsionsschicht vollflächig an der einer zu behandelnden Oberfläche zugewandten Seite des Plasmaapplikators angeordnet ist. Die Steckvorrichtung ist vorzugsweise fest mit einem elektrotechnischen Kern verbunden und ausgebildet, im Betrieb ein Spannungssignal an eine getriebene Elektrodenstruktur des elektrotechnischen Kerns zu übertragen.

Solche Steckvorrichtungen sind weiter unten im Detail beschrieben. Die Steckvorrichtung ist vorzugsweise laschenförmig ausgebildet. Eine laschenförmige Steckvorrichtung kann dadurch gebildet werden, dass die Elektrodenstrukturen und Isolationsschichten des elektrotechnischen Kerns auf diese Lasche erweitert werden. Die Dimensionen der Steckvorrichtung sind vorzugsweise in Abhängigkeit von der Länge der Kriechstrecken so bemessen, dass im Betrieb keine Teilentladungen innerhalb einer zusammengesteckten Steckvorrichtung und einer Einschubvorrichtung entstehen.

Ein weiterer Aspekt, der sowohl eigenständig und unabhängig von den übrigen hierin beschriebenen Aspekten als auch in Kombination mit einzelnen oder mehreren dieser Aspekte zu einem verbesserten Plasmaapplikator beitragen kann, betrifft eine Adhäsionsschicht für einen Plasmaapplikator, die ausgebildet ist, gleichzeitig die Funktion einer Distanzstruktur zu erfüllen. Die Adhäsionsschicht gemäß diesem Aspekt stellt insbesondere ein eigenes Produkt dar, und kann als Modul zusammen mit verschieden ausgebildeten Plasmaapplikatoren verwendet werden. Solche Plasmaapplikatoren können beispielsweise eine integrierte Energieversorgungseinheit und/oder einen Zugangsanschluss und/oder eine Umschließung mit Tasche und/oder ein Merkmal zum Sicherstellen einer Einmalverwendbarkeit und/oder einen elektrotechnischen Kern, der wenigstens einen isolierten elektrisch leitenden Draht umfasst oder einen gemäß dem ersten Aspekt ausgebildeten elektrotechnischen Kern aufweisen.

Dieser Aspekt betrifft eine Adhäsionsschicht für einen Plasmaapplikator, die ausgebildet ist, gleichzeitig die Funktion einer Distanzstruktur zu erfüllen. Eine Adhäsionsschicht gemäß diesem Aspekt ist so ausgebildet, dass mit ihr ein Plasmaapplikator auf einer zu behandelnden Oberfläche fixiert werden kann und dann zwischen einem elektrotechnischen Kern eines Plasmaapplikators und einer zu behandelnden Oberfläche im Betrieb ein definierter Abstand hergestellt wird, sodass sich ein erzeugtes Plasma über der zu behandelnden Oberfläche verteilen kann. Eine solche Adhäsionsschicht kann beispielweise von einem Klebstoff, insbesondere einem Silikon- oder einem Polyurethan (PU)-Klebstoff gebildet sein. Ein die Adhäsionsschicht bildender Klebstoff kann beispielsweise eine spezielle Geometrie haben, beispielsweise ein Wabenmuster oder X-, O-, Z-, M-, E-, oder eine W-Form haben, und auf die einer zu behandelnden Oberfläche zugewandte Seite eines Plasmaapplikators aufgebracht werden. Für eine Plasmabehandlung wird der Plasmaapplikator mit aufgebrachter Adhäsionsschicht auf eine zu behandelnde Oberfläche aufgelegt und mittels der Adhäsionsschicht auf dieser fixiert. In Kontakt mit der zu behandelnden Oberfläche haftet der die Adhäsionsschicht bildende Klebstoff in der speziellen Geometrie auf der zu behandelnden Oberfläche, sodass in den Bereichen ohne Klebstoff jeweils ein definiertes Gasvolumen geschaffen wird, in dem sich ein erzeugtes Plasma verteilen kann.

Alternativ kann für eine Plasmabehandlung die Adhäsionsschicht auf der zu behandelnden Oberfläche bevorzugt um die zu behandelnde Wunde herum aufgebracht werden und im Anschluss die wundzugewandte Seite des Plasmaapplikator auf die wundabgewandte Seite der auf der zu behandelnden Oberfläche fixierten Adhäsionsschicht fixiert werden, sodass in den Bereichen ohne Klebstoff jeweils ein definiertes Gasvolumen geschaffen wird, in dem sich ein erzeugtes Plasma verteilen kann.

Ein weiterer Aspekt, der eigenständig und unabhängig von den übrigen hierin beschriebenen Aspekten oder in Kombination mit wenigstens einem dieser Aspekte zu einem verbesserten Plasmaapplikator beitragen kann, betrifft eine Umschließung die Einschubschlitze aufweist, in die eine Energieversorgungseinheit oder eine Einschubvorrichtung eingeschoben werden kann. Eine Umschließung gemäß diesem Aspekt kann bei verschiedenen Plasmaapplikatoren verwirklicht sein, die beispielsweise eine integrierte Energieversorgungseinheit und/oder einen Zugangsanschluss und/oder eine Umschließung mit Tasche und/oder ein Merkmal zum Sicherstellen einer Einmalverwendbarkeit und/oder einen elektrotechnischen Kern, der wenigstens einen isolierten elektrisch leitenden Draht umfasst oder einen gemäß dem ersten Aspekt ausgebildeten elektrotechnischen Kern aufweisen. Die Energieversorgungseinheit, die in die Einschubschlitze eingeschoben werden kann, kann insbesondere eine autarke, mobile Energieversorgungseinheit sein. Die Einschubvorrichtung, die in die Einschubschlitze eingeschoben werden kann, kann insbesondere über ein Kabel mit einer eher stationär genutzten Energieversorgungseinheit verbunden sein.

Gemäß diesem Aspekt weist ein Plasmaapplikator mit einem elektrotechnischen Kern eine Umschließung mit Einschubschlitzen auf. Der elektrotechnische Kern kann ein elektrotechnischer Kern gemäß einer der hier beschriebenen Ausführungsvarianten sein. Der elektrotechnische Kern ist vorzugsweise flexibel ausgebildet. Der elektrotechnische Kern kann in einer beliebigen Grundform gestaltet sein. Bevorzugt ist jedoch eine viereckige oder runde Grundform. Vorzugsweise ist der elektrotechnische Kern ein sechslagiger elektrotechnischer Kern, der bereits selbst berührungssicher ausgebildet ist, gemäß dem ersten Aspekt. Wenigstens eine Elektrodenstruktur des elektrotechnischen Kerns kann eine spezielle Geometrie aufweisen. Der elektrotechnische Kern kann aber auch vierlagig und somit nicht berührungssicher sein. Der elektrotechnische Kern kann auch mehrere Elektrodenstrukturen mit einer speziellen Geometrie aufweisen. Wenn ein elektrotechnischer Kern mehrere Elektrodenstrukturen mit spezieller Geometrie aufweist, können die Elektrodenabschnitte der jeweiligen Elektrodenstrukturen zueinander in einer definierten Überlappung angeordnet sein. Wenn der elektrotechnische Kern mehrere Elektrodenstrukturen aufweist, ist zwischen jeweils zwei benachbarten Elektrodenstrukturen vorzugsweise wenigstens eine Isolationsschicht angeordnet, um die zwei entsprechenden Elektrodenstrukturen galvanisch voneinander zu trennen. Beispielsweise kann ein geeigneter elektrotechnischer Kern im Rollensiebdruckverfahren hergestellt werden, indem auf eine oder beide Seiten eines Elektrodenträgers jeweils eine Elektrodenstruktur gedruckt wird. Vorzugsweise weist der elektrotechnische Kern eine weitere Isolationsschicht auf, die auf der einer zu behandelnden Oberfläche zugewandten Seite des Plasmaapplikators angeordnet ist. Diese weitere Isolationsschicht ist vorzugsweise von einem biokompatiblen Material gebildet. Die Umschließung kann so gestaltet sein, dass sie den elektrotechnischen Kern komplett umschließt. Die Umschließung kann beispielsweise Silikon, Lacke oder eine Parylenbeschichtung aufweisen. Vorzugsweise weist die Umschließung Einschubschlitze auf, die an der der zu behandelnden Oberfläche abgewandten Seite des Plasmaapplikators angeordnet und so ausgebildet sind, dass eine zu den Einschubschlitzen komplementäre Energieversorgungseinheit oder eine Einschubvorrichtung in die Einschubschlitze eingeschoben werden kann, um dann mit Kontaktierungen des elektrotechnischen Kerns elektrisch verbunden zu sein.

Ein weiterer Aspekt, der eigenständig und unabhängig von den übrigen hierin beschriebenen Aspekten oder in Kombination mit wenigstens einem dieser Aspekte zu einem verbesserten Plasmaapplikator beitragen kann, betrifft eine Umschließung mit absorbierenden Eigenschaften. Die Umschließung kann zusätzlich auch eine Tasche aufweisen, in die ein elektrotechnischer Kern eingeschoben werden kann und/oder Einschubschlitze, in die eine Energieversorgungseinheit oder eine Einschubvorrichtung eingeschoben werden kann, aufweisen. Eine Umschließung gemäß diesem Aspekt kann bei verschiedenen Plasmaapplikatoren verwirklicht sein, die beispielsweise eine integrierte Energieversorgungseinheit und/oder einen Zugangsanschluss und/oder ein Merkmal zum Sicherstellen einer Einmalverwendbarkeit und/oder einen elektrotechnischen Kern, der wenigstens einen isolierten elektrisch leitenden Draht umfasst oder einen gemäß dem ersten Aspekt ausgebildeten elektrotechnischen Kern aufweisen.

Gemäß diesem Aspekt kann ein Plasmaapplikator einen elektrotechnischen Kern, eine Umschließung mit absorbierenden Eigenschaften, eine Adhäsionsschicht, eine Distanzstruktur und eine Steckvorrichtung aufweisen. Der elektrotechnische Kern ist vorzugsweise gemäß einer der hier beschriebenen Variante eines elektrotechnischen Kerns ausgebildet. Beispielsweise kann der elektrotechnische Kern gemäß dem elektrotechnischen Kern des ersten Aspekts ausgebildet sein und bereits selbst einen Berührungsschutz gewährleisten. Ein geeigneter elektrotechnischer Kern kann im Rollensiebdruckverfahren hergestellt sein, bei dem auf eine oder auf beide Seiten eines Elektrodenträgers jeweils eine Elektrodenstruktur gedruckt wird. Bevorzugt ist der elektrotechnische Kern flexibel ausgebildet. Der elektrotechnische Kern kann in verschiedenen Grundformen verwirklicht werden. Der elektrotechnische Kern kann eine Grundform haben, die beispielsweise rund oder vieleckig, vorzugsweise viereckig, ist. Wenigstens eine Elektrodenstruktur des elektrotechnischen Kerns kann eine spezielle Geometrie aufweisen. Wenn mehrere Elektrodenstrukturen des elektrotechnischen Kerns eine spezielle Geometrie aufweisen können insbesondere diese Elektrodenstrukturen so zueinander angeordnet sein, dass die Elektrodenabschnitte der jeweiligen Elektrodenstrukturen zueinander eine definierte Überlappung aufweisen. Wenn der elektrotechnische Kern mehrere Elektrodenstrukturen aufweist, sind jeweils benachbarte Elektrodenstrukturen vorzugsweise durch eine Isolationsschicht galvanisch voneinander getrennt. Vorzugsweise ist eine weitere Isolationsschicht auf der einer zu behandelnden Oberfläche zugewandten Seite angeordnet und befindet sich im Anwendungsfall zwischen einer zu behandelnden Oberfläche und derjenigen Elektrodenstruktur, die im Anwendungsfall den geringsten Abstand zu der zu behandelnden Oberfläche aufweist. Auch wenn der elektrotechnische Kern lediglich eine Elektrodenstruktur aufweist, ist vorzugsweise eine Isolationsschicht benachbart zu dieser Elektrodenstruktur auf der einer zu behandelnden Oberfläche zugewandten Seite des Plasmaapplikators angeordnet. Eine Isolationsschicht, die auf der einer zu behandelnden Oberfläche zugewandten Seite des Plasmaapplikators angeordnet ist, ist vorzugsweise von einem biokompatiblen Material gebildet.

Die Umschließung umschließt den elektrotechnischen Kern vorzugsweise vollständig und hat wenigstens in einem Teilbereich absorbierende Eigenschaften. Die Umschließung kann auch eine Tasche aufweisen, in der der elektrotechnische Kern herausnehmbar angeordnet ist. Die Umschließung mit absorbierenden Eigenschaften besteht vorzugsweise aus mindestens einer Lage von flüssigkeitsabsorbierenden und/oder flüssigkeitsabführenden und/oder flüssigkeitsverteilenden Materialien, wie z.B. Textil, Mull, PU-Schaum, Verteilerschicht, Wundkontaktschicht, Distanzstruktur. Die Distanzstruktur ist vorzugsweise von einem biokompatiblen Material gebildet und kann durch die Umschließung selbst realisiert sein. Vorzugsweise ist die Distanzstruktur von einem luftdurchlässigen Material gebildet. Die Distanzstruktur kann auch Mull, Absorber, Textilien, Zellstoff, oder PU-Schaum aufweisen. Die Adhäsionsschicht ist vorzugsweise vollflächig als letzte Schicht auf der einer zu behandelnden Oberfläche zugewandten Seite des Plasmaapplikators angeordnet und ausgebildet, den Plasmaapplikator auf einer zu behandelnden Oberfläche zu fixieren. Die Adhäsionsschicht kann auch größere und/oder kleinere Aussparungen aufweisen. Die Steckvorrichtung kann fest mit dem elektrotechnischen Kern verbunden sein und ist vorzugsweise laschenförmig ausgebildet. Eine laschenförmige Steckvorrichtung kann dadurch realisiert sein, dass die Elektrodenstrukturen und Isolationsschichten des elektrotechnischen Kerns in Laschenform erweitert werden. Die Dimensionen der Steckvorrichtung sind vorzugsweise in Abhängigkeit von der Länge der Kriechstrecken so bemessen, dass im Betrieb keine Teilentladungen innerhalb einer zusammengesteckten Steckvorrichtung und einer Einschubvorrichtung entstehen.

Ein weiterer Aspekt, der eigenständig und unabhängig von den übrigen hierin beschriebenen Aspekten oder in Kombination mit wenigstens einem dieser Aspekte zu einem verbesserten Plasmaapplikator beitragen kann, betrifft einen Plasmaapplikator der dafür vorgesehen ist, über einen längeren Zeitraum auf einer zu behandelnden Oberfläche zu verbleiben. Der Plasmaapplikator gemäß diesem Aspekt kann auch eine integrierte Energieversorgungseinheit und/oder einen Zugangsanschluss und/oder ein Merkmal zum Sicherstellen einer Einmalverwendbarkeit und/oder einen elektrotechnischen Kern, der wenigstens einen isolierten elektrisch leitenden Draht umfasst oder einen gemäß dem ersten Aspekt ausgebildeten elektrotechnischen Kern und/oder eine Umschließung mit absorbierenden Eigenschaften und/oder einer Tasche und/oder Einschubschlitze aufweisen.

Dieser Aspekt betrifft also einen Plasmaapplikator, der dafür vorgesehen ist, über einen längeren Zeitraum auf einer zu behandelnden Oberfläche zu verbleiben und einen elektrotechnischen Kern, eine Umschließung, eine Adhäsionsschicht, eine Distanzstruktur, eine Steckvorrichtung sowie wenigstens einen Sensor aufweist. Der Plasmaapplikator kann mit verschiedenen elektrotechnischen Kernen verwirklicht werden. Vorzugsweise ist der elektrotechnische Kern jedoch ein elektrotechnischer Kern gemäß dem ersten Aspekt, also sechslagig und selbst berührungssicher ausgebildet. Vorzugsweise ist der elektrotechnische Kern derart flexibel ausgebildet, dass dieser in seiner Form an eine Form einer zu behandelnden Oberfläche angepasst werden kann. Der elektrotechnische Kern kann beispielweise eine runde, eine viereckige oder eine andere vieleckige Grundform haben. Wenigstens eine Elektrodenstruktur des elektrotechnischen Kerns kann eine spezielle Geometrie aufweisen. Wenn mehrere Elektrodenstrukturen des elektrotechnischen Kerns eine spezielle Geometrie aufweisen, können insbesondere diese Elektrodenstrukturen so zueinander angeordnet sein, dass die Elektrodenabschnitte der jeweiligen Elektrodenstrukturen zueinander eine definierte Überlappung aufweisen. Wenn der elektrotechnische Kern mehrere Elektrodenstrukturen aufweist, sind jeweils benachbarte Elektrodenstrukturen vorzugsweise durch eine Isolationsschicht galvanisch voneinander getrennt. Vorzugsweise ist eine weitere Isolationsschicht auf der einer zu behandelnden Oberfläche zugewandten Seite angeordnet und befindet sich im Anwendungsfall zwischen einer zu behandelnden Oberfläche und derjenigen Elektrodenstruktur, die im Anwendungsfall in dem kleinsten Abstand zu der zu behandelnden Oberfläche angeordnet ist. Auch wenn der elektrotechnische Kern lediglich eine Elektrodenstruktur aufweist, ist vorzugsweise eine Isolationsschicht benachbart zu dieser Elektrodenstruktur auf der einer zu behandelnden Oberfläche zugewandten Seite des Plasmaapplikators angeordnet. Eine Isolationsschicht, die auf der einer zu behandelnden Oberfläche zugewandten Seite des Plasmaapplikators angeordnet ist, ist vorzugsweise von einem biokompatiblen Material gebildet.

Die Umschließung umschließt den elektrotechnischen Kern wenigstens teilweise. Vorzugsweise ist die Umschließung von einem biokompatiblen Material gebildet. Die Umschließung kann beispielsweise Silikon, Lacke oder eine Parylenbeschichtung aufweisen. Insbesondere dann, wenn der elektrotechnische Kern des Plasmaapplikator bereits selbst berührungssicher ausgebildet ist, kann die Umschließung einlagig ausgebildet sein und selbst keinen Berührungsschutz gewährleisten. Die Distanzstruktur ist vorzugsweise benachbart zu dem elektrotechnischen Kern auf der einer zu behandelnden Oberfläche zugewandten Seite des Plasmaapplikators angeordnet. Die Distanzstruktur ist vorzugsweise von einem biokompatiblen Material gebildet. Die Adhäsionsschicht ist vorzugsweise als letzte Schicht auf der einer zu behandelnden Oberfläche zugewandten Seite des Plasmaapplikators angeordnet. Die Adhäsionsschicht kann beispielweise auf der einer zu behandelnden Oberfläche zugewandten Seite des Plasmaapplikators wie ein Rahmen entlang des Umfangs des Plasmaapplikators geführt sein und so einen Kleberand bilden.

Die Steckvorrichtung kann fest mit dem elektrotechnischen Kern verbunden sein. Die Steckvorrichtung ist vorzugsweise laschenförmig ausgebildet, indem die Elektrodenstrukturen und Isolationsschichten des elektrotechnischen Kerns in einer Laschenform erweitert werden. Der wenigstens eine Sensor ist vorzugsweise ausgebildet, im Betrieb eine für eine Plasmabehandlung und/oder eine Wundheilung relevante Messgrößen, insbesondere physiologische und/oder physikalische Messgrößen eines im Anwendungsfall von dem Plasmaapplikator bedeckten Körperabschnitts, zu erfassen und auszugeben. Für eine Behandlung einer Oberfläche über einen längeren Zeitraum, der insbesondere die Zeitdauer einer Wundheilung umfassen kann, kann der Plasmaapplikator derart ausgebildet sein, dass eine zu behandelnde Oberfläche im Anwendungsfall durch den Plasmaapplikator versiegelt wird. Die Adhäsionsschicht ist dann vorzugsweise von einem Silikon- oder einem PU-Klebstoff und die Umschließung von einem luftundurchlässigen Material gebildet. Insbesondere kann die Adhäsionsschicht einen Klebstoff aufweisen, der durch Bestrahlung mit UV-Licht oder durch Kontakt mit Alkohol seine Hafteigenschaften verliert, sodass der Plasmaapplikator nach einer längeren Behandlung von einer zu behandelnden Oberfläche abgenommen werden kann. Während der Zeitdauer einer Langzeitbehandlung können von dem wenigstens einen Sensor ausgegebene Messgrößen beispielsweise im Rahmen eines Telemonitoring oder eines home-monitoring ausgewertet und anhand dessen ein Verlauf einer Wundheilung beurteilt werden.

Ein weiterer Aspekt, der eigenständig und unabhängig von den übrigen hierin beschriebenen Aspekten oder in Kombination mit wenigstens einem dieser Aspekte zu einem verbesserten Plasmaapplikator beitragen kann, betrifft eine Distanzstruktur für einen Plasmaapplikator, die selbst eine Plasmaquelle ist. Eine Distanzstruktur gemäß diesem Aspekt stellt ein eigenständiges Produkt dar und kann als Modul zusammen mit verschieden ausgebildeten Plasmaapplikatoren verwendet werden. Solche Plasmaapplikatoren können beispielsweise eine integrierte Energieversorgungseinheit und/oder einen Zugangsanschluss und/oder ein Merkmal zum Sicherstellen einer Einmalverwendbarkeit und/oder einen elektrotechnischen Kern, der wenigstens einen isolierten elektrisch leitenden Draht umfasst oder einen gemäß dem ersten Aspekt ausgebildeten elektrotechnischen Kern und/oder eine Umschließung mit absorbierenden Eigenschaften und/oder einer Tasche und/oder Einschubschlitze aufweisen.

Dieser Aspekt betrifft eine Distanzstruktur für einen Plasmaapplikator, die selbst eine Plasmaquelle ist. Die Distanzstruktur erfüllt also sowohl die Funktion einer Distanzstruktur also auch die einer Plasmaquelle. Eine solche Distanzstruktur weist wenigstens eine Elektrodenstruktur auf und ist vorzugsweise in einer speziellen Geometrie gestaltet. Insbesondere kann die Distanzstruktur eine Reihe von Hohlräumen oder Durchführungen aufweisen, die so gestaltet sind, dass ein im Anwendungsfall erzeugtes Plasma mit einer zu behandelnden Oberfläche wechselwirken kann. Wenn die Distanzstruktur lediglich eine Elektrodenstruktur aufweist, wird diese im Anwendungsfall zum Zünden eines Plasmas mit einem Spannungssignal beaufschlagt. Die Gegenelektrode ist dann vorzugsweise durch die zu behandelnde Oberfläche selbst realisiert. Die Distanzstruktur kann auch eine im Anwendungsfall getriebene Elektrodenstruktur und eine Gegenelektrode aufweisen. Die Distanzstruktur kann beispielsweise von einem Flachkabel gebildet sein, das gleichzeitig eine Plasmaquelle ist. Das Flachkabel kann auch ein zwei-adriges Flachkabel sein. Eine Isolation eines Flachkabels hat vorzugsweise eine Dicke, die zwischen 5 µm und einigen 100 µm beträgt, vorzugsweise weniger als 100 µm beträgt, vorzugsweise zwischen 40 µm und 60 µm beträgt, bevorzugt 50 µm beträgt.

Wenigstens diejenigen Bereiche der Distanzstruktur, die im Anwendungsfall in Kontakt mit einer zu behandelnden Oberfläche sind, sind vorzugsweise von einem biokompatiblen Material gebildet.

Die Distanzstruktur kann mit einer Steckvorrichtung verbunden sein. Bei einer Distanzstruktur mit einer Steckvorrichtung ist insbesondere wenigstens eine Elektrodenstruktur der Distanzstruktur elektrisch leitfähig mit einer Leiterbahn der Steckvorrichtung verbunden, sodass wenn die Steckvorrichtung beispielsweise über ein Kabel mit einer Energieversorgungseinheit verbunden ist, im Anwendungsfall ein zum Zünden eines Plasmas ausreichendes Spannungssignal an die wenigstens eine Elektrodenstruktur übertragen werden kann.

Im Anwendungsfall kann die Distanzstruktur mittels einer Adhäsionsschicht auf einer zu behandelnden Oberfläche fixiert werden. Es ist jedoch bevorzugt, dass die Distanzstruktur für eine Plasmabehandlung mittels einer Folie, insbesondere einer Folie, die auf einer Seite haftenden Eigenschaften aufweist, auf einer zu behandelnden Oberfläche fixiert wird. Die Folie wird dann so über die Distanzstruktur und die zu behandelnde Oberfläche gezogen, dass die Distanzstruktur durch die Folie auf der zu behandelnden Oberfläche fixiert wird. Vorzugsweise wird durch die aufgebrachte Folie ein abgeschlossener Gasraum gebildet, in dem ein Plasma im Anwendungsfall erzeugt werden und mit der zu behandelnden Oberfläche wechselwirken kann. Es kann auch eine Folie verwendet werden, die keine Oberfläche mit haftenden Eigenschaften aufweist. Eine solche Folie, die selbst nicht auf einer zu behandelnde Oberfläche haftet, kann beispielsweise durch eine Adhäsionsschicht auf der zu behandelnden Oberfläche fixiert werden. Weiterhin kann eine solche Folie auf der zu behandelnden Oberfläche fixiert werden, indem ein Vakuum im abgeschlossenen Gasraum erzeugt wird. Es kann auch eine Folie verwendet werden, bei der durch Zusammenbringen von Abschnitten dieser Folie ein Haftkontakt zwischen diesen Abschnitten entsteht. Eine solche Folie kann mit Überlapp um beispielsweise ein Körperteil eines Patienten gewickelt werden, um so die Distanzstruktur auf einer zu behandelnden Oberfläche zu fixieren und einen abgeschlossenen Gasraum zu bilden.

Ein weiterer Aspekt, der eigenständig und unabhängig von den übrigen hierin beschriebenen Aspekten oder in Kombination mit wenigstens einem dieser Aspekte zu einem verbesserten Plasmaapplikator beitragen kann, betrifft einen Plasmaapplikator der dafür vorgesehen ist, in bestimmten dreidimensionalen Formen auf eine zu behandelnde Oberfläche aufgebracht zu werden. Ein Plasmaapplikator gemäß diesem Aspekt kann auch eine integrierte Energieversorgungseinheit und/oder einen Zugangsanschluss und/oder ein Merkmal zum Sicherstellen einer Einmalverwendbarkeit und/oder einen elektrotechnischen Kern, der wenigstens einen isolierten elektrisch leitenden Draht umfasst oder einen gemäß dem ersten Aspekt ausgebildeten elektrotechnischen Kern und/oder eine Umschließung mit absorbierenden Eigenschaften und/oder einer Tasche und/oder Einschubschlitze aufweisen.

Ein weiterer Aspekt betrifft einen Plasmaapplikator der ausgebildet ist, für eine Plasmabehandlung in einer speziellen Form, beispielsweise der Form eines Zeltes oder eines Kegels, auf einer zu behandelnden Oberfläche aufgebracht zu werden. Ein Plasmaapplikator gemäß diesem Aspekt weist einen elektrotechnischen Kern, eine Umschließung, eine Adhäsionsschicht und eine Steckvorrichtung auf. Der Plasmaapplikator kann mit verschiedenen elektrotechnischen Kernen verwirklicht werden. Vorzugsweise ist der elektrotechnische Kern jedoch ein elektrotechnischer Kern gemäß dem ersten Aspekt, also bereits selbst berührungssicher. Ein geeigneter elektrotechnischer Kern kann beispielsweise im Rollensiebdruckverfahren hergestellt werden, bei dem auf eine oder auf beide Seite eines Elektrodenträgers jeweils eine Elektrodenstruktur gedruckt wird. Die Grundform des Plasmaapplikators kann sich insbesondere aus derjenigen dreidimensionalen Form ergeben, in die der Plasmaapplikator für eine Plasmabehandlung gebracht werden soll. Wenn der Plasmaapplikator beispielsweise dafür vorgesehen ist, für eine Plasmabehandlung in eine Kegelform gebracht zu werden, kann die Grundform des Plasmaapplikators der ausgerollten Mantelfläche des Kegels entsprechen. Ein solcher Plasmaapplikator ist vorzugsweise flexibel in seiner Form. Ein flexibler Plasmaapplikator kann für eine Plasmabehandlung beispielsweise um einen Schlauch oder ein Kabel eines bereits bestehenden Zugangs eines Patienten gelegt werden, ohne dass der bereits gelegte Zugang zum zu behandelnden Körper für eine Plasmabehandlung entfernt werden braucht.

Der Plasmaapplikator kann aber auch eine starre Form aufweisen, beispielsweise bereits die Form eines Kegels. Insbesondere ein solcher Plasmaapplikator mit starrer Form kann ein Loch oder einen Schlitz aufweisen, durch den ein Schlauch oder Kabel geführt werden kann. Ein solches Loch oder ein solcher Schlitz kann sich beispielsweise an der Kegelspitze oder in der Mantelfläche eines kegelförmigen Plasmaapplikators befinden.

Wenigstens eine Elektrodenstruktur des elektrotechnischen Kerns kann eine spezielle Geometrie aufweisen. Wenn mehrere Elektrodenstrukturen des elektrotechnischen Kerns eine spezielle Geometrie aufweisen, sind die Elektrodenabschnitte der jeweiligen Elektrodenstrukturen vorzugsweise zueinander in einer definierten Überlappung angeordnet. Zwischen benachbarten Elektrodenstrukturen ist vorzugsweise eine Isolationsschicht angeordnet, die ausgebildet ist, die beiden benachbarten Elektrodenstrukturen galvanisch voneinander zu trennen. Auf der einer zu behandelnden Oberfläche zugewandten Seite weist der Plasmaapplikator vorzugsweise eine weitere, vorzugsweise von einem biokompatiblen Material gebildete Isolationsschicht auf.

Die Umschließung kann den elektrotechnischen Kern teilweise oder sogar vollständig umschließen. De Umschließung ist vorzugsweise von einem biokompatiblen Material gebildet.

Wenn der elektrotechnische Kern selbst bereits berührungssicher ausgebildet ist, kann es ausreichen, wenn die Umschließung von lediglich einer Lage gebildet ist und selbst keinen Berührungsschutz gewährleistet. Die Umschließung kann beispielsweise Silikon, Lacke Textilien und/oder eine Parylenbeschichtung aufweisen. Die Adhäsionsschicht ist vorzugsweise derart angeordnet, dass der Plasmaapplikator in der für eine Plasmabehandlung vorgesehenen Form auf einer zu behandelnden Oberfläche fixiert werden kann. Wenn der Plasmaapplikator beispielsweise dafür vorgesehen ist, in einer Kegelform auf einer zu behandelnden Oberfläche aufgebracht zu werden, ist die Adhäsionsschicht vorzugsweise an der der zu behandelnden Oberfläche zugewandten Seite der Mantelfläche angeordnet.

Ein weiterer Aspekt, der eigenständig und unabhängig von den übrigen hierin beschriebenen Aspekten oder in Kombination mit wenigstens einem dieser Aspekte zu einem verbesserten Plasmaapplikator beitragen kann, betrifft einen Plasmaapplikator, der insbesondere für die Behandlung von großflächigen Wunden mit einer Fläche von einigen und/oder mehreren Quadratdezimetern (z.B. Brandwunden und insbesondere von großflächigen Brandwunden) geeignet ist. Ein solcher Plasmaapplikator kann auch eine integrierte Energieversorgungseinheit und/oder einen Zugangsanschluss und/oder ein Merkmal zum Sicherstellen einer Einmalverwendbarkeit und/oder einen elektrotechnischen Kern, der wenigstens einen isolierten elektrisch leitenden Draht umfasst oder einen gemäß dem ersten Aspekt ausgebildeten elektrotechnischen Kern und/oder eine Umschließung mit absorbierenden Eigenschaften und/oder einer Tasche und/oder mit Einschubschlitzen aufweisen.

Ein weiterer Aspekt betrifft einen Plasmaapplikator, der insbesondere für die Behandlung von großflächigen Wunden (z.B. großflächige Brandwunden) geeignet ist. Der Plasmaapplikator ist geeignet, mit der einer zu behandelnden Oberfläche abgewandtem Seite an einem Trägermaterial befestigt und zusammen mit diesem Trägermaterial verwendet zu werden. Ein Trägermaterial kann zum Beispiel eine Rettungsdecke oder ein Verband oder eine große Folie sein. Der Plasmaapplikator gemäß diesem Aspekt weist einen elektrotechnischen Kern, eine Umschließung und eine Adhäsionsschicht auf. Vorzugsweise umfasst der Plasmaapplikator weiterhin eine integrierte Energieversorgungseinheit. Der Plasmaapplikator kann mit verschiedenen elektrotechnischen Kernen verwirklicht werden. Ein geeigneter elektrotechnischer Kern beispielsweise im Rollensiebdruckverfahren hergestellt werden. Der elektrotechnischen Kern kann auch wenigstens eine Schicht aufweisen, in der mehrere unabhängige Elektrodenstrukturen angeordnet und nicht elektrisch miteinander verbunden sind und jeweils elektrisch voneinander unabhängige Behandlungsgebiete bilden. Die mehreren Elektrodenstrukturen innerhalb einer Schicht können zeitlich versetzt, d.h. kaskadiert, angesteuert werden. Vorzugsweise ist der elektrotechnische Kern jedoch ein elektrotechnischer Kern gemäß dem ersten Aspekt und bereits selbst berührungssicher. Der elektrotechnische Kern kann auch in verschiedenen Grundformen verwirklicht sein. Wenigstens eine Elektrodenstruktur des elektrotechnischen Kerns kann eine spezielle Geometrie aufweisen. Wenn mehrere Elektrodenstrukturen des elektrotechnischen Kerns eine spezielle Geometrie aufweisen, können insbesondere diese Elektrodenstrukturen so zueinander angeordnet sein, dass die Elektrodenabschnitte der jeweiligen Elektrodenstrukturen zueinander eine definierte Überlappung aufweisen. Wenn der elektrotechnische Kern mehrere Elektrodenstrukturen aufweist, sind jeweils benachbarte Elektrodenstrukturen vorzugsweise durch eine Isolationsschicht galvanisch voneinander getrennt. Vorzugsweise ist wenigstens eine weitere Isolationsschicht auf der einer zu behandelnden Oberfläche zugewandten Seite des Plasmaapplikators angeordnet und bevorzugt von einem biokompatiblen Material gebildet. Die Umschließung umschließt den elektrotechnischen Kern wenigstens teilweise und ist bevorzugt von einem biokompatiblen Material gebildet. Wenn der elektrotechnische Kern selbst bereits berührungssicher ausgebildet ist, kann die Umschließung einlagig ausgebildet sein und selbst keinen Berührungsschutz gewährleisten. Die Umschließung kann beispielsweise Silikon, Lacke, Textilien und/oder eine Parylenbeschichtung aufweisen.

Die Adhäsionsschicht ist vorzugsweise als letzte Schicht auf der einer zu behandelnden Oberfläche abgewandten Seite des Plasmaapplikators angeordnet. Die Adhäsionsschicht ist vorzugsweise ausgebildet, den Plasmaapplikator auf seiner einer zu behandelnden Oberfläche abgewandten Seite an einem Trägermaterial, beispielsweise einer Rettungsdecke oder einer Folie, zu befestigen.

Der Plasmaapplikator kann auch eine Steckvorrichtung aufweisen, die vorzugsweise laschenförmig ausgebildet ist. Die Steckvorrichtung kann fest mit dem elektrotechnischen Kern verbunden sein.

Die optionale in den Plasmaapplikator integrierte Energieversorgungseinheit umfasst eine Energiequelle, die ein Akkumulator, eine Batterie oder ein Kondensator sein kann. Die Energieversorgungseinheit ist vorzugsweise mit einer Elektrodenstruktur des elektrotechnischen Kerns verbunden und ausgebildet, ein zum Zünden eines Plasmas ausreichendes Spannungssignal bereitzustellen. Die Energieversorgungseinheit kann eine elektrische Schaltung aufweisen, die ausgebildet ist, ein Spannungssignal in ein zum Zünden eines Plasmas ausreichendes Spannungssignal zu wandeln. Die integrierte Energieversorgungseinheit kann auch eine mit dem Energiespeicher elektrisch verbundene Empfangs-Spulenanordnungen aufweisen und derart ausgebildet sein, dass der Energiespeicher geladen werden kann, indem von einer Sende-Spulenanordnung elektrische Energie von der Sende-Spulenanordnung auf die Empfangs-Spulenanordnung im Plasmaapplikator induktiv übertragen wird. Wenn der Plasmaapplikator eine Steckvorrichtung aufweist, ist die Steckvorrichtung vorzugsweise elektrisch leitfähig mit der Energieversorgungseinheit verbunden, sodass die Energiequelle, insbesondere ein Akkumulator oder ein Kondensator, der Energieversorgungseinheit durch Verbinden der Steckvorrichtung mit einer externen Energieversorgungseinheit geladen werden kann.

### Bevorzugte Ausführungsvarianten eines Plasmaapplikators

In einer Ausführungsvariante in der ein Plasmaapplikator eine Umschließung aufweist, kann diese von einem biokompatiblen Material gebildet sein, wie z.B. medizinischem Silikon, einem Lack, einem Klebstoff, einer Folie, einem Textil, einem Kompressionstextil oder organischem Material wie z.B. Mull, Zellstoff oder Baumwolle.

In einer Ausführungsvariante, in der ein Plasmaapplikator eine Umschließung aufweist, kann die Umschließung des Plasmaapplikators wenigstens eine Lage von flüssigkeitsabsorbierenden und/oder flüssigkeitsabführenden und/oder flüssigkeitsverteilenden Materialien umfassen.

In einer Ausführungsvariante in der ein Plasmaapplikator einen Zugangsanschluss aufweist, ist der Zugangsanschluss derart angeordnet und ausgebildet, dass ein fluides Medium während einer Plasmabehandlung einem zwischen dem elektrotechnischen Kern und einer zu behandelnden Oberfläche durch die Umschließung gebildeten abgeschlossenen Gasraum zu- oder aus dem Gasraum abgeführt werden kann. Ein fluides Medium ist ein gasförmiges oder flüssiges Medium.

In einer Ausführungsvariante in der ein Plasmaapplikator eine Umschließung aufweist, kann die Umschließung des Plasmaapplikators Einschubschlitze umfassen, die an der der zu behandelnden Oberfläche abgewandten Seite des Plasmaapplikators angeordnet und ausgebildet sind, dass eine zu den Einschubschlitzen komplementäre Energieversorgungseinheit bzw. Einschubvorrichtung in die Einschubschlitze eingeschoben werden kann, um dann elektrisch mit den Kontaktierungen des elektrotechnischen Kerns elektrisch verbunden zu sein.

Ein hier beschriebener und auch anderer Plasmaapplikator kann auf einer einer zu behandelnden Oberfläche zugewandten Seite des Plasmaapplikators einen mit Wiederhaken besetzen Teil eines Klettverschlusses aufweisen.

In einer Ausführungsvariante in der ein Plasmaapplikator eine integrierte Energieversorgungseinheit aufweist, kann die Energieversorgungseinheit einen Energiespeicher umfassen, der elektrisch mit den Kontaktierungen des elektrotechnischen Kerns verbunden ist, um im Betrieb ein zum Zünden eines Plasmas ausreichendes Spannungssignal an die zweite Elektrodenstruktur zu übertragen.

In einer Ausführungsvariante in der ein Plasmaapplikator eine integrierte Energieversorgungseinheit aufweist, kann die integrierte Energieversorgungseinheit eine elektrische Schaltung aufweisen, die ausgebildet ist, eine von dem Energiespeicher bereitgestellte Spannung in ein zum Zünden eines Plasmas ausreichendes Spannungssignal zu wandeln und dieses an die Kontaktierung der zweiten Elektrodenstruktur zu übertragen.

In einer Ausführungsvariante in der ein Plasmaapplikator eine mit wenigstens der Kontaktierung der zweiten Elektrodenstruktur elektrisch verbundene Energieaufnahmevorrichtung aufweist, kann die Energieaufnahmevorrichtung jeweils eine oder mehrere Empfangs-Spulenanordnungen enthalten. Mittels elektromagnetischer Induktion kann elektrische Energie von einer Sende-Spulenanordnungen einer Energieabgabevorrichtung auf die Empfangs-Spulenanordnungen im Plasmaapplikator übertragen werden.

### Plasmabehandlung

Eine Plasmabehandlung mit einem Plasmaapplikator wird über eine bestimmte Behandlungszeit durchgeführt. Diese Behandlungszeit beträgt typischerweise 1 bis 10 Minuten, bevorzugt 2 Minuten. Für den Fall, dass das Plasma während der Plasmabehandlung gepulst bereitgestellt wird, entspricht die aufsummierte Zeitdauer, in der Plasma während einer Plasmabehandlung tatsächlich gezündet wird, einem vergleichsweise geringen Teil, beispielsweise 10 %, der Gesamtzeitdauer einer Plasmabehandlung. Wenn die Gesamtdauer einer Plasmabehandlung z.B. zwei Minuten dauert und eine Pulsung von Plasma-An zu Plasma-Aus gleich 1 zu 9 beträgt, wird ein Plasma also lediglich während einer aufsummierten Zeitdauer von 12 Sekunden gezündet. Mit dem Ende der Plasmabehandlung endet typischerweise der Gebrauch des Plasmaapplikators. Obwohl sich der Gebrauch eines Plasmaapplikators auf den Zeitraum einer Plasmabehandlung beschränkt, kann ein Plasmaapplikator selbst bereits vor und auch nach einer Plasmabehandlung auf einer zu behandelnden Oberfläche angebracht sein. Es kann z.B. vorteilhaft für den Behandlungserfolg sein, wenn ein Plasmaapplikator auch einige Zeit nach einer Plasmabehandlung über einer Wunde aufgebracht ist. Beispielsweise kann nach einer eine Minute dauernden Plasmabehandlung, ein Plasmaapplikatorweitere fünf Minuten überderWunde verbleiben, um so den Behandlungserfolg zu verbessern. Ein Tragen eines Plasmaapplikators über einen längeren Zeitraum kann vorteilhaft sein, da ein Plasmaapplikator die zu behandelnde Oberfläche abdeckt und ein Wundareal gegenüber einer Neubesiedlung von externen Mikroorganismen abschließt. Falls ein Plasmaapplikator über einen längeren Zeitraum, z.B. über mehrere Tage oder auch mehrere Wochen, auf einer Wunde aufgebracht getragen wird, ist es vorteilhaft, wenn ein Plasmaapplikator derart ausgebildet ist, dass mit dem Plasmaapplikator mehrfach eine Plasmabehandlung durchgeführt werden kann.

Ein erstmaliger Gebrauch eines Plasmaapplikators bezieht sich auf die erste mit dem Plasmaapplikator durchgeführte Plasmabehandlung.

### Plasmaapplikator

Ein Plasmaapplikator dient im Wesentlichen zur Behandlung von menschlichen oder tierischen Oberflächen. Ein Plasmaapplikator eignet sich insbesondere zum Behandeln von Wunden, wie z. B. chronischen und/oder postoperativen Wunden. Darüber hinaus ist ein Plasmaapplikator auch zum Behandeln von Verbrennungen, Abschürfungen usw. geeignet. Auch der Einsatz zur Desinfektion, Faltenbehandlung, Narbenreduktion und/oder anderer kosmetischer Behandlungen ist denkbar. Eine Anwendung im Bereich der Plasmabehandlung von technischen Oberflächen ist ebenfalls möglich. Zum Beispiel können technische Oberflächen durch eine Plasmabehandlung veredelt werden. In diesem Zusammenhang sind die Plasmaaktivierung, plasma-gestützte chemische Gasphasenabscheidung sowie physikalische Gasphasenabscheidung als primäres Wirkprinzip zu nennen.

Ein Plasmaapplikatorwird zur Plasmabehandlung einer menschlichen oder tierischen oder technischen Oberfläche bevorzugt so angebracht, dass sich der elektrotechnische Kern auf oder nahe der zu behandelnden menschlichen oder tierischen oder technischen Oberfläche befindet.

### Weitere bevorzugte Ausführungsvarianten eines Plasmaapplikators

Ein Plasmaapplikator kann einen elektrotechnischen Kern und eine Steckvorrichtung, eine Umschließung und eine Adhäsionsschicht aufweisen. Die Steckvorrichtung ist dann vorzugsweise elektrisch mit wenigstens einer Elektrodenstruktur des elektrotechnischen Kerns verbunden und zum Übertragen von Spannungssignalen, bevorzugt mit einer Amplitude im Bereich von einigen hundert Volt bis zu 10 kV, geeignet.

Ein Plasmaapplikator kann einen flächigen elektrotechnischen Kern mit wenigstens einer Elektrodenstruktur aufweisen. Vorzugsweise umfasst ein solcher Plasmaapplikator eine Steckvorrichtung, die zum Übertragen eines Spannungssignals an die wenigstens eine Elektrodenstruktur mit wenigstens dieser Elektrodenstruktur elektrisch leitfähig verbunden ist.

Ein Plasmaapplikator kann derart ausgebildet sein, dass er sich flexibel, insbesondere formschlüssig, an beliebig gekrümmte Oberflächen anpassen kann und somit auch zum Behandeln von schwer zu behandelnden Hautareale, wie z. B. dem Fuß, für eine Plasmabehandlung verwendet werden kann. In einer solchen Ausführungsvariante weist der Plasmaapplikator einen elektrotechnischen Kern auf, der flexibel in eine entsprechende Form gebracht werden kann.

Alternativ kann ein Plasmaapplikator auch nicht flexibel und biegsam, sondern starr mit einer vorgegebenen Form ausgebildet. In diesem Fall kann ein elektrotechnischer Kern wenigstens eine Schicht oder Struktur aufweisen, die nicht flexibel ist und dem Plasmaapplikator eine starre Form vorgibt. Die vorgegebene Form ist vorteilhafterweise für die Verwendung des Plasmaapplikators an eine speziell geformte Oberfläche oder an einem bestimmten Körperteil angepasst. Ein Plasmaapplikator mit starrer Form kann zum Beispiel für die Fixierung an weichem Gewebe oder weichen Wundstrukturen vorteilhaft sein.

Ein Plasmaapplikator kann auch ausgebildet sein, kegelförmig um einen Schlauch oder ein Kabel angebracht zu werden. In diesem Fall wird ein Plasmaapplikator vorzugsweise um einen Schlauch oder ein Kabel gelegt, sodass ein abgeschlossener Gasraum unter einem Plasmaapplikator mit der Form eines Kegels entsteht, wobei die zu behandelnde Oberfläche unter dem Kegel liegt und ein Plasma im Anwendungsfall im abgeschlossenen Gasraum zwischen Kegelinnenraum und der zu behandelnden Oberfläche gezündet wird. Vorteilhafterweise ist es dann nicht notwendig, einen bereits gelegten Zugang zum zu behandelnden Körper zu entfernen, um an dem Zugang zum zu behandelnden Körper eine Behandlung mit einem Plasmaapplikator durchzuführen. Falls vor dem Legen eines Zugangs bekannt ist, dass eine Behandlung mit einem Plasmaapplikator stattfinden soll, kann es vorteilhaft sein, wenn ein Plasmaapplikator ein Loch oder einen Schlitz aufweist, z.B. in einer Kegelspitze, durch den eine Kabel oder ein Schlauch geführt werden kann. Dadurch kann zunächst ein Zugang gelegt werden und zu einem späteren Zeitpunkt eine Plasmabehandlung stattfinden, ohne dass der Zugang entfernt werden muss.

Es kann vorteilhaft sein, wenn eine Wundauflagefläche eines Plasmaapplikators oder ein Plasmaapplikator selbst skalierbar ausgebildet sind. Ein skalierbarer Plasmaapplikator kann eine Vielzahl von Formen haben, wie z.B. eckig, rund, 8-förmig *(butterfly),* oder eine an spezielle Körperstrukturen, z.B. dem Bereich unter der Brust oder der Ferse, angepasste Form. Insbesondere kann die Wundauflagefläche eines Plasmaapplikators im Bereich von einigen Zentimetern bis hin zu vielen Zehnzentimetern skalierbar sein. Hierbei ist bevorzugt, dass ein Plasmaapplikator mit einer Fläche oberhalb von ungefähr 20 cm mal 20 cm bevorzugt rechteckig oder rund ist. Ein Plasmaapplikator mit einer kleineren Fläche ist insbesondere geeignet, eine Form aufzuweisen, die an spezielle Körperstrukturen angepasst ist.

### Elektrotechnische Kern

Im Rahmen dieser Beschreibung wird ein im Betrieb ein Plasma erzeugendes Mehrschichtsystem aus aneinander angrenzenden Elektrodenstrukturen und Isolationsschichten als elektrotechnischer Kern bezeichnet. Die Anordnung der Strukturen und Schichten in einem solchen elektrotechnischen Kern kann hierbei in verschiedenen elektrotechnischen Kernen unterschiedlich sein. In verschiedenen Varianten eines elektrotechnischen Kerns können die einzelnen Strukturen und Schichten mit einer geschlossenen Fläche oder mit einer speziellen Geometrie, ausgebildet sein.

Im Folgenden werden verschiedene bevorzugte Ausführungsformen eines elektrotechnischen Kerns beschrieben, die als elektrotechnischer Kern eines hier beschriebenen und/oder eines anderen Plasmaapplikators geeignet sind. Ein elektrotechnischer Kern weist typischerweise eine Folge von Isolationsschichten und Elektrodenstrukturen auf, die in einer Stapelfolge aus aneinander angrenzenden Schichten angeordnet sind und somit ein Mehrschichtsystem bilden.

In einer Ausführungsform ist ein elektrotechnischer Kern flächig ausgebildet und umfasst wenigstens eine Elektrodenstruktur, die bevorzugt im Betrieb durch ein Spannungssignal getrieben wird, und wenigstens eine Isolationsschicht, die sich im Anwendungsfall zwischen der getriebenen Elektrodenstruktur und einer zu behandelnden Oberfläche befindet. Die getriebene Elektrodenstruktur befindet sich im Anwendungsfall also auf der der zu behandelnden Oberfläche abgewandten Seite des elektrotechnischen Kerns.

In der Regel hat ein elektrotechnischer Kern eine rechteckige Form. In verschiedenen Varianten hat ein elektrotechnischer Kern eine kreisförmige, eine ovale Form, eine hexagonale Form oder eine andere Polygone Form. In verschiedenen weiteren Varianten hat ein elektrotechnischer Kern eine dreidimensionale Form, beispielsweise die Form eines Zylinders oder eines Quaders. In weiteren Varianten hat der elektrotechnische Kern eine Form, die speziell an einen bestimmten Körperteil (z.B. einen Hacken, einen Finger, eine Brust) angepasst ist.

In einer Ausführungsform weist ein elektrotechnischer Kern wenigstens eine erste und eine zweite Elektrodenstruktur auf, wobei die erste und die zweite Elektrodenstruktur ein jeweils unterschiedliches und vom Massepotential abweichendes Potential haben (*floating electrodes).*

In einer Ausführungsform umfasst ein elektrotechnischer Kern von der der zu behandelnden Oberfläche zugewandten Seite beginnend eine erste Isolationsschicht, welche vorzugsweise biokompatibel und somit besonders für eine Behandlung einer menschlichen oder tierischen Oberfläche geeignet ist. Auf der Isolationsschicht ist eine erste Elektrodenstruktur angeordnet, die typischerweise auf Massepotential liegt. Auf die erste Elektrodenstruktur folgt eine zweite Isolationsschicht, welche eine Dielektrikumsschicht ist, und zur galvanischen Entkopplung der ersten Elektrodenstruktur und einer auf der zweiten Isolationsschicht angeordneten zweiten Elektrodenstruktur dient. Die zweite Elektrodenstruktur wird im Anwendungsfall typischerweise mit einem Spannungssignal zum Zünden eines Plasmas getrieben.

In einer Ausführungsform umfasst ein elektrotechnischer Kern lediglich eine zweite Elektrodenstruktur, die im Anwendungsfall durch ein Spannungssignal getrieben wird, und eine zweite Isolationsschicht, die im Anwendungsfall zwischen der zweiten Elektrodenstruktur und der zu behandelnden Oberfläche angeordnet ist. Die Gegenelektrode wird dann im Betrieb des elektrotechnischen Kerns, insbesondere als Bestandteil eines Plasmaapplikators, durch den menschlichen oder tierischen oder technischen Körper bzw. die zu behandelnde Oberfläche selbst realisiert.

Vorteilhafterweise sind alle Schichten und/oder Strukturen eines elektrotechnischen Kerns untereinander formschlüssig und ohne Lufteinschlüsse, Fremdkörpern oder Fremdmaterialien mindestens durch Adhäsion miteinander verbunden.

Ein elektrotechnischer Kern kann auch in einem Teilbereich oder auch über die gesamte Fläche des elektrotechnischen Kerns verteilt mit Löchern bzw. Durchführungen versehen sein. Durch die Löcher bzw. Durchführungen kann ein Medientransport durch den elektrotechnischen Kern von der einer zu behandelnden Oberfläche zugewandten Seite zu der einer zu behandelnden Oberfläche abgewandten Seite des Plasmaapplikators oder in umgekehrter Richtung von der einer zu behandelnden Oberfläche abgewandten Seite zu der einer zu behandelnden Oberfläche zugewandten Seite des Plasmaapplikators ermöglicht werden.

Bei einer Ausführungsvariante, in der der elektrotechnische Kern Löcher oder Durchführungen partiell oder über die gesamte Fläche des elektrotechnischen Kerns verteilt aufweist, haben die Löcher oder Durchführungen einen Durchmesser, der zwischen 1 mm und 10 mm beträgt.

Bei einer Ausführungsvariante, in der ein elektrotechnischer Kern Löcher oder Durchführungen partiell oder über die gesamte Fläche des elektrotechnischen Kerns verteilt aufweist, sind die Löcher oder Durchführungen in denjenigen Bereichen angeordnet, in denen sich keine Elektrodenstruktur befindet. Die Elektrodenstrukturen werden dann bei einer Bohrung oder Stanzung der Löcher nicht beschädigt.

Vorzugsweise werden bei der Anordnung und dem Durchmesser von Löchern oder Durchführungen eines elektrotechnischen Kerns mit Löchern oder Durchführungen die Kriechstrecken berücksichtigt, um eine homogene Plasmaausbildung auf der einer zu behandelnden Oberfläche zugewandten Seite des elektrotechnischen Kerns zu ermöglichen.

Ein im Rahmen dieser Beschreibung beschriebener elektrotechnischer Kern kann z.B. mittels einem Dispensverfahren, im 3D-Druck oder im Sieb- bzw. Rollensiebdruck hergestellt werden. Hierbei werden in mehreren Prozessschritten eine oder auch mehrere Elektrodenstrukturen Schritt für Schritt aufgebaut. Die Elektrodenstrukturen sind durch Isolationsschichten getrennt. Es kann dann abwechselnd mit leitfähigem und elektrisch isolierendem Material dispenst bzw. gedruckt werden. Vorzugsweise wird ein im Rahmen dieser Beschreibung beschriebener elektrotechnischer Kern im Rollensiebdruck hergestellt. Bevorzugt ist wenigstens eine Isolationsschicht des elektrotechnischen Kerns von einer Folie gebildet, auf die im Rollensiebdruckverfahren weitere Schichten des elektrotechnischen Kerns, also Elektrodenstrukturen und/oder weitere von Lacken und/oder Folien gebildete Isolationsschichten, übereinander gedruckt werden. Diese von einer Folie und/oder einem Lack gebildeten Isolationsschichten des elektrotechnischen Kerns haben vorzugsweise eine Dicke, die zwischen 10 µm und 500 µm beträgt. Vorzugweise ist die Dicke einer Isolationsschicht so gewählt, dass im Anwendungsfall keine Durchschläge zwischen den auf jeweils eine Seite der Folie oder des Lackes gedruckte Elektrodenstrukturen erfolgen. Zum Bestimmen einer geeigneten Dicke können unter anderem die Durchschlagsfestigkeit der Lacke oder der Folie von der diese Isolationsschicht gebildet sind als die Größe derjenigen Spannung berücksichtigt werden, die im Anwendungsfall zwischen den auf die Folie gedruckten Elektrodenstrukturen anliegt. Eine von einer Folie gebildete Isolationsschicht ist vorzugsweise frei von Poren auch *pinholes* genannt.

Ein elektrotechnischer Kern kann in seiner lateralen Ausdehnung größer als eine Elektrodenstruktur des elektrotechnischen Kerns sein. Beispielsweise kann ein elektrotechnischer Kern eine Grundfläche haben, die 20 cm x 20 cm oder auch 30 cm x 30 cm oder noch größer beträgt, wohingegen die Fläche einer Elektrodenstruktur des elektrotechnischen Kerns lediglich 10 cm x 10 cm beträgt.

Ein elektrotechnischer Kern, der eine größere Fläche als die Elektrodenstrukturen des elektrotechnischen Kerns hat, kann für eine Plasmabehandlung in eine gewünschte Größe zugeschnitten werden. Die minimale Größe ist allerdings durch die Fläche der Elektrodenstrukturen bestimmt. Insbesondere bei einem elektrotechnischen Kern, der eine größere Fläche als die Elektrodenstrukturen des elektrotechnischen Kerns hat, können die Elektrodenstrukturen in Bezug auf die Grundfläche des elektrotechnischen Kerns innerhalb der Grundfläche beliebig angeordnet sein.

### Elektrodenstruktur

Im Folgenden werden bevorzugte Ausführungsformen einer Elektrodenstruktur beschrieben, die als Elektrodenstruktur eines hier beschriebenen und/oder eines anderen elektrotechnischen Kerns geeignet ist. Eine hier beschriebene Elektrodenstruktur ist typischerweise von einer elektrisch leitfähigen Struktur gebildet. Die elektrisch leitfähige Struktur stellt dann eine Elektrodenstruktur dar.

Eine Elektrodenstruktur, oder im Falle mehrerer Elektrodenstrukturen, die Elektrodenstrukturen eines elektrotechnischen Kerns sind bevorzugt flächig ausgebildet und bilden eine Schicht eines elektrotechnischen Kerns. Eine Elektrodenstruktur kann auf oder in einem Elektrodenträger angeordnet sind. Eine Elektrodenstruktur kann von einem leitfähigen Material, insbesondere mit einem Metall, beispielsweise in Form einer dünnen Metallschicht, -folie, -gitter und/oder von einer leitfähigen Polymerschicht gebildet sein. Ein Elektrodenträger kann beispielsweise eine unbedruckte Folie sein. Eine Elektrodenstruktur, oder Elektrodenstrukturen, können dann auf die unbedruckte Folie oder einen alternativen Elektrodenträger gedruckt sein, beispielsweise mit Silberleitlack.

In einer Ausführungsform ist beispielsweise eine 10 µm dicke Elektrodenstruktur aus Silberleitlack direkt auf jeweils eine der beiden Seiten einer Isolationsschicht, insbesondere einer Dielektrikumsschicht, gedruckt, sodass die Dielektrikumsschicht selbst der Elektrodenträger ist.

Eine Elektrodenstruktur eines elektrotechnischen Kerns kann auch von elektrisch leitfähigen Fäden, die in ein Textil eingewoben sind, gebildet sein. In diesem Fall ist der Elektrodenträger durch das Textil realisiert und die Elektrodenstruktur in dem Elektrodenträger angeordnet. Es kann auch vorteilhaft sein, wenigstens eine Elektrodenstruktur eines elektrotechnischen Kerns aus einem leitfähigen, vorzugsweise flexiblen Material zu bilden, wie zum Beispiel einem leitfähigen Kunststoff, einem mit leitfähigen Partikeln angereicherten Material, einer metallischen Folie oder Graphit. Für eine derart ausgebildete Elektrodenstruktur wird in der Regel kein zusätzlicher Elektrodenträger benötigt. Eine derart ausgebildete Elektrodenstruktur kann bspw. mittels einem Dispensverfahren hergestellt werden.

Ein elektrotechnischer Kern umfasst vorzugsweise wenigstens zwei Elektrodenstrukturen, nämlich eine erste und eine zweite Elektrodenstruktur, wobei die zweite Elektrodenstruktur im Anwendungsfall mittels einer in Bezug auf Erdpotential angelegten Spannung in Form eines Spannungssignals getrieben wird. Die erste Elektrodenstruktur ist typischerweise geerdet. Wenn ein elektrotechnischer Kern eine erste und eine zweite Elektrodenstruktur aufweist, ist die erste Elektrodenstruktur im Anwendungsfall typischerweise der zu behandelnden Oberfläche zugewandt. Der Abstand zwischen der ersten Elektrodenstruktur und der zweiten Elektrodenstruktur ist bevorzugt kleiner als 1 mm, insbesondere kleiner als 200 µm, bevorzugt kleiner als 100 µm. Vorteilhafterweise ist durch einen kleinen Abstand ein geringeres Spannungssignal zum Zünden eines Plasmas notwendig.

Eine Elektrodenstruktur eines elektrotechnischen Kerns kann als eine Schicht mit geschlossener Fläche ausgebildet sein, die sich komplett oder auch nur teilweise über die Fläche des elektrotechnischen Kerns erstreckt. Insbesondere eine zweite und eine dritte Elektrodenstruktur sind bevorzugt als solche Flächenelektroden ausgebildet, da durch diese Elektrodenstrukturen während einer Plasmabehandlung keine elektrischen Feldlinien zur Erzeugung eines Plasmas hindurchtreten soll. Eine Elektrodenstruktur und insbesondere die erste Elektrodenstruktur, die sich auf der der zu behandelnden Oberfläche zugewandten Seite eines elektrotechnischen Kerns befindet, kann auch eine spezielle Geometrie aufweisen und innerhalb einer Schicht angeordnet sein. Eine Elektrodenstruktur mit einer speziellen Geometrie kann z.B. mäanderförmig, spiralförmig, durch eine Fläche mit Löchern gebildet, quadratisch, U-förmig, E-förmig, M-förmig, L-förmig, C-förmig, X-förmig oder O-förmig sein und sich lateral innerhalb einer Schicht eines elektrotechnischen Kerns eines Plasmaapplikators ausdehnen. Eine Elektrodenstruktur mit einer speziellen Geometrie ist vorzugsweise von regelmäßig angeordneten Elektrodenabschnitten gebildet, die bevorzugt ein regelmäßiges Muster bilden.

In einer Ausführungsform eines elektrotechnischen Kerns mit mehreren Elektrodenstrukturen, haben insbesondere eine erste und eine zweite Elektrodenstruktur dieselbe spezielle Geometrie und ihre Elektrodenabschnitte sind zueinander mit einer definierten Überlappung versetzt, in unterschiedlichen Schichten eines elektrotechnischen Kerns angeordnet. Insbesondere befindet sich zwischen zwei Elektrodenstrukturen wenigstens eine Isolationsschicht, z.B. in Form einer Folie, eines Klebers oder eines Lackes. Es kann auch vorteilhaft sein, wenn die mehreren Elektrodenstrukturen eines elektrotechnischen Kerns unterschiedliche Geometrien aufweisen.

Die Überlappung der Elektrodenabschnitte kann einen deutlichen Einfluss auf die Kapazität eines elektrotechnischen Kerns und die Größe der vom elektrotechnischen Kern im Betrieb erzeugten elektrischen Feldstärke haben. Wenn die Elektrodenabschnitte einer ersten und einer zweiten Elektrodenstruktur kongruent zueinander angeordnet sind, sodass sich die Elektrodenabschnitte vollständig überlappen, ist die Feldstärke typischerweise klein und die Kapazität maximal. Typischerweise gilt, dass die Kapazität am größten ist, wenn sich die Elektrodenabschnitte vollständig überlappen, während die Kapazität umso kleiner wird, je weniger sich die Elektrodenabschnitte überlappen. Für die Zündung eines Plasmas ist eine hohe elektrische Feldstärke bei einem geringen Abstand zwischen den beiden Elektrodenstrukturen generell vorteilhaft. Je größer der Abstand, desto größer muss auch die Amplitude des Spannungssignals zum Zünden eines Plasmas sein. Jedoch ist eine vergleichsweise kleine Kapazität vorteilhaft, da ein elektrotechnischer Kern dann schneller reagiert und Verzerrungen im Spannungssignal aufgrund eines geringen kapazitiven Anteils schwach sind.

In einem elektrotechnischen Kern eines Plasmaapplikators können auch mehrere Elektrodenstrukturen innerhalb einer Schicht eines elektrotechnischen Kerns angeordnet sein. Jede der Elektrodenstrukturen stellt für sich eine individuelle Elektrodenstruktur dar, die sich in ihrer lateralen Ausdehnung lediglich über einen Teil der Fläche eines elektrotechnischen Kerns erstreckt. Bevorzugt sind die mehreren Elektrodenstrukturen in dieser Schicht gleichmäßig über die Fläche eines elektrotechnischen Kerns, d.h. über die Fläche der Wundauflage, verteilt. Die mehreren Elektrodenstrukturen innerhalb einer Schicht können alle elektrisch miteinander verbunden sein. Es ist auch denkbar, dass nur bestimmte individuelle Elektrodenstrukturen innerhalb der Schicht elektrisch miteinander verbunden, sodass Gruppen von elektrisch miteinander verbunden Elektrodenstrukturen gebildet werden. Des Weiteren kann es vorteilhaft sein, wenn Elektrodenstrukturen innerhalb einer Schicht nicht elektrisch miteinander verbunden sind und jeweils elektrisch voneinander unabhängige Behandlungsgebiete bilden. Die mehreren Elektrodenstrukturen innerhalb einer Schicht können zeitlich versetzt, d.h. kaskadiert, angesteuert werden. Eine kaskadierte Ansteuerung kann vorteilhafterweise zu einer Verringerung einer Leistungsaufnahme pro Zeitintervall und/oder zu einer Verringerung einer Behandlungszeit führen.

Die mehreren Elektrodenstrukturen innerhalb einer Schicht eines elektrotechnischen Kerns eines Plasmaapplikators können aus den vorher genannten Materialien und in den vorher genannten Formen gebildet sein, die in Bezug auf die vorher beschriebenen Elektrodenstrukturen genannt wurden.

Die hier beschriebenen Elektrodenstrukturen haben bevorzugt eine Dicke von wenigen µm bis hin zu einigen hundert µm.

Wenn eine Elektrodenstruktur nicht vollflächig, also nicht als Flächenelektrode, ausgebildet ist, sondern eine der oben beschriebenen speziellen Geometrien aufweist, sind die Dicke und die Breite der Elektrodenabschnitte der Elektrodenstruktur mit einer der speziellen Geometrien vorteilhafterweise derart gewählt, dass die Elektrodenstruktur durch die thermische Belastung im Betrieb eines Plasmaapplikators nicht zerstört wird bzw. der Plasmaapplikatorwährend des Betriebes eine Temperatur von 40°C nicht überschreitet. Es sei denn, dass eine Einmalverwendung eines elektrotechnischen Kerns sichergestellt werden soll.

Die hierbei entscheidende Materialeigenschaft ist der elektrische Widerstand bzw. die Impedanz, der material- und geometrieabhängig einen bestimmten Wert von in der Regel wenigen Ohm nicht übersteigen sollte. In Abhängigkeit vom verwendeten Material und dessen Leitwert weisen die Elektrodenabschnitte einer solchen Elektrodenstruktur vorteilhafterweise einen entsprechend gewählten Querschnitt auf. Als vorteilhaft haben sich Elektrodenabschnitte mit einer Breite von 5 mm und einer Dicke von 14 µm herausgestellt. Diese Werte können aber in verschiedenen Varianten von den genannten Werten abweichen und dennoch zu Materialeigenschaften führen, die für spezielle Anwendungen vorteilhaft sind. Für manche Anwendungen kann es z.B. vorteilhaft sein, wenn die Elektrodenabschnitte einer Elektrodenstruktur eine Breite von 1 mm und eine Dicke von 70 µm haben. Für andere Anwendungen kann es z.B. vorteilhaft sein, wenn die Elektrodenabschnitte einer Elektrodenstruktur eine Breite von 10 mm und eine Dicke von 7 µm haben.

In einer Ausführungsform ist eine Elektrodenstruktur aus langen Polymerschlangen gebildet. Das Innere einer derart hergestellten Polymerschlange umfasst bevorzugt ein leitfähiges Polymer, wie z.B. mit leitfähigen Partikeln wie Kohlenstoff oder Carbon-Nano-Tubes angereichertes Silikon. Bevorzugt wird um die Elektrodenstruktur ein biokompatibles Material (z.B. Silikon) gedruckt bzw. dispenst. Vorteilhafterweise kann eine derart hergestellte Elektrodenstruktur eine Vielzahl unterschiedlicher Formen aufweisen und ist nicht notwendiger Weise flach.

In einer Ausführungsform ist eine Elektrodenstruktur von einem Drahtgitter, einem Drahtgewebe, oder einem Drahtgeflecht gebildet. Ein solches Drahtgitter, Drahtgewebe oder Drahtgeflecht kann von einem einzelnen isolierten Draht oder von mehreren isolierten Drähten gebildet sein. Wenn die Drähte isoliert sind, ist eine gesonderte Isolationsschicht, in die die Drähte eingebettet sind, obsolet. Ein entsprechendes Drahtgitter, Drahtgewebe, oder Drahtgeflecht stellt also bereits einen vergleichsweise einfachen elektrotechnischen Kern dar. Durch ein Drahtgitter, Drahtgewebe, oder Drahtgeflecht kann also bereits eine vergleichsweise einfache Plasmaquelle realisiert werden. Wenn ein Drahtgitter, Drahtgewebe, oder Drahtgeflecht von einem einzelnen Draht gebildet ist, wird eine Gegenelektrode im Anwendungsfall durch eine zu behandelnde Oberfläche selbst realisiert. Wenn ein Drahtgitter, Drahtgewebe, oder Drahtgeflecht von mehreren Drähten gebildet ist, kann wenigstens ein Draht im Anwendungsfall durch ein Spannungssignal getrieben werden und wenigstens ein weiterer Draht geerdet sein, der dann eine Gegenelektrode darstellt.

Ein Draht eines Drahtgitters, Drahtgewebes, oder Drahtgeflechts stellt einen vergleichsweise einfachen elektrischen Leiter dar. Ein Drahtgitter, Drahtgewebe, oder Drahtgeflecht kann auch von einem einfachen elektrischen Leiter in Form wenigstens eines Flachkabels gebildet sein. Ein Flachkabel in diesem Sinne kann einen quadratischen oder rechteckigen Querschnitt aufweisen. Ein einfacher elektrischer Leiter weist vorzugsweise eine Isolationsummantelung auf.

Es ist auch denkbar, dass eine Plasmaquelle von einem einzelnen isolierten elektrischen Leiter, beispielsweiseeinem isolierten Draht gebildet ist, ohne diesen in einer vergleichsweise komplizierten Struktur wir einem Gewebe oder Gitter anzuordnen. Wenn ein elektrischer Leiter, z.B. ein isolierter Draht, über einer zu behandelnden Oberfläche angeordnet und mit Strom beaufschlagt wird, kann die zu behandelnde Oberfläche als Gegenelektrode wirken wird und zwischen der zu behandelnden Oberfläche und dem isolierten Draht ein Plasma zünden. In diesem Fall wird also ein isolierter Draht über oder auf eine zu behandelnde Oberfläche gelegt, z.B. als Schlaufe oder in eindimensional.

### Isolationsschicht

Im Folgenden werden bevorzugte Ausführungsformen einer Isolationsschicht beschrieben, die im Rahmen dieser Beschreibung auch als Dielektrikumsschicht bezeichnet wird und als Bestandteil eines hier beschriebenen und/oder eines anderen elektrotechnischen Kerns geeignet ist. Eine hier beschriebene Isolationsschicht kann von einer elektrisch isolierenden Struktur gebildet werden. Die elektrisch isolierende Struktur stellt dann eine Isolationsschicht dar.

Um einen Stromfluss zwischen einer im Anwendungsfall getriebenen Elektrodenstruktur und einer möglichen weiteren Elektrodenstruktur, die auf Massepotential liegt, zu verhindern, befindet sich in einem elektrotechnischen Kern vorzugsweise wenigstens eine Isolationsschicht zwischen den jeweiligen Elektrodenstrukturen oder der wenigstens einen, im Betrieb mit einem Spannungssignal getriebenen Elektrodenstruktur und einer zu behandelnden menschlichen oder tierischen oder technischen Oberfläche.

Eine Isolationsschicht kann z.B. aus Kunststoff oder Keramik oder beidem bestehen. Bevorzugt hat eine Isolationsschicht eine Dicke zwischen wenigen µm bis hin zu wenigen 100 µm. Wie auch bei einer Elektrodenstruktur, hängt die Wahl der Dicke einer Isolationsschicht von den elektrischen Materialkonstanten, insbesondere der Dielektrizitätskonstanten und der Durchschlagsfestigkeit, ab.

Es kann vorteilhaft sein, wenn die Dicke einer Isolationsschicht vergleichsweise gering gewählt ist und zum Beispiel im Bereich von 50 µm liegt. Eine geringe Dicke führt in der Regel dazu, dass eine geringere Amplitude eines Spannungssignals zum Zünden eines Plasmas benötigt wird. Außerdem kann eine geringe Dicke vorteilhaft sein, um eine hohe Flexibilität des Plasmaapplikators zu gewährleisten.

Je nach Anwendung, z.B. bei einem Plasmaapplikator mit starrer Form, kann eine Dicke von 200 µm und größer vorteilhaft sein. Insbesondere bei der Verwendung von unflexible Materialien, wie z.B. Keramiken die nicht beliebig dünn hergestellt werden können, kann eine Dicke von mindestens 200 µm vorteilhaft sein.

Bevorzugt weist eine Isolationsschicht eine Dicke auf, die weniger als 1 mm, insbesondere weniger als 200 µm, bevorzugt weniger als 100 µm beträgt.

Vorzugsweise ist eine Isolationsschicht als vollflächige Schicht ausgebildet.

Bevorzugt ist eine Isolationsschicht porenfrei, d.h. es sind keine oder nur sehr wenige Löcher oder Hohlräume vorhanden, sodass sich keine Entladungskanäle durch die Isolationsschicht ausbilden. Bevorzugt weist eine Isolationsschicht eine Spannungsfestigkeit von wenigstens 5 kV pro mm Dicke auf. Des Weiteren ist bevorzugt, dass die laterale Ausdehnung einer Isolationsschicht der Dimension einer Elektrodenstruktur in einem elektrotechnischen Kern zuzüglich eines darüber hinausragenden Randes entspricht, wobei der Rand vorzugsweise so dimensioniert ist, dass er wenigstens die Länge der Kriechstrecken abdeckt.

In einer Ausführungsform ist die lateralen Ausdehnung einer Isolationsschicht derart gewählt, dass es zwischen einer im Anwendungsfall getriebenen, zweiten Elektrodenstruktur und einer auf Erdpotential liegenden ersten und/oder dritten Elektrodenstruktur oder einer zu behandelnden Oberfläche zu keiner Bogenentladung kommt.

Vorteilhafterweise können durch ein wenigstens teilweises Umschließen eines elektrotechnischen Kerns mit einer Umschließung Kriechstrecken unterschritten werden. In einer Ausführungsform in der ein elektrotechnischer Kern wenigstens teilweise von einer Umschlie-βung umschlossen ist, kann eine laterale Ausdehnung einer Isolationsschicht in Abhängigkeit von der verwendeten Umschließung derart bemessen sein, dass ein über eine Elektrodenstruktur hinausragender Rand der Isolationsschicht kleiner bemessen ist als eine Kriechstrecke, die die zum Zünden eines Plasmas notwendige Amplitude eines Spannungssignals an sich vorgeben würde.

Eine erste Isolationsschicht, welche im Anwendungsfall direkt einer zu behandelnden Oberfläche zugewandt ist, weist bevorzugt ein biokompatibles Material wie z.B. Lack, Silikon, PU oder Beschichtungen auf. Beschichtungen können z.B. mittels nasschemischer Prozesse, plasmaunterstützte chemische Gasphasenabscheidung (PACVD), chemische Gasphasenabscheidung (CVD), Eloxal-Verfahren oder mittels Galvanotechnik aufgebracht werden.

### Steckvorrichtung

Im Folgenden werden bevorzugte Ausführungsformen einer Steckvorrichtung beschrieben. Eine hier beschriebene Steckvorrichtung kann Bestandteil eines hier beschriebenen und/oder eines anderen Plasmaapplikators und dient dazu, insbesondere einen elektrotechnischen Kern eines Plasmaapplikators durch Verbinden mit einer komplementär ausgebildeten Einschubvorrichtung mit einer Energieversorgungseinheit zu verbinden. Da eine Steckvorrichtung Bestandteil eines Plasmaapplikators ist, stellt sie eine Applikator-Steckkontaktvorrichtung dar.

Eine Steckvorrichtung ist bevorzugt fest mit einem elektrotechnischen Kern, und insbesondere mit einer im Anwendungsfall getriebenen zweiten Elektrodenstruktur - und falls vorhanden - mit den geerdeten Elektrodenstrukturen eines elektrotechnischen Kerns elektrisch leitfähig verbunden. Bevorzugt ist eine Steckvorrichtung zum Übertragen eines Spannungssignals im kV-Bereich, insbesondere im Bereich von einigen 100 Volt bis 10 kV, an eine zweite Elektrodenstruktur geeignet.

Eine Steckvorrichtung weist vorzugsweise wenigstens eine elektrische Leiterbahn, die eine elektrisch leitfähige Leiterstruktur ist, auf, die zu wenigstens einer Elektrodenstruktur führt. Eine Leiterbahn ist also in elektrisch leitfähigem Kontakt mit einer Elektrodenstruktur vorzugsweise an einer Längsseite der entsprechenden Elektrodenstruktur angeordnet und erstreckt sich bevorzugt senkrecht in Bezug auf die Längsseite der entsprechenden Elektrodenstruktur in einer gemeinsamen Ebene mit der Elektrodenstruktur. Wenn ein elektrotechnischer Kern mehrere Elektrodenstrukturen umfasst, ist in der Regel an jeder der Elektrodenstrukturen wenigstens eine Leiterbahn angeordnet. Um die Leiterbahnen elektrisch voneinander zu isolieren, ist zwischen den Leiterbahnen jeweils wenigstens eine isolierende Lasche angeordnet. Vorzugsweise ist die fest mit einer Isolationsschicht verbunden und von demselben elektrisch isolierenden Material wie die Isolationsschicht gebildet. Insbesondere kann die Lasche integraler Bestandteil der jeweiligen Isolationsschicht sein.

Eine Steckvorrichtung kann z.B. mittels Laminieren, Kaschieren, Kleben, Löten oder einem alternativen materialverbindenden Verfahren fest mit einem elektrotechnischen Kern verbunden werden. Eine Leiterbahn einer Steckvorrichtung hat bevorzugt eine vergleichbare Dicke und besteht bevorzugt aus dem gleichen Material oder aus den gleichen Materialien wie eine Elektrodenstruktur eines entsprechenden elektrotechnischen Kerns und hat bevorzugt einen Leitwert in einer vergleichbaren Größenordnung wie diese Elektrodenstruktur.

Vorzugsweise werden ein elektrotechnischer Kern und eine Steckvorrichtung in einem gemeinsamen Fertigungsprozess hergestellt. In einem Fertigungsschritt werden dann beispielsweise eine Leiterbahn und eine mit der Leiterbahn elektrisch verbundene Elektrodenstruktur gleichzeitig und direkt als eine Elektrodenstruktur mit Leiterbahn mithergestellt. In einem weiteren Fertigungsschritt werden dann beispielsweise eine Isolationsschicht und eine Lasche gleichzeitig und direkt als eine Isolationsschicht mit integraler Lasche hergestellt und darauf eine Elektrodenstruktur mit Leiterbahn gemeinsam aufgebracht. In einem solchen Fertigungsprozess wird also ein elektrotechnischer Kern mit Steckvorrichtung als ein gemeinsames Produkt hergestellt. Eine Elektrodenstruktur mit Leiterbahn besteht dann aus denselben Materialien und hat eine homogene Dicke. Genauso besteht dann eine Isolationsschicht mit angeformter Lasche aus einem Material und hat überall dieselbe Dicke. Vorteilhafterweise müssen dann also ein elektrotechnischer Kern und eine Steckvorrichtung nicht in getrennten Fertigungsprozessen hergestellt und nachträglich miteinander verbunden werden.

Ein solcher elektrotechnischer Kern mit Steckvorrichtung weicht also dadurch von den vorher genannten Grundformen eines elektrotechnischen Kerns ohne Steckvorrichtung ab, dass die Grundform durch eine z.B. laschenförmige Steckvorrichtung ergänzt wird. Bevorzugt werden dabei die Elektrodenstrukturen und Isolationsschichten des elektrotechnischen Kerns auf diese Lasche erweitert.

An dem Übergang von Steckvorrichtung zu elektrotechnischem Kern oder an derjenigen Stelle, an der eine Steckvorrichtung mit einem elektrotechnischen Kern verbunden ist, kann zwischen elektrotechnischem Kern und Steckvorrichtung eine Perforation vorgesehen sein. Die Funktion der Perforation ist die Reduktion der Festigkeit zwischen Steckvorrichtung und elektrotechnischem Kern. Die Perforation stellt eine Sollbruchstelle dar. An dieser Perforation kann die Steckvorrichtung nach einer Plasmabehandlung von dem elektrotechnischen Kern abgerissen bzw. entfernt werden. Dadurch kann ein Plasmaapplikator weiterhin, für einen längeren Zeitraum von Tagen bis hin zu Wochen, unabhängig von einer Energieversorgungseinheit auf einer zu behandelnden Oberfläche verbleiben, da die nicht mehr benötigte Steckvorrichtung entfernt werden kann.

Eine Steckvorrichtung hat bevorzugt die Form einer Chipkarte, d.h. sie hat eine Länge von ungefähr 5 cm bis 16 cm, eine Breite von 1 cm bis 3 cm und eine Höhe zwischen ungefähr 0.2 mm und 1 mm. Die Dimensionen einer Steckvorrichtung können auch von den genannten Werten abweichen und sind insbesondere abhängig von der Länge der Kriechstrecken in Abhängigkeit von der Amplitude eines Spannungssignals zum Zünden eines Plasmas bemessen. Vorzugsweise ist die laterale Ausdehnung einer Steckvorrichtung derart gewählt, dass es zwischen der im Anwendungsfall getriebenen zweiten Elektrodenstruktur und einerweiteren auf Erdpotenzial liegenden Elektrodenstruktur, beispielsweise einer ersten oder dritten Elektrodenstruktur, oder einer zu behandelnden Oberfläche zu keiner Bogenentladung kommt.

In einer bevorzugten Ausgestaltung ist eine Steckvorrichtung oder eine Einschubvorrichtung als Steckvorrichtung mit Versteifung mit einer Höhe zwischen 0.2 mm und 1,5 cm, mit einer Länge zwischen 5 cm und 20 cm und mit einer Breite zwischen 1 cm und 3 cm ausgebildet. Eine entsprechende Einschubvorrichtung ist komplementär zur Aufnahme des Steckers ausgebildet. Durch eine chipkartenähnliche Form der Steckvorrichtung, d.h. eine geringe Höhe und eine verhältnismäßig große Länge, können insbesondere Kriechstrecken derart eingehalten werden, dass keine Teilentladungen innerhalb der zusammengesteckten Steckvorrichtung und der Einschubvorrichtung entstehen. Die angegebenen Maße für die Länge, die Breite und die Höhe können vorteilhafterweise auch unabhängig voneinander so realisiert werden, dass die Kriechstrecken weiterhin eingehalten werden. Weiterhin ist bevorzugt, dass die Einschubvorrichtung eine Isolationsummantelung zur Reduktion der ausgestrahlten elektromagnetischen Wellen unter Berücksichtigung der Kriechstrecke aufweist. Mit den angegebenen Maßen können insbesondere Kriechstrecken zwischen aufgebrachten Leiterbahnen von mehreren Zentimetern realisiert werden. Die hierbei entscheidende Größe ist die angelegte Spannung zur Erzeugung des Plasmas. Je höher die vorgesehene, anzulegende Spannung zum Erzeugen eines Plasmas ist, desto größer müssen typischerweise auch die Kriechstrecken sein. Weiterhin entscheidend sind die Eigenschaften, insbesondere die dielektrischen Eigenschaften, des verwendeten Materials sowie der Verschmutzungsgrad des verwendeten Materials. Auch bei einer verschmutzten Oberfläche des verwendeten Materials müssen typischerweise Kriechstrecken vergleichsweise groß ausgelegt werden.

Eine Steckvorrichtung mit Versteifung in Chipkartenform hat vorzugsweise eine feste Verbindung mit einem elektrotechnischen Kern, die z. B. durch Laminieren, Kleben, Löten oder als direkte Verlängerung des elektrotechnischen Kerns in Form einer Lasche realisiert sein kann. Bevorzugt ist die Steckvorrichtung teilweise mit dem Material einer Umschließung eins Plasmaapplikators umschlossen, indem er z. B. mit Silikon umspritzt wird. Die Steckvorrichtung kann weiterhin bedruckte oder bedampfte oder geätzte Leiterbahnen aufweisen. Eine Steckvorrichtung kann weiterhin eine Versteifung aufweisen, die vorzugsweise oberhalb und/oder unterhalb der Lasche aufgebracht ist und die Steckvorrichtung mechanisch verstärkt. Die Leiterbahnen können auch als Spulendraht mit isolierender Ummantelung des Drahtes ausgebildet sein. Optional kann eine Steckvorrichtung auch eine Datenleitung z. B. als Leiterbahn oder als Flachkabel aufweisen. Es kann z.B. vorteilhaft sein, einen Speicher in einen Plasmaapplikator oder in eine entsprechende Kupplung oder in eine Energieversorgungseinheit einzubauen, um Daten über die Verwendung des Plasmaapplikators zu sammeln.

Mit einem entsprechenden Datenkabel mit Kupplung kann auch ein RFID-Transponder oder ein nichtflüchtiger elektronischer Speicherbaustein, der sich z.B. in einer Steckvorrichtung befindet, von einem in einer komplementär ausgebildeten Einschubvorrichtung integrierten Lesegerät ausgelesen werden, um einer Einmalverwendung zu gewährleisten.

Vorteilhafterweise können in einem entsprechenden Speicherbaustein auch Daten zu einem Plasmaapplikator selbst hinterlegt sein. Wenn der Plasmaapplikator mit einem Speicherbaustein über ein entsprechendes Datenkabel mit einer Energieversorgungseinheit verbunden ist, kann die Energieversorgungseinheit die gespeicherten Daten auslesen und automatisch eine bestimmte Spannung, ein bestimmtes Pulsmuster, eine bestimmte Behandlungszeit oder weitere Behandlungsparameter zum Betreiben des bestimmten Plasmaapplikators bereitstellen. Bevorzugt sind die verwendeten Parameter gezielt auf unterschiedliche Plasmaapplikator-Formen und -Größen, oder einen bestimmten Aufbau des elektrotechnischen Kerns eines Plasmaapplikators in der Energieversorgungseinheit gespeichert und werden entsprechend des angeschlossenen Plasmaapplikators von der Energieversorgungseinheit abgerufen und verwendet.

Vorzugsweise ist eine Steckvorrichtung an einer Längsseite eines elektrotechnischen Kerns angebracht und mit wenigstens einer Elektrodenstruktur des elektrotechnischen Kerns elektrisch verbunden. Es ist auch denkbar, dass eine Steckvorrichtung an der Oberseite eines elektrotechnischen Kerns, also derjenigen Seite eines elektrotechnischen Kerns, die von einer zu behandelnden Seite abgewandt ist, in Form eines Einschubschlitzes angebracht und mit wenigstens einer Elektrodenstruktur verbunden ist.

Bevorzugt ist die Steckvorrichtung als Steckvorrichtung mit Versteifung mit einer Höhe zwischen 0.5 mm und 1,5 cm, eine Länge zwischen 5 cm und 20 cm und eine Breite zwischen 1 cm und 3 cm ausgebildet und eine entsprechende zu der Steckvorrichtung komplementäre Einschubvorrichtung als Aufnahmebuchse zur Aufnahme der Steckvorrichtung ausgebildet.

Um beispielsweise an eine zweite Elektrodenstruktur mittels einer Energieversorgungseinheit ein Spannungssignal zu übertragen, kann z.B. eine zu einer Steckvorrichtung komplementäre Einschubvorrichtung mit der Steckvorrichtung verbunden werden. Die Einschubvorrichtung kann mit einem Kabel verbunden sein, das an eine vorwiegend stationäre Ener-gieversorgungseinheit angeschlossen ist. Eine entsprechende Energieversorgungseinheit kann z.B. ein Hochspannungsgenerator mit einer Steuereinheit sein.

### Einmalverwendung

Im Folgenden werden verschiedene Mittel und Merkmale beschrieben, mit denen eine Einmalverwendung eines elektrotechnischen Kerns und insbesondere eines Plasmaapplikators sichergestellt werden kann. Vorzugsweise sind die Mittel und Merkmale, die eine Einmalverwendung sicherstellen als Bestandteil eines Plasmaapplikators insbesondere als Bestandteil einer hier beschriebenen und/oder einer anderen Steckvorrichtung oder als Bestandteil eines hier beschriebenen und/oder eines anderen elektrotechnischen Kerns eines Plasmaapplikators realisiert.

In einer Ausführungsform eines Plasmaapplikators weist ein elektrotechnischer Kern wenigstens ein Merkmal auf, welches sich infolge eines erstmaligen Gebrauchs derart ändert, dass zwischen der im Anwendungsfall getriebenen Elektrodenstruktur und der geerdeten Elektrodenstruktur kein ausreichend starkes elektrisches Feld zur Zündung eines Plasmas mehr ausgebildet werden kann. Dadurch wird vorteilhafterweise eine einmalige Verwendung eines Plasmaapplikators sichergestellt. Ein Merkmal eines elektrotechnischen Kerns, welches eine Einmalverwendung des Plasmaapplikators sicherstellt, kann z.B. eine selbstzerstörende Vorrichtung wie eine elektrische Sicherung sein. Eine solche elektrische Sicherung kann zum Beispiel durch eine Verjüngung eines Elektrodenabschnitts einer Elektrodenstruktur im elektrotechnischen Kern sein, die am Ende der Behandlung durch einen dann bereitgestellten hohen Strompuls zerstört wird, da sie einen höheren Widerstand als der Rest der Elektrodenstruktur aufweist und sich daher schneller erwärmt. Vorzugsweise befindet sich eine solche Verjüngung in der im Anwendungsfall getriebenen Elektrodenstruktur.

Die im Folgenden in Bezug auf eine Steckvorrichtung beschriebenen Mittel und Merkmale, welche eine Einmalverwendung eines Plasmaapplikators sicherstellen, können auch als Merkmale einer Elektrodenstruktur, insbesondere als Bestandteil eines Elektrodenabschnitts einer Elektrodenstruktur eines elektrotechnischen Kerns realisiert sein. Vorzugsweise weist eine im Betrieb durch ein Spannungssignal getriebene Elektrodenstruktur wenigstens ein wie in Bezug auf eine Steckvorrichtung beschriebenes Merkmal oder Mittel auf, um eine Einmalverwendung eines elektrotechnischen Kerns insbesondere als Bestandteil eines Plasmaapplikators sicherzustellen.

In einer Ausführungsform eines Plasmaapplikators weist eine Steckvorrichtung wenigstens ein Merkmal auf, welches sich infolge eines erstmaligen Gebrauchs derart ändert, dass eine Steckvorrichtung kein zum Zünden eines Plasmas ausreichendes Spannungssignal mehr an eine Elektrodenstruktur eines elektrotechnischen Kerns übertragen kann bzw., dass der elektrotechnische Kern kein ausreichend starkes elektrisches Feld zum Zünden eines Plasmas mehr ausbilden kann. Dadurch wird vorteilhafterweise eine einmalige Verwendung eines Plasmaapplikators sichergestellt. Ein Merkmal einer Steckvorrichtung, welches eine Einmalverwendung eines Plasmaapplikators sicherstellt, kann z.B. eine selbstzerstörende Vorrichtung wie eine elektrische Sicherung sein. Eine solche elektrische Sicherung kann zum Beispiel durch eine Verjüngung einer Leiterbahn sein, die am Ende der Behandlung durch einen dann bereitgestellten hohen Strompuls zerstört wird, da sie einen höheren Widerstand als der Rest der Leiterbahn aufweist und sich daher schneller erwärmt.

In einer Ausführungsform weist ein Plasmaapplikator zum Erzeugen eines kalten Plasmas für die Behandlung von menschlichen oder tierischen oder technischen Oberflächen einen flächigen elektrotechnischen Kern mit wenigstens einer Elektrodenstruktur und einer Isolationsschicht auf. Der Plasmaapplikator weist weiterhin eine Steckvorrichtung auf, die zum Übertragen eines Spannungssignals an die wenigstens eine Elektrodenstruktur mit wenigstens dieser Elektrodenstruktur elektrisch leitfähig verbunden ist. Die Steckvorrichtung ist vorzugsweise dazu ausgebildet, eine einmalige Verwendung des Plasmaapplikators dadurch sicherzustellen, dass die Steckvorrichtung wenigstens eine mechanische und/oder elektrische Komponente aufweist, die derart gestaltet ist, dass sie infolge eines erstmaligen Gebrauchs des Plasmaapplikators ihre technischen Eigenschaften derart ändert, dass die Steckvorrichtung nach dem erstmaligen Gebrauch kein zum Zünden eines Plasmas ausreichendes Spannungssignal mehr an die wenigstens eine Elektrodenstruktur übertragen kann.

Das Sicherstellen der einmaligen Verwendung kann also beispielsweise dadurch realisiert sein, dass infolge des erstmaligen Gebrauchs eine elektrische Komponente aufgrund des Stromflusses zerstört wird oder dass eine mechanische oder elektrische Komponente beim mechanischen Trennen der Steckvorrichtung von einer komplementären Einschubvorrichtung zerstört wird.

Dadurch, dass die mechanische und/oder elektrische Komponente infolge eines erstmaligen Gebrauchs des Plasmaapplikators ihre Struktur derart ändert, dass die Steckvorrichtung nach erstmaligem Gebrauch kein zum Zünden eines Plasmas ausreichendes Spannungssignal mehr an die wenigstens eine Elektrodenstruktur übertragen kann, wird sichergestellt, dass der Plasmaapplikator nur einmal verwendet werden kann. Vorteilhafterweise kann auf diese Weise eine Mehrfachverwendung eines bereits benutzten Plasmaapplikators, der als Folge des ersten Gebrauchs bestimmte Hygieneanforderungen nicht mehr erfüllt, verhindert werden. Entsprechend wird sichergestellt, dass eine Wunde ausschließlich mit einem unbenutzten Plasmaapplikator behandelt wird.

Der Gebrauch eines Plasmaapplikators bezieht sich auf die Durchführung einer Plasmabehandlung. Das heißt, dass zunächst mittels einer Energieversorgungseinheit ein Spannungssignal bereitgestellt wird, das an eine Elektrodenstruktur zum Zünden eines Plasmas übertragen wird.

Ein entsprechendes Merkmal der Einmalverwendung kann auch durch chemische Reaktion mit Luftsauerstoff bzw. -stickstoff oder eine elektrochemische Reaktion, die durch ein angelegtes Spannungssignal während einer Plasmabehandlung induziert wird, realisiert sein. Zum Beispiel kann durch ein angelegtes Spannungssignal eine elektrochemische Re-aktion in den z.B. aus Silberleitlack bestehenden Leiterbahnen einer Steckvorrichtung ausgelöst werden, die eine Oxidation einer Leiterbahn, insbesondere an freiliegenden Kontaktflächen, beschleunigt und somit den Widerstand an dieser Stelle erhöht. Durch einen erhöhten oder verringerten Widerstand wird ein Spannungssignal zum Betreiben eines Plasmaapplikators deutlich verändert. Diese Änderung kann in einer Energieversorgungseinheit detektiert und somit eine Freigabe des Spannungssignals unterbunden werden. Generell ist es also denkbar, dass z.B. eine Oxidation oder Nitrierung der Leiterbahn an Luft ihre Leitfähigkeit reduziert und dadurch ein Spannungssignal derart z.B. in der Amplitude, in der Frequenz und/oder im Signalverlauf (z.B. dadurch, dass keine reine Sinusschwingung mehr vorliegt) verändert, dass es als Fehlsignal durch eine Energieversorgungseinheit detektiert werden kann und zu einem automatischen Beenden der Energieabgabe durch die Energieversorgungseinheit führt. Eine weitere Möglichkeit besteht darin, eine durch ein Spannungssignal induzierte elektrochemische Reaktion zum Ändern der Leiterbahn der Steckvorrichtung auszunutzen, die dazu führt, dass das Spannungssignal derart z.B. in der Amplitude, in der Frequenz und/oder im Signalverlauf (z.B. dadurch, dass keine reine Sinusschwingung mehr vorliegt) verändert wird, dass es als Fehlsignal durch eine Energieversorgungseinheit detektiert werden kann und zu einem automatischen Beenden der Energieabgabe durch die Energieversorgungseinheit führt.

Eine Steckvorrichtung ist bevorzugt so ausgebildet, dass sie mit einer zu der Steckvorrichtung komplementär ausgebildeten Einschubvorrichtung elektrisch leitfähig und mechanisch verbunden werden kann. Die mechanische Komponente der Steckvorrichtung kann derart ausgebildet sein, dass sie beim mechanischen Trennen der Steckvorrichtung von der Einschubvorrichtung ihre Struktur derart ändert, dass nach dem Trennen kein elektrisch leitfähiges Verbinden der Steckvorrichtung mit der Einschubvorrichtung zum Übertragen einer Hochspannung möglich ist.

Ein derartiges mechanisches Ändern kann ein Abbrechen von Klemmen eines Klemmkontakts oder von Verriegelungselementen und/oder ein Zerkratzen und/oder ein Zerschneiden der Leiterstruktur der Steckvorrichtung umfassen.

Eine Steckvorrichtung kann wenigstens ein Verriegelungselement aufweisen, das so ausgebildet und angeordnet ist, dass es beim Verbinden der Steckvorrichtung mit der Einschubvorrichtung mit dieser verriegelt und beim Trennen der Steckvorrichtung von einer Einschubvorrichtung irreversibel unbrauchbar wird.

Durch das Verbinden einer Einschubvorrichtung mit der Steckvorrichtung wird bevorzugt eine mechanisch stabile Verbindung hergestellt, die nur durch eine aktive Kraftaufwendung einer Person wieder getrennt werden kann. Die dafür benötigte Kraft liegt typischerweise zwischen 5 und 50N, bevorzugt zwischen 10 und 30N. Damit eine mechanische Komponente der Steckvorrichtung infolge des erstmaligen Gebrauchs des Plasmaapplikators ihre Struktur derart ändert, dass der elektrotechnische Kern kein ausreichend starkes elektrisches Feld zum Zünden eines Plasmas mehr ausbilden kann, kann z.B. vorgesehen sein, dass Verriegelungselemente der Steckvorrichtung abbrechen oder zerstört werden. Entsprechend kann dann keine stabile mechanische Verbindung zwischen der Steckvorrichtung und der Einschubvorrichtung mehr hergestellt werden. Der mit der Steckvorrichtung verbundene elektrotechnische Kern ist somit nach dem Trennen, also infolge des erstmaligen Gebrauchs, nicht mehr in der Lage, ein ausreichend starkes elektrisches Feld zur Zündung eines Plasmas auszubilden.

In einer Ausführungsform eines Plasmaapplikators weisen eine Steckvorrichtung oder ein elektrotechnischer Kern eine elektrische Komponente auf, die ihre Struktur infolge eines erstmaligen Gebrauchs eines Plasmaapplikators derart ändert, dass die Steckvorrichtung bzw. der elektrotechnische Kern nach erstmaligem Gebrauch kein zum Zünden eines Plasmas ausreichendes elektrisches Feld mehr erzeugen kann. Zum Beispiel kann die Steckvorrichtung eine Leiterbahn aufweisen, die zum Übertragen eines Spannungssignals an eine Elektrodenstruktur vorgesehen ist, und die am Ende eines erstmaligen Gebrauchs eines Plasmaapplikators durch einen hohen Strompuls zerstört wird. Danach kann die Steckvorrichtung kein zum Zünden eines Plasmas ausreichendes Spannungssignal mehr an eine Elektrodenstruktur übertragen.

In einer Ausführungsform eines Plasmaapplikators weist eine zweite Elektrodenstruktur eines elektrotechnischen Kerns einen Bereich auf, der am Ende eines erstmaligen Gebrauchs eines Plasmaapplikators durch einen hohen Strompuls zerstört wird. Danach kann der elektrotechnische Kern kein ausreichend starkes elektrisches Feld zum Zünden eines Plasmas mehr ausbilden.

In einer Ausführungsform weist ein Plasmaapplikator eine Steckvorrichtung auf, die mit einer zu der Steckvorrichtung komplementär ausgebildeten Einschubvorrichtung elektrisch leitfähig und mechanisch verbunden werden kann, wobei eine mechanische Komponente der Steckvorrichtung beim mechanischen Trennen der Steckvorrichtung von der Einschubvorrichtung ihre technischen Eigenschaften derart ändert, dass nach dem Trennen kein elektrisch leitfähiges Zusammenführen der Steckvorrichtung mit der Einschubvorrichtung zum Übertragen eines zum Zünden eines Plasmas ausreihenden Spannungssignals mehr möglich ist. Ein Ändern der technischen Eigenschaften der mechanischen Komponente umfasst vorzugsweise ein Abbrechen von Klemmen eines Klemmkontakts, ein Zerkratzen oder ein Zerschneiden.

Vorzugsweise sind eine Steckvorrichtung oder ein elektrotechnischer Kern eines Plasmaapplikators derart gestaltet, dass sie infolge eines Stromflusses am Ende eines erstmaligen Gebrauchs derart verändert sind, dass die Steckvorrichtung nach dem erstmaligen Gebrauch kein zum Zünden eines Plasmas ausreichendes Spannungssignal mehr an eine Elektrodenstruktur des elektrotechnischen Kerns übertragen kann.

Eine Energieversorgungseinheit kann ausgebildet sein, zum Ende einer Plasmabehandlung einen hohen Stromimpuls bereitzustellen, der Teile einer Leiterbahn in der Steckvorrichtung verändert oder zerstört, sodass bei erneutem Anschließen einer Energieversorgung keine Übertragung eines zum Zünden eines Plasmas ausreichenden Spannungssignals an die wenigstens eine Elektrodenstruktur im elektrotechnischen Kern mehr möglich ist. Bevorzugt sendet die mit dem Plasmaapplikator zusammengeführte Energieversorgungseinheit am Ende der Plasmabehandlung automatisch für bevorzugt deutlich unter einer Sekunde, bevorzugt im Millisekunden- oder sogar Mikrosekunden-Bereich, einen überhöhten Strompuls aus. Um sicherzustellen, dass entsprechende Teile einer Leiterbahn in der Steckvorrichtung zerstört werden, ist die Stromstärke des bereitgestellten Strompulses vorzugsweise derart gewählt, dass der Schmelzpunkt der Leiterbahn der Steckvorrichtung deutlich überschritten wird und die Leiterbahn zerstört wird. Vorteilhafterweise wird so eine Einmalverwendung eines Plasmaapplikators sichergestellt.

Vorzugsweise weist eine Steckvorrichtung eine Leiterbahn auf, die von einer Kontaktfläche in der Steckvorrichtung zu wenigstens einer Elektrodenstruktur führt, wobei diese Leiterbahn wenigstens an einer Stelle durch einen Strom mit einer Stromstärke zerstört werden kann, die größer als eine im Betrieb zum Erzeugen des Plasmas auftretenden Stromstärke ist. Die wenigstens eine Stelle weist bevorzugt einen höheren elektrischen Widerstand als der Rest der Leiterbahn auf und/oder die wenigstens eine Stelle weist eine geringere thermische Festigkeit als der Rest der Leiterbahn auf und/oder die wenigstens eine Stelle weist eine Verjüngung mit einem kleineren Leiterbahnquerschnitt als der Rest der Leiterbahn auf. Eine entsprechende Leiterbahn besteht vorzugsweise z. B. aus Silberleitlack, Metall, Metallfolie, einem Polymer angereichert mit leitfähigen Partikeln oder einem leitfähigen Polymer.

Insbesondere weist die wenigstens eine Stelle einen höheren elektrischen Widerstand als der Rest der Leiterbahn auf. Die wenigstens eine Stelle kann alternativ oder zusätzlich auch eine geringere thermische Festigkeit als der Rest der Leiterbahn aufweisen. Wiederum zusätzlich oder alternativ kann die wenigstens eine Stelle auch eine Verjüngung mit einem kleineren Querschnitt als der Rest der Leiterbahn aufweisen. An der Stelle der Leiterbahn mit einem höheren elektrischen Widerstand als im Rest der Leiterbahn, kann die erhöhte Stromstärke zu einem starken Erhitzen der Leiterbahn und somit zu einer Zerstörung der Leiterbahn führen. Somit wird sichergestellt, dass der Plasmaapplikator nur einmal verwendet werden kann.

Eine Einmalverwendbarkeit eines Plasmaapplikators mit einer Steckvorrichtung mit einer entsprechenden Leiterbahn kann auch durch eine Stelle der Leiterbahn mit gleichem Widerstand wie in dem Rest der Leiterbahn aber mit einer geringeren thermischen Festigkeit realisiert sein. Wenn ein Stromimpuls mit erhöhter Stromstärke bereitgestellt wird, bevorzugt am Ende einer Plasmabehandlung mit einer Dauer deutlich unterhalb von ca. 1 Sekunde, ist die thermische Belastung der Leiterbahn erhöht. Die Stelle mit einer geringeren thermischen Festigkeit kann so ausgebildet sein, dass sie durch den bereitgestellten Stromimpuls schmilzt, wodurch eine Einmalverwendbarkeit eines Plasmaapplikators sichergestellt wird.

Eine Einmalverwendbarkeit eines Plasmaapplikators kann auch durch eine Verjüngung der entsprechenden Leiterbahn sichergestellt werden. Durch die Verjüngung ist der Widerstand an dieser Stelle erhöht, sodass die am Ende der Behandlung bereitgestellte erhöhte Stromstärke zu einem Schmelzen der Leiterbahn an dieser Stelle führt. Die Überspannung führt also zu einer Temperaturerhöhung an der Stelle mit der Verjüngung und somit zum Schmelzen der Leiterbahn. Zum Beispiel kann die Dimension der Leiterbahn entsprechend des verwendeten Materials so berechnet werden, dass bei einem bestimmten Stromfluss über einen definierten Zeitraum das Material auf eine Temperatur ansteigt, die bevorzugt deutlich über dem Schmelzpunkt des Materials liegt, sodass die Leiterbahn an dieser Stelle schmilzt.

Bevorzugt weist eine Leiterbahn eine Höhe auf, die kleiner als 0.8 mm ist und eine Breite auf, die kleiner als 1 cm ist. Solche Leiterbahnen können z.B. mittels Siebdruck mit einem leitfähigen Material, durch gezieltes Ätzen von Leiterbahnen oder durch aufgedruckte Leiterbahnen (z.B. *printed electronics*) realisiert sein.

Eine Steckvorrichtung kann einen nichtflüchtigen elektronischen Speicherbaustein aufweisen, der von einem entsprechenden Kontakt in der Einschubvorrichtung ausgelesen werden kann, wenn die Einschubvorrichtung und die Steckvorrichtung miteinander verbunden sind, wobei der nichtflüchtige elektronische Speicherbaustein eine Information bereitstellt, die eine angeschlossene Energieversorgungseinheit dazu veranlasst, im Betriebsfall eine Energieabgabe an einen angeschlossenen Plasmaapplikator zu unterbinden.

In einer Ausgestaltung weist eine Steckvorrichtung einen RFID-Transponder auf. Der RFID-Transponder ist derart ausgebildet, dass er von einem in eine Einschubvorrichtung integrierten Lesegerät ausgelesen werden kann, wenn die entsprechende Einschubvorrichtung und die Steckvorrichtung miteinander verbunden sind.

Der RFID-Transponder stellt bevorzugt eine Information bereit, durch die keine Freigabe einer Hochspannung durch eine Energieversorgungseinheit erfolgt. Denkbar ist, dass einem Plasmaapplikator eine Identität zugewiesen ist, die durch ein Lesegerät, das in eine Einschubvorrichtung integriert ist, ausgelesen werden kann. In einem Steuergerät einer Energieversorgungseinheit können z.B. eindeutige und individuelle Kennungen für jeden Plasmaapplikator hinterlegt sein, der mit der Energieversorgungseinheit verbunden war. Ein Lesegerät kann so erkennen, ob ein Plasmaapplikator bereits verwendet wurde oder nicht. Zusätzlich oder alternativ kann auch ein Schreib-Lesegerät vorgesehen sein, um einen in dem RFID-Transponder gespeicherten Wert - z.B. ein Flag - zu verändern bzw. zu setzen, der anzeigt, dass der Plasmaapplikator verwendet wurde.

In einer Ausführungsform ist ein Plasmaapplikator ausgebildet, auf einem Speicher einen speziellen Code oder Hash-Wert zu speichern. Vorzugsweise ist eine Energieversorgungseinheit ausgebildet, von einem ausgelesenen Code oder Hash-Wert abhängig bestimmte Werte für Behandlungsparameter einzustellen und im Betrieb ein entsprechendes Spannungssignal an einen mit der Energieversorgungseinheit verbundenen Plasmaapplikator abzugeben. Vorzugsweise ist eine Energieversorgungseinheit ausgebildet, Werte der Behandlungsparameter für die Größe eines Plasmaapplikators oder an die Art der Krankheit, die mit dem angeschlossenen Plasmaapplikator behandelt werden soll, aus einer in der Energieversorgungseinheit gespeicherten Liste auszulesen. Vorzugsweise ist eine Energieversorgungseinheit ausgebildet, zu überprüfen, ob ein ausgelesener Plasmaapplikator bereits verwendet wurde oder für eine bestimmte Plasmabehandlung geeignet ist. Vorzugsweise ist ein Schreib-Lesegerät ausgebildet, nach einer Plasmabehandlung entweder den Chip mit dem Wert zu zerstören oder einen neuen Wert auf den Speicher zu schreiben. Der neue Wert beinhaltet z.B. nur Nullen, sodass der spezielle Plasmaapplikator nicht mehr für eine Plasmabehandlung verwendet werden kann, da eine angeschlossene Energieversorgungseinheit bevorzugt ausgebildet ist, im Falle der Verwendung ungültiger Nummern die Freigabe eines Spannungssignals zu verweigern.

Ein RFID-Transponder kann auch nicht in eine Steckvorrichtung, sondern in den übrigen Plasmaapplikator integriert sein.

In einer Ausgestaltung weist eine Steckvorrichtung einen nichtflüchtigen elektronischen Speicherbaustein, z.B. ein EPROM (erasable programable read-only memory), auf, der von einem entsprechenden Kontakt in der Einschubvorrichtung ausgelesen werden kann, wenn die Einschubvorrichtung und die Steckvorrichtung miteinander verbunden sind. Ähnlich wie im Fall eines RFID-Transponders kann in den nichtflüchtigen elektronischen Speicherbaustein bei dem erstmaligen Gebrauch ein Wert eingeschrieben werden, der beim späteren Auslesen anzeigt, dass der Plasmaapplikator bereits verwendet wurde. Nach dem erstmaligen Gebrauch kann eine Freigabe einer Hochspannung durch eine Energieversorgungseinheit nicht mehr erfolgen. In Verbindung mit einem entsprechenden Steuergerät kann so eine Mehrfachverwendung unterbunden werden.

Anstatt in eine Steckvorrichtung kann ein nichtflüchtiger elektronischer Speicherbaustein auch in den übrigen Plasmaapplikator integriert sein.

### Einschubvorrichtung

Im Folgenden werden Ausführungsformen einer Einschubvorrichtung beschrieben. Eine Einschubvorrichtung stellt eine zu einer Steckvorrichtung komplementären Einheit dar und ist ausgebildet, eine feste mechanische und elektrische Verbindung mit einer Steckvorrichtung einzugehen.

Entsprechend stellt eine Einschubvorrichtung eine zu einer Steckvorrichtung komplementär ausgebildete Komplementär-Steckkontaktvorrichtung dar. Eine Einschubvorrichtung stellt also ein Gegenstück zu einer Steckvorrichtung dar. Eine Einschubvorrichtung kann vorzugsweise an einem Ende eines Kabels angeordnet oder Teil einer Energieversorgungseinheit sein.

Es ist vorzugsweise eine Einschubvorrichtung vorgesehen, die ausgebildet ist, ein Spannungssignal an eine mit der Einschubvorrichtung zusammen gesteckte Steckvorrichtung eines elektrotechnischen Kerns insbesondere eine Plasmaapplikators zu übertragen.

Eine Einschubvorrichtung ist bevorzugt derart ausgebildet, dass sie mit einer komplementären Steckvorrichtung elektrisch leitfähig verbunden werden kann. Durch Zusammenführen einer Steckvorrichtung und einer Einschubvorrichtung werden gleichzeitig eine elektrische und eine mechanische Verbindung hergestellt. Die Leiterbahnen der Einschubvorrichtung und der Steckvorrichtung sind dabei im zusammengeführten Zustand nach außen elektrisch isoliert.

Eine Einschubvorrichtung kann mit einem Kabel verbunden sein. Über das Kabel kann ein Plasmaapplikator, der mit einer entsprechenden Einschubvorrichtung verbunden ist, mit einer vorwiegend stationären Energieversorgungseinheit verbunden werden. Eine vorwiegend stationäre Energieversorgungseinheit kann auch ein Steuergerät umfassen. Eine Einschubvorrichtung kann jederzeit von einer Steckvorrichtung getrennt werden, sodass sich ein Nutzer eines Plasmaapplikators mit dem Plasmaapplikator unabhängig von der Energieversorgungseinheit und der Einschubvorrichtung bewegen kann. Für den Fall, dass eine Steckvorrichtung in Form eines Steckers, insbesondere in Form einer Chipkarte, ausgebildet ist, ist eine Einschubvorrichtung als eine entsprechende Kupplung zur Aufnahme der Steckvorrichtung ausgebildet.

Eine Einschubvorrichtung kann auch als Teil einer mobilen Energieversorgungseinheit ausgebildet sein, die vorzugsweise einen Energiespeicher, z.B. eine Batterie, einen Akkumulator oder einen Kondensator, aufweist. Eine Einschubvorrichtung ist dann typischerweise elektrisch mit einem Energiespeicher verbunden, um - wenn sie mit einer Steckvorrichtung verbunden ist - eine durch den Energiespeicher bereitgestellte elektrische Energie an einen elektrotechnischen Kern zu übertragen. Vorteilhafterweise muss eine Einschubvorrichtung dann nicht mit einem vergleichsweise langen Kabel verbunden sein, um die Einschubvorrichtung mit einer vorwiegend stationären Energieversorgungseinheit zu verbinden.

Um einen Plasmaapplikator mit einem Spannungssignal zu versorgen, kann eine Einschubvorrichtung einer mobilen Energieversorgungseinheit mit einer Steckvorrichtung verbunden werden und nach einer Plasmabehandlung von dieser wieder getrennt werden. Im zusammengeführten Zustand wird dann eine durch einen Energiespeicher bereitgestellte Gleichspannung mittels einer in der mobilen Energieversorgungseinheit integrierten elektrischen Schaltung in ein zum Zünden des Plasmas ausreichendes Spannungssignal transformiert und über die Einschubvorrichtung an die Steckvorrichtung übertragen.

Da eine Batterie oder ein Akkumulator typischerweise eine Gleichspannung von einigen Volt bereitstellt, kann in einem Plasmaapplikator oder in einer mobilen Energieversorgungseinheit eine elektrische Schaltung vorgesehen sein, die die durch die Batterie oder den Akkumulator der kompakten und mobilen Energieversorgungseinheit bereitgestellte Gleichspannung in ein zum Zünden eines Plasmas ausreichendes Spannungssignal transformiert, welches an wenigstens eine Elektrodenstruktur zum Zünden eines Plasmas übertragen wird. Vorteilhafterweise bildet eine mobile Energieversorgungseinheit eine im Vergleich zum Plasmaapplikator kleine und kompakte Einheit, die über weite Strecken und über einen großen Zeitraum von mehreren Wochen mitgeführt werden kann, auch dann, wenn sie mit dem Plasmaapplikator verbunden bleibt.

Im zusammengeführten Zustand bilden also der Plasmaapplikator und die im Vergleich zum Plasmaapplikator verhältnismäßig kleine mobile Energieversorgungseinheit eine während einer Plasmabehandlung von einem Patienten leicht zu tragende Einheit. Eine mobile Energieversorgungseinheit stellt also eine autonome Energieversorgung dar, die es nicht notwendig macht, eine vorwiegend stationär genutzte Energieversorgungseinheit, wie z.B. einen Hochspannungsgenerator, über ein Kabel und eine Einschubvorrichtung mit dem Plasmaapplikator zu verbinden, um ein Spannungssignal an wenigstens eine Elektrodenstruktur zu übertragen und ein Plasma zu zünden.

Dadurch wird ein Nutzer einer mobilen Energieversorgungseinheit unabhängig von einer im Vergleich zu einer mobilen Energieversorgungseinheit großen, vorwiegend stationär genutzten Energieversorgungseinheit, die an eine örtliche Stromversorgung angeschlossen wird, wie einem Hochspannungsgenerator, und die typischerweise nur über kurze Distanzen, z.B. innerhalb eines Krankenhauses, transportiert wird. Insbesondere kann ein Nutzer einer mobilen Energieversorgung selbst entscheiden, wo und wann er eine Plasmabehandlung durchführen möchte. Ein Nutzer einer mobilen Energieversorgung ist entsprechend unabhängig von lokaler Infrastruktur, wir z.B. dem Stromnetz und verfügbaren Steckdosen. Dies ist besonders dann von Vorteil, wenn sich ein Nutzer über einen längeren Zeitraum von mehreren Wochen in einem Gebiet aufhält, in dem das nächste Krankenhaus weit entfernt ist und weiterhin nur begrenzt Gepäck mitgeführt werden kann oder allgemein für den Homecare-Bereich, also die Anwendung außerhalb einer Klinik bzw. im nicht-klinischen Umfeld.

Ein durch eine mobile Energieversorgungseinheit bereitgestelltes Spannungssignal kann z.B. durch Herstellen einer galvanischen Kopplung von einer Einschubvorrichtung an eine Steckvorrichtung übertragen werden.

In einer weiteren Ausführungsform weist ein Plasmaapplikator keine Steckvorrichtung auf und kann dementsprechend nicht mittels einer Einschubvorrichtung an eine vorwiegend stationäre oder eine mobile Energieversorgungseinheit angeschlossen werden. Zum Bereitstellen eines Spannungssignals kann ein Energiespeicher, z.B. eine Batterie oder ein Akkumulator, in den Plasmaapplikator selbst intergiert und elektrisch über eine geeignete elektrische Schaltung, z.B. einer Sperrschwingschaltung, zum Erzeugen einer zum Zünden eines Plasmas ausreichenden Spannung, mit wenigstens einer Elektrodenstruktur eines elektrotechnischen Kerns verbunden sein.

Falls ein Akkumulator oder ein Kondensator in einen Plasmaapplikator mit einer Steckvorrichtung integriert sind, können der Akkumulator oder der Kondensator durch ein Verbinden der Steckvorrichtung mit einer Energieversorgung geladen werden. Auch hier ist der Akkumulator oder der Kondensator elektrisch über eine geeignete elektrische Schaltung, z.B. einer Sperrschwingschaltung, zum Erzeugen einer zum Zünden eines Plasmas ausreichenden Spannung, mit wenigstens einer Elektrodenstruktur eines elektrotechnischen Kerns verbunden. Durch Schalten eines Schaltkontakts kann dann zu einem späteren Zeitpunkt die in dem Akkumulator oder dem Kondensator gespeicherte Energie an wenigstens eine Elektrodenstruktur abgegeben werden.

### Induktive Energieübertragung

In einer Ausführungsform weist ein Plasmaapplikator keine Steckvorrichtung, sondern eine Energieaufnahmevorrichtung auf, die jeweils eine oder mehrere Empfangs-Spulenanordnungen enthält. Mittels einer mobilen Energieversorgungseinheit, die eine oder mehrere Sende-Spulenanordnungen enthält, kann durch elektromagnetische Induktion elektrische Energie von den Sende-Spulenanordnungen in der mobilen Energieversorgungseinheit auf die Empfangs-Spulenanordnungen im Plasmaapplikator übertragen werden. Ebenfalls denkbar ist, dass eine Sende-Spulenanordnung in einer Energieabgabevorrichtung enthalten ist. Die Energieabgabevorrichtung ist vorzugsweise mit einem Kabel verbunden und kann an eine stationäre Energieversorgungseinheit angeschlossen werden. Die von einer stationären Energieversorgungseinheit bereitgestellte elektrische Energie kann mittels elektromagnetischer Induktion von einer Sende-Spulenanordnung in der Energieabgabevorrichtung auf eine Empfangs-Spulenanordnung im Plasmaapplikator übertragen werden. Vorteilhafterweise kann in dieser Ausführungsform die Energieabgabevorrichtung vollständig durch z.B. ein Silikon oder einen Lack umschlossen werden und es kann auf frei zugängliche elektrische Kontakte sowohl in bzw. an der Energieabgabevorrichtung als auch im bzw. am Plasmaapplikator verzichtet werden. Ein auf diese Weise ausgeführter Plasmaapplikator bzw. eine Energieabgabevorrichtung lassen sich vergleichsweise einfach reinigen, desinfizieren und sterilisieren bzw. autoklavieren.

Im Gegensatz zu den vorher beschriebenen Varianten eines Plasmaapplikators mit einer Steckvorrichtung, die mit einer Einschubvorrichtung zusammengeführt werden kann, findet eine Energieübertragung in der hier beschrieben Variante eines Plasmaapplikators nicht über eine galvanische Kopplung, sondern über elektromagnetische Induktion statt. Bevorzugt ist entweder im Plasmaapplikator oder in einer mobilen Energieversorgungseinheit oder in beiden eine elektrische Schaltung vorgesehen, die ausgebildet ist, ein zum Zünden eines Plasmas ausreichendes Spannungssignal zu erzeugen. Beispielweise kann in einer mobilen Energieversorgungseinheit eine elektrische Schaltung vorgesehen sein, die aus einer Gleichspannung von wenigen Volt, welche typischerweise von Akkumulatoren und/oder Batterien bereitgestellt wird, in ein zur induktiven Übertragung geeignetes Wechselspannungssignal transformiert werden. Im Plasmaapplikator kann dann eine elektrische Schaltung integriert sein, die das in der im Plasmaapplikator enthaltenen Empfangs-Spulenanordnungen induzierte Spannungssignal in ein zum Zünden eines Plasmas ausreichendes Spannungssignal transformiert.

In einerweiteren Ausführungsform weist ein Plasmaapplikator keine Steckvorrichtung aber einen integrierten Energiespeicher, z.B. einen Akkumulator oder einen Kondensator, und eine integrierte Spulenanordnung zum Laden des Akkumulators oder des Kondensators auf. In dieser Ausführungsform kann zusätzlich eine elektrische Schaltung im Plasmaapplikator integriert sein, welche den in der Spulenanordnung im Plasmaapplikator induzierten Strom in einen zum Laden eines Akkumulators oder Kondensators ausreichendes Strom-Spannungssignal transformiert. Ein entsprechender Plasmaapplikator benötigt keine Steckvorrichtung und kann ohne frei zugängliche elektrische Kontakte ausgeführt sein. Ein auf diese Weise ausgeführter Plasmaapplikator kann vergleichsweise einfach gereinigt, desinfiziert und sterilisiert bzw. autoklaviert werden. Weiterhin kann ein solcher Plasmaapplikator z.B. in den menschlichen oder tierischen Körper implantiert werden. In einer Ausführungsform ist ein Plasmaapplikator mit einem oder mehreren pharmakologisch und/oder nicht-pharmakologisch wirksamen Wirkstoffen in Form von einzelnen Molekülen, Agglomeraten oder Tabletten (z.B. Morphinen, Koagulanzien, Cytokinen, Hydrokolloiden) beschichtet bzw. ausgestattet.

In Ausführungsformen eines Plasmaapplikators ohne eine Steckvorrichtung kann ein elektrotechnischer Kern mit den vorher genannten Merkmalen, insbesondere mit den Markmalen, die in Bezug auf die Elektrodenstruktur und die Isolationsschicht genannt wurden, ausgebildet sein. Auch in diesen Ausführungsformen ohne Steckvorrichtung ist es möglich durch Ändern von elektrischen oder mechanischen Komponenten eine Einmalverwendbarkeit des Plasmaapplikators sicherzustellen. Zum Beispiel kann eine elektrische Komponente, die für die Übertragung eines zum Zünden eines Plasmas ausreichendes Spannungssignals an wenigstens eine Elektrodenstruktur vorgesehen ist, infolge eines erstmaligen Gebrauchs derart verändert werden, dass nach dem erstmaligen Gebrauch kein zum Zünden eines Plasmas ausreichendes Spannungssignal mehr an eine Elektrodenstruktur übertragen werden kann.

### Umschließung

Bevorzugt weist ein Plasmaapplikator für die Behandlung von menschlichen oder tierischen Oberflächen eine Umschließung insbesondere aus einem biokompatiblen Material, wie z.B. medizinischem Silikon, einem Lack, einem Klebstoff, einer Folie, einem Textil, einem Kompressionstextil oder organischem Material wie z.B. Mull, Zellstoff oder Baumwolle auf. Auch eine Umschließung, die eine Kombinationen aus den genannten Materialien umfasst, kann für manche Anwendungen vorteilhaft sein. Insbesondere kann ein elektrotechnischer Kern eines Plasmaapplikators komplett oder wenigstens teilweise von einer Umschließung umschlossen sein.

Üblicherweise umfasst eine Umschließung mehrere Silikonumspritzungen. Auf der der zu behandelnden Seite zugewandten Seite ist eine erste Silikonumspritzung vorgesehen, die die Funktion einer elektrischen Isolierung erfüllt. Auf der der zu behandelnden Seite abgewandten Seite folgt auf die erste Silikonumspritzung eine zweite Silikonumspritzung, die ein elektrisch leitfähiges Silikon umfasst. Diese zweite Silikonumspritzung wird auf Massepotential gelegt und erfüllt die Funktion eines Berührungsschutzes, sodass ein Plasmaapplikator berührt werden kann, ohne dass es zu einem elektrischen Durchschlag zwischen dem elektrotechnischen Kern und dem direkt auf der Außenseite anliegenden Erdpotential oder einem virtuellen Erdpotential in Form der zu behandelnden Oberfläche kommt. Auf der zweiten Silikonumspritzung ist eine dritte Silikonumspritzung aus einem elektrisch isolierenden Silikon aufgebracht. Die Herstellung einer solchen Umschließung erfordert eine vergleichsweise hohe Fertigungstiefe.

Eine Umschließung kann beispielsweise im Spritzgussverfahren, im Tauchverfahren oder im Lackierverfahren ausgeführt werden. Die Verwendung von anderen Beschichtungsverfahren wie Plasmabeschichtung oder Parylenebeschichtung sind auch denkbar. Eine Umschließung kann vollständig oder nur teilweise ausgeführt sein. Zum Beispiel kann es vorteilhaft sein, wenn die der Wunde zugewandte Seite nicht oder nur teilweise umschlossen ist und die der Wunde abgewandte Seite komplett umschlossen ist.

In einer bevorzugten Ausführungsform umfasst ein elektrotechnische Kern eine erste Isolationsschicht, eine erste Elektrodenstruktur, die im Betrieb geerdet ist, eine zweite Isolationsschicht, die ausgebildet ist, die erste Elektrodenstruktur und eine zweite Elektrodenstruktur galvanisch voneinander zu trennen, eine zweite Elektrodenstruktur, die im Betrieb durch ein von einer Energieversorgungseinheit bereitgestelltes und zum Zünden eines Plasmas ausreichendes Spannungssignal getrieben wird, eine dritte Isolationsschicht, die ausgebildet ist, die zweite Elektrodenstruktur und eine dritte Elektrodenstruktur galvanisch voneinander zu trennen und eine dritte Elektrodenstruktur, die im Betrieb geerdet ist. Ein solcher elektrotechnischer Kern bietet an sich bereits einen Berührungsschutz. Ein Berührungsschutz muss also nicht nachträglich durch eine aufgebrachte Umschließung gewährleistet werden. Ein elektrotechnischer Kern entsprechend dieser bevorzugten Ausführungsform kann vorzugsweise durch eine Umschließung in Form von lediglich einer einzigen Silikonschicht realisiert sein, die vorteilhafterweise vergleichsweise dünn und somit flexibel ausgebildet sein kann. Die Umschließung muss insbesondere nicht mehr einen Berührungsschutz gewährleisten, da diese Funktion von der ersten und dritten Elektrodenstruktur erfüllt wird. Eine solche Umschließung ist also vergleichsweise einfach und kann in lediglich einem Fertigungsschritt aufgebracht werden. Dadurch reduziert sich die Fertigungstiefe bei der Herstellung eines Plasmaapplikators im Vergleich zu einem Plasmaapplikator mit einer wie oben beschriebenen üblichen Umschließung deutlich, da eine Umschließung typischerweise auf jeden elektrotechnischen Kern separat aufgebracht werden muss. Ein wie hier beschrieben ausgebildeter elektrotechnischer Kern lässt sich hingegen durch den Einsatz von Laminiermaschinen und anschließendem Ausstanzen vergleichsweise einfach in großen Stückzahlen produzieren.

Ein elektrotechnischer Kern gemäß dieser bevorzugten Ausführungsform kann modulartig in verschiedene Umschließungen integriert werden, ohne dass an eine verwendete Umschließung spezielle Anforderungen gestellt werden müssen. Eine mögliche Umschließung kann beispielsweise durch ein herkömmliches Pflaster ohne Umspritzung realisiert sein in das der elektrotechnische Kern integriert wird. Eine mögliche Umschließung kann auch durch eine Saugkompresse realisiert sein in die der elektrotechnische Kern integriert wird. Eine mögliche Umschließung kann auch durch einen Kompressionsstrumpf realisiert sein. Der elektrotechnische Kern kann auch in einer Tasche eingenäht oder mit der Rückseite an eine Decke, ein Tuch, Thermofolie etc. angeklebt oder in ein Unterdruck-Wundtherapie-System (V.A.C.) integriert sein.

Eine Umschließung kann strukturiert ausgebildet sein, z.B. in Form eines Gitters oder rautenförmig oder mit Aussparungen. Eine derart strukturierte Umschließung ist insbesondere dann vorteilhaft, wenn sie auf einer körperzugewandten Seite eines elektrotechnischen Kerns z.B. mit einem Klebesilikon (nicht vollständig ausvulkanisiertes Silikon) oder einem anderen Kleber z.B. auf Acrylatbasis oder Polyurethan-basis ausgeführt ist. Weiterhin kann eine Umschließung so ausgebildet sein, dass in gewissen Bereichen um einen elektrotechnischen Kern eine Umschließung vorliegt (die Form kann dann z.B. rund, eckig, L-, M-, E-, X- oder auch O-förmig sein) und in anderen Bereichen keine Umschließung vorliegt.

Eine kann Umschließung auch so ausgeführt sein, dass außerhalb eines Bereiches, in dem ein Plasma erzeugt wird, mindestens eine Durchschlagfestigkeit und somit eine Berührungssicherheit zwischen der wenigstens einen, im Anwendungsfall mit einem Spannungssignal getriebenen Elektrodenstruktur und einem potentiell direkt auf der Außenseite anliegenden Erdpotential oder einem virtuellen Erdpotential in Form der zu behandelnden Oberfläche gewährleistet ist. Für die Gewährleistung ist eine Abstimmung der elektrischen Eigenschaften eines Umschließungsmaterials und dessen Dicke mit dem in einem elektrotechnischen Kern vorliegenden elektrischen Potential notwendig. Vorteilhafterweise wird ein zusätzlicher Sicherheitsfaktor berücksichtigt. Beispielsweise hat medizinisches Silikon typischerweise eine Durchschlagsfestigkeit von ungefähr 20 kV pro mm. Wenn eine entsprechende Umschließung aus medizinischem Silikon eine Dicke von 1 mm hat, kann z.B. eine Spannung von 20 kV angelegt werden, ohne dass es zu Materialschäden oder Durchschlägen kommt. Eine Durchschlagsfestigkeit ist aber von Material zu Material unterschiedlich. Es gibt spezielle Lacke, die eine deutlich höhere Durchschlagsfestigkeit aufweisen als medizinisches Silikon. Angenommen, ein Spannungssignal, mit dem wenigstens eine Elektrodenstruktur getrieben wird, weist eine Amplitude von 5 kV auf, d.h. 10 kVvon Spitze zu Spitze auf. Dann wäre die Durchschlagsfestigkeit einer Silikonschicht mit einer Dicke von 250 µm ausreichend damit es nicht zu Durchschlägen im Material kommt. Der Sicherheitsfaktor wird typischerweise mit einem Faktor 2 angegeben. In dem Fall, in dem eine Umschließung aus medizinischem Silikon besteht und die Amplitude des Spannungssignals mit dem eine Elektrodenstruktur getrieben wird 5 kV hat, muss eine Umschließung entsprechend eine Dicke haben, die mindestens 500 µm beträgt. Bei Umschließungen aus anderen Materialien, wie z.B. Lacken und/oder Polyurethan, ist es vorteilhaft die Dicke auf die Materialeigenschaften des für die Umschließung verwendeten Materials angepasst werden.

Eine Umschließung kann auch von einem Textil z.B. in Form einer Tasche gebildet sein. In diese Tasche kann ein elektrotechnischer Kern geschoben oder auch eingenäht/geprägt werden. Eine solche Tasche mit eingeschobenem oder eingenähtem/geprägtem elektrotechnischem Kern kann direkt auf einer zu behandelnden Oberfläche befestigt werden.

Eine Tasche mit eingeschobenem oder eingenähtem/geprägtem elektrotechnischen Kern kann auch Teil eines anderen Textils sein. Die Tasche kann fest an das andere Textil z.B. genäht oder genietet oder mittels z.B. Klebstoff, Klettband oder Klebeband befestigt sein.

Eine Umschließung eines elektrotechnischen Kerns kann auch so ausgebildet sein, dass diese absorbierende Eigenschaften hat. Eine Umschließung mit absorbierenden Eigenschaften besteht vorzugsweise aus mindestens einer Lage von flüssigkeitsabsorbierenden und/oder flüssigkeitsabführenden und/oder flüssigkeitsverteilenden Materialien, wie z.B. Textil, Mull, PU-Schaum, Verteilerschicht, Wundkontaktschicht, Distanzstruktur. Bevorzugt befindet sich eine solche Umschließung mit absorbierenden Eigenschaften auf der körperzugewandten Seite eines Plasmaapplikators.

Eine Umschließung kann auch durch eine Kombination der oben genannten Materialien (z.B. Textilien, Silikon, Lacke, Kleber, Parylenebeschichtung, Plasmabeschichtung, Mull, Kompresse) gebildet sein. Diese Kombination kann sowohl eine Mischung der Materialien sein (z.B. Textil und Silikon als Verbundmatrix) als auch in gestapelter Form (z.B. unterschiedliche Textilien übereinander auf Silikon, Mull, PU-Schaum, Verteilerschicht, Wundkontaktschicht) oder auch nebeneinander bzw. auf unterschiedlichen Seiten sein. So kann z.B. auf der patientenabgewandten Seite eine Umschließung von einer dünnen Folie gebildet sein, während auf der patientenzugewandten Seite eine Umschließung aus einem oder mehreren Lagen Textil und /oder einem oder mehreren Absorbern besteht. Je nach Anwendungsfall kann auch eine Befestigung eines elektrotechnischen Kerns an einer zu behandelnden Oberfläche durch eine Umschließung in Form eines Verbandes erfolgen. Ein elektrotechnischer Kern wird in diesem Fall aufgelegt und mit einem Fixierverband durch mehrmaliges Umwickeln des auf der zu behandelnden Oberfläche aufliegenden elektrotechnischen Kerns und der zu behandelnden Oberfläche fixiert.

Oben genannte Textilien für eine Umschließung können sowohl aus organischem als auch aus anorganischem Material sowie aus einem Mix beider Materialien bestehen.

Wenn ein Plasmaapplikator eine Steckvorrichtung aufweist, ist eine Umschließung der Steckvorrichtung und eines elektrotechnischen Kerns bevorzugt formschlüssig und ohne Lufteinschlüsse. Ist eine Umschließung von einem Textil oder einem Material mit absorbierenden Eigenschaften gebildet, ist es eine formschlüssige Umschließung ohne Lufteinschlüsse nicht notwendig. Eine Umschließung einer Steckvorrichtung ist vorteilhafterweise unter Berücksichtig einer entsprechenden Einschubvorrichtung und der Art der Kopplung zwischen diesen gestaltet. Falls zum Beispiel eine galvanische Kopplung zwischen einer Steckvorrichtung und einer Einschubvorrichtung vorgesehen ist, sollten wenigstens die elektrischen Kontaktflächen der Steckvorrichtung frei zugänglich für die elektrischen Kontaktflächen der Einschubvorrichtung sein. Wenn allerdings eine induktive Kopplung mittels elektromagnetischer Induktion vorgesehen ist, kann ein Plasmaapplikator auch vollständig mit einer Umschließung umschlossen sein.

Für den Fall, dass ein Plasmaapplikator keine Steckvorrichtung aufweist und ein Energiespeicher direkt in den Plasmaapplikator intergiert ist, kann der Plasmaapplikator ebenfalls vollständig mit einer Umschließung umschlossen sein.

Eine Umschließung eines Plasmaapplikators kann mit einem oder mehreren pharmakologisch bzw. nicht-pharmakologisch wirksamen Wirkstoffen in Form von einzelnen Molekülen, Agglomeraten oder Tabletten (z.B. Morphinen, Koagulanzien, Cytokinen, Hydrokolloiden) angereichert und/oder beschichtet sein. Eine Anreicherung mit einem pharmakologisch wirksamen Wirkstoff kann insbesondere dann vorteilhaft sein, wenn ein Plasmaapplikator in einen menschlichen oder tierischen Körper integriert werden soll.

Ein Plasmaapplikator kann mit reinem Mull oder Zellstoff oder vergleichbaren Materialien umschlossen sein. In einer entsprechenden Ausführungsform wird ein Plasmaapplikator nicht mit einem biokompatiblen Material wie Silikon umschlossen, sondern ein elektrotechnischer Kern eines Plasmaapplikators wird in eine Mullbinde oder Medizinkissen oder in Zellstoff eingewoben bzw. eingenäht/geprägt. Hierbei ist es besonders vorteilhaft, wenn der elektrotechnische Kern selbst einen Berührungsschutz, beispielweise durch zwei geerdete Elektrodenstrukturen (eine erste und eine dritte Elektrodenstruktur) zwischen denen eine im Betrieb getriebene Elektrodenstruktur (zweite Elektrodenstruktur) angeordnet ist, aufweist.

In einer Ausführungsvariante, in der ein Plasmaapplikator einen elektrotechnischen Kern mit Löchern oder Durchführungen aufweist, die in einem Teilbereich des elektrotechnischen Kerns oder über die gesamte Fläche des elektrotechnischen Kerns verteilt angeordnet sind, kann eine Umschließung derart gestaltet sein, dass die Umschließung einen Medientransport durch den elektrotechnischen Kern hindurch von der einer zu behandelnden Oberfläche zugewandten Seite des Plasmaapplikators zu der einer zu behandelnden Oberfläche abgewandten Seite des Plasmaapplikators zulässt oder selbst ermöglicht. Beispielsweise kann eine geeignete Umschließung von einem medientransportierenden Material gebildet sein oder ebenfalls Löcher und Durchführungen aufweisen.

In einer Ausführungsvariante, in der eine Steckvorrichtung von dem elektrotechnischen Kern an einer Perforation abgetrennt werden kann, kann die Perforation als Teil einer Umschließung ausgebildet sein. Die Funktion der Perforation ist die Reduktion der Festigkeit der Umschließung an dieser Stelle, sie soll eine Sollbruchstelle darstellen. An dieser Perforation kann die Steckvorrichtung nach der Behandlung abgerissen bzw. entfernt werden.

Dadurch kann ein Plasmaapplikator weiterhin auf einer zu behandelnden Oberfläche verbleiben, zum Beispiel über einen längeren Zeitraum, der einige Tagen bis hin zu Wochen umfassen kann, und die nicht mehr benötigte Steckvorrichtung entfernt werden.

Eine Umschließung kann in ihrer lateralen Ausdehnung deutlich größer als eine Elektrodenstruktur eines elektrotechnischen Kerns sein. Eine Umschließung kann beispielweise eine Grundfläche haben, die 20 cm x 20 cm oder auch 30 cm x 30 cm und größer beträgt, wohingegen die Fläche einer Elektrodenstruktur 10 cm x 10 cm beträgt.

In einer Ausführungsvariante, in der eine Umschließung eines Plasmaapplikators eine größere Grundfläche als eine Elektrodenstruktur eines elektrotechnischen Kerns hat, kann ein Nutzer vor einer Plasmabehandlung den Plasmaapplikator auf eine gewünschte Größe zurechtschneiden, die beispielsweise der Fläche einer Wunde entsprechen kann, wobei die minimale Größe durch die Größe der Elektrodenstruktur gegeben ist.

### Adhäsionsschicht

Es kann vorteilhaft sein, wenn ein Plasmaapplikator als letzte Schicht auf der der zu behandelnden Oberfläche zugewandten Seite eine Adhäsionsschicht aufweist, um einen Plasmaapplikator auf oder über einem zu behandelnden Areal zu fixieren und einen abgeschlossenen Gasraum zwischen Plasmaapplikator und der zu behandeln Oberfläche zu erzeugen. Eine Adhäsionsschicht besteht bevorzugt aus einem biokompatiblen Material, wie Silikon oder einem Klebstoff auf Acrylatbasis und hat eine bevorzugte Dicke, die zwischen wenigen µm und einigen hundert µm beträgt. Über die Dicke einer Adhäsionsschicht kann direkt deren Adhäsionskraft eingestellt werden. Bevorzugt liegt die Dicke einer Adhäsionsschicht unterhalb von 150 µm, insbesondere unterhalb von 60 µm, insbesondere unterhalb von 20 µm.

Bevorzugt besitzt eine Adhäsionsschicht eine Adhäsionskraft, die ausreicht, dass ein Plasmaapplikator ohne zusätzliche Hilfsmittel oder Befestigungsmaterial an einer zu behandelnden Oberfläche haftet. Insbesondere kann es vorteilhaft sein, wenn eine Adhäsionsschicht einen Haftkontakt zwischen einer zu behandelnden Oberfläche und einem Plasmaapplikator herstellt, der einige Tage oder sogar mehrere Wochen hält.

Eine Adhäsionsschicht kann auch von einem Material gebildet sein oder ein Material aufweisen, welches photoaktiv ist. Photoaktiv bedeutet in diesem Zusammenhang, dass die Adhäsionskraft des Materials mittels Photonen beeinflusst werden kann. Vor einer Bestrahlung eignet sich das Adhäsionsmaterial für ein Herstellen eines Haftkontakts mit einer anderen Oberfläche. Nach Bestrahlung mit Photonen einer speziellen Wellenlänge verliert das Adhäsionsmaterial seine Adhäsionskraft und eignet sich nicht mehr zum Herstellen eines Haftkontakts. Ein Plasmaapplikator mit einer photoaktiven Adhäsionsschicht ist besonders für den Einsatz über einen längeren Zeitraum von einigen Tagen bis hin zu Wochen geeignet.

Eine Adhäsionsschicht kann zum Beispiel im Siebdruckverfahren oder im Spritzgussverfahren aufgebracht werden. Es ist auch denkbar, dass eine Adhäsionsschicht als Rollenware durch beispielsweise ein Transferklebeband oder durch ein doppelseitiges Klebeband realisiert ist. Ein Transfer- bzw. doppelseitige Klebeband kann elastisch und somit flexibel ausgelegt sein, sodass ein entsprechender Plasmaapplikator flexibel an verschiedene Oberflächen angepasst und angebracht werden kann.

Eine Adhäsionsschicht kann zum Beispiel den Rand einer Umschließung eines Plasmaapplikators bedecken, sodass ein Haftkontakt zwischen einer zu behandelnden Oberfläche und einem Plasmaapplikator nur an diesem Rand hergestellt wird. Eine Adhäsionsschicht kann auch die komplette der zu behandelnden Oberfläche zugewandten Seite eines Plasmaapplikators bedecken, sodass ein flächiger Haftkontakt über die gesamte Kontaktfläche zwischen dem Plasmaapplikator und der zu behandelnden Oberfläche hergestellt wird.

Eine Adhäsionsschicht kann auch löchrig sein oder auch größere Aussparungen, z.B.in Form von Kreisen oder anderen geometrischen Formen aufweisen. Vorteilhafterweise kann bei einer vollflächigen Ausführung einer Adhäsionsschicht, die löchrig ist oder grö-ßere Aussparungen hat, in den Löchern oder Aussparungen ein Plasma zünden. Gleichzeitig stellen Löcher und Aussparungen Möglichkeiten zur Diffusion der Wirkkomponenten eines Plasmas dar. Des Weiteren kann über die Anzahl und Größe der Löcher oder Aussparungen zusätzlich die Haftkraft eingestellt werden.

Für den Fall, dass mehrere Elektrodenstrukturen innerhalb einer Schicht angeordnet sind, kann eine Adhäsionsschicht als negatives Abbild der Verteilung der Elektrodenstrukturen in dieser Schicht ausgebildet sein. Somit wird ein Gasraum, in dem sich ein Plasma verteilen kann, möglichst groß gehalten.

Es kann vorteilhaft sein, wenn eine Adhäsionsschicht ein Gewebe oder ein Textil aufweist oder eine Adhäsionsschicht auf einem Gewebe oder Textil aufgebracht ist.

In einer weiteren Ausführungsform ist eine Adhäsionsschicht auf der der zu behandelnden Oberfläche abgewandten Seite eines Plasmaapplikators aufgebracht. Dies ist besonders von Vorteil, wenn ein Plasmaapplikator mit der der zu behandelnden Oberfläche abgewandten Seite an z.B. Decken, Tüchern oder Thermodecken in der Unfallversorgung fixiert werden soll, um z.B. ein Verrutschen zu verhindern. In diesem Fall entsteht zwischen der Decke, dem Tuch oder der Thermodecke und der zu behandelnden Oberfläche in abgeschlossener Gasraum in dem im Betrieb ein Plasma zündet.

In einerweiteren bevorzugte Ausführungsform ist auf der der zu behandelnden Oberfläche zugewandten Seite eines Plasmaapplikators ein aktiv haftender Teil eines Klettverschlusses mit Widerhaken befestigt (z.B. angeklebt, angenäht). Ein solcher aktiv haftender Teil eines Klettverschlusses kann besonders vorteilhaft für das Aufbringen eines Plasmaapplikators auf einer mit Textil verbundenen bzw. bedeckten Oberfläche (z.B. Kleidung, Wundverband, Fixierverband, Kompressionskleidung) verwendet werden, da die Widerhaken des Klettverschlusses dann am Textil haften und somit eine Fixierung eines Plasmaapplikators über einer Wunde ermöglichen.

### Distanzstruktur

Es kann vorteilhaft sein, wenn ein hier beschriebener Plasmaapplikator an der der zu behandelnden Oberfläche zugewandten Seite eine Distanzstruktur aufweist. Eine Distanzstruktur hat die Funktion, zwischen einem Plasmaapplikator und einer zu behandelnden Oberfläche einen definierten Abstand zu schaffen und somit ein definiertes Gasvolumen, in dem ein Plasma zünden kann.

Eine Distanzstruktur kann sowohl fest als auch nicht-fest mit einem Plasmaapplikator verbunden sein. Eine feste Verbindung kann z.B. durch Klebung und/oder Anspritzung realisiert sein. Bei einer nicht-festen Verbindung besteht keine mechanisch und/oder chemisch feste Verbindung zwischen einem Plasmaapplikator und einer Distanzstruktur.

Da eine Distanzstruktur während einer Plasmabehandlung in direktem Kontakt mit einer Wunde ist, besteht eine Distanzstruktur bevorzugt aus einem biokompatiblen Material (z.B. Silikon, Textil/Silikon-Verbundmatrix, Mull, Absorber, Zellstoff, Wundgaze, PU-Schaum) und/oder aus einer Kombination der genannten Materialien.

Bevorzugt ist eine Distanzstruktur nicht vollflächig ausgebildet. Vorzugsweise ist eine laterale Ausdehnung einer Distanzstruktur vergleichbar mit einer lateralen Ausdehnung einer Elektrodenstruktur eines elektrotechnischen Kerns.

In einer Ausführungsform eines Plasmaapplikators mit einer Distanzstruktur kann die Distanzstruktur Aussparungen aufweisen, in denen ein Plasma zünden kann. Solche Aussparungen können z.B. eine Wabenform haben oder von Löchern mit unterschiedlicher Größe oder von einer Gitterstruktur gebildet sein. In weiteren bevorzugten Ausführungsformen ist eine Distanzstruktur von einer X-, O-, Z-, M-, E- oder W-förmigen Struktur gebildet.

In einer Ausführungsform eines Plasmaapplikators mit einer Distanzstruktur kann die Distanzstruktur auch von dünnen Stegen oder Raupen mit einer Breite von einigen 100µm bis hin zu wenigen mm und mit einer Höhe von einigen 100µm bis hin zu wenigen mm gebildet sein.

Eine Distanzstruktur ist vorzugsweise so ausgebildet, dass sie nur einen vergleichsweise geringen Teil einer der zu behandelnden Oberfläche zugewandten Seite eines Plasmaapplikators bedeckt, sodass ein Plasma über eine vergleichsweise große Fläche verteilt zünden kann.

Eine Distanzstruktur, welche lösbar an einem Plasmaapplikator angeordnet werden kann, kann unabhängig von einem Plasmaapplikator als separates Bauteil ausgeführt sein.

Eine Distanzstruktur, welche lösbar an einem Plasmaapplikator angeordnet werden kann, kann durch ein oder mehrere Klebepads realisiert sein. Diese Klebepads weisen typischerweise eine glatte bzw. ebene Seite auf, welche mit einem Adhäsionsmaterial versehen ist. Diese adhäsive Seite kann auf der einer zu behandelnden Oberfläche zugewandten Seite des Plasmaapplikators aufgebracht werden, wodurch ein Klebepad am Plasmaapplikator haftet.

Ein Klebepad kann unterschiedliche Formen und Größen aufweisen. So kann die Form eine Halbkugel, ein Quader, eine Pyramide, L-, O, M, E, X-förmig oder eine Kombination aus typischen 3-dimensionalen geometrischen Formen darstellen. Der Durchmesser eines Klebepads beträgt vorzugsweise zwischen 1 mm und 6 cm. Die Höhe eines Klebepads beträgt vorzugsweise zwischen 100 µm und 8 mm.

Ein Klebepad kann von Silikon, Filz, Mull, einem ausgehärteten Polymer-Schaum, Textilien, PE, PP, PET bzw. vergleichbaren Materialien sowie aus Materialkombinationen gebildet sein.

Ein Klebepad ist bevorzugt von einem biokompatiblen Material gebildet.

Ein Klebepad kann auch rahmenförmig ausgebildet sein Dabei kann der Innendurchmesser des Rahmens in der Größenordnung der lateralen Ausdehnung einer Elektrodenstruktur eines elektrotechnischen Kerns liegen. Überspannt eine Elektrodenstruktur beispielsweise eine Fläche von 10 cm x 10 cm, so kann der Innendurchmesser des Rahmens ebenfalls im cm-Bereich liegen. Bevorzugt ist der Innendurchmesser jedoch größer als eine Längsseite einer Elektrodenstruktur, also in diesem Fall größer als 10 cm. Die Stegbreite des Rahmens beträgt vorzugsweise zwischen 3 mm und 6 cm. Die Höhe des Rahmens beträgt vorzugsweise zwischen 100 µm und 8 mm.

Eine als Klebepad in Rahmenform ausgeführte Distanzstruktur ist bevorzugt so ausgebildet, dass die Elektrodenstruktur durch die Distanzstruktur nicht bedeckt und die Plasmabildung am Plasmaapplikator nicht durch die aufgebrachte Distanzstruktur behindert wird.

Eine als Klebepad in Rahmenform ausgeführte Distanzstruktur ist vorzugsweise sowohl auf einer der zu behandelnden Seite zugewandten Seite der Distanzstruktur als auch auf der einer der zu behandelnden Seite abgewandten Seite der Distanzstruktur mit einem Adhäsionsmaterial benetzt. Mit der einer der zu behandelnden Seite zugewandten Seite der Distanzstruktur kann die Distanzstruktur für eine Plasmabehandlung auf einer zu behandelnden Oberfläche aufgebracht werden und ein Plasmaapplikator auf der einer der zu behandelnden Seite abgewandten Seite der Distanzstruktur auf der Distanzstruktur angeordnet werden. Die Distanzstruktur kann das Adhäsionsmaterial auch selbst aufweisen oder von diesem gebildet sein. Die Distanzstruktur mit Adhäsionsschicht oder adhäsiven Eigenschaften kann ein von einem Plasmaapplikator unabhängiges Produkt bilden und kann an verschiedenen Plasmaapplikatoren angeordnet und mit diesen für eine Plasmabehandlung verwendet werden.

In einer Ausführungsform eines Plasmaapplikators mit einer Distanzstruktur kann die Distanzstruktur mit einem oder auch mehreren pharmakologischen und/oder nicht-pharmakologischen Wirkstoffen (z.B. Morphinen, Koagulanzien, Cytokinen, Hydrokolloiden) ausgestattet bzw. angereichert sein. Eine Ausstattung in diesem Sinne kann z.B. durch eine Beschichtung der Oberfläche einer Distanzstruktur mit den Wirkstoffen und/oder durch eine Anreicherung des Materials einer Distanzstruktur mit den Wirkstoffen umgesetzt sein. Für eine Beschichtung und/oder Anreicherung des Materials einer Distanzstruktur mit einem oder mehreren pharmakologischen bzw. nicht-pharmakologischen Wirkstoffen kann es vorteilhaft sein, wenn zusätzliche Materialien und/oder Mittel in die Beschichtung und/oder Anreicherung eingemischt werden. Dadurch kann z.B. die Beständigkeit und/oder die Freisetzungskinetik der pharmakologischen bzw. nicht-pharmakologischen Wirkstoffe verzögert (z.B. Retardierung) oder beschleunigt werden.

In einer Ausführungsform eines Plasmaapplikators mit einer Distanzstruktur, kann eine Distanzstruktur als Plasmaquelle ausgebildet sein. Vorteilhafterweise befinden sich dann innerhalb einer Distanzstruktur mindestens eine Elektrodenstruktur, welche im Anwendungsfall mit einem zum Zünden eines Plasmas ausreichendem Spannungssignal beaufschlagt wird. In dieser Konfiguration mit einer Elektrodenstruktur als Bestandteil einer Distanzstruktur stellt eine zu behandelnde Oberfläche im Betrieb eine Gegenelektrode dar.

In einer Ausführungsform eines Plasmaapplikators mit einer Distanzstruktur, kann die Distanzstruktur gleichzeitig eine Elektrodenstruktur sein. Eine solche Elektrodenstruktur erfüllt also zusätzlich die Funktion einer Distanzstruktur. Eine geeignete Elektrodenstruktur kann ein einfacher elektrischer Leiter sein, der von einem Draht mit einem runden oder ovalen Querschnitt gebildet ist. Ein einfacher elektrischer Leiter kann auch von einem Flachkabel gebildet sein. Ein Flachkabel in diesem Sinne kann einen quadratischen oder rechteckigen Querschnitt aufweisen. In diesem Fall ist die Distanzstruktur gleichzeitig eine Plasmaquelle. Auch in dieser Ausführungsform ist eine Gegenelektrode im Betrieb vorzugsweise durch die zu behandelnde Oberfläche selbst realisiert.

In einer Ausführungsform weist ein Plasmaapplikator mit einer als Plasmaquelle ausgebildeten Distanzstruktur, die also entweder eine Elektrodenstruktur umfasst oder selbst eine Elektrodenstruktur ist und zum Zünden eines Plasmas mit einem Spannungssignal beaufschlagt wird, eine weitere Elektrodenstruktur auf. Die weitere Elektrodenstruktur kann z.B. als Flächenelektrode zwischen der Distanzstruktur und dem übrigen Plasmaapplikator angeordnet sein. Eine derart ausgebildete weitere Elektrodenstruktur ist bevorzugt geerdet, sodass zwischen einer als Plasmaquelle ausgebildeten Distanzstruktur und der weiteren Elektrode ein Plasma zünden kann. In einer Variante dieser Ausführungsform ist eine weitere Elektrodenstruktur nicht vollflächig ausgebildet, sondern weist Aussparungen z.B. in Form von Waben, unterschiedlich großen Löchern oder polygonalen Formen auf.

In einer Ausführungsform eines Plasmaapplikators mit einer Distanzstruktur kann die Distanzstruktur mindestens zwei Elektrodenstrukturen aufweisen. Eine bevorzugte Anordnung der Elektrodenstrukturen ist in Schichten übereinander in Bezug zu einer zu behandelnden Oberfläche. Bevorzugt wird eine der Elektrodenstrukturen auf Erdpotential gelegt und mindestens eine weitere der Elektrodenstrukturen wird im Betrieb mit einem zum Zünden eines Plasmas ausreichenden Spannungssignal beaufschlagt. Bevorzugt ist eine geerdete Elektrodenstruktur näher an einer zu behandelnden Oberfläche angeordnet als eine im Betrieb getriebene Elektrodenstruktur. Der Abstand einer im Betrieb mit einem zum Zünden eines Plasmas ausreichendem Spannungssignal beaufschlagten Elektrodenstruktur zu einer zu behandelnden Oberfläche ist also vorzugsweise größer als der Abstand einer geerdeten Elektrodenstruktur zu einer zu behandelnden Oberfläche. Vorzugsweise zündet ein Plasma im Betrieb im Wesentlichen an zwei sich gegenüberliegenden Längsseiten einer Distanzstruktur.

In einer bevorzugten Ausführungsform eines Plasmaapplikators mit einer als Plasmaquelle ausgebildeten Distanzstruktur und einer weiteren Elektrodenstruktur innerhalb und/oder zwischen der Distanzstruktur und dem übrigen Plasmaapplikator, weist der Plasmaapplikator keinen als Mehrschichtsystem ausgebildeten elektrotechnischen Kern auf, da eine Plasmaquelle bereits durch die als Plasmaquelle ausgebildete Distanzstruktur realisiert ist. Ein Plasmaapplikator weist in diesem Fall z.B. eine Umschließung und eine als Plasmaquelle ausgebildete Distanzstruktur auf.

In einer bevorzugten Ausführungsform weist eine als Plasmaquelle ausgebildete Distanzstruktur eine Steckvorrichtung auf. Die Steckvorrichtung kann mit einer Einschubvorrichtung galvanisch verbunden werden, um an eine Elektrodenstruktur der Distanzstruktur ein zum Zünden eines Plasmas ausreichendes Spannungssignal zu übertragen. Eine als Plasmaquelle ausgebildete Distanzstruktur kann eine erste Elektrodenstruktur innerhalb der Distanzstruktur und eine zweite Elektrodenstruktur innerhalb oder außerhalb der Distanzstruktur oder nur eine einzige Elektrodenstruktur innerhalb der Distanzstruktur aufweisen.

In einer bevorzugten Ausführungsform ist eine als Plasmaquelle ausgebildete Distanzstruktur eigenständig ohne weitere Bestandteile eines Plasmaapplikators ausgebildet. Für eine Plasmabehandlung kann dann ein abgeschlossener Gasraum, in dem ein Plasma gezündet werden soll, z.B. durch Auflegen oder Aufkleben einer Folie über der Distanzstruktur realisiert werden. Dadurch kann die Distanzstruktur über der zu behandelnden Oberfläche befestigt werden. Eine als Plasmaquelle ausgebildete Distanzstruktur kann eine erste Elektrodenstruktur innerhalb der Distanzstruktur und eine zweite Elektrodenstruktur innerhalb oder außerhalb der Distanzstruktur oder nur eine einzige Elektrodenstruktur innerhalb der Distanzstruktur aufweisen. Wenigstens eine der Elektrodenstrukturen kann mit einer Steckvorrichtung galvanisch verbunden sein.

### Zugangsanschluss

Ein hier beschriebener Plasmaapplikator kann zusätzlich einen Zugangsanschluss, beispielsweise für eine Absaugvorrichtung, aufweisen. Der Zugangsanschluss ist dann vorzugsweise in diesen Plasmaapplikator integriert. Ein Zugangsanschluss kann für mehrere Funktionen genutzt werden:
- Spülen einer Wunde während ein Plasmaapplikator auf einer zu behandelnden Oberfläche aufgebracht ist,
- Absaugen von evtl. aus einer Wunde austretendem Exsudat während ein Plasmaapplikator auf einer zu behandelnden Oberfläche aufgebracht ist,
- Durchführen einer V.A.C.-Therapie während ein Plasmaapplikator auf einer zu behandelnden Oberfläche aufgebracht ist,
- sowie eine Kombination der oben genannten Funktionen vor, nach und/oder während einer Plasmabehandlung.

Der Zugangsanschluss ist bevorzugt als Tülle oder Nippel ausgebildet, sodass an eine solche Tülle oder einen solchen Nippel ein Schlauch angeschlossen werden kann, welcher z.B. mit einer Vakuumpumpe in Verbindung steht und zwar derart, dass ein durch die Vakuumpumpe erzeugter Unterdruck über den Schlauch an einen Nippel oder eine Tülle an einen Plasmaapplikator geleitet werden kann, wodurch im abgeschlossenen Gasraum zwischen einem Plasmaapplikator und einer zu behandelnden Oberfläche die oben genannten Funktionen (Spülen, Absaugung, V.A.C.-Therapie sowie die Kombination der einzelnen Funktionen untereinander vor, nach und/oder während einer Plasmabehandlung) erfüllt werden können.

Eine Tülle oder ein Nippel ist bevorzugt schlauchförmig und innen hohl ausgeführt. Bevorzugt befindet sich das eine Ende dieser schlauchförmigen Vorrichtung im abgeschlossenen Gasraum zwischen einem Plasmaapplikator und einer zu behandelnden Oberfläche und das andere Ende außerhalb des Plasmaapplikators, sodass, wenn ein Plasmaapplikator auf einer zu behandelnden Oberfläche angeordnet ist, durch die schlauchförmige Tülle ein oder auch mehrere Medien in den abgeschlossenen Gasraum hinzugefügt oder aus dem abgeschlossenen Gasraum abtransportiert werden können.

Eine Tülle oder ein Nippel kann vom Querschnitt her rund, oval, rechteckig oder auch polygonal sein.

In einer bevorzugten Ausführungsform ist ein Innen- und Außendurchmesser einer Tülle bzw. eines Nippels so gewählt, dass ein Schlauch aufgeschoben und an der Tülle befestigt werden kann.

In einer Ausführungsform umfasst eine schlauchförmige Tülle ein männliches Gewinde oder eine weibliche Muffe, um einen Schlauch mit einem komplementären Gewinde zum Hinzufügen und/oder Abtransportieren von Medien in ein bzw. aus einem abgeschlossenen Gasraum über eine Schraubverbindung an der schlauchförmigen Tülle zu befestigen. In einer Variante dieser Ausführungsform wird diese schlauchförmige Tülle durch einen elektrotechnischen Kern geführt. In einem elektrotechnischen Kern kann dafür speziell ein Loch oder eine Aussparung vorgesehen sein, welche vom Durchmesser dem Außendurchmesser der schlauchförmigen Tülle entspricht. In einer Variante wird diese schlauchförmige Tülle durch eine Umschließung geführt. In einer Umschließung kann dafür speziell ein Loch oder eine Aussparung vorgesehen sein, welche vom Durchmesser dem Außendurchmesser der schlauchförmigen Tülle entspricht.

In einer bevorzugten Ausführungsform ist eine Tülle oder ein Nippel in eine Steckvorrichtung integriert. In einer komplementären Einschubvorrichtung befindet sich dann bevorzugt ein Gegenstück zur Tülle, sodass im zusammengefügten Zustand von Steckvorrichtung und Einschubvorrichtung das Gegenstück in der Einschubvorrichtung mit der Tülle in der Steckvorrichtung eine wasser- und luftdichte Verbindung eingeht.

In einer bevorzugten Ausführungsform weisen eine schlauchförmige Tülle bzw. ein schlauchförmiger Nippel ein integriertes Ventil auf, sodass mittels dieses Ventils ein Durchfluss des oder der Medien durch die schlauchförmige Tülle geregelt und gestoppt werden kann. Ein solches Ventil kann manuell, maschinell oder elektronisch regelbar sein.

### Plasmaapplikator mit Sensorik

Bei allen hier beschriebenen oder herkömmlichen Plasmaapplikatoren kann ein wie im Folgenden beschriebener Sensor und gegebenenfalls eine entsprechende Sensorik vorgesehen sein. Lediglich beispielhaft werden im Folgenden einige bevorzugte Ausführungsformen eines Plasmaapplikators mit einem oder auch mehreren, insbesondere unterschiedlichen Sensoren beschrieben.

In einer Ausführungsform umfasst ein Plasmaapplikator einen elektrotechnischen Kern, der als Plasmaquelle ausgebildet ist, eine Umschließung, durch die ein abgeschlossener Gasraum zwischen einem zu behandelnden Körperabschnitt und dem Plasmaapplikator hergestellt werden kann und wenigstens einen Sensor, der ausgebildet ist, für eine Plasmabehandlung und/oder eine Wundheilung relevante Messgrößen, insbesondere physiologische Messgrößen eines im Anwendungsfall von dem Plasmaapplikator bedeckten Körperabschnitts, zu erfassen und auszugeben.

Ein Plasmaapplikator mit einem Sensor kann gerade dann besonders vorteilhaft verwendet werden, wenn ein Plasmaapplikator über einen längeren Zeitraum, beispielsweise von einigen Tagen bis hin zu mehreren Wochen, auf einem zu behandelnden Körperabschnitt verbleiben soll, um diesen gegenüber äußeren Einflüssen zu versiegeln. Wenn zum Beispiel zu Beginn einer Behandlung einer Wunde eine Plasmabehandlung durchgeführt wird, kann es vorteilhaft für eine Wundheilung sein, wenn ein Plasmaapplikator über einen längeren Zeitraum von typischerweise mehreren Tagen bis hin zu mehreren Wochen, beispielsweise bis eine entsprechende Wunde verheilt ist, dauerhaft auf einem entsprechenden Körperabschnitt angebracht ist und die Wunde versiegelt, wodurch vorteilhafterweise eine Rekontamination der Wunde verhindert werden kann. Insbesondere nach einer Plasmabehandlung können dann mittels mindestens einem Sensor für einen abgeschlossenen Gasraum und/oder für eine zu behandelnde Wunde charakteristische Messgrößen erfasst und ausgelesen werden. Ein Erfassen beinhaltet ein Messen einer Messgröße und ein Wandeln in ein die Messgröße repräsentierendes Datensignal. Dadurch kann ein Heilungsverlauf einer Wunde anhand ausgelesener Messgrößen verfolgt werden, ohne dass ein Plasmaapplikator hierzu während eines Heilungsprozesses von einem zu behandelnden Körperabschnitt entfernt werden muss. Eine Wunde kann also während eines Heilungsprozesses dauerhaft versiegelt bleiben.

Der wenigstens eine Sensor ist vorzugsweise ausgebildet, eine Messgröße, beispielsweise einen Gasdruck, eine Temperatur, eine Sauerstoffsättigung des Blutes (SpO2-Wert), Leitwerte eines Wundsekretes, eine Bakterienkolonisation, einen pH-Wert, eine Wundgröße, etc., zu erfassen. Erfasste Messgrößen können dann beispielsweise als die Messgrößen repräsentierende Daten in einem Speicherbaustein einer Sensorik mit wenigstens einem Sensor gespeichert werden. Die Daten können dann zu einem späteren Zeitpunkt von einem externen Lesegerät ausgelesen werden. Beispielsweise kann eine den wenigstens einen Sensor umfassende Sensorik einen RFID (*radio-frequency identification*)-*Transpon*der aufweisen, der auf in einem Speicherbaustein gespeicherte Daten zugreifen und diese an ein Lesegerät übermitteln kann, wenn eine entsprechende Anfrage von einem Lesegerät an den Transponder gestellt wird.

Ein die Messgrößen repräsentierendes Datensignal kann auch, vorzugsweise drahtlos, von einer Sende-Einheit einer Sensorik direkt an eine komplementäre Empfänger-Einheit eines tragbaren oder stationären Datenverarbeitungsgeräts übermittelt wird.

Durch ein Auslesen von von einem Sensor erfassten Messgrößen kann besonders vorteilhaft ein Behandlungserfolg einer Plasmabehandlung und/oder ein Fortschritt einer Wundheilung beurteilt werden, ohne dass hierzu ein auf einem zu behandelnden Körperabschnitt angeordneter Plasmaapplikator von dem zu behandelnden Körperabschnitt entfernt werden muss. Dies ist insbesondere dann vorteilhaft, wenn ein Plasmaapplikator über einen längeren Zeitraum auf einem zu behandelnden Körperabschnitt aufgebracht bleiben sollen. Ein längerer Zeitraum ist vorzugsweise ein Zeitraum von mehreren Tagen oder mehreren Wochen. Ein Zeitraum von mehreren Tagen kann auch aufsummiert einem Zeitraum von mehreren Wochen entsprechen. Bevorzugt umfasst ein längerer Zeitraum einen Zeitraum, in dem eine Wunde wenigstens weitestgehend verheilt ist. Während dieses Zeitraums ist eine zu behandelnde Wunde von einer umgebenden Atmosphäre weitestgehend isoliert. In diesem Zeitraum ist eine Wunde also dauerhaft versiegelt.

Vorteilhafterweise können erfasste Messgrößen beispielsweise direkt von einem Arzt oder Krankenhauspersonal ausgelesen und interpretiert werden. Anhand der ausgelesenen Messgrößen kann dann zum Beispiel die Notwendigkeit einer erneuten Plasmabehandlung beurteilt oder ein geeigneter Zeitpunkt zum Abnehmen eines Plasmaapplikators bestimmt werden.

Es kann auch vorteilhaft sein, wenn erfasste Messgrößen von einem Patienten selbst ausgelesen werden. Beispielsweise kann ein Patient zu Hause die Messgrößen repräsentierende Daten auslesen und über ein Netzwerk einem Arzt zu Verfügung stellen, sodass dieser einen Behandlungserfolg beurteilen kann, ohne einen Patienten persönlich zu konsultieren. Mit einem Plasmaapplikator mit wenigstens einem Sensor wird also besonders vorteilhaft ein Telemonitoring, manchmal auch *home-monitoring* genannt, eines Patienten durch einen Arzt ermöglicht.

Es ist auch denkbar, dass eine Amplitude eines zum Zünden eines Plasmas ausreichenden Spannungssignals in Abhängigkeit von einem Sensor oder mehreren, insbesondere unterschiedlichen Sensoren erfassten Messgrößen geregelt wird. Hierfür können beispielsweise die Messgrößen repräsentierende Daten oder ein die Messgrößen repräsentierendes Datensignal vorzugsweise drahtlos aber auch via Kabel an eine entsprechende Schnittstelle einer Energieversorgungseinheit übertragen werden, sodass eine Amplitude eines bereitzustellenden Spannungssignals entsprechend moduliert werden kann. In einer Ausführungsform weist ein Plasmaapplikator eine elektrische Schaltung auf, die ein zum Zünden eines Plasmas bereitgestelltes Spannungssignal, insbesondere eine Amplitude des Spannungssignals, in Abhängigkeit von wenigstens einer erfassten Messgröße moduliert.

Eine elektrische Schaltung, die ausgebildet ist, ein zum Zünden eines Plasmas bereitgestelltes Spannungssignal, insbesondere eine Amplitude des Spannungssignals, in Abhängigkeit von wenigstens einer erfassten Messgröße zu modulieren, kann auch in eine Energieversorgungseinheit integriert sein. Eine Energieversorgungseinheit kann dann beispielweise ausgebildet sein, ein die erfassten Messgrößen repräsentierendes Datensignal via Kabel oder drahtlos entweder direkt von einem oder mehreren Sensoren eines Plasmaapplikators oder von einem Datenverarbeitungsgerät, auf welchem die erfassten Messgrößen repräsentierende Daten gespeichert sind, zu empfangen und zu verarbeiten und ein entsprechendes Ausgangssignal an die elektrische Schaltung zu übermitteln.

Entsprechend ist eine Energieversorgungseinheit vorgesehen, die ausgebildet ist, ein zum Zünden eines Plasmas ausreichendes Spannungssignal bereitzustellen und eine Schnittstelle zum Empfangen eines die erfassten Messgrößen repräsentierenden Datensignals und eine elektrische Schaltung umfasst, wobei die elektrische Schaltung ausgebildet ist, ein zum Zünden eines Plasmas bereitgestelltes Spannungssignal, insbesondere eine Amplitude des Spannungssignals, in Abhängigkeit von dem empfangenen Datensignal zu modulieren.

Ein Sensor kann beispielsweise ein Gasdrucksensor sein, der ausgebildet und derart angeordnet ist, einen Gasdruck in einem abgeschlossenen Gasraum zu messen oder ein Drucksensor, der ausgebildet und derart angeordnet ist, den Druck eines Kompressionsverbandes zu messen oder ein Temperatursensor sein, der ausgebildet und derart angeordnet ist, eine Temperatur insbesondere in einem abgeschlossenen Gasraum zu messen, oder ein pH-Wert-Sensor sein, der ausgebildet und derart angeordnet ist, einen pH-Wert insbesondere einer Wunde zu messen, ein Feuchtigkeitssensor sein, der ausgebildet und derart angeordnet ist, eine Feuchtigkeit eines Wund-Milieus zu messen, oder ein Stoffwechselprodukt-Sensor sein, der ausgebildet und derart angeordnet ist, insbesondere Stoffwechselprodukte zu erfassen, die charakteristisch für eine Wundheilung sind. Solche Stoffwechselprodukte können beispielsweise Proteine wie Fibrin, oder Lactate sein. Für eine Wundheilung charakteristische Stoffwechselprodukte können auch solche Stoffwechselprodukte sein, die von Bakterien eines Bakterienbelags einer Wunde abgegeben werden.

Eine Sensorik kann auch mehrere, insbesondere unterschiedliche Sensoren aufweisen, und beispielsweise als Mikrosystem (engl. Microelectromechanical system (MEMS)) ausgebildet sein. Solche Mikrosysteme stellen eine kompakte Einheit dar, die besonders vorteilhaft in einen Plasmaapplikator integriert werden kann.

In einer Ausführungsform weist ein Plasmaapplikator eine Sensorik auf, die mehrere, insbesondere unterschiedliche Sensoren umfasst. Die Sensoren sind vorzugsweise ausgebildetjeweils unterschiedliche physiologische Messgrößen eines im Anwendungsfall von dem Plasmaapplikator bedeckten Körperabschnitts zu erfassen und auszugeben. Die mehreren Sensoren einer Sensorik sind vorzugsweise an verschiedenen Stellen an einem Plasmaapplikator angeordnet. Vorzugsweise ist ein Sensor der mehreren Sensoren an einer Stelle angeordnet, die besonders geeignet zum Erfassen einer entsprechenden Messgröße ist. Ein Sensor, der vorgesehen ist, einen Gasdruck zu messen, ist vorzugsweise in einem Abstand zu der Wunde und zu einem Gasraum zugänglich an dem Plasmaapplikator angeordnet. Ein Temperatursensor, der vorgesehen ist Leitwerte eines Wundsekretes zu messen, ist vorzugsweise derart angeordnet, dass er sich im Anwendungsfall in Kontakt mit einer zu behandelnden Wunde befindet.

Eine Sensorik mit mehreren, insbesondere unterschiedlichen Sensoren kann auch als Mikrofluidiksystem, auch lab-on-a-chip-system genannt, ausgebildet sein, welches beispielsweise ausgebildet sein kann, einen Bakterienbelag der Wunde oder Art und Konzentration von Krankheitserregern im Blut oder in einem Wundsekret zu erfassen. In einer Ausführungsform weist ein Plasmaapplikator eine als Mikrofluidiksystem ausgebildete Sensorik auf, wobei die Sensorik derart an dem Plasmaapplikator angeordnet ist, dass sie sich im Anwendungsfall in Kontakt mit einer zu behandelnden Wunde befindet, um beispielsweise Wundsekret oder Blut aufnehmen und analysieren zu können.

Ein Sensor ist vorzugsweise so an dem Plasmaapplikator angeordnet, dass mit dem Sensor für eine Plasmabehandlung und/oder eine Wundheilung relevante insbesondere physiologische Messgrößen erfasst werden können. In einer Ausführungsform ist ein Sensor derart angeordnet, dass dieser sich während einer Plasmabehandlung in einem abgeschlossenen Gasraum in einem Abstand zu einem zu behandelnden Körperabschnitt befindet. Ein derart angeordneter Sensor kann besonders vorteilhaft für den abgeschlossenen Gasraum spezifische Messgrößen, wie Temperatur oder Gasdruck messen. In einer Ausführungsform ist ein Sensor derart angeordnet, dass dieser sich während einer Plasmabehandlung in direktem Kontakt mit einem zu behandelnden Körperabschnitt befindet. Ein derart angeordneter Sensor kann besonders vorteilhaft für den zu behandelnden Körperabschnitt spezifische Messgrößen, wie ein Bakterienbelag oder eine Sauerstoffsättigung der Wunde messen. Es kann auch vorteilhaft sein, einen Sensor derart anzuordnen, dass für einen elektrotechnischen Kern charakteristische Messgrößen erfasst werden können.

In einer Ausführungsform umfasst ein Plasmaapplikator einen elektrotechnischen Kern, eine Umschließung mit Tasche, wobei der elektrotechnische Kern in die Tasche eingeschoben und aus dieser herausgenommen werden kann, und wenigstens einen Sensor, der ausgebildet ist, für eine Plasmabehandlung und/oder eine Wundheilung relevante insbesondere physiologische Messgrößen zu erfassen und auszugeben. Vorzugsweise ist die Umschließung wenigstens teilweise soweit durchsichtig bzw. optisch transparent, dass von einer Person visuell ein Zustand einer Wunde beurteilt werden kann, wenn der Plasmaapplikator auf einem zu behandelnden Körperabschnitt, also über der zu behandelnden Wunde, aufgebracht ist. Es kann dann zunächst eine Plasmabehandlung durchgeführt werden. Nach der Plasmabehandlung kann der elektrotechnische Kern aus der Tasche herausgenommen werden, sodass eine Sicht durch die Umschließung hindurch auf die Wunde ermöglicht wird. Falls eine erneute Plasmabehandlung durchgeführt werden soll, kann ein elektrotechnischer Kern als Modul wieder in die Tasche der Umschließung eingeschoben werden. Dadurch kann ein Fortschritt einer Wundheilung, zusätzlich zu einer Interpretation von von einer Sensorik erfassten Messgrößen, auch visuell beurteilt werden. In einer Ausführungsform weist ein Plasmaapplikator eine Umschließung mit einer Tasche auf, in der der elektrotechnische Kern herausnehmbar angeordnet ist. Die Umschließung ist derart ausgebildet, dass, wenn der elektrotechnische Kern herausgenommen und der Plasmaapplikator auf einem zu behandelnden Körperabschnitt angeordnet ist, der Körperabschnitt durch die Umschließung hindurch erkennbar ist.

Eine visuelle Begutachtung einer Wunde, die sich unter einem aufgebrachten Plasmaapplikator befindet kann auch dadurch ermöglicht werden, dass eine Umschließung eines Plasmaapplikators ein Sichtfenster aufweist, das in demjenigen Bereich der Umschließung angeordnet ist, der es ermöglicht, dass eine Person durch das Sichtfenster einen Zustand einer zu behandelnden Wunde beurteilen kann, ohne dass ein Plasmaapplikator hierzu entfernt werden muss. In einer Ausführungsform eines Plasmaapplikators weist eine Umschließung deshalb ein Sichtfenster auf, das derart angeordnet ist, dass, wenn der Plasmaapplikator auf einem Körperabschnitt angeordnet ist, der Körperabschnitt durch das Sichtfenster erkennbar ist.

In einer Ausführungsform umfasst ein Plasmaapplikator einen elektrotechnischen Kern, eine Umschließung mit einem Sichtfenster, wobei das Sichtfenster derart angeordnet ist, dass ein Körperabschnitt, auf dem der Plasmaapplikator angebracht ist, durch das Sichtfenster visuell begutachtet werden kann, und mindestens einen Sensor, der ausgebildet ist, für eine Plasmabehandlung und/oder eine Wundheilung relevante insbesondere physiologische Messgrößen zu erfassen und auszugeben. Ein Sichtfenster ist vorzugsweise derart in einer Umschließung angeordnet, dass eine Sicht auf eine Wunde nicht durch einen elektrotechnischen Kern versperrt wird. Hierfür kann es vorteilhaft sein, wenn ein elektrotechnischer Kern mittig eine Durchführung aufweist, über der das Sichtfenster angeordnet ist.

In einer Ausführungsform umfasst ein Plasmaapplikator einen elektrotechnischen Kern, eine Umschließung, wenigstens einen Sensor und eine Gel-Schicht, wobei die Gel-Schicht auf der einem zu behandelnden Körperabschnitt zugewandten Seite des Plasmaapplikators angeordnet ist. Wenn ein Plasmaapplikator auf einem zu behandelnden Körperabschnitt angeordnet ist, bewirkt die Gel-Schicht vorzugsweise das ein von dem Plasmaapplikator auf den zu behandelnden Körperabschnitt ausgeübter Auflagedruck gleichmäßig über eine Fläche des zu behandelnden Körperabschnitts verteilt wird. Dies ist besondere dann vorteilhaft, wenn ein Plasmaapplikator über einen längeren Zeitraum auf einem Körperabschnitt verbleiben soll.

In einer Ausführungsform umfasst ein Plasmaapplikator einen elektrotechnischen Kern, eine Umschließung, wenigstens einen Sensor und ein Luftpolster, wobei das Luftpolster auf der einem zu behandelnden Körperabschnitt zugewandten Seite des Plasmaapplikators angeordnet ist. Ein Luftpolster ist bevorzugt ringförmig ausgebildet und ist mit Luft gefüllt. Das ringförmige Luftpolster hat dabei einen inneren Durchmesser, der dem Durchmesser der Öffnung des ringförmigen Luftpolsters entspricht und einen äußeren Durchmesser, der dem Durchmesser des Gesamtumfangs des Luftpolsters entspricht. Der innere Durchmesser entspricht bevorzugt wenigstens einer lateralen Ausdehnung einer Elektrodenstruktur des elektrotechnischen Kerns. Der äußere Durchmesser entspricht vorzugsweise den äußeren Dimensionen des Plasmaapplikators. Wenn ein Plasmaapplikator auf einem zu behandelnden Körperabschnitt angeordnet ist, bewirkt das Luftpolster vorzugsweise, dass ein von dem Plasmaapplikator auf den zu behandelnden Körperabschnitt ausgeübter Auflagedruck gleichmäßig verteilt wird. Dies ist besondere dann vorteilhaft, wenn ein Plasmaapplikator über einen längeren Zeitraum auf einem Körperabschnitt verbleiben soll.

In einer Ausführungsform umfasst ein Plasmaapplikator einen elektrotechnischen Kern, eine Umschließung, wenigstens einen Sensor und eine Schicht, die mit pharmakologisch und/oder nicht-pharmakologisch wirksamen Wirkstoffen angereichert oder beschichtet ist und sich während einer Plasmabehandlung direkt auf dem zu behandelnden Körperabschnitt befindet. Eine solche Schicht kann besonders vorteilhaft durch eine Distanzstruktur realisiert sein. Insbesondere bei einer Versiegelung einer Wunde über einen längeren Zeitraum, vorzugsweise bis zur Heilung einer Wunde, können dann während dieses Zeitraums Wirkstoffe an die zu behandelnde Wunde abgegeben werden, um eine Wundheilung zu unterstützen sowie spezielle und/oder zusätzliche Indikationen zu behandeln.

In einer Ausführungsform umfasst ein Plasmaapplikator einen elektrotechnischen Kern, eine Umschließung, wenigstens einen Sensor und eine Adhäsionsschicht, wobei die Adhäsionsschicht ausgebildet ist, einen Haftkontakt zwischen dem übrigen Plasmaapplikator und einer zu behandelnden Oberfläche über einen Zeitraum von mehreren Tagen, insbesondere bis zu einem Heilungserfolg einer Wunde, zu gewährleisten. Ein solcher Zeitraum kann unter Umständen auch mehrere Wochen umfassen. Vorzugsweise ist die Adhäsionsschicht selbstklebend ausgebildet. Eine selbstklebende Adhäsionsschicht kann beispielsweise von einem geeigneten Klebstoff, z.B. Silikon, gebildet sein. Vorzugsweise stellt eine Adhäsionsschicht einen festen Haftkontakt zwischen einem Plasmaapplikator und einem zu behandelnden Körperabschnitt sicher. Bevorzugt ist eine Adhäsionsschicht derart ausgebildet, dass sie sich in einem bestimmten Zeitraum, vorzugsweise von mehreren Tagen, abbaut oder sich durch Zugabe von Lösungsmitteln, beispielweise Alkohol, löst.

In einer Ausführungsform umfasst ein Plasmaapplikator einen elektrotechnischen Kern, eine Umschließung, wenigstens einen Sensor und einen Zugangsanschluss, der derart angeordnet ist, dass, wenn der Plasmaapplikator auf einem zu behandelnden Körperabschnitt angeordnet ist, ein fluides Medium in einen abgeschlossenen Gasraum zugeführt oder aus dem Gasraum abgeführt werden kann. Wenn ein Plasmaapplikator über einen längeren Zeitraum auf einem zu behandelnden Körperabschnitt verbleibt, kann ein fluides Medium so gezielt in verschiedenen Stadien einer Wundheilung entweder zugeführt oder abgeführt werden, um so eine Wundheilung zu unterstützen. Vorteilhafterweise ermöglicht ein Zugangsanschluss ein Durchführen einer V.A.C-Therapie. Eine V.A.C-Therapie kann beispielsweise durch Flüssigkeitssensoren gesteuert werden. Wenn ein Plasmaapplikator einen Zugangsanschluss aufweist, kann vorteilhafterweise trotz einer Versiegelung eines zu behandelnden Körperabschnitts Exsudat aus dem Gasraum abtransportiert werden. Durch einen Zugangsanschluss ist es vorteilhafterweise möglich, in dem Gasraum ein aerobes oder anaerobes Luftklima herzustellen.

In einer Ausführungsform umfasst ein Plasmaapplikator einen elektrotechnischen Kern, eine Umschließung und wenigstens einen Sensor, wobei die Umschließung von einem wasserabweisenden Material gebildet oder mit einer wasserabweisenden Beschichtung beschichtet ist. Dadurch wird vorteilhafterweise ein Eindringen von Flüssigkeiten in den Gasraum verhindert, während ein Plasmaapplikator über mehrere Tage auf einem zu behandelnden Körperabschnitt angeordnet ist.

Ein Verfahren zur dauerhaften Wundversiegelung einer Wunde mit einem Plasmaapplikator, wobei der Plasmaapplikator eine Umschließung, einen elektrotechnischen Kern und wenigstens einen Sensor aufweist, umfasst wenigstens die folgenden Schritte:
- Aufbringen eines Plasmaapplikators auf einem zu behandelnden Körperabschnitt, sodass zwischen dem Plasmaapplikator und dem zu behandelnden Körperabschnitt ein abgeschlossener Gasraum gebildet wird,
- Durchführen einer Plasmabehandlung, wobei das Durchführen umfasst, dass an eine Elektrodenstruktur des elektrotechnischen Kerns eine zum Zünden eines Plasmas geeignete elektrische Spannung angelegt wird,
- Belassen des Plasmaapplikators auf dem zu behandelnden Körperabschnitt, sodass der abgeschlossene Gasraum über die Plasmabehandlung hinaus bestehen bleibt, und
- während des Belassens des Plasmaapplikators auf dem zu behandelnden Körperabschnitt, Erfassen und Ausgeben einer physiologischen Messgröße eines von dem Plasmaapplikator bedeckten Körperabschnitts mittels dem wenigstens einen Sensor.

Der Schritt des Belassens erstreckt sich vorzugsweise über einen Zeitraum, der einer Zeitdauer der Wundheilung einer zu behandelnden Wunde entspricht. Die Zeitdauer dieses Schrittes kann sich also über mehrere Wochen erstrecken. Während dieser Zeitdauer werden dann für die Wundheilung relevante physiologische Messgrößen mittels einem Sensor erfasst und ausgegeben. Bevorzugt werden einzelne Schritte des Verfahrens während des Belassens mehrfach durchgeführt. So kann es vorteilhaft sein, wenn z.B. während des Belassens mehrmals eine Plasmabehandlung durchgeführt wird.

Für eine Versiegelung eines Körperabschnitts, insbesondere einer Wunde, kann auch ein Plasmaapplikator verwendet werden, der weder einen Sensor noch eine Sensorik aufweist. Ein solches Verfahren umfasst die Schritte:
- Aufbringen eines Plasmaapplikators, der einen elektrotechnischen Kern und eine Umschließung umfasst, auf einem zu behandelnden Körperabschnitt, sodass zwischen dem Plasmaapplikator und dem zu behandelnden Körperabschnitt ein abgeschlossener Gasraum gebildet wird,
- Durchführen einer Plasmabehandlung, wobei das Durchführen umfasst, dass an eine Elektrodenstruktur des elektrotechnischen Kerns eine zum Zünden eines Plasmas geeignete elektrische Spannung angelegt wird,
- Belassen des Plasmaapplikators auf dem zu behandelnden Körperabschnitt, sodass der abgeschlossene Gasraum über die Plasmabehandlung hinaus über mehrere Tage bestehen bleibt.

Ein solches Verfahren kann mit den im Rahmen dieser Beschreibung beschriebenen Plasmaapplikatoren und anderen herkömmlichen Plasmaapplikatoren durchgeführt werden. Durch das Belassen des Plasmaapplikators auf dem zu behandelnden Körperabschnitt wird ein zu behandelnder Körperabschnitt versiegelt und somit gegenüber äußeren Einflüssen isoliert. Das Belassen über einen Zeitraum von mehreren Tagen erstreckt sich insbesondere über einen Zeitraum, in dem eine Wunde weitestgehend abheilt. Vorzugsweise wird ein Plasmaapplikator auf einem zu behandelnden Körperabschnitt angeordnet, eine Plasmabehandlung durchgeführt und der Plasmaapplikator so lange auf dem zu behandelnden Körperabschnitt belassen, bis eine Wunde abgeheilt ist. Ein solcher Zeitraum umfasst typischerweise mehrere Tage, die in der Summe auch einen Zeitraum von mehreren Wochen ergeben können.

### Zünden eines Plasmas

Zum Zünden des Plasmas wird im Betrieb mittels einer Energieversorgungseinheit ein zum Zünden eines Plasmas ausreichendes Spannungssignal an wenigstens eine Elektrodenstruktur eines elektrotechnischen Kerns bereitgestellt.

Bevorzugt ist eine Energieversorgungseinheit ausgebildet, ein zum Zünden eines Plasmas ausreichendes Spannungssignal als eine Rechteck-, eine Sägezahn- oder eine Sinusspannung bereitzustellen. Bevorzugt ist eine Energieversorgungseinheit ausgebildet, einzelne repetitive Pulse bereitzustellen, beispielsweise kann eine Wechselspannung gepulst bereitgestellt werden.

Es kann für eine Plasmabehandlung vorteilhaft sein, wenn eine Sinusspannung mit 9 kV Spitze-Spitze, gepulst mit 20 µs an, 180 µs aus, 5x pro Sekunde (5 Hz) bereitgestellt wird. Vorteilhafterweise kann so die Temperatur eines gezündeten Plasmas niedrig gehalten werden. Vorteilhafterweise wird ein Plasma während einer Gesamtzeit von ungefähr 10% der Behandlungszeit gezündet.

In alternativen Varianten kann es vorgesehen sein, ein Spannungssignal von wenigen 100 V bis 5 kV Spitze-Spitze zu verwenden. In weiteren Varianten kann es vorgesehen sein, ein anderes Pulsmuster vorzusehen. Beispielsweise kann es vorteilhaft sein eine Anzahl kurzer Pulse zu verwenden, um eine entsprechende Konzentration einer Wirkspezies zu erreichen und nach der Folge kurzer Pulse zunächst für einige Sekunden kein Plasma mehr zu zünden. Eine solche Plasmabehandlung kann bei besonderen Krankheitsbildern zu einem verbesserten Behandlungsergebnis führen. Gleichzeitig können über die Dauer der Pulse und über die Pausen zwischen den Pulsen der Energieverbrauch gezielt eingestellt werden. Besonders für den Betrieb mit einer mobilen Energieversorgungseinheit ist eine kurze Zündung eines Plasmas mit vergleichsweise langer Pause vorteilhaft, da der Energiebedarf dadurch deutlich sinkt und eine längere Behandlungsdauer mit einem vergleichsweise kleinen Energiespeicher ermöglicht wird.

Falls eine erste Elektrodenstruktur in einem elektrotechnischen Kern vorgesehen ist, liegt diese dann vorzugsweise auf Erd- oder Massenpotential und bildet damit einen elektrischen Gegenpol für die mit dem Spannungssignal getriebene, zweite Elektrodenstruktur.

Zwischen den beiden Elektrodenstrukturen oder wenigstens der zweiten Elektrodenstruktur und der zu behandelnden Oberfläche, liegt dann ein elektrisches Feld an, wobei ein Kurzschluss zwischen den Elektrodenstrukturen durch die zwischen den Elektrodenstrukturen angeordnete Isolationsschicht verhindert oder unterbunden wird. Stattdessen bildet sich ein großflächiges, dielektrisch behindertes Plasma aus. Da die Plasmaeigenschaften stark von der Gasraumdicke, insbesondere von dem Gasvolumen zwischen einer geerdeten Elektrodenstruktur und einer zu behandelnden Oberfläche, insbesondere einer menschlichen oder tierischen Oberfläche, abhängen, kann eine Distanzstruktur vorgesehen sein, welche ein zuverlässiges und reproduzierbares Bereitstellen einer ausreichenden Gasmenge im abgeschlossenen Gasraum zwischen Plasmaapplikator und zu behandelnder Oberfläche für das Erzeugen eines Plasmas mit stets gleichen Wirkeigenschaften erlaubt. Ein zu ionisierendes Gas oder Gasgemisch ist dabei ein zugeführtes Arbeitsgas und/oder die Umgebungs- oder Außenluft.

Ausführungsbeispiele der Erfindung werden nachfolgend anhand der Figuren beschrieben. Diese sollen die Ausführungsbeispiele nicht notwendigerweise maßstäblich darstellen, vielmehr sind die Figuren in schematischer und/oder leicht verzerrter Form ausgeführt. Die in der Beschreibung, in den Figuren sowie in den Ansprüchen offenbarten Merkmale können sowohl einzeln als auch in beliebiger Kombination für die Realisierung der Erfindung wesentlich sein. Hierbei sind identische und/oder ähnliche Merkmale mit identischer oder ähnlicher Funktion, dort wo sinnvoll, mit gleichen Bezugszeichen versehen. Weitere Vorteile, Merkmale und Einzelheiten der Erfindung ergeben sich aus der nachfolgenden Beschreibung der bevorzugten Ausführungsbeispiele sowie anhand der Figuren.

Im Einzelnen zeigt:
- Fig. 1:: einen Plasmaapplikator mit einer Steckvorrichtung, die mit einer Einschubvorrichtung zusammengeführt ist,
- Fig. 2A:: eine Seitenansicht auf einen Schnitt entlang der Breite durch eine mit einer Einschubvorrichtung zusammengeführte Steckvorrichtung,
- Fig. 2B:: eine Seitenansicht auf einen Schnitt entlang der Höhe durch eine mit einer Einschubvorrichtung zusammengeführte Steckvorrichtung,
- Fig. 3:: eine Seitenansicht auf einen Schnitt entlang der Breite einer Steckvorrichtung in Form eines Steckers, die elektrisch mit einer Elektrodenstruktur verbunden ist,
- Fig. 4:: einen Mechanismus für eine sichere Verbindung zwischen einer Steckvorrichtung und einer Einschubvorrichtung,
- Fig. 5A:: einen Plasmaapplikator mit einem elektrotechnischen Kern und mit einer Steckvorrichtung, die mit einer Einschubvorrichtung zusammengeführt ist,
- Fig. 5B:: eine Seitenansicht auf einen Schnitt entlang der Breite durch die mit der Einschubvorrichtung zusammengeführte Steckvorrichtung,
- Fig. 6:: eine Steckvorrichtung im Chipkartenformat mit einer Verjüngung in einer Leiterbahn,
- Fig. 7:: eine Explosionsdarstellung einer bekannten Vorrichtung zur Erzeugung eines kalten Atmosphärendruckplasmas für die Behandlung von Oberflächen,
- Fig. 8A: einen Plasmaapplikator, der mit einer mobilen Energieversorgungseinheit mit einer Einschubvorrichtung zusammengeführt ist,
- Fig. 8B: einen Plasmaapplikator mit einer integrierten mobilen Energieversorgungseinheit und einer Steckvorrichtung,
- Fig. 8C: einen Plasmaapplikator mit einer integrierten mobilen Energieversorgungseinheit ohne eine Steckvorrichtung,
- Fig. 8D: einen Plasmaapplikator mit einem Einschubschlitz für eine mobile Energieversorgungseinheit,
- Fig. 8E: einen Plasmaapplikator mit einer integrierten Empfangs-Spulenanordnung und mit einem Einschubschlitz in den eine mobile Energieversorgungseinheit mit einer Sende-Spulenanordnung geschoben werden kann,
- Fig. 8F: einen Plasmaapplikator mit einer integrierten Energieversorgungseinheit mit einem Akkumulator, der mittels einer ebenfalls intergierten Ladevorrichtung induktiv geladen werden kann,
- Fig. 9: einen Plasmaapplikator mit skalierbarer Behandlungsfläche,
- Fig. 10A: eine Distanzstruktur, die gleichzeitig als Plasmaquelle zur Erzeugung einer dielektrisch behinderten Entladung (DBE) ausgebildet ist,
- Fig. 10B: einen Querschnitt der in Fig. 10A gezeigten Distanzstruktur,
- Fig. 11: einen geschlossenen Stromkreis bestehend aus einem zweiadrigen Kabel,
- Fig. 12: eine Draufsicht auf die patientenzugewandte Seite eines elektrotechnischen Kerns,
- Fig. 13: eine Draufsicht auf die patientenabgewandte Seite des in Fig. 12 gezeigten elektrotechnischen Kerns,
- Fig. 14: eine Draufsicht auf die patientenabgewandte Seite des in Fig. 13 gezeigten elektrotechnischen Kerns,
- Fig. 15: eine Einschubvorrichtung mit einem Spannungsanschluss und einem Masseanschluss,
- Fig. 16: angedeutet, wie ein mechanisch fester Sitz zwischen einer Steckvorrichtung und einer durch Einrastelemente angedeutete Einschubvorrichtung hergestellt werden,
- Fig. 17: einen elektrotechnischen Kern der in eine Saugkompresse geschoben ist,
- Fig. 18: einen elektrotechnischen Kern, der in eine bereits vorhandene Lasche eines Verbands eingeschoben ist,
- Fig. 19: einen Querschnitt durch einen Plasmaapplikator mit einem elektrotechnischen Kern mit jeweils drei Elektrodenstrukturen und drei Isolationsschichten,
- Fig. 20: eine Draufsicht auf die Seite eines elektrotechnischen Kerns, die während einer Plasmabehandlung von der zu behandelnden Oberfläche abgewandt ist,
- Fig. 21: eine Draufsicht auf die Seite eines elektrotechnischen Kerns, die während einer Plasmabehandlung von der zu behandelnden Oberfläche abgewandt ist, wobei insbesondere die dritte Isolationsschicht zu sehen ist,
- Fig. 22: eine Draufsicht auf die Seite eines elektrotechnischen Kerns gezeigt, die während einer Plasmabehandlung von der zu behandelnden Oberfläche abgewandt ist, wobei insbesondere die dritte Elektrodenstruktur zu sehen ist,
- Fig. 23: eine Draufsicht auf die Seite eines elektrotechnischen Kerns gezeigt, die während einer Plasmabehandlung von der zu behandelnden Oberfläche abgewandt ist, wobei der elektrotechnische Kern eine dritte Elektrodenstruktur und eine Versteifung umfasst,
- Fig. 24: eine Steckvorrichtung, die mit einer Einschubvorrichtung zusammengesteckt ist,
- Fig. 25: einen Plasmaapplikator mit einem elektrotechnischen Kern, einer Umschließung und einem Zugangsanschluss,
- Fig. 26: eine Steckvorrichtung mit einem Zugangsanschluss und eine zu der Steckvorrichtung komplementär ausgebildete Einschubvorrichtung,
- Fig. 27: eine Steckvorrichtung mit einem Zugangsanschluss und eine zu der Steckvorrichtung komplementär ausgebildete Einschubvorrichtung,
- Fig. 28: einen Plasmaapplikator, der eine Umschließung, einen elektrotechnischen Kern und eine Steckvorrichtung mit einem Zugangsanschluss aufweist,
- Fig. 29: einen Plasmaapplikator mit einer Sensorik,
- Fig. 30A: einen Plasmaapplikator mit einem elektrotechnischen Kern, der über sein Fläche verteilt eine Anzahl Durchführungen aufweist,
- Fig. 30B: einen vergrößert dargestellten Teilbereich des in Fig. 30A gezeigten Plasmaapplikators,
- Fig. 31: einen Plasmaapplikator mit einem elektrotechnischen Kern und einer Steckvorrichtung, wobei an dem Übergang von elektrotechnischem Kern und Steckvorrichtung eine Perforation ausgebildet ist,
- Fig. 32: einen an einem Beutel befestigten Plasmaapplikator,
- Fig. 33: einen an einem Beutel befestigten Plasmaapplikator, wobei der Plasmaapplikator über einem Loch in dem Beutel angeordnet ist.

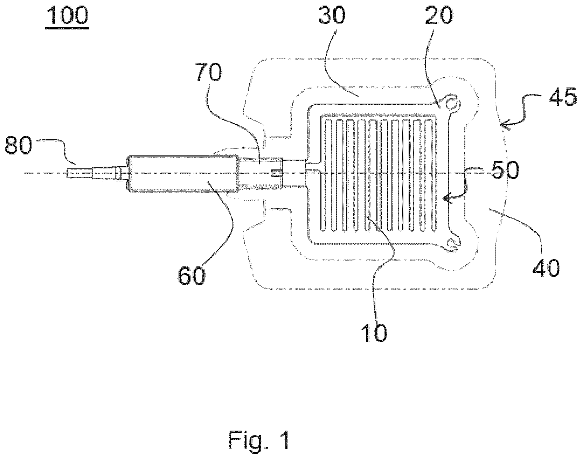

**Figur 1** zeigt eine bevorzugte Ausführungsform eines Plasmaapplikators 100 mit einem elektrotechnischen Kern 50 und mit einer Steckvorrichtung 70, die als chipkartenähnlicher Stecker ausgebildet ist. Die Steckvorrichtung 70 ist mit einer als eine komplementäre Aufnahmebuchse ausgebildeten Einschubvorrichtung 60 zusammengeführt. Die Einschubvorrichtung 60 und die Steckvorrichtung 70 weisen elektrisch leitfähige Leiterbahnen auf, die im zusammengeführten Zustand an entsprechenden Kontaktflächen galvanisch gekoppelt sind. Insbesondere weist die Leiterbahn der Steckvorrichtung 70 einen Leiter auf, der von einer Kontaktfläche in der Steckvorrichtung zu wenigstens einer Elektrodenstruktur 10 führt.

Die Elektrodenstruktur 10 ist Teil des elektrotechnischen Kerns 50, der in der gezeigten Ausführungsform weiterhin eine Isolationsschicht 20 umfasst. Der Rand der Isolationsschicht 20 überragt die Elektrodenstruktur 10 lateral um die Länge der Kriechstrecken bei einer für die Anwendung typischen Spannung.

In der gezeigten Ausführungsform besteht die Elektrodenstruktur 10 aus einem Silberleitlack, welcher Kamm-förmig ausgebildet ist. In verschiedenen Varianten der gezeigten Ausführungsform kann eine Elektrodenstruktur auch in Form von dünnen Metallschichten, - folien, -gittern und/oder mit leitfähigen Polymerschichten gebildet sein. Denkbar ist auch eine Variante, in der eine Elektrodenstruktur eines entsprechenden elektrotechnischen Kerns durch elektrisch leitfähige Fäden, die in ein Textil eingewoben sind, gebildet ist. In einer weiteren Variante ist eine Elektrodenstruktur eines entsprechenden elektrotechnischen Kerns als elektrisch leitfähige Struktur aus einem leitfähigen, flexiblen Material gebildet, wie zum Beispiel einem leitfähigen Kunststoff, einem mit leitfähigen Partikeln angereicherten Material, einer metallischen Folie oder Graphit.

Die Elektrodenstruktur 10 der gezeigten Ausführungsform ist elektrisch mit einer Leiterbahn der Steckvorrichtung 70 verbunden. Die Einschubvorrichtung 60 ist mit einem Kabel 80 verbunden. An dem anderen Ende des Kabels 80, ist das Kabel 80 typischerweise mit einer vorwiegend stationär genutzten Energieversorgungseinheit (nicht gezeigt), wie z.B. einem Hochspannungsgenerator verbunden. Die Energieversorgungseinheit (nicht gezeigt) stellt ein zum Zünden eines Plasmas ausreichendes Spannungssignal bereit und kann ein Steuergerät und ein Lesegerät für digitale Daten umfassen. Zum Betrieb wird die Steckvorrichtung 70 des Plasmaapplikators 100 mit der Einschubvorrichtung 60 zusammengeführt. Ein durch die Energieversorgungseinheit (nicht gezeigt) bereitgestelltes Spannungssignal wird im Betrieb des Plasmaapplikators 100 über das Kabel 80 und die mit der Einschubvorrichtung 60 zusammengeführte Steckvorrichtung 70 an die Elektrodenstruktur 10 zum Zünden eines Plasmas übertragen.

Die Elektrodenstruktur 10 des gezeigten elektrotechnischen Kerns hat die Funktion einer durch das Spannungssignal getrieben Elektrodenstruktur und ist bevorzugt flexibel ausgebildet. Typischerweise wird eine weitere Elektrodenstruktur benötigt, die die Funktion einer Massenelektrode hat. In der gezeigten Ausführungsform weist der elektrotechnische Kern 50 nur eine Elektrodenstruktur 10 auf und die Gegenelektrode ist, wenn der Plasmaapplikator an oder auf einer menschlichen oder tierischen oder technischen Oberfläche angebracht ist, durch den menschlichen oder tierischen Körper oder die technische Oberfläche selbst realisiert. In einer Variante des gezeigten Ausführungsbeispiels ist die Gegenelektrode als weitere Elektrodenstruktur Bestandteil des flexiblen, flächigen elektrotechnischen Kerns und befindet sich an der der zu behandelnden Oberfläche zugewandten Seite. In dieser Variante bestehen die getriebene Elektrodenstruktur und die geerdete Elektrodenstruktur aus dem gleichen Material und haben die gleiche spezielle Geometrie. Allerdings sind die Elektrodenabschnitte der getriebenen Elektrodenstruktur und der geerdeten Elektrodenstruktur zueinander mit einer definierten Überlappung versetzt angeordnet. Ein Elektrodenabschnitt einer entsprechenden Elektrodenstruktur hat in der gezeigten Ausführungsform und in den beschriebenen Varianten bevorzugt eine Breite von 5 mm und eine Dicke von 14 µm.

Als relevante Größe hat sich die Querschnittsform der Elektrodenabschnitte einer Elektrodenstruktur herausgestellt. Die Leitfähigkeit in Kombination mit einer Querschnittsform der Elektrodenabschnitte einer Elektrodenstruktur ist bevorzugt derart bemessen, dass eine einen jeweiligen Elektrodenabschnitt bildende Leiterbahn einen Widerstand im einstelligen Ohm-Bereich aufweist. Daraus resultiert, dass zwischen dem Anfang einer Leiterbahn einer Elektrodenstruktur und dem Ende nur wenige Volt Spannung abfallen und somit eine homogene Entladung über die komplette Fläche einer Elektrodenstruktur bereitgestellt werden kann. Bevorzugt ist derzeit ein Widerstand in der Elektrodenstruktur, der 2 Ohm beträgt. Denkbar sind auch höhere Widerstandswerte beispielsweise bis 50 Ohm. Bei höheren Widerständen ist jedoch ein großer Spannungsabfall zu beobachten und die Elektrodenstrukturen erwärmen sich deutlich.

Für manche Anwendungen kann es aber auch vorteilhaft sein, wenn ein Elektrodenabschnitt einer Elektrodenstruktur eines elektrotechnischen Kerns eine Breite von 1 mm und eine Dicke von 70 µm hat. Für wiederum andere Anwendungen kann es vorteilhaft sein, wenn ein Elektrodenabschnitt einer Elektrodenstruktur eines elektrotechnischen Kerns eine Breite von 10 mm und eine Dicke von 7 µm aufweist.

Zum Erzeugen eines flächigen Plasmas, insbesondere eines kalten Plasmas, befindet sich in dem elektrotechnischen Kern 50 Isolationsschicht 20, die zwischen der getriebenen Elektrodenstruktur 10 und der zu behandelnden Oberfläche angeordnet ist.

In der gezeigten Ausführungsform besteht die Isolationsschicht 20 aus einem elektrisch nicht-leitfähigen Kunststoff. Die Isolationsschicht 20 kann aber auch aus einer Keramik oder einem Kunststoff-Keramik-Gemisch oder aus einem Naturfaserverbund oder auch anderen natürlichen Materialien bestehen. Bevorzugt hat die Isolationsschicht 20 eine Dicke, die zwischen wenigen µm bis hin zu wenigen 100 µm beträgt. Die Isolationsschicht 20 ist bevorzugt porenfrei, d.h. sie hat keine oder nur sehr wenige Löcher oder Hohlräume. Die Isolationsschicht 20 hat weiterhin eine Spannungsfestigkeit von wenigstens 5 kV pro mm Dicke. Die laterale Ausdehnung der Isolationsschicht 20 entspricht der Dimension der Elektrodenstruktur 10 im elektrotechnischen Kern 50 zuzüglich einem darüber hinausragenden Rand, wobei der Rand so dimensioniert ist, dass er wenigstens die Länge der Kriechstrecken bei typischen angewandten Spannungswerten zum Zünder des Plasmas abdeckt.

In einer nicht gezeigten Variante der hier gezeigten Ausführungsform ist die laterale Ausdehnung einer Isolationsschicht derart gewählt, dass es zwischen einer im Anwendungsfall getriebenen Elektrodenstruktur und einer weiteren auf Erdpotential liegenden Elektrodenstruktur oder der zu behandelnden Oberfläche zu keiner Bogenentladung kommt. Typischerweise können durch den Einsatz spezieller Isolationsmechanismen (z.B. Umspritzung) die Kriechstrecken unterschritten werden, ohne dass es zu einem Fehlerfall kommt. Je nach Umschließung eines elektrotechnischen Kerns kann also eine laterale Ausdehnung einer Isolationsschicht auch dahingehend ausgelegt sein, dass der über eine Elektrodenstruktur hinausragende Rand der Isolationsschicht kleiner ist, als die zum Zünden eines Plasmas notwendige Amplitude des Spannungssignals als Kriechstrecke vorgibt.

In einer weiteren nicht gezeigten Variante der gezeigten Ausführungsform, in der der elektrotechnische Kern eine erste und eine zweite Elektrodenstruktur aufweist, weist der elektrotechnische Kern bevorzugt eine weitere Isolationsschicht auf, die zwischen der geerdeten Elektrodenstruktur und der zu behandelnden Oberfläche angeordnet ist. Die weitere Isolationsschicht besteht bevorzugt aus einem biokompatiblen Material wie z.B. Lack, Silikon, Polyurethan oder einer Beschichtung. Die Beschichtung kann mittels plasmaunterstützte chemische Gasphasenabscheidung (PACVD), chemische Gasphasenabscheidung (CVD), Eloxal-Verfahren oder mittels Galvanotechnik aufgebracht werden.

In dem gezeigten Ausführungsbeispiel ist der Plasmaapplikator 100 teilweise mit einem biokompatiblen Material 45, wie z.B. medizinischem Silikon oder einem Lack, umschlossen. Hierbei ist die Unterseite des elektrotechnischen Kerns 50, also die der zu behandelnden Oberfläche zugewandte Seite, nicht umschlossen und die Oberseite des elektrotechnischen Kerns 50, also die der zu behandelnden Oberfläche abgewandte Seite, komplett umschlossen. Die Umschließung ist so ausgeführt, dass mindestens eine Durchschlagfestigkeit zwischen der getriebenen Elektrodenstruktur 10 und einem direkt auf der Außenseite anliegenden Erdpotenzial gewährleistet ist.

Die Steckvorrichtung 70 ist ebenfalls teilweise umschlossen. Insbesondere ist die Umschließung der Steckvorrichtung 70 und des elektrotechnischen Kerns 50 formschlüssig und ohne Lufteinschlüsse. Um eine galvanische Kopplung zwischen der Steckvorrichtung 70 und der Einschubvorrichtung 60 zu ermöglichen, sind die elektrischen Kontaktflächen der Steckvorrichtung 70 frei für die elektrischen Kontaktflächen der Einschubvorrichtung 60 zugänglich, also nicht umschlossen.

Entlang des Randes der Umschließung 45 ist in der gezeigten Ausführungsform auf der der zu behandelnden Oberfläche zugewandten Seite eine Adhäsionsschicht 40 aufgebracht. Durch die Adhäsionsschicht 40 kann der Plasmaapplikator 100 auf einer zu behandelnden menschlichen oder tierischen oder technischen Oberfläche fixiert werden. Die Adhäsionsschicht 40 besteht bevorzugt aus einem biokompatiblen Material, wie zum Beispiel Silikon oder einem Klebstoff auf Acrylatbasis und hat eine bevorzugte Dicke, die zwischen wenigen µm und einigen hundert µm beträgt. Wenn der Plasmaapplikator mittels einer Adhäsionsschicht an einer zu behandelnden Oberfläche angebracht ist, erzeugt die Adhäsionsschicht eine Adhäsionskraft, die ausreicht, dass der Plasmaapplikator ohne zusätzliche Hilfsmittel an der zu behandelnden Oberfläche haftet. Die Adhäsionsschicht kann zum Beispiel im Siebdruckverfahren oder im Spritzgussverfahren aufgebracht werden. Es ist auch denkbar, dass die Adhäsionsschicht durch ein Transferklebeband oder durch ein doppelseitiges Klebeband realisiert ist. Das Transfer- bzw. doppelseitige Klebeband kann elastisch und somit flexibel ausgelegt sein, sodass ein entsprechender Plasmaapplikator flexibel an verschiedene Oberflächen angepasst und angebracht werden kann.

Zum Zünden des Plasmas wird an die getriebene Elektrodenstruktur des elektrotechnischen Kerns ein Spannungssignal angelegt. Falls eine weitere Elektrodenstruktur in dem elektrotechnischen Kern vorgesehen ist, liegt diese dann auf Erd- oder Massenpotential und bildet damit eine Gegenelektrode für die im Betrieb durch ein Spannungssignal getriebene Elektrodenstruktur. Zwischen den beiden Elektrodenstrukturen oder der im Betrieb getriebenen Elektrodenstruktur und der zu behandelnden Oberfläche liegt dann ein elektrisches Feld, wobei ein Kurzschluss zwischen den Elektrodenstrukturen durch die Isolationsschicht zwischen den beiden Elektrodenstrukturen verhindert oder unterbunden wird. Stattdessen bildet sich ein großflächiges, dielektrisch behindertes Plasma aus.

In einer hier nicht gezeigten Ausführungsform weist ein elektrotechnischer Kern eine im Betrieb getriebene Elektrodenstruktur und eine Gegenelektrode auf, wobei die Gegenelektrode ausgebildet ist, im Betrieb mittels einer an die Gegenelektrode angelegten Gleichspannung einen Spannungsoffset zu erzeugen und geladene Teilchen aus einem Plasma zu einer Wunde hin zu beschleunigen. In einer hier nicht gezeigten Ausführungsform ist eine Gegenelektrode ausgebildet, eine mittels einer Gleichspannung einen Spannungsoffset zu erzeugen. Die Gegenelektrode ist in diesem Fall dazu ausgebildet, an eine entsprechende Spannungsquelle abgeschlossen zu werden.

Der Plasmaapplikator 100 - wenn im Anwendungsfall auf einer Oberfläche aufgebracht - definiert einen abgeschlossenen Raum, den Behandlungsbereich 30, in dem ein Plasma erzeugt wird. Der Behandlungsbereich 30 ist vorteilhafterweise Luftdicht abgeschlossen. Vorzugsweise befindet sich der Behandlungsbereich 30 in einem Abstand von einigen Millimetern zu der zu behandelnden Oberfläche, sodass sich ein kaltes Plasma flächig über der zu behandelnden menschlichen oder tierischen oder technischen Oberfläche verteilt. Eine typische Zeitdauer einer Plasmabehandlung beträgt hierbei wenige Minuten.

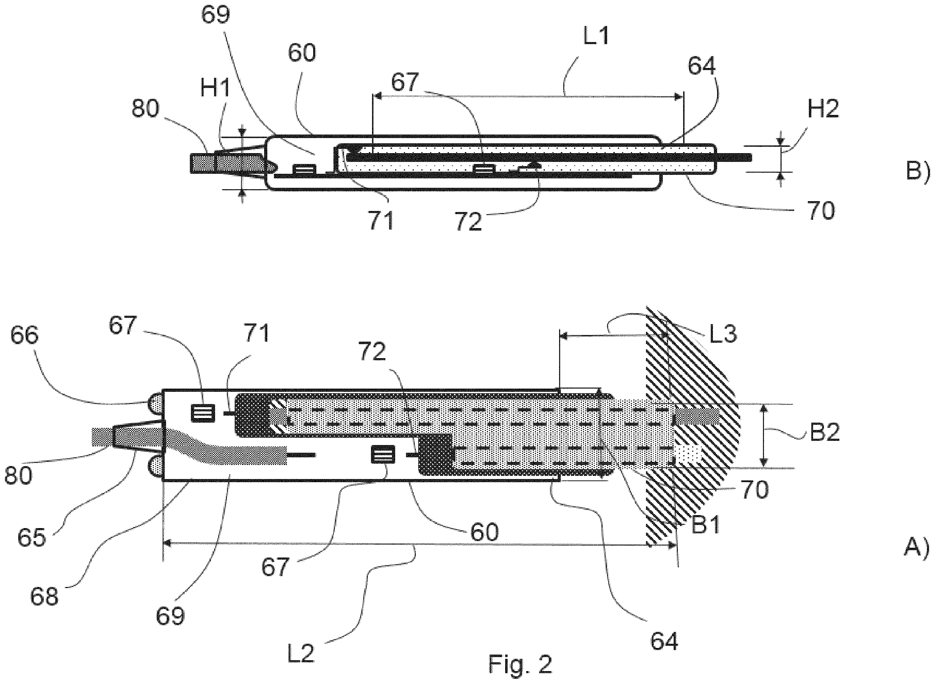

**Figur 2A** zeigt eine bevorzugte Ausführungsform einer Steckvorrichtung 70, die als Stecker ausgebildet ist, und mit einer als Aufnahmebuchse ausgebildeten Einschubvorrichtung 60 zusammengeführt ist. In der gezeigten Darstellung ist eine Seitenansicht auf einen Schnitt entlang der Breite durch eine mit einer Einschubvorrichtung zusammengeführten Steckvorrichtung dargestellt.

Die Einschubvorrichtung 60 hat eine Breite B1 von 30 mm. Über einen Anschluss ist ein Kabel 80 mit der Einschubvorrichtung 60 verbunden. An dem anderen Ende des Kabels 80 kann eine Energieversorgungseinheit (nicht gezeigt) angeschlossen sein, die im Betrieb das Spannungssignal zum Zünden eines Plasmas bereitstellt. Der Anschluss 65 weist eine isolierende Struktur aus Polyethylen auf, die außen mit einer elektromagnetischen Abschirmung (EMV-Schirm) versehen ist. Mittels der Abschirmung kann ein verstärktes Aussenden von Störwellen unterbunden werden, die potentiell andere elektrische Geräte z.B. in einem Krankenhaus stören könnten. Weiterhin kann mithilfe dieser Abschirmung die Empfindlichkeit gegen äußere Störeinflüsse von anderen Strahlquellen minimiert werden.

In verschiedenen Varianten kann die isolierende Struktur aber auch aus anderen flexiblen und porenfreien Isolatoren (z.B. Kunststoffen oder Keramiken) bestehen. Das Kabel 80 ist mit einer Silikontülle ummantelt. Die Einschubvorrichtung 60 weist weiterhin Verschlussstopfen 66 auf, die dafür vorgesehen sind, das Gehäuse mit einem Isolator (z.B. Epoxid oder Silikon) komplett auszufüllen. Hierbei ist ein erster Verschlussstopfen 66 als Eingang für das Epoxid oder das Silikon vorgesehen und ein zweiter Verschlussstopfen 66 als Luftausgang, damit Luft während des Füllens aus dem Gehäuse entweichen kann.

Weiterhin sind Induktivitäten 67 vorgesehen, die als Filter für spezielle Störfrequenzen dienen. Für eine vergleichsweise gute EMV-Schirmung ist das Gehäuse 68 der Einschubvorrichtung 60 metallisiert. Das gesamte Gehäuse 68 besteht bevorzugt aus einem elektrisch leitfähigen Material. Alternativ kann das Gehäuse 68 auch innen z.B. metallisiert oder mit einem Gitter geschirmt sein. In beiden Varianten wird die Abschirmung des Gehäuses 68 auf PE gelegt und somit das Gehäuse 68 elektrisch abgeschirmt (faradayscher Käfig).

Der Innenraum 69 der Einschubvorrichtung 60 ist mit Silikon oder einem anderen Material mit hoher Durchschlagsfestigkeit (z.B. Epoxidharz) vergossen, um eine Spannungsfestigkeit zu gewährleisten und Teilentladungen zu vermeiden. Ein weiterer Vorteil ist, dass die mechanischen und elektrischen Komponenten in der Einschubvorrichtung klein und kompakt gebaut werden können. Des Weiteren wird ein Eindringen von Feuchtigkeit z.B. beim Dampfsterilisieren verhindert.

Die gezeigte Einschubvorrichtung 60 weist einen Hochspannungsanschluss (HV-Anschluss) 71 und einen Masseanschluss (GND-Anschluss) 72 auf. Die Breite B2 der Steckvorrichtung beträgt in der hier gezeigten Variante 24 mm. Bei vollständiger Aufnahme der Steckvorrichtung 70 in die Einschubvorrichtung 60 beträgt die Länge L2 des Systems aus den beiden zusammengesteckten Vorrichtungen in der hier gezeigten Variante 124 mm. Dabei ragt die Steckvorrichtung 70 in der hier gezeigten Variante mit einer Länge L3 von 36 mm aus der Einschubvorrichtung 60. Der Teil der Steckvorrichtung 70, der aus der Einschubvorrichtung herausragt, kann vorzugsweise mit der Umschließung, z.B. aus Silikon umschlossen sein und dient dann zur Befestigung der Steckvorrichtung 70 an einem elektrotechnischen Kern (nicht gezeigt).

In einer nicht gezeigten Ausführungsform hat eine Steckvorrichtung eine runde Form. Auch andere Steckerformen sind denkbar, wobei ein Vermeiden von Teilentladungen und in der Regel die Abschirmung zu berücksichtigen sind.

Es ist anzumerken, dass Größe und Form eines geeigneten Systems bestehend aus Steckvorrichtung und Einschubvorrichtung typischerweise von der Amplitude des für den Betrieb vorgesehenen Spannungssignals abhängen. Für den Fall, dass im Betrieb mittels einer Energieversorgungseinheit ein Spannungssignal von 1 kV zum Zünden eines Plasmas bereitgestellt wird, können die in Bezug auf Fig. 2A beschriebenen Größenangaben deutlich kleiner ausfallen, sodass eine entsprechende Steckvorrichtung kleiner und kompakter gestaltet ist.

Die Steckvorrichtung weist eine Einrastvorrichtung 64 auf, die im zusammengeführten Zustand von einer komplementär ausgebildeten Einschubvorrichtung aufgenommen wird. Dadurch werden Steckvorrichtung und Einschubvorrichtung mechanisch miteinander verbunden.

Figur 2B zeigt eine Seitenansicht auf einen Schnitt entlang der Höhe durch die in Fig. 2A gezeigte mit der Einschubvorrichtung zusammengeführte Steckvorrichtung. Die Einschubvorrichtung 60 ist auf der einen Seite mit einem Kabel 80 verbunden und hat eine Höhe H1 von 14 mm. Auf der gegenüberliegenden Seite weist die Einschubvorrichtung 60 eine Öffnung zur Aufnahme der Steckvorrichtung 70 auf. Die Steckvorrichtung 70 hat eine maximale Höhe H2 von 6,8 mm. Bei vollständiger Aufnahme der Steckvorrichtung 70 in die Einschubvorrichtung 60 hat der minimale Kriechweg patientenseitig eine Länge L1 von 85 mm. Im zusammengeführten Zustand sind Steckvorrichtung 70 und Einschubvorrichtung 60 mittels der Einrastvorrichtung 64 mechanisch verbunden.

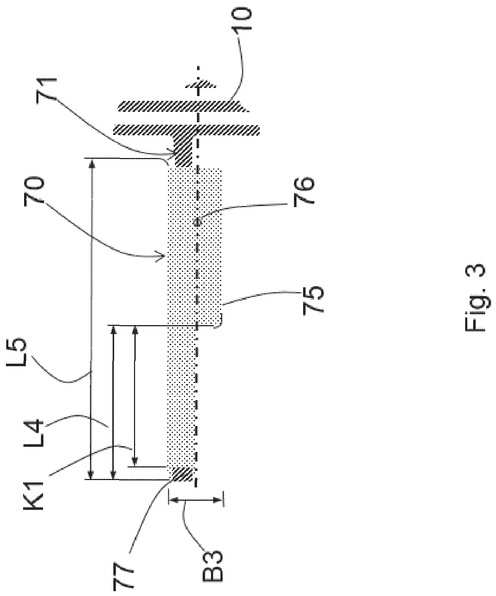

**Figur 3** zeigt eine bevorzugte Ausführungsform einer Steckvorrichtung 70. In der gezeigten Darstellung ist eine Draufsicht auf einen Schnitt entlang der Breite der Steckvorrichtung 70 zu sehen. Die Steckvorrichtung 70 ist mit einer Elektrodenstruktur 10 über wenigstens eine Leiterbahn 71 elektrisch leitend verbunden, wobei die Leiterbahn 71 von der Kontaktfläche des Spannungsanschlusses (HV-Anschluss) 77 zu der Elektrodenstruktur 10 führt. In der gezeigten Ausführungsform beträgt die maximale Breite B3 der Steckvorrichtung 70 21 mm. Die Steckvorrichtung 70 weist in der gezeigten Ausführungsform eine optionale Versteifung 75 auf, die z.B. aus einer Polyethylen (PE)-Folie bestehen kann und die beispielsweise eine Höhe von ca. 0,2 mm bis 1 mm haben kann. In dem gezeigten Ausführungsbeispiel hat die Versteifung die Funktion der Erhöhung des Elastizitätsmoduls. Dadurch werden ein Durchbiegen bzw. eine Formänderung durch eine Einwirkung von äußeren mechanischen Kräften verringert und die Steckvorrichtung lässt sich leicht und unkompliziert in die Einschubvorrichtung schieben.

Weiterhin weist die Steckvorrichtung 70 eine Bohrung 76 mit einer Einrastfunktion auf, die dazu ausgebildet ist, die Steckvorrichtung mechanisch mit einer hier nicht-gezeigten Einschubvorrichtung zu verriegeln. Auf der der Elektrodenstruktur 10 abgewandten Seite weist die Steckvorrichtung 70 über eine Länge L4 von 58 mm eine schmalere Breite als die maximale Breite B3 der Steckvorrichtung 70 auf. Die beschriebene Form ist insbesondere aufgrund der Kriechstrecken und der Vermeidung von Teilentladungen so gewählt, dass in der über ein Kabel mit einer Energieversorgungseinheit verbundenen und mit Spannung beaufschlagten Kupplung, wenn keine Steckvorrichtung eingeführt ist, keine Bogenentladung entsteht. An der der Elektrodenstruktur 10 abgewandten Seite weist die Steckvorrichtung 70 eine Kontaktfläche 77 auf, um die Steckvorrichtung 70 mit einem hier nicht-gezeigten HV-Anschluss zu verbinden. In der gezeigten Darstellung der Steckvorrichtung 70 beträgt der minimale Kriechweg K1 zwischen HV-Anschluss (nicht gezeigt) und GND-Anschluss (nicht gezeigt) 53 mm und die Gesamtlänge L5 der Steckvorrichtung 70 beträgt 119 mm.

Durch eine chipkartenähnliche Form der Steckvorrichtung, d.h. eine geringe Höhe und eine verhältnismäßig große Länge, können insbesondere Kriechstrecken derart eingehalten werden, dass keine Teilentladungen innerhalb der zusammengesteckten Steckvorrichtung und der Einschubvorrichtung entstehen. Die angegebenen Maße für die Länge, die Breite und die Höhe können vorteilhafterweise auch unabhängig voneinander derart realisiert werden, dass die Kriechstrecken für die für die Erzeugung des Plasmas notwendige Spannungsamplitude weiterhin eingehalten werden. Entsprechend können Länge, Breite und Höhe in Varianten der beschriebenen Ausführungsform von den genannten Werten abweichen.

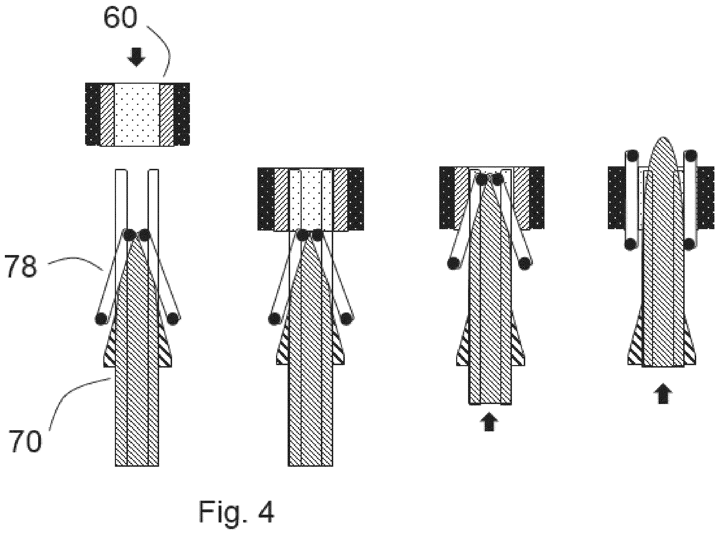

**Figur 4** zeigt einen Mechanismus für eine sichere Verbindung zwischen einer Steckvorrichtung 70 und einer Einschubvorrichtung 60 mittels eines Klemmkontakts 78, der sich an der Steckvorrichtung 70 befindet. Durch das Zusammenstecken von Steckvorrichtung 70 und Einschubvorrichtung 60 wird eine sichere Steckverbindung durch das Einrasten von als Verriegelungselemente dienenden Klemmen in der Einschubvorrichtung 70 hergestellt. In einem hier nicht-gezeigten Ausführungsbeispiel kann eine sichere Verbindung zwischen einer Steckvorrichtung und einer Einschubvorrichtung durch als Verriegelungselemente dienenden Spreizzungen an der Steckvorrichtung sichergestellt werden.

Um eine Einmalverwendung sicherzustellen, ist eine Steckvorrichtung bevorzugt derart gestaltet, dass sie beim mechanischen Trennen von der Einschubvorrichtung infolge des erstmaligen Gebrauchs derart verändert wird, dass kein erneutes elektrisches Anschließen mit einer Einschubvorrichtung möglich ist, da kein ausreichend festes mechanisches Verbinden mehr möglich ist. In verschiedenen Varianten kann eine Einmalverwendung einer Steckvorrichtung dadurch realisiert sein, dass beim mechanischen Trennen von der Einschubvorrichtung Klemmen abbrechen, Einrastelemente abbrechen, Verriegelungselemente unbrauchbar werden oder die Leiterbahnen der Steckvorrichtung zerkratzt oder zerschnitten werden.

In einem weiteren hier nicht-gezeigten Ausführungsbeispiel kann eine ausreichend zugfeste Verbindung zwischen einer Steckvorrichtung und einer Einschubvorrichtung durch magnetische Kontakte sichergestellt werden. In diesem Fall befinden sich in der Steckvorrichtung und in der Einschubvorrichtung jeweils mindestens 1 Magnet. Vorteilhafterweise haben die Magnete in der Steckvorrichtung eine gegensätzliche Polung zu den Magneten in der Einschubvorrichtung.

Bevorzugt ist die Steckverbindung zwischen Steckvorrichtung und Einschubvorrichtung derart ausgebildet, dass die mit einem Kabel verbundene Einschubvorrichtung mehrfach verwendbar ist. In einer hier nicht-gezeigten Ausführungsform ist zwischen der Einschubvorrichtung und einem Kabel ein Knickschutz vorgesehen.

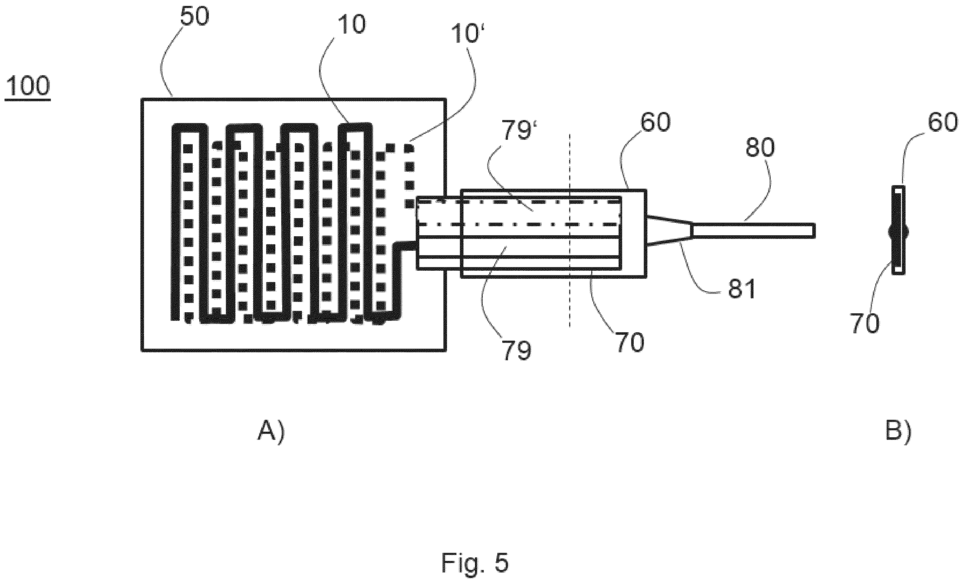

**Figur 5A** zeigt einen Plasmaapplikator 100 mit einem elektrotechnischen Kern 50 und einer Steckvorrichtung 70. Der elektrotechnischen Kern 50 umfasst eine zweite Elektrodenstruktur 10 und eine erste Elektrodenstruktur 10`. Die zweite Elektrodenstruktur 10 wird bevorzugt im Betrieb durch ein angelegtes Spannungssignal getrieben und die erste Elektrodenstruktur 10' ist bevorzugt geerdet. Die Elektrodenabschnitte der zweiten Elektrodenstruktur 10 und der ersten Elektrodenstruktur 10' sind übereinander mit einer definierten Überlappung angeordnet. Vorteilhafterweise sind Elektrodenabschnitte der geerdeten

Elektrodenstruktur und der im Betrieb getriebenen Elektrodenstruktur derart zueinander versetzt angeordnet, dass sich ein für die Plasmazündung vorteilhaftes elektrisches Feld ausbildet. Bevorzugt überlappen sich die Elektrodenabschnitte entsprechender Elektrodenstrukturen jeweils in einem vergleichsweise geringen Bereich. Das heißt, dass der jeweils übrige Bereich der Elektrodenabschnitte, der sich nicht in Überlappung mit einem Elektrodenabschnitt einer weiteren Elektrodenstruktur befindet, im Vergleich zu dem überlappten Bereich signifikant größer ist. Durch eine vergleichsweise geringe Überlappung wird zwischen den Elektrodenstrukturen ein im Wesentlichen homogen verteiltes Plasma erzeugt.

Die Steckvorrichtung 70 ist fest mit dem elektrotechnischen Kern 50 verbunden und weist eine erste Leiterbahn 79' und eine zweite Leiterbahn 79 auf. Die erste Leiterbahn 79' ist elektrisch leitend mit der ersten Elektrodenstruktur 10' verbunden und die zweite Leiterbahn 79 ist elektrisch leitend mit der zweiten Elektrodenstruktur 10 verbunden. Die ersten und zweiten Leiterbahnen können z. B. als einfache Leiter ausgebildet sein. Die ersten und zweiten Leiterbahnen bestehen bevorzugt aus dem gleichen Material wie die Elektrodenstrukturen. Die Steckvorrichtung 70 hat bevorzugt eine Breite von 3 cm, eine Höhe von 1 mm und eine Länge von 10 cm. In der gezeigten Darstellung ist die Steckvorrichtung 70 mit einer Einschubvorrichtung 60 zusammengeführt. Die Einschubvorrichtung 60 ist über ein Kabel 80 mit einer hier nicht-gezeigten, vorwiegend stationär genutzten Energieversorgungseinheit, wie einen Hochspannungsgenerator, verbunden. An der der Einschubvorrichtung 60 zugewandten Seite weist das Kabel 80 einen Knickschutz 81 auf. **Figur 5B** zeigt eine Seitenansicht auf einen Schnitt entlang der Höhe durch die Steckvorrichtung.

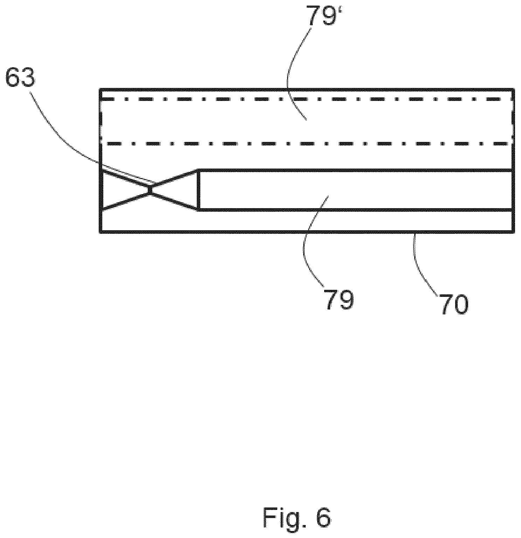

**Figur 6** zeigt eine Steckvorrichtung 70 mit einer zweiten Leiterbahn 79, die elektrisch mit einer im Betrieb getriebene Elektrodenstruktur (nicht gezeigt) verbunden ist, und einer ersten Leiterbahn 79`, die elektrisch mit einer geerdeten Elektrodenstruktur (nicht gezeigt) verbunden ist. Die zweite Leiterbahn 79 weist an einer Stelle eine Verjüngung 63 auf. Aus dem kleineren Durchmesser der Leiterbahn an der Verjüngung 63 resultiert ein höherer elektrischer Widerstand als in dem Rest der Leiterbahn 79. Die gezeigte Verjüngung 63 kann grundsätzlich sowohl in der Steckvorrichtung 70 als auch im elektrotechnischen Kern 50 integriert sein. Um eine Einmalverwendung eines Plasmaapplikators 100 zu gewährleisten, kann am Ende einer Behandlung ein Strompuls auf die Leiterbahn 79 gegeben werden, dessen Stromstärke so bemessen ist, dass sich die Leiterbahn 79 an dieser Verjüngung 63 soweit erwärmt, dass er schmilzt. Beispielsweise kann eine mit dem Plasmaapplikator zusammengeführte Energieversorgungseinheit am Ende der Plasmabehandlung automatisch für deutlich unter 1 Sekunde einen überhöhten Strompuls mit einer entsprechenden Stromstärke aussenden.

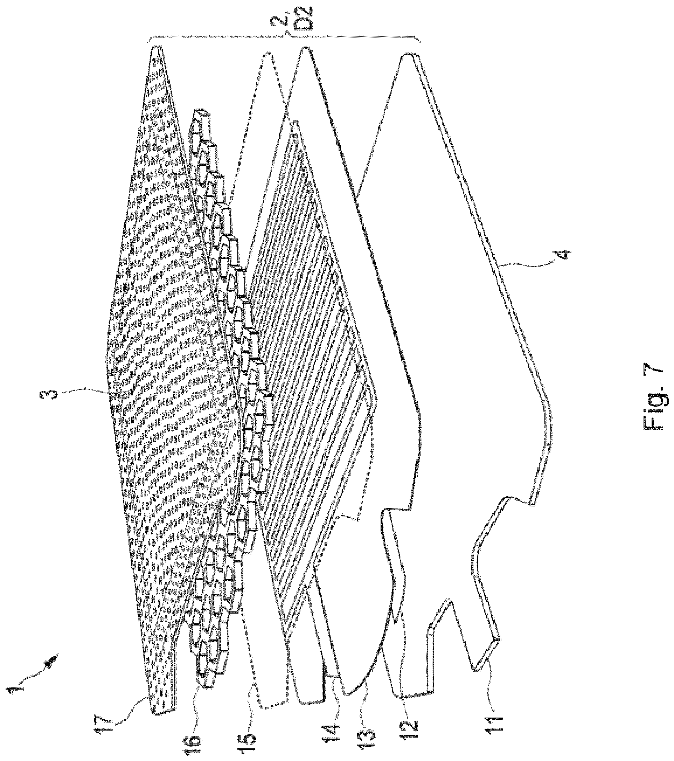

**Figur 7** zeigt eine Explosionsdarstellung einer bereits bekannten Vorrichtung 1 zum Erzeugen eines kalten Atmosphärendruckplasmas für die Behandlung von Oberflächen mit einem Mehrschichtsystem 2. Das Mehrschichtsystem 2 bildet einen Plasmaapplikator und umfasst die folgenden schichtförmig ausgebildeten Strukturen, nämlich (von unten beginnend):
- eine erste isolierende Struktur 11,
- eine erste Elektrodenstruktur 12,
- eine Dielektrikumsschicht 13,
- eine zweite Elektrodenstruktur 14,
- eine zweite isolierende Struktur 15,
- eine Abstandshalterstruktur 16, und
- eine Adhäsionsschicht 17.

Die erste isolierende Struktur 11, die erste Elektrodenstruktur 12, die Dielektrikumsschicht 13, die zweite Elektrodenstruktur 14 und die zweite isolierende Struktur 15 bilden jeweils eine Schicht des elektrotechnischen Kerns des Plasmaapplikators. Die erste isolierende Struktur 11 ist dabei auf der von der zu behandelnden Oberfläche abgewandten Seite 4 des Mehrschichtsystem 2 angeordnet und weist eine Höhe zwischen 0,5 mm und 2,5 mm, bevorzugt von 2 mm auf. Die erste isolierende Struktur 11 dient im Wesentlichen zur Isolierung der ersten Elektrodenstruktur 12, die bevorzugt als Hochspannungsschicht gebildet ist, d. h. eine Elektrodenstruktur, an die eine Hochspannung angelegt wird.

Die Dielektrikumsschicht 13 ist zwischen der ersten Elektrodenstruktur 12 und der zweiten Elektrodenstruktur 14 angeordnet, wobei die zweite Elektrodenstruktur 14 bevorzugt als eine Massenelektrodenschicht ausgebildet ist. Die Dielektrikumsschicht 13 verhindert dabei im Wesentlichen einen Kurzschluss zwischen der ersten und zweiten Elektrodenstruktur, insbesondere in Form eines Lichtbogens.

Weiterhin ist in einer bevorzugten Ausgestaltungsform auf der zweiten Elektrodenstruktur 14 eine zweite isolierende Struktur 15 angeordnet, die eine Dicke zwischen 50 µm und 300 µm aufweist.

Oberhalb der zweiten Elektrodenstruktur 14 oder der zweiten isolierenden Struktur 15, also auf der von der zu behandelnden Oberflächen zugewandten Seite 3 des Mehrschichtsystems 2 ist dann die Abstandshalterstruktur 16 angeordnet, die dafür Sorge trägt, dass ausreichend Gasvolumen bereitgestellt wird, so dass ein Plasma zünden kann.

Abschließend ist auf der zu behandelnden Oberfläche zugewandten Seite 3 des Mehrschichtsystems 2 und oberhalb der Abstandshalterstruktur 16 eine Adhäsionsschicht 17 angeordnet, die eine Dicke zwischen 100 µm bis 300 µm, bevorzugt von 200 µm aufweist und die im direkten Kontakt mit der zu behandelnden Oberfläche steht. Bevorzugt ist die Adhäsionsschicht 17 dann mit einem haut- und/oder wundverträglichen Material bevorzugt mit antiseptischen und/oder atraumatischen Eigenschaften, gebildet.

Vorliegend, wie in der Fig. 7 dargestellt, ist die zweite Elektrodenstruktur 14 mit einer Vielzahl von Aussparungen, insbesondere gitterartig, ausgebildet. In weiteren Ausführungsformen können die Aussparungen aber auch loch-, streifen-, mäander-, waben-, kreisförmig und/oder quadratisch ausgebildet sein.

Weiterhin kann auch die Abstandshalterstruktur 16 wabenförmig gebildet sein, wobei die Abstandshalterstruktur 16 auch durch Vorsprünge oder Stege realisiert sein kann. Mögliche Materialien für die Abstandshalterstruktur 16 sind Polymere, Elastomere und/oder Silikone oder dergleichen. Grundsätzlich können eine Vielzahl von möglichen Materialien verwendet werden, wie zum Beispiel anorganische oder organische Materialien, insbesondere natürliche und/oder synthetische Materialien, wie Thermoplaste, Duroplaste und/oder Elastomere. Für weitere mögliche Materialien wird beispielhaft auch auf das Buch "Kunststoff-Taschenbuch" (28. Auflage) von Karl Oberbach und Hansjürgen Saechtling verwiesen. In einer bevorzugten Ausgestaltungsform der in Fig. 7 gezeigten Vorrichtung ist die Abstandshalterstruktur mit Vorsprüngen und/oder Stegen gebildet, die eine Höhe zwischen 0,5 mm und 5 mm aufweisen.

Insgesamt weist das in der Fig. 7 dargestellte Mehrschichtsystem eine Dicke von 2 mm bis 4 mm auf. Hierbei ist vorgesehen, dass die Schichten, die mit der zu behandelnden Oberfläche in direktem Kontakt sind, aus einem hitzebeständigen, biokompatiblen und chemisch beständigen Kunststoff gebildet sind.

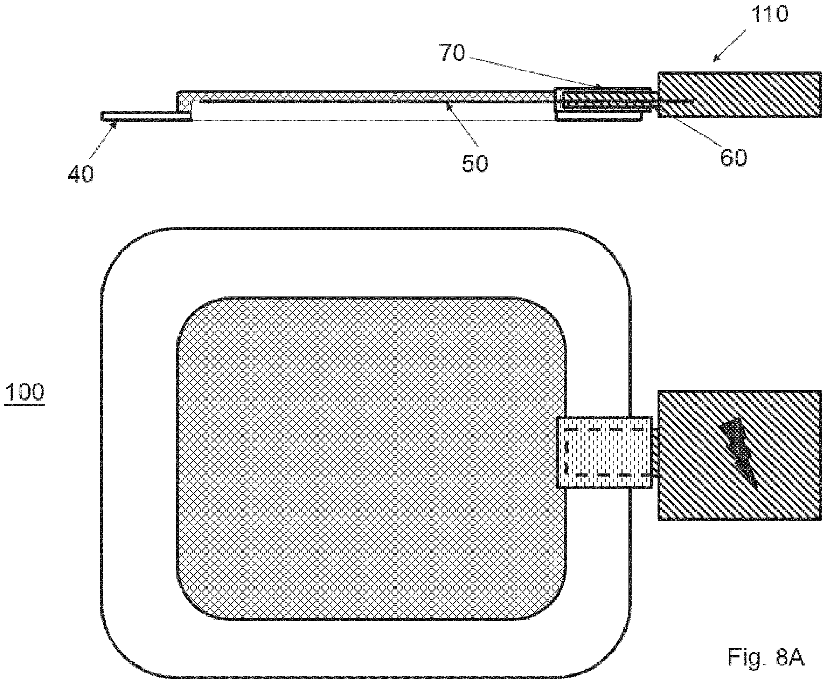

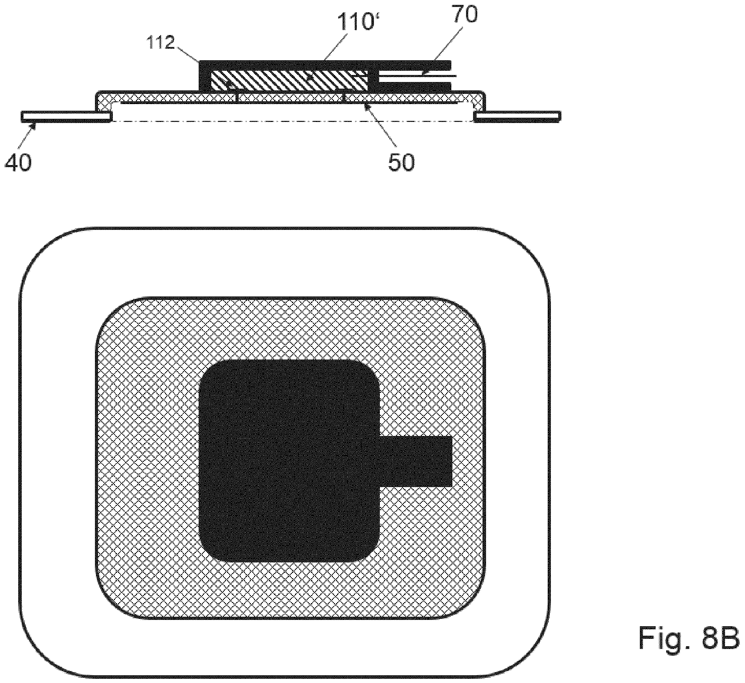

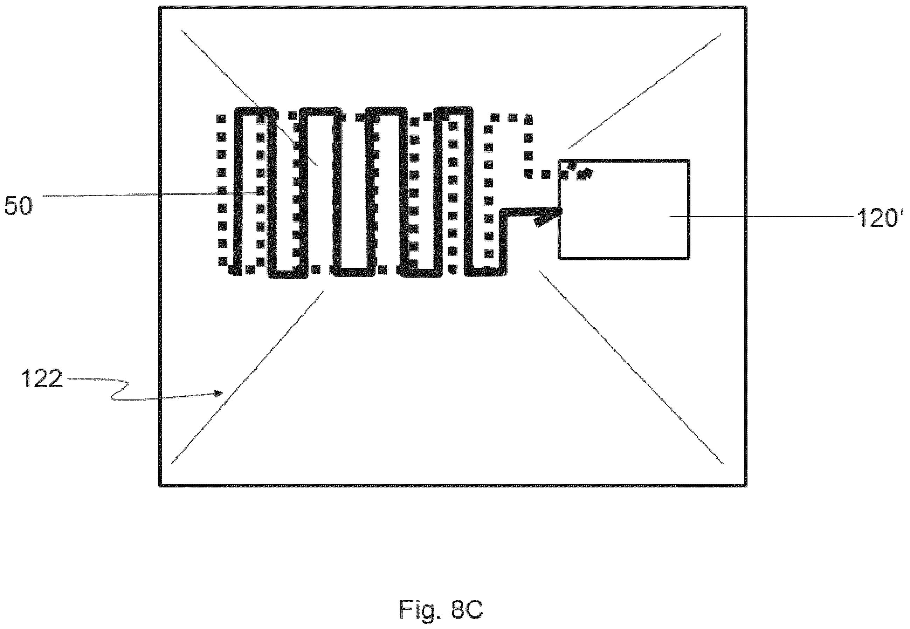

**Figuren 8A****,** **8B** **und** **8C** zeigen einen Plasmaapplikator mit einer mobilen Energieversorgung, die durch eine im Vergleich zum Plasmaapplikator verhältnismäßig kleine Energieversorgungseinheit gewährleistet wird. Aufgrund der mobilen Energieversorgungseinheit ist es nicht notwendig, eine vorwiegend stationär genutzte Energieversorgungseinheit, wie z.B. einen Hochspannungsgenerator, über ein Kabel mit dem Plasmaapplikator zu verbinden, um ein zum Zünden eines Plasmas ausreichendes Spannungssignal an die Elektrodenstruktur zu übertragen und ein Plasma zu Zünden. Die notwendige Energie zum Zünden eines Plasmas wird durch einen in der mobilen Energieversorgungseinheit umfassten Energiespeicher bereitgestellt. Ein solcher Energiespeicher kann z.B. eine Batterie, ein Ackumulator oder ein Kondensator sein.

Der in den **Figuren 8A bis 8F** gezeigte Plasmaapplikator umfasst mindestens eine erste Elektrodenstruktur und eine zweite Elektrodenstruktur. Zwischen den Elektrodenstrukturen ist jeweils mindestens eine Isolationsschicht angeordnet. Die Isolationsschicht kann z.B. ein Polymer mit dielektrischen Eigenschaften sein. Der Abstand zwischen den Elektrodenstrukturen ist bevorzugt kleiner als 1 mm. Vorteilhafterweise ist durch den kleinen Abstand eine geringere Amplitude des Spannungssignals zum Zünden eines Plasmas notwendig. An Stelle der gezeigten Mäanderform der ersten Elektrodenstruktur und der zweiten Elektrodenstruktur, können die Elektrodenstrukturen auch eine Spiralform haben, können durchgängig sein oder Löcher haben. In der gezeigten Ausführungsform bestehen die Elektrodenstrukturen aus einem Metall. In verschiedenen Varianten der gezeigten Ausführungsform sind Elektrodenstrukturen aus einem leitfähigen Kunststoff oder aus einem Textil mit leitfähigen Drähten vorgesehen. Der gezeigte Plasmaapplikator weist auf der der zu behandelnden Oberfläche zugewandten Seite als Schutzschicht eine Isolationsschicht aus einem Polymer auf, die vorzugsweise von einem biokompatiblen Material gebildet ist.

Optional kann der gezeigte Plasmaapplikator auch eine Distanzstruktur aufweisen. Eine entsprechende Distanzstruktur kann z.B. aus einem Polymer, Textil, Hydrogel, auf Stärkebasis, z.B. als Maisflips, Standardwundflies bzw. -mull sowie einem Absorber realisiert sein und elektrisch isolierend, und insbesondere biokompatibel sein. Eine Distanzstruktur kann auch von einer Kombination der vorher genannten Materialien gebildet ein. Eine Distanzstruktur kann auch in Form eines Flachkabels zum Zünden eines Plasmas ausgebildet sein. Insbesondere kann die Distanzstruktur als Flachkabel ausgebildet sein, das gleichzeitig eine Plasmaquelle ist.

Optional weist der gezeigte Plasmaapplikator auf der der zu behandelnden Oberfläche abgewandten Seite wenigstens eine Isolationsschicht als Schutzschicht auf.

Der gezeigte Plasmaapplikator kann auf der der zu behandelnden Oberfläche zugewandten Seite eine Adhäsionsschicht oder einen Aufkleber zur Fixierung des Plasmaapplikators über oder auf der zu behandelnden Oberfläche aufweisen.

Neben der gezeigten rechteckigen Form kann der elektrotechnische Kern des Plasmaapplikators auch alternative Geometrien aufweisen. In verschiedenen Varianten hat der elektrotechnische Kern des Plasmaapplikators eine Kreisform, eine Form, die speziell an einen bestimmten Körperteil (z.B. einen Fuß) angepasst ist oder ist zylinderförmig. In einer Variante ist der Plasmaapplikator dafür vorgesehen, kegelförmig um einen Schlauch oder ein Kabel angebracht zu werden. In diesem Fall wird der Plasmaapplikator um einen Schlauch oder ein Kabel gelegt, sodass ein abgeschlossener Gasraum unter einem Plasmaapplikator mit der Form eines Kegels entsteht. Vorteilhafterweise muss so ein gelegter Zugang nicht abgenommen werden, um eine Behandlung mit einem Plasmaapplikator zu ermöglichen. Falls vor dem Legen eines Zugangs bekannt ist, dass eine Behandlung mit einem Plasmaapplikator durchgeführt werden soll, kann es vorteilhaft sein, wenn der Plasmaapplikator ein Loch oder einen Schlitz aufweist durch den ein Kabel oder ein Schlauch geführt werden kann. Dadurch kann zunächst ein Zugang gelegt werden und zu einem späteren Zeitpunkt eine Plasmabehandlung stattfinden ohne, dass der Zugang entfernt werden muss.

Die im Folgenden mit Bezug auf die **Figuren 8A bis 8F** beschriebenen Varianten einer mobilen Energieversorgung für einen Plasmaapplikator oder die Varianten derjenigen Merkmale, die eine Einmalverwendung eines Plasmaapplikators sicherstellen sollen, können in Kombination mit elektrotechnischen Kernen einer beliebigen der vorgenannten oder anderen Geometrien zu verschiedenen Varianten eines Plasmaapplikators kombiniert werden. Insbesondere kann ein elektrotechnischer Kern eine erste Isolationsschicht, gefolgt von einer ersten, geerdeten Elektrodenstruktur, gefolgt von einer zweiten Isolationsschicht, gefolgt von einer im Betrieb getriebenen zweiten Elektrodenstruktur, gefolgt von einer dritten Isolationsschicht, gefolgt von einer dritten, geerdeten Elektrodenstruktur aufweisen und somit an sich bereits einen Berührungsschutz gewährleisten.

**Figur 8A** zeigt einen Plasmaapplikator mit einer Steckvorrichtung 70, die mit einer Einschubvorrichtung 60 einer mobilen Energieversorgungseinheit 110 zusammengeführt ist. Die verhältnismäßig kleine mobile Energieversorgungseinheit 110 umfasst einen Energiespeicher und eine Einschubvorrichtung. Im Gegensatz zu den mit Bezug auf die Figuren 1, 2, 3 und 5 beschriebenen Varianten einer Einschubvorrichtung ist es nicht notwendig, dass die Einschubvorrichtung einer mobilen Energieversorgung am Ende eines längeren Kabels angeordnet ist, welches für einen Anschluss an eine vorwiegend stationär genutzte Energieversorgungseinheit, wie einen Spannungsgenerator, vorgesehen ist. Die mobile Energieversorgungseinheit 110 kann mittels der Einschubvorrichtung 60 mit der Steckvorrichtung 70 eines Plasmaapplikators mechanisch und elektrisch verbunden werden. Die gezeigte Steckvorrichtung 70 und/oder der elektrotechnische Kern 50 können Varianten derjenigen Merkmale aufweisen, die eine Einmalverwendbarkeit sicherstellen, wie sie z.B. in Bezug auf Figuren 4 und 6 beschrieben werden. Im verbundenen Zustand bilden der Plasmaapplikator und die mobile Energieversorgungseinheit eine kompakte Einheit, die auch im Betrieb von einem Patienten leicht getragen werden kann.

Der Energiespeicher der mobilen Energieversorgungseinheit 110 liefert typischerweise kein Spannungssignal im kV-Bereich, sondern ein Spannungssignal von einigen Volt, z.B. zwischen 5 und 20 Volt. Das bereitgestellte Spannungssignal kann z.B. in der Größenordnung einer von einer handelsüblichen Batterie bereitgestellten Spannung liegen, z.B. 9V eines 9V-Blocks. Da zum Zünden eines Plasmas jedoch in der Regel ein Spannungssignal mit einer Amplitude von einigen hundert Volt bis hin zu 10 kV benötigt wird, muss das durch den Energiespeicher der mobilen Energieversorgungseinheit gelieferte Spannungssignal in ein Spannungssignal von einigen hundert Volt bis hin zu 10 kV transformiert werden.

Zu diesem Zweck weist der Plasmaapplikator in der hier beschriebenen Ausführungsform weiterhin eine elektrische Schaltung (nicht gezeigt) auf, die ein bereitgestelltes Spannungssignal der mobilen Energieversorgungseinheit 110 in eine (gepulste) Wechselspannung in einem Spannungsbereich von bevorzugt einigen 100 V bis 10 kV transformiert. Eine zu diesem Zweck ausgebildete elektrische Schaltung weist z.B. einen Wechselrichter oder einen VDC-VAC Inverter in Kombination mit einem Spannungstransformator sowie einem Pulser mit z.B. einem Tastverhältnis von 1s "An" und 9s "Aus" auf. Je nach Anwendungsfall kann ein hier nicht gezeigter Plasmaapplikator ein abweichendes Tastverhältnis aufweisen. Die elektrische Schaltung ist elektrisch mit wenigstens einer Elektrodenstruktur des Plasmaapplikators verbunden und ist geeignet, ein zum Zünden eines Plasmas ausreichend hohe Amplitude des Spannungssignals an die Elektrodenstruktur zu liefern.

Alternativ kann die elektrische Schaltung zum Transformieren eines Spannungssignals mit 5 - 20V in ein Spannungssignal mit einer Amplitude von einigen 100 V bis 10 kV auch in die mobile Energieversorgungseinheit zusammen mit dem Energiespeicher und der Einschubvorrichtung intergiert sein. Der Energiespeicher der mobilen Energieversorgungseinheit liefert ein Spannungssignal, welches von der entsprechenden in die Energieversorgungseinheit integrierten elektrischen Schaltung in ein zum Zünden eines Plasmas ausreichendes Spannungssignal mit entsprechender Amplitude transformiert wird. Wenn die Einschubvorrichtung der mobilen Energieversorgungseinheit mit der Steckvorrichtung eines Plasmaapplikators verbunden ist, kann das Spannungssignal über Leiterbahnen der Steckvorrichtung an wenigstens eine Elektrodenstruktur zum Zünden eines Plasmas übertragen werden. Der Plasmaapplikator selbst benötigt dann keine elektrische Schaltung zum Transformieren eines Spannungssignals in ein Spannungssignal mit einer Amplitude im kV-Bereich.

In einer hier nicht gezeigten Ausführungsform ist sowohl in einer mobilen Energieversorgungseinheit als auch einem Plasmaapplikator jeweils eine elektrische Schaltung integriert. Wenn eine Steckvorrichtung des Plasmaapplikators mit einer Einschubvorrichtung der mobilen Energieversorgungseinheit zusammengesteckt ist und eine elektrische und mechanische Verbindung hergestellt ist, bilden die beiden elektrischen Schaltungen ein Schaltungssystem. Das Schaltungssystem transformiert dann eine Gleichspannung des Energiespeichers der mobilen Energieversorgungseinheit in ein zum Zünden eines Plasmas ausreichendes Spannungssignal und leitet das Spannungssignal an mindestens eine Elektrodenstruktur im elektrotechnischen Kern.

Wenn der Energiespeicher in der mobilen Energieversorgungseinheit 110 ein Akkumulator ist, ist es bevorzugt, dass der Akkumulator möglichst flach ausgebildet ist und z.B. eine Länge von 9 cm, eine Breite von 9 cm und eine Höhe von 0,2 cm hat. Bevorzugt weist ein entsprechender Akkumulator eine hohe Kapazität, bevorzugt mehr als 4000 mAh, und eine hohe Stromabgabe von mehr als 500 mA, insbesondere zwischen 1 bis 2 A, auf. Alternativ können auch eine Anzahl von kleineren Akkumulatoren parallelgeschaltet werden, um einen ausreichend hohen Strom erzeugen zu können.

Beim Transformieren einer Gleichspannung in ein zum Zünden eines Plasmas ausreichendes Spannungssignal wird eine Spannung typischerweise um einen Faktor 100 oder mehr erhöht. Dies bedeutet wiederum eine Reduktion eines abgegebenen Stroms an eine Sekundärspule eines Transformators um einen Faktor 100. Mit einem von mehreren parallelgeschalteten Akkumulatoren gebildeten Energiespeicher kann es möglich sein, in einem kurzen Zeitraum einen vergleichsweise hohen Strom abzugeben, ohne dass der Energiespeicher dabei zu heiß wird. Eine Verwendung eines solchen Energiespeichers, der in einem kurzen Zeitraum einen hohen Strom abgegeben kann, ohne dabei zu heiß zu werden, kann deshalb vorteilhaft sein, da während einer Plasmaentladung kurzzeitig Ströme im Milliampere-Bereich bis in den Ampere-Bereich entstehen können.

Der Energiespeicher der mobilen Energieversorgungseinheit 110 kann auch ein Kondensator sein. Hierbei sind insbesondere die Größe bzw. das Gewicht und die Kapazität des verwendeten Kondensators entscheidend. Bevorzugt hat der verwendete Kondensator ein Gewicht von wenigen Gramm, eine kompakte Größe im Bereich von einigen Zentimetern, eine Kapazität im Bereich von µF bis mF und eine Halbwertszeit der Entladung von effektiv wenigen Sekunden. Der Kondensator kann über die Einschubvorrichtung der mobilen Energieversorgungseinheit durch Verbinden mit einer Energieversorgung, beispielsweise einem Ladegerät, aufgeladen werden. Wenn die Einschubvorrichtung mit einer Steckvorrichtung verbunden ist, kann die in dem Kondensator gespeicherte Energie mittels einer in die Einschubvorrichtung oder in die Steckvorrichtung integrierten elektrischen Schaltung in ein zum Zünden eines Plasmas ausreichendes Spannungssignal über Leiterbahnen in der Steckvorrichtung an eine Elektrodenstruktur des Plasmaapplikators abgeben werden. Vorteilhafterweise ist zwischen dem Kondensator und der Leiterbahnin der Steckvorrichtung noch mindestens ein elektrisches Bauteil, bevorzugt ein elektrischer Widerstand, in Reihe oder auch parallelgeschaltet, um den Entladungsstrom vom Kondensator zu begrenzen.

Durch Verbinden der mobilen Energieversorgungseinheit mit einem Plasmaapplikator und einer resultierenden Spannungsübertragung an wenigstens eine Elektrodenstruktur des Plasmaapplikators kann ein Patient ein Plasma zu einem beliebigen Zeitpunkt nach dem Aufbringen eines Plasmaapplikators über der Wunde an einem beliebigen Ort zünden. Der Patient ist also unabhängig von einer vorwiegend stationär verwendeten, von einer örtlichen Stromversorgung abhängigen Energieversorgung und kann den Plasmaapplikator mit Hilfe der mobilen Energieversorgungseinheit an einem beliebigen Ort für eine Plasmabehandlung nutzen.

Vorteilhafterweise ist eine wie in Fig. 8A gezeigte mobile Energieversorgungseinheit 110 wiederverwendbar. Insbesondere auch dann, wenn der Plasmaapplikator selbst nur einmal verwendet werden kann.

**Figur 8B** zeigt einen Plasmaapplikator mit einer integrierten Energieversorgungseinheit 110' und einer Steckvorrichtung 70. In der gezeigten Ausführungsform ist also die mobile Energieversorgungseinheit 110' in den Plasmaapplikator integriert. Der elektrotechnische Kern 50 des Plasmaapplikators ist mittels einer Kontaktierung 112 elektrisch mit der integrierten Energieversorgungseinheit 110' verbunden. Der Plasmaapplikator weist weiterhin eine Steckvorrichtung 70 auf. Wenn die mobile Energieversorgungseinheit einen Akkumulator oder einen Kondensator aufweist, können diese über die Steckvorrichtung mit einer mobilen oder einer stationären Energieversorgung verbunden und geladen werden. Wenn der Akkumulator oder der Kondensator ausreichend geladen ist, kann die Verbindung mit der Energieversorgung getrennt werden. Ein Patient kann sich dann unabhängig von einer stationären Energieversorgung bewegen und ein Plasma unabhängig von einer stationären Energieversorgung an einem beliebigen Ort und zu einem späteren Zeitpunkt zünden.

Eine Einmalverwendbarkeit des gezeigten Plasmaapplikators kann dadurch sichergestellt werden, dass die Steckvorrichtung oder der elektrotechnische Kern Varianten derjenigen Merkmale aufweist, die eine Einmalverwendbarkeit sicherstellen, wie sie z.B. in Bezug auf Figuren 4 und 6 beschrieben werden. Ein elektrotechnischer Kern kann insbesondere eine erste Isolationsschicht, gefolgt von einer ersten, geerdeten Elektrodenstruktur, gefolgt von einer zweiten Isolationsschicht, gefolgt von einer im Betrieb getriebenen zweiten Elektrodenstruktur, gefolgt von einer dritten Isolationsschicht, gefolgt von einer dritten, geerdeten Elektrodenstruktur aufweisen und somit an sich bereits einen Berührungsschutz gewährleisten.

**Figur 8C** zeigt einen Plasmaapplikator mit integrierter Energieversorgungseinheit 120` und ohne eine Steckvorrichtung. Die integrierte Energieversorgungseinheit 120` ist mit dem elektrotechnischen Kern 50 des Plasmaapplikators elektrisch verbunden. Der gezeigte Plasmaapplikator umfasst eine mobile, in den Plasmaapplikator integrierte, Energieversorgungseinheit 120` mit einem Energiespeicher. Im Gegensatz zu den in Fig. 8A und 8B gezeigten Ausführungsformen, weist der gezeigte Plasmaapplikator keine Steckvorrichtung auf.

Der Energiespeicher kann z.B. eine Batterie mit einer Kapazität zwischen 0.5 und 20 Ah, z.B. ein handelsüblicher 9V-Block sein. Die von dem Energiespeicher bereitgestellte Gleichspannung wird an eine in den Plasmaapplikator integrierten elektrischen Schaltung übertragen und dort in ein Spannungssignal bevorzugt mit einer Amplitude im kV-Bereich transformiert. Zum Zünden eines Plasmas kann auch ein Spannungssignal mit einer Amplitude von einigen hundert Volt ausreichend sein. Das transformierte Spannungssignal wird dann an die wenigstens eine Elektrodenstruktur zum Zünden eines Plasmas übertragen.

Mit einer Energieversorgungseinheit, die in den Plasmaapplikator integriert ist und einen Energiespeicher mit begrenzter Ladung hat, der nicht wieder aufladbar ist, kann ebenfalls eine Einmalverwendung des Plasmaapplikators sichergestellt werden.

Eine Einmalverwendbarkeit des gezeigten Plasmaapplikators mit integrierter Energieversorgungseinheit 120` kann dadurch sichergestellt werden, dass eine Leiterbahn zum Übertragen eines zum Zünden eines Plasmas ausreichenden Spannungssignals an eine Elektrodenstruktur eine Verjüngung aufweist, wie sie z.B. in Bezug auf Fig. 6 beschrieben wird. Im Bereich der Verjüngung weist die Leiterbahn einen höheren elektrischen Widerstand als in dem Rest der Leiterbahn auf. Am Ende einer Behandlung kann von der Energieversorgungseinheit ein Strompuls bereitgestellt werden, dessen Stromstärke so bemessen ist, dass sich die Leiterbahn an der Verjüngung soweit erwärmt. dass er in dem Bereich der Verjüngung schmilzt.

Die in dem Energiespeicher, z.B. eine Batterie, der integrierten Energieversorgungseinheit gespeicherte Energie kann auch gerade so bemessen sein, dass sie lediglich für eine einmalige Behandlung ausreicht. Eine einmalige Behandlung hat typischerweise eine Dauer von wenigen Minuten.

Der gezeigte Plasmaapplikator weist auch eine Distanzstruktur 122 auf.

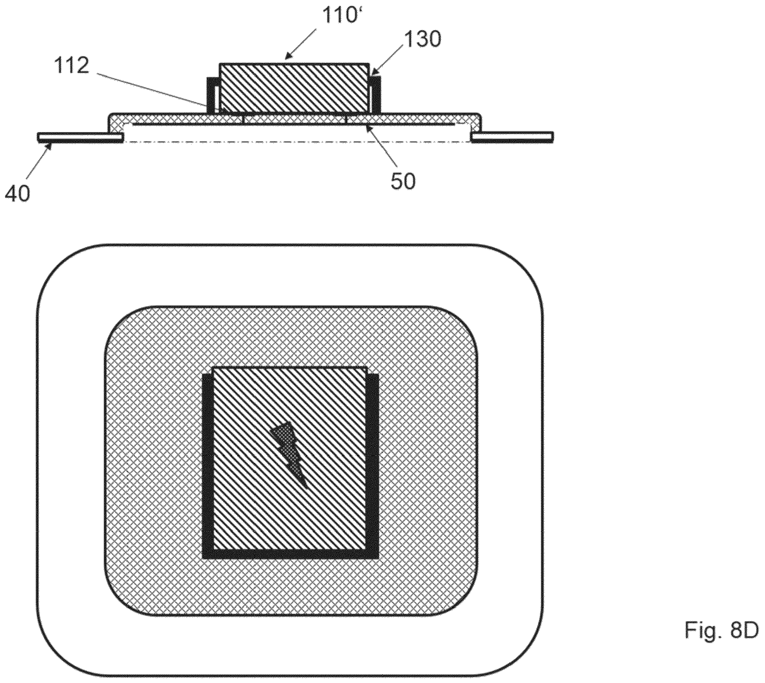

**Figur 8D** zeigt einen Plasmaapplikator mit einem Einschubschlitz 130 für eine mobile Energieversorgungseinheit 110`. In der gezeigten Ausführungsform weist der Plasmaapplikator einen Einschubschlitz 130 auf der Oberseite, d.h. der einer Wunde abgewandten Seite, auf, mit dem eine mobile Energieversorgungseinheit 110` auf dem Plasmaapplikator befestigt werden kann. Der Plasmaapplikator weist Kontakte 112 auf, die eine Elektrodenstruktur des elektrotechnischen Kerns 50 mit der Oberseite des Plasmaapplikators verbinden. Insbesondere weisen die Kontakte 112 an der Oberfläche freie Kontaktflächen auf, durch die eine galvanische Kopplung mit dem Energiespeicher der mobilen Energieversorgungseinheit 110' hergestellt werden kann, wenn die mobile Energieversorgungseinheit 110' in den Einschubschlitz 130 des Plasmaapplikators eingeschoben ist.

Der Energiespeicher der mobilen Energieversorgungseinheit ist in der gezeigten Ausführungsform ein Akkumulator. Vorteilhafterweise kann die mobile Energieversorgungseinheit durch Laden eines leeren Akkumulators mehrmals zur Energieversorgung eines Plasmaapplikators verwendet werden. Die mobile Energieversorgungseinheit weist weiterhin eine elektrische Schaltung auf, die ausgebildet ist, eine durch den Akkumulator bereitgestellte Gleichspannung in ein zum Zünden eines Plasmas ausreichendes Spannungssignal zu transformieren. In der gezeigten Ausführungsform weist die mobile Energieversorgungseinheit keine Einschubvorrichtung auf. Zum Laden des Akkumulators ist deshalb eine in die mobile Energieversorgungseinheit integrierte Ladevorrichtung vorgesehen. Die Ladevorrichtung umfasst in dem gezeigten Ausführungsbeispiel eine Empfangs-Spulenanordnung zum induktiven Laden des Akkumulators.

Es ist auch denkbar, dass ein Energiespeicher einer Energieversorgungseinheit über Kontakte aufgeladen werden kann. Beispielsweise kann eine mobile Energieversorgungseinheit in einen dafür vorgesehenen Einschubschlitz einer stationären Energieversorgungseinheit geschoben werden und der Energiespeicher der Energieversorgungseinheit über Kontakte aufgeladen werden. Es ist auch denkbar, dass ein Ladegerät Einschubschlitze aufweist, in die eine Energieversorgungseinheit eingeschoben werden kann, um einen elektrischen Kontakt zwischen dem Energiespeicher und einer Energieversorgung des Ladegeräts herzustellen.

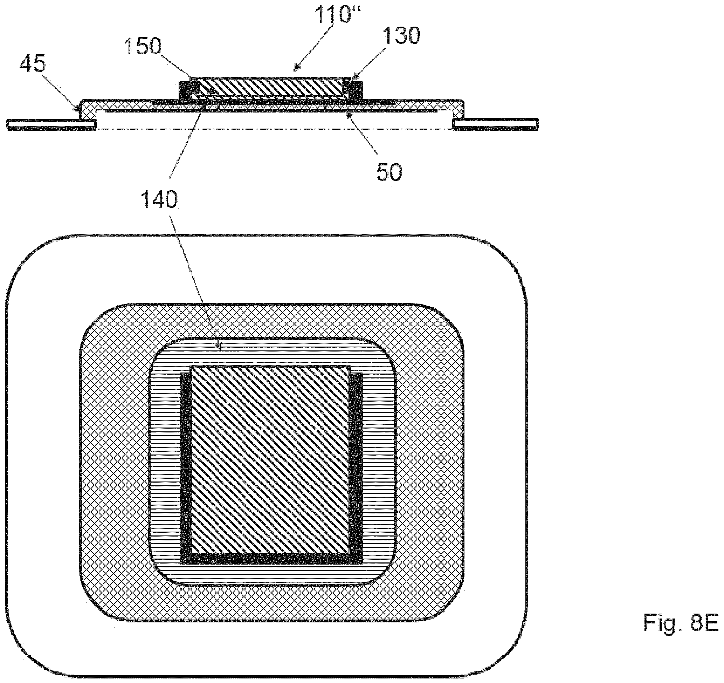

**Figur 8E** zeigt einen Plasmaapplikator mit einer integrierten Empfangs-Spulenanordnung 140 und mit einem Einschubschlitz 130 in den eine mobile Energieversorgungseinheit 110" eingeschoben ist. Die Einschubschlitze 130, in die eine mobile Energieversorgungseinheit 110" eingeschoben ist, befinden sich auf der der zu behandelnden Oberfläche abgewandten Seite des Plasmaapplikators. An der der zu behandelnden Oberfläche abgewandten Seite des Plasmaapplikators zugewandten Seite der mobilen Energieversorgungseinheit weist die mobile Energieversorgungseinheit eine Sende-Spulenanordnung 150 auf, die die durch einen in die mobile Energieversorgungseinheit 110" integrierten Energiespeicher (nicht dargestellt) bereitgestellte elektrische Energie an die Empfangs-Spulenanordnung 140 des Plasmaapplikators mittels induktiver Kopplung überträgt. Die mobile Energieversorgungseinheit 110" weist also einen Energiespeicher (nicht dargestellt) auf, welcher Energie bereitstellt, welche mittels induktiver Kopplung von der Sende-Spulenanordnung 150 an die Empfangs-Spulenanordnung 140 übertragen wird.

Es ist auch denkbar, dass die Energieversorgungseinheit 110" keine mobile Energieversorgungseinheit ist und keinen Energiespeicher aufweist. Eine solche Energieversorgungseinheit kann beispielweise mit einem Kabel verbunden sein, welches an dem anderen Ende mit einer stationären Energieversorgungseinheit verbunden ist. In diesem Fall wird über eine stationäre Energieversorgungseinheit Energie bereitgestellt, die mittels induktiver Kopplung von der Sende-Spulenanordnung 150 an die Empfangs-Spulenanordnung 140 übertragen wird.

Der elektrotechnische Kern 50 befindet sich auf der der zu behandelnden Oberfläche zugewandten Seite des Plasmaapplikators und ist mittels Kontakten mit einer darüber liegenden flachen Empfangs-Spulenanordnung 140 elektrisch verbunden. Die Empfangs-Spulenanordnung 140 befindet sich auf der der zu behandelnden Oberfläche abgewandten Seite des Plasmaapplikators und ist vollständig mit einer Umschließung 45 des Plasmaapplikators bedeckt. Die Umschließung 45 des Plasmaapplikators kann zum Beispiel im Spritzgussverfahren hergestellt werden.

Vorteilhafterweise kann der Plasmaapplikator der gezeigten Ausführungsform komplett mit einer Umschließung 45 umspritzt werden. Insbesondere gibt es keine freiliegenden elektrischen Kontakte. Somit ist der Plasmaapplikator leicht zu reinigen, zu desinfizieren und/oder zu sterilisieren.

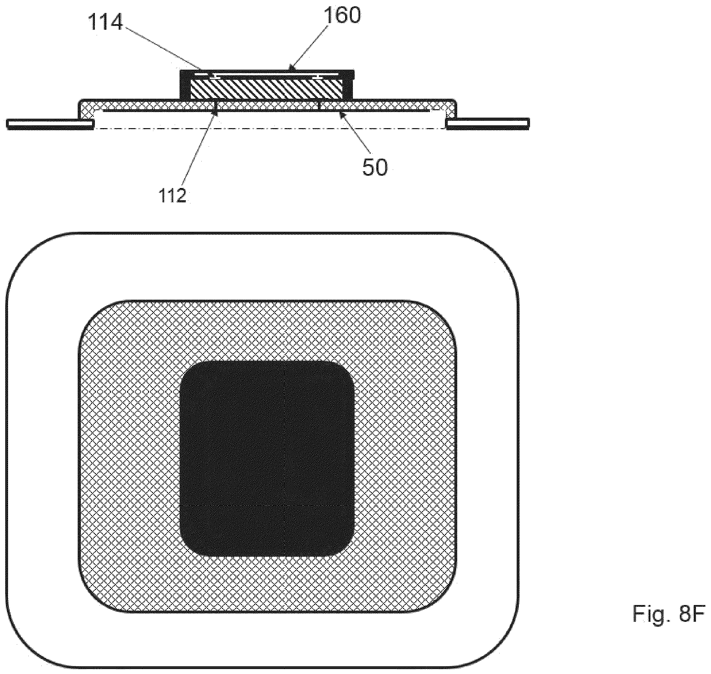

**Figur 8F** zeigt einen Plasmaapplikator mit einer integrierten Energieversorgungseinheit 120" mit einem Energiespeicher, der mittels einer ebenfalls intergierten, induktiven Ladevorrichtung 160 geladen werden kann.

In der gezeigten Ausführungsform befindet sich der elektrotechnische Kern 50 an der der zu behandelnden Oberfläche zugewandten Seite des Plasmaapplikators und ist mittels Kontakten 112 elektrisch mit einem in den Plasmaapplikatorintegrierten aufladbaren Energiespeicher verbunden. Der aufladbare Energiespeicher kann z.B. ein Akkumulator oder ein Kondensator sein.

An seiner Oberseite weist der Energiespeicher zwei separate Kontakte 114 auf, die den Energiespeicher elektrisch mit der Ladevorrichtung 160, insbesondere einer Empfangs-Spulenanordnung, verbinden. Mittels induktiver Kopplung kann elektrische Energie von einer handelsüblichen Ladestation an die Ladevorrichtung 160 zum Laden des Energiespeichers gesendet werden. In den Energiespeicher ist eine elektrische Schaltung integriert, die ausgebildet ist, ein durch den Energiespeicher bereitgestelltes Gleichspannungssignal in ein zum Zünden eines Plasmas ausreichendes Spannungssignal zu transformieren. Das transformierte Spannungssignal wird dann mittels der elektrischen Kontakte 112 an die wenigstens eine Elektrodenstruktur im elektrotechnischen Kern 50 übertragen.

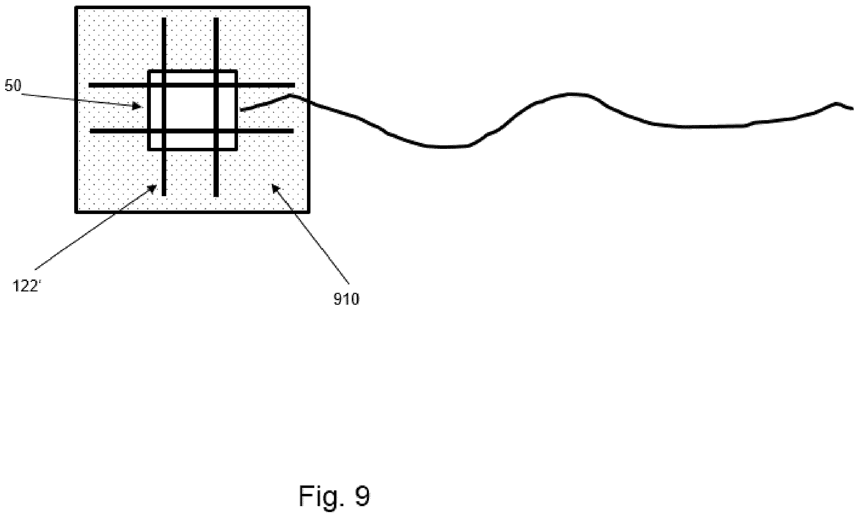

**Figur 9** zeigt einen Plasmaapplikator mit elektrotechnischem Kern 50, der mit Varianten derjenigen Merkmale, die eine Einmalverwendung sicherstellen und mit den beschriebenen Varianten für eine mobile Energieversorgung kombiniert werden kann. Der elektrotechnische Kern 50 kann insbesondere eine erste Isolationsschicht, gefolgt von einer ersten, geerdeten Elektrodenstruktur, gefolgt von einer zweiten Isolationsschicht, gefolgt von einer im Betrieb getriebenen zweiten Elektrodenstruktur, gefolgt von einer dritten Isolationsschicht, gefolgt von einer dritten, geerdeten Elektrodenstruktur aufweisen und somit an sich bereits einen Berührungsschutz gewährleisten.

Der gezeigte Plasmaapplikator weist eine skalierbare Distanzstruktur 122` auf. Die skalierbare Distanzstruktur 122` kann z.B. aus Silikon, Kunststoff oder Textil bestehen und hat eine Stützfunktion für eine Wundabdeckung 910, um einen definierten Abstand zwischen der zu behandelnden Oberfläche und der der zu behandelnden Oberfläche zugewandten Seite des Plasmaapplikators herzustellen. Durch eine skalierbare Distanzstruktur kann ein Plasmaapplikator durch z.B. reißen oder schneiden einer Distanzstruktur an unterschiedliche Wundgrößen angepasst werden. Grundsätzlich sind alle in der Klinik oder der ambulanten Versorgung vorrätigen mechanischen Trennwerkzeuge bzw. -verfahren möglich, um die Größe einer Distanzstruktur an eine Wundgröße anzupassen. Der Plasmaapplikator selbst bleibt von der Skalierungsmethode unberührt. In der gezeigten Ausführungsform ist die Distanzstruktur gasdurchlässig und biegsam. Durch einen Anwender kann die Größe einer Distanzstruktur durch Schneiden oder eine alternative Methode an eine finale Form angepasst werden.

Optional kann ein Plasmaapplikator an eine Distanzstruktur über eine definierte Verbindung, durch Klammern oder Klebstoff oder durch an der Oberfläche einer Distanzstruktur vorgesehene Klebestellen befestigt werden. Eine Distanzstruktur ist bevorzugt gitterförmig ausgeführt und weist mittig einen Bereich auf, in dem der elektrotechnische Kern angeordnet ist bzw. befestigt werden kann. In diesem Bereich sind bevorzugt eine höhere Anzahl an Gitterstrukturen vorgesehen, sodass der Plasmaapplikator einen ausreichend festen Halt auf der Distanzstruktur hat. Mit z.B. einem Wundpflaster, einer Klebefolie, einem Duschpflaster, einer Mullbinde oder sonstigem Verbandmaterial kann die mit einem Plasmaapplikator verbundene und auf die Wundgröße angepasste Distanzstruktur über oder auf der Wunde fixiert werden, sodass ein abgeschlossener Gasraum um den Plasmaapplikator und die Distanzstruktur zwischen der zu behandelnden Oberfläche und der Wundabdeckung 910 entsteht.

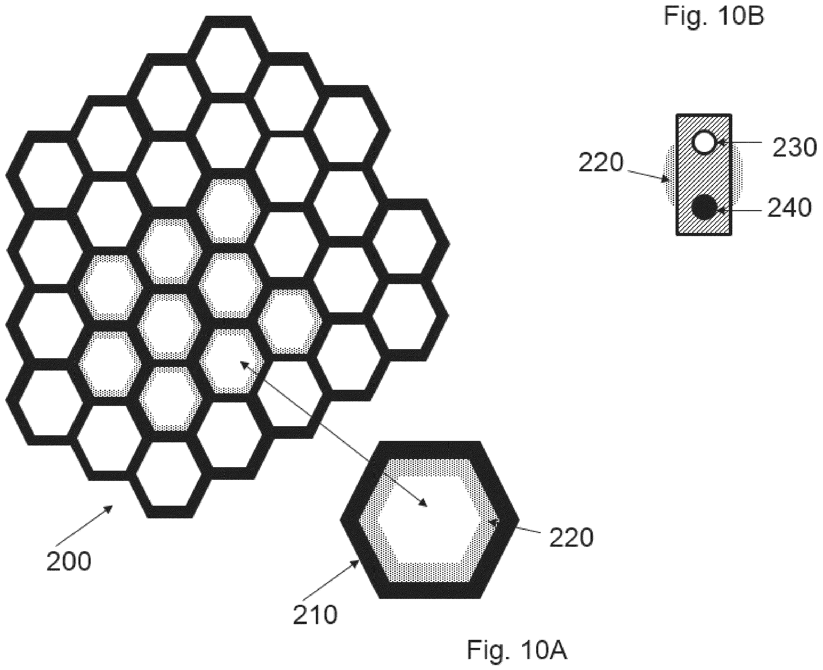

In **Figur 10A** ist eine Distanzstruktur 200 gezeigt, die gleichzeitig die dielektrisch behinderte Entladung (DBE) ist. Die Distanzstruktur weist die Form mehrerer aneinandergrenzender Waben auf. Wie in Bezug auf einen Ausschnitt 210 einer Wabe der Distanzstruktur gezeigt ist, breitet sich ein gezündetes Plasmas 220 (durch den schraffierten Bereich angedeutet) an den Rändern einer Wabe 210 aus. Eine Distanzstruktur kann z.B. aus einem 2-adrigen Flachkabel hergestellt sein. In **Figur 10B** ist ein Querschnitt der Distanzstruktur 200 gezeigt. Hier sind die getriebene Elektrodenstruktur 230 und die Gegenelektrode 240, welche typischerweise auf Erdpotenzial liegt, sichtbar. Ein Plasma 220 brennt dann jeweils rechts und links vom Kabel bzw. innerhalb und außenhalb der Wabe.

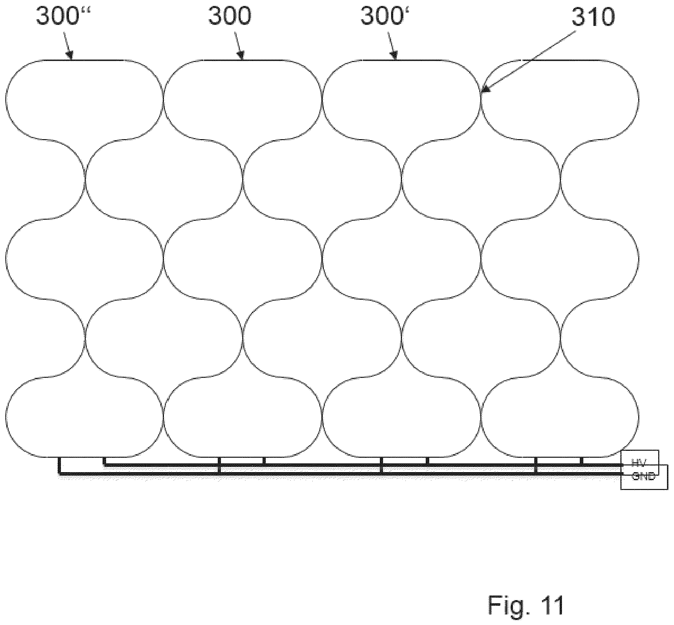

In **Figur 11** zeigt einen geschlossenen Stromkreis 300, der ein zweiadriges Kabel aufweist. Der geschlossene Stromkreis 300 weist Verbindungsstellen 310 auf, die z.B. Klebestelle, Schweißstelle, Lötstelle sein können. An den Verbindungsstellen 310 ist die Ummantelungen der beiden Kabel fest miteinander verbunden. Der in Fig. 11 gezeigte Stromkreis 300 stellt eine Möglichkeit dar, wie ein Abstandhalter einer Distanzstruktur aus Flachkabeln aufgebaut werden kann. Die Kabelabschnitte liegen hierbei hochkant, das Plasma brennt also rechts und links vom Kabel, wie in Fig. 10B gezeigt. Die Kabelabschnitte sind wellenartig geschwungen, ein geschlossener Stromkreis 300 ist vollkommen autark von anderen geschlossenen Stromkreisen, z.B. 300' und 300", die jeweils von einem anderen Kabel gebildet werden. An einer Längsseite der von den Kabeln gebildeten Elektrodenstruktur sind alle Kabel elektrisch miteinander kontaktiert. Die Form ist so gewählt, um möglichst einfach mit wenig Fertigungsaufwand hergestellt werden zu können.

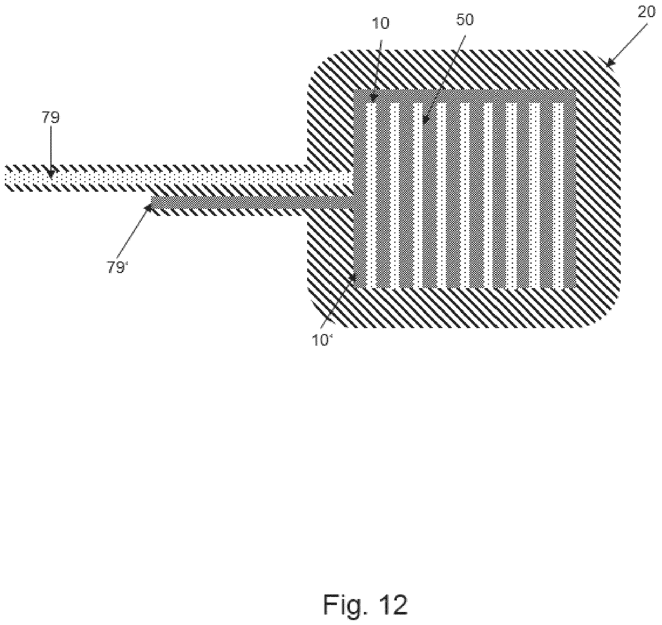

**Figur 12** zeigt eine Draufsicht auf die einer zu behandelnden Oberfläche zugewandten Seite eines elektrotechnischen Kerns 50 mit einer Isolationsschicht 20, die zwischen einer getriebenen Elektrodenstruktur 10 und einer Gegenelektrode 10' angeordnet ist. Jeweils eine der Leiterbahnen 79, 79' ist mit der getriebenen Elektrodenstruktur 10 und der Gegenelektrode 10' elektrisch verbunden und sind Leiterbahnen einer Chipkarten-förmigen Steckvorrichtung.

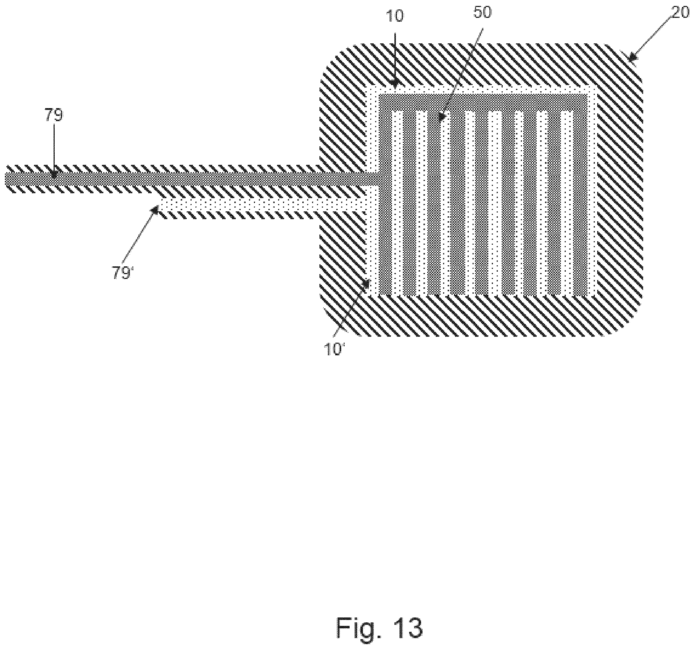

**Figur 13** zeigt eine Draufsicht auf eine der zu behandelnden Oberfläche abgewandten Seite des in Figur 12 gezeigten elektrotechnischen Kerns 50 mit einer Isolationsschicht 20, die zwischen einer im Betrieb durch ein Spannungssignal getriebenen Elektrodenstruktur 10 und einer Gegenelektrode 10' angeordnet ist. Jeweils eine der Leiterbahnen 79, 79' ist mit der getriebenen Elektrodenstruktur 10 und der Gegenelektrode 10' elektrisch verbunden und sind Leiterbahnen einer Chipkarten-förmigen Steckvorrichtung.

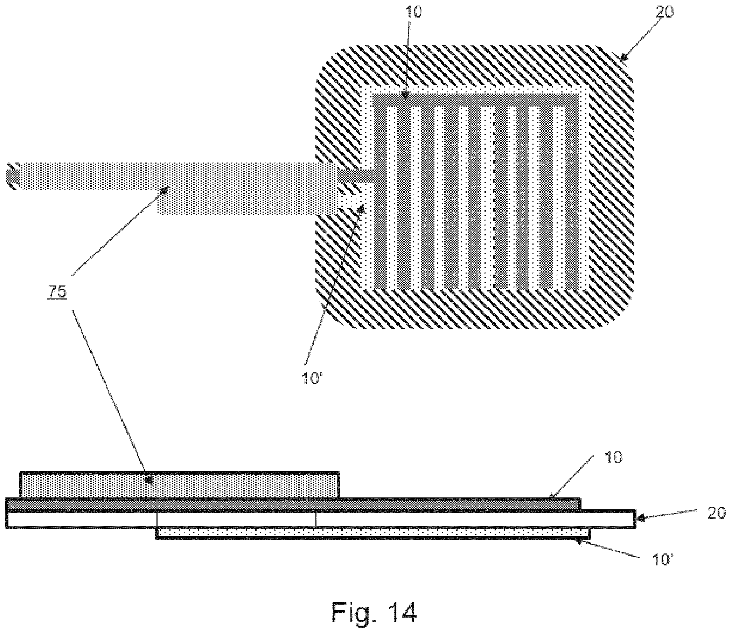

**Figur 14** zeigt eine Draufsicht auf die der zu behandelnden Oberfläche abgewandten Seite des in Figur 13 gezeigten elektrotechnischen Kerns 50 mit einer Isolationsschicht 20, die zwischen einer getriebenen Elektrodenstruktur 10 und einer Gegenelektrode 10` angeordnet ist. In der gezeigten Darstellung ist eine chipkartenförmige Versteifung 75 auf die Leiterbahnen 79, 79' der Steckvorrichtung geleimt, laminiert, geklebt etc. Die Gegenelektrode 10` befindet sich auf der der zu behandelnden Oberfläche zugewandten Seite.

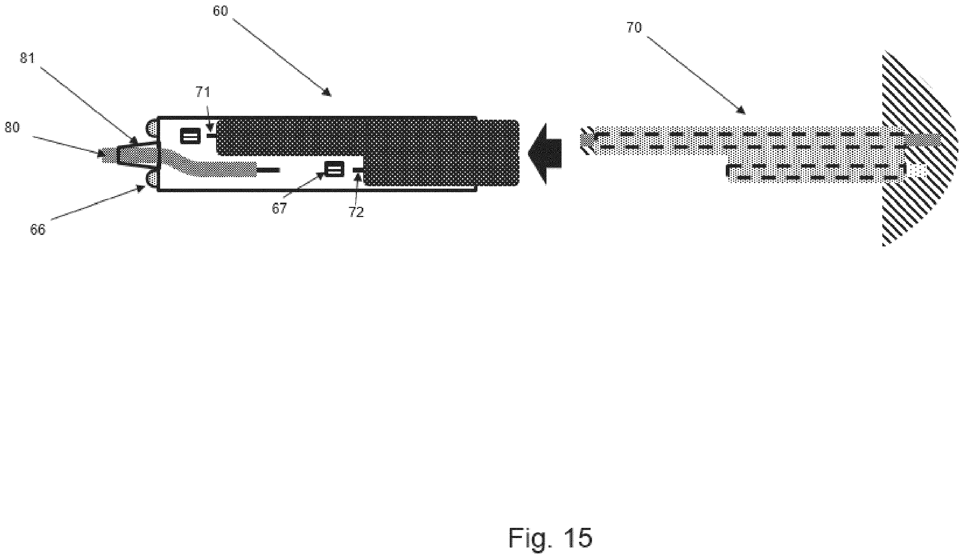

**Figur 15** zeigt eine Einschubvorrichtung 60 mit einem Spannungsanschluss 71 und einem Masseanschluss 72. Weiterhin weist die Einschubvorrichtung 60 Induktivitäten 67 und Verschlusstopfen 66 auf. Die Einschubvorrichtung 60 ist über ein mehrfach geschirmtes Kabel 80 mit einer nicht gezeigten Energieversorgungseinheit verbunden. Zur Stabilisierung des Kabels 80 ist ein Knickschutz 81 vorgesehen. In Fig. 15 ist angedeutet, wie eine Steckvorrichtung 70 in die Einschubvorrichtung 60 geschoben wird, um ein durch eine Energieversorgungseinheit bereitgestelltes Spannungssignal an eine durch das Spannungssignal getriebene Elektrodenstruktur (nicht gezeigt) zu übertragen.

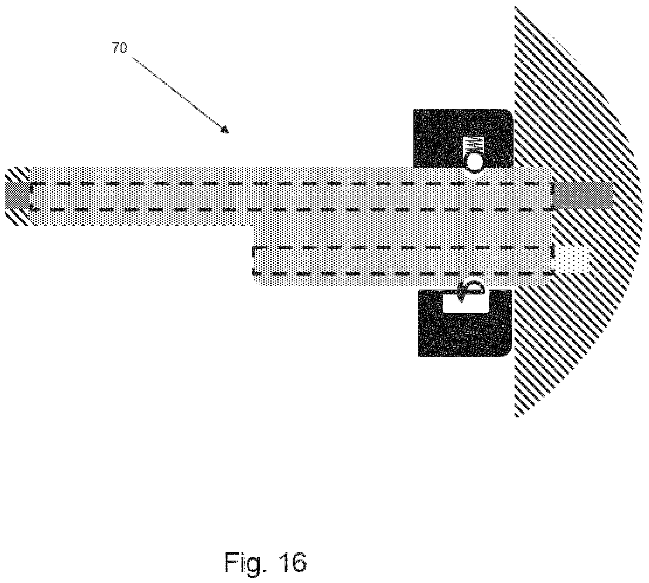

In **Figur 16** ist angedeutet, wie ein mechanisch fester Sitz zwischen einer Steckvorrichtung 70 und einer hier lediglich durch Einrastelemente angedeutete Einschubvorrichtung hergestellt werden kann. Über eine Feder mit Kugel auf beiden Seiten einer Einschubvorrichtung und einer entsprechenden Aussparung in der Steckkontaktvorrichtung 70 wird in der gezeigten Ausführungsform ein mechanisch fester Sitz realisiert. In einer hier nicht gezeigten Ausführungsform kann auch ein Federarm mit entsprechend passender Beule in eine dafür vorgesehene Aussparung einrasten.

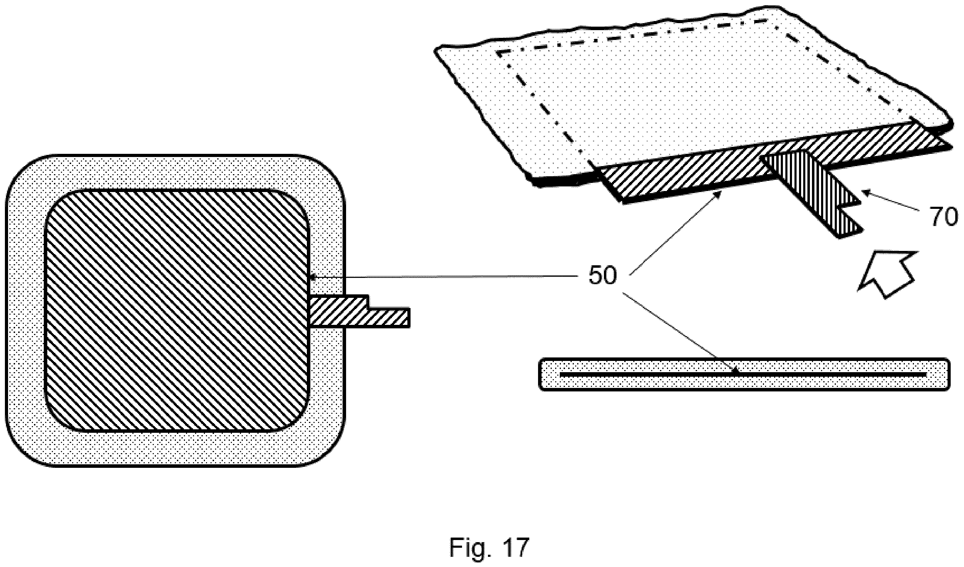

**Figur 17** zeigt einen elektrotechnischen Kern 50, der in eine Saugkompresse integriert ist. In einer hier nicht gezeigten Ausführungsform ist eine Kompresse um einen elektrotechnischen Kern herum gepresst bzw. genäht. Die Kompresse stellt in diesem Fall die Umschließung des elektrotechnischen Kerns dar. Bevorzugt ist der elektrotechnische Kern 50 vollständig von Mull bzw. einer Kompresse und/oder einem Textil umschlossen. In einer hier nicht gezeigten Ausführungsform ist die Oberseite (körperabgewandt) mit einer Folie luftdicht abgeschlossen und an der der zu behandelnden Oberfläche zugewandten Seite eine Adhäsionsschicht aufgebracht.

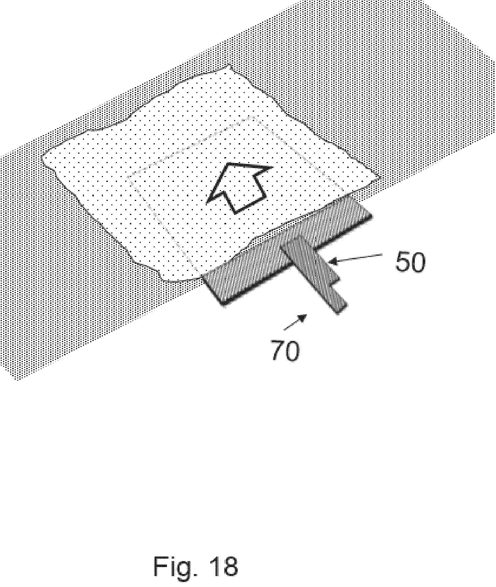

**Figur 18** zeigt einen elektrotechnische Kern 50 der in eine bereits vorhandene Lasche eines Verbands bzw. eines Textils z.B. eingeschoben oder auch eingenäht ist. Die Lasche ist bevorzugt derart ausgebildet, dass der elektrotechnische Kern 50 im vollständig eingeschobenen bzw. eingenähten Zustand komplett in der Lasche verschwindet.

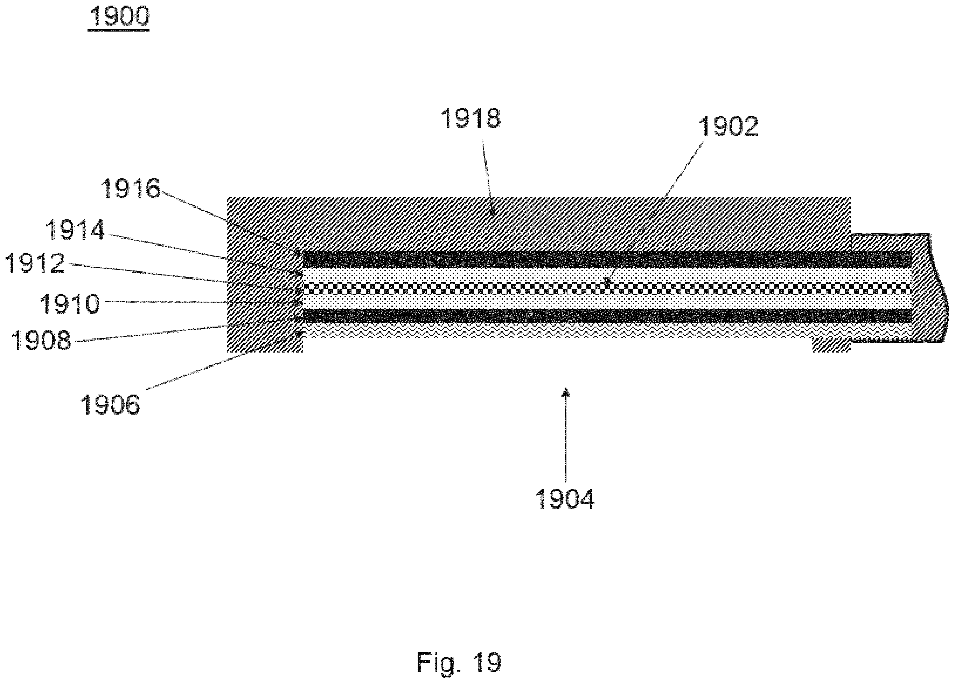

**Figur 19** zeigt einen Querschnitt durch einen besonders bevorzugten Plasmaapplikator 1900 mit einem elektrotechnischen Kern 1902. Der elektrotechnische Kern 1902 umfasst sechs flache, schichtartige Strukturen, die flächig ausgebildet und in einer Stapelfolge als Schichten übereinander angeordnet sind. Auf der Seite 1904 des elektrotechnischen Kerns 1902, die im Anwendungsfall der menschlichen oder tierischen oder technischen Oberfläche zugewandt ist, befindet sich eine flächig ausgebildete erste Isolationsschicht 1906 aus einem biokompatiblen Material. Wenn der Plasmaapplikator 1900 auf der menschlichen oder tierischen oder technischen Oberfläche aufgebracht ist, kann die erste Isolationsschicht 1906 in direktem Kontakt mit der entsprechenden Oberfläche sein. In verschiedenen Varianten umfasst die erste Isolationsschicht eine elektrisch isolierende Folie und/oder einen elektrisch isolierenden Lack und/oder eine elektrisch isolierenden Klebstoffschicht und/oder Silikon.

In Richtung der der zu behandelnden Oberfläche abgewandten Seite folgt auf die erste Isolationsschicht 1906 eine erste Elektrodenstruktur 1908, die die Funktion einer Massenelektrode hat. In der gezeigten Ausführungsform weist die erste Elektrodenstruktur 1908 eine spezielle Geometrie auf, die in verschiedenen Varianten der gezeigten Ausführungsform mäanderförmig, spiralförmig, durch eine Fläche mit Löchern gebildet, quadratisch, U-förmig, E-förmig, M-förmig, L-förmig, C-förmig, X-förmig oder O-förmig ist. Die erste Elektrodenstruktur 1908 wird bevorzugt im Siebdruckverfahren hergestellt und hat eine Dicke, die zwischen 5 µm und 200 µm beträgt. In einer hier nicht gezeigten Ausführungsform ist die erste Elektrodenstruktur 1908 als Flächenelektrode mit einer geschlossenen Fläche ausgebildet.

Auf die erste Elektrodenstruktur in Form einer Massenelektrode 1908 folgt eine zweite Isolationsschicht 1910, die vollflächig, d.h. als geschlossene Fläche, ausgebildet ist. Die zweite Isolationsschicht 1910 umfasst in verschiedenen Varianten z.B. eine elektrisch isolierende Folie und/oder einen elektrisch isolierenden Lack und/oder einen elektrisch isolierenden Klebstoffschicht und/oder Silikon und hat eine Dicke, die zwischen 50 µm und 200 µm, vorzugsweise zwischen 75 µm und 100 µm beträgt

Auf der zweiten Isolationsschicht 1910 ist eine zweite Elektrodenstruktur 1912 angeordnet, die im Anwendungsfall durch ein Spannungssignal zum Erzeugen eines Plasmas getrieben wird. Diese im Anwendungsfall durch ein Spannungssignal getriebene zweite Elektrodenstruktur 1912 weist ebenfalls eine spezielle Geometrie auf. Optional kann die zweite Elektrodenstruktur auch als Flächenelektrode ausgebildet sein. Die im Anwendungsfall getriebene, zweite Elektrodenstruktur 1912 wird bevorzugt im Siebdruckverfahren hergestellt und hat eine Dicke, die zwischen 5 µm und 200 µm vorzugsweise zwischen 5 µm und 100 µm, bevorzugt zwischen 5 µm und 20 µm beträgt.

Die zwischen der ersten auf Massepotential liegenden Elektrodenstruktur 1908 und der im Anwendungsfall getriebenen zweiten Elektrodenstruktur 1912 angeordnete zweite Isolationsschicht 1910, bewirkt eine galvanische Trennung der beiden Elektrodenstrukturen.

Auf die im Anwendungsfall durch ein Spannungssignal getriebene zweite Elektrodenstruktur 1912 folgt eine dritte Isolationsschicht 1914, die bevorzugt eine elektrisch isolierende Folie und/oder einen elektrisch isolierenden Klebstoffschicht umfasst.

Auf der dritten Isolationsschicht 1914 ist eine dritte Elektrodenstruktur 1916 angeordnet. Diese dritte Elektrodenstruktur 1916, die als Flächenelektrode, bevorzugt als eine elektrisch leitfähige Folie, ausgebildet ist, wird im Anwendungsfall auf Erdpotential gelegt. Die dritte Elektrodenstruktur 1916 hat eine Dicke, die bevorzugt zwischen 20 µm und 200 µm, vorzugsweise zwischen 20 µm und 100 µm beträgt. Die dritte Elektrodenstruktur 1916 erfüllt im Anwendungsfall die Funktion eines Berührungsschutzes sowie zur EMV-Abschirmung. Das heißt, dass die dritte Elektrodenstruktur 1916 im Betrieb eine Feldfreiheit zwischen der getriebenen, zweiten Elektrodenstruktur 1912 und einem direkt auf der Außenseite des elektrotechnischen Kerns anliegenden Erdpotential gewährleistet.

Die zwischen der im Anwendungsfall getriebenen, zweiten Elektrodenstruktur 1912 und der dritte Elektrodenstruktur 1916 angeordnete dritte Isolationsschicht 1914 ist vollflächig ausgebildet und bewirkt eine vollflächige elektrische Isolation bzw. galvanische Trennung der dritte Elektrodenstruktur 1916 von der zweiten Elektrodenstruktur 1912.

In dem gezeigten elektrotechnischen Kern 1902 ist also zusätzlich zu einer ersten Massenelektrode 1908 eine zweite Massenelektrode 1916 vorgesehen, die durch eine dritte Isolationsschicht 1914 galvanisch von der im Anwendungsfall getriebenen, zweite Elektrodenstruktur 1912 getrennt ist. Dadurch ist im Betrieb ein Berührungsschutz bereits durch den elektrotechnischen Kern 1902 selbst realisiert. Durch die dritte Elektrodenstruktur 1916 wird verhindert, dass es im Betrieb zu einem elektrischen Durchschlag zwischen der getriebenen, zweiten Elektrodenstruktur 1912 und einem außerhalb des elektrotechnischen Kerns anliegenden Erdpotential oder einem virtuellen Erdpotential in Form der zu behandelnden Oberfläche oder eines Menschen oder Tieres kommt. Vorteilhafterweise kann dadurch eine Umschließung 1918 vergleichsweise einfach aufgebaut sein, da eine Umschließung 1918 nicht mehr notwendigerweise die Funktion eines Berührungsschutzes erfüllen muss. Insbesondere kann bei dem hier beschrieben elektrotechnischen Kern auf eine aufwendige Umschließung aus einer ersten und zweiten und dritten Spritzgussschicht verzichtet werden. Bei herkömmlichen elektrotechnischen Kernen ohne eine dritte Isolationsschicht und eine dritte Elektrodenstruktur, ist eine Umschließung aus einer ersten und zweiten und dritten Spritzgussschicht typischerweise notwendig, da von der patientenabgewandten Seite beginnend die erste Spritzgussschicht aus biokompatiblem Silikon, die zweite Spritzgussschicht aus leitfähigem Silikon, welches im Betrieb auf Massepotenzial liegt, und die dritte Spritzgussschicht aus biokompatiblem Silikon besteht. Die Umschlie-βung soll also eine Verträglichkeit im Kontakt mit einer zu behandelnden Oberfläche und gleichzeitig einen Berührungsschutz gewährleisten. Ein solche Umschließung ist vergleichsweise aufwendig in der Fertigung.

In dem hier beschrieben elektrotechnischen Kern sind die Funktionen der ersten und zweiten Spritzgussschicht als Strukturen und Schichten in den elektrotechnischen Kern selbst in Form von dünnen Folien integriert. Insbesondere kann ein hier beschriebener elektrotechnischer Kern mit nur einer Spritzgussschicht aus Silikon umspritzt werden.

Ein solcher elektrotechnischer Kern ist vorteilhafterweise selbst berührungssicher und EMV-sicher. Besonders vorteilhaft kann ein solcher elektrotechnischer Kern als Modul verwendet und in beliebige Plasmaapplikatoren oder Umschließungen intergiert werden. Beispielsweise kann ein hier beschriebener elektrotechnischer Kern in eine Kompresse, in einen Superabsorber, in Schuhsohlen, in Kompressionsstrümpfe, in Bekleidung, integriert werden.

Besonders vorteilhaft ist, dass ein solcher elektrotechnischer Kern mit einer Dicke von 300 µm und weniger hergestellt werden kann. Ein solcher elektrotechnischer Kern hat eine vergleichsweise geringe Fertigungstiefe und kann beispielsweise als Folienlaminat hergestellt werden. Vorteilhafterweise lässt sich ein hier beschriebener elektrotechnischer Kern deutlich einfacher und preiswerter herstellen, ist vorzugsweise nach wie vor vergleichsweise flach, biegsam und sehr flexibel bzgl. eines Einsatzes. Vorteilhafterweise kann ein hier beschriebener elektrotechnischer Kern in demselben Fertigungsprozess mit einer Steckvorrichtung in Form einer Lasche hergestellt werden.

Entsprechend weist der hier gezeigte elektrotechnische Kern 1902 sechs Schichten auf, wobei sich in der Stapelfolge der sechs Schichten jeweils eine Isolationsschicht 1906, 1910, 1914 und eine Elektrodenstruktur 1908, 1912, 1916 abwechseln. Ein wie in Figur 19 gezeigter elektrotechnischer Kern 1902 lässt sich mit einem vergleichsweise geringen Produktionsaufwand und mit vergleichsweise geringen Produktionskosten herstellen.

Zum Herstellen des gezeigten elektrotechnischen Kerns 1902 wird auf die im Betrieb durch ein Spannungssignal getriebene, zweite Elektrodenstruktur 1912 eine durch eine Folie gebildete dritte Isolationsschicht 1914 auflaminiert. Insbesondere durch einen zum Laminieren verwendeten Klebstoff kann die elektrisch isolierende Wirkung der dritten Isolationsschicht verstärkt werden. Auf die dritte Isolationsschicht 1914 wird dann die dritte Elektrodenstruktur 1916 aufgebracht. Es ist auch denkbar, dass die dritte Isolationsschicht durch den Kleber zum Laminieren und nicht als gesonderte Folie gebildet ist. Die dritte Elektrodenstruktur kann in diesem Fall direkt auf die zweite Elektrodenstruktur laminier werden, wobei der Kleber zwischen der zweiten und der dritten Elektrodenstruktur die dritte Isolationsschicht darstellt.

In der gezeigten Darstellung sind die Dicken der einzelnen Schichten des Mehrschichtsystems derart gewählt, dass die Gesamtdicke entlang der Stapelfolge des gezeigten elektrotechnischen Kerns 1902, innerhalb der üblichen Fehlertoleranzen, etwa 200 µm bis 300 µm beträgt. Somit wird garantiert, dass der elektrotechnische Kern 1902 vergleichsweise gut elastisch verformbar ist und vergleichsweise leicht an verschiedene Körper- und/oder Oberflächenformen angepasst werden kann.

In dem dargestellten Ausführungsbeispiel sind die beschriebenen Schichten des elektrotechnischen Kerns 1902 als Laminat hergestellt. Der elektrotechnische Kern 1902 besteht also aus einem Folienlaminat.

Die gezeigte Ausführungsform weist eine Umschließung 1918 aus einem biokompatiblen Material auf. Ein geeignetes biokompatibles Material 1918 ist beispielsweise medizinisches Silikon, Lack, Mull, Textilien, Absorber oder Klebstoff oder eine Kombination aus den genannten Materialien.

Da die im Anwendungsfall geerdete, dritte Elektrodenstruktur 1916 die Funktion eines Berührungsschutzes und der EMV-Verträglichkeit erfüllt, kann die Umschließung 1918 der gezeigten Ausführungsform z.B. durch eine einfache Silikonumspritzung realisiert sein. Die gezeigte Umschließung 1918 ist also vergleichsweise einfach aufgebaut.

In der gezeigten Ausführungsform ist der elektrotechnische Kern 1902 nur teilweise von der Umschließung 1918 umschlossen. Insbesondere auf derjenigen Seite 1904 des elektrotechnischen Kerns 1902, die während einer Plasmabehandlung der zu behandelnden Oberfläche zugewandt ist, kann keine Umschließung 1918 vorgesehen sein. Die Umschlie-βung 1918 kann beispielsweise im Spritzgussverfahren, im Tauchverfahren oder im Lackierverfahren hergestellt sein. In einer hier nicht gezeigten Ausführungsform ist ein elektrotechnischer Kern vollständig von einer Umschließung z.B. in Form eines Textils oder Mull oder Kompressen umschlossen.

In einer hier nicht gezeigten Ausführungsform ist ein elektrotechnischer Kern und insbesondere die im Anwendungsfall getriebene, zweite Elektrodenstruktur mit einer Steckvorrichtung elektrisch verbunden. Eine solche Steckvorrichtung ist bevorzugt chipkartenförmig und wie in Bezug auf Fig. 2 beschrieben ausgebildet. In nicht gezeigten Varianten der hier gezeigten Ausführungsform weist eine Steckvorrichtung oder ein elektrotechnischer Kern wenigstens ein Merkmal auf, welches eine Einmalverwendung des Plasmaapplikators sicherstellt. Ein solches Merkmal kann beispielsweise durch eine wie in Fig. 6 gezeigte Verjüngung einer Leiterbahn der Steckvorrichtung oder eine Verjüngung eines Elektrodenabschnitts einer Elektrodenstruktur im elektrotechnischen Kern oder wie in Bezug auf Fig. 16 beschriebene Einrastelemente realisiert sein.

In einer hier nicht gezeigten Ausführungsform umfasst ein Plasmaapplikator eine integrierte Energieversorgungseinheit und eine Steckvorrichtung. Wie in Bezug auf Fig. 8B beschrieben, kann die integrierte Energieversorgungseinheit über die Steckvorrichtung mit einer mobilen oder einer stationären Energieversorgung verbunden und geladen werden.

In einer weiteren hier nicht gezeigten Ausführungsform umfasst ein Plasmaapplikator eine integrierte Energieversorgungseinheit mit einem Energiespeicher aber keine Steckvorrichtung. Wie in Bezug auf Fig. 8C beschrieben, ist die integrierte Energieversorgungseinheit mit dem elektrotechnischen Kern zur Energieversorgung desselben zum Zünden eines physikalischen Plasmas elektrisch verbunden.

In einerweiteren hier nicht gezeigten Ausführungsform umfasst ein Plasmaapplikator einen Einschubschlitz, der zur Aufnahme einer mobilen Energieversorgungseinheit ausgebildet ist. Wie in Bezug auf Fig. 8D beschrieben, kann der Plasmaapplikator Kontakte aufweisen, die insbesondere die im Anwendungsfall getriebene Elektrodenstruktur des elektrotechnischen Kerns mit der Oberseite des Plasmaapplikators verbinden. Die Kontakte weisen an der Oberfläche freie Kontaktflächen auf, durch die eine galvanische Kopplung mit einem Energiespeicher einer mobilen Energieversorgungseinheit hergestellt werden kann, wenn eine mobile Energieversorgungseinheit in den Einschubschlitz des Plasmaapplikators eingeschoben ist.

In einer weiteren hier nicht gezeigten Ausführungsform umfasst ein Plasmaapplikator eine integrierte Empfangs-Spulenanordnung und einen Einschubschlitz, in den eine mobile Energieversorgungseinheit mit einer Sende-Spulenanordnung geschoben werden kann.

Wie in Bezug auf Fig. 8E beschrieben, kann mittels der Sende-Spulenanordnung durch einen in die mobile Energieversorgungseinheit integrierten Energiespeicher bereitgestellte elektrische Energie an die Empfangs-Spulenanordnung des Plasmaapplikators mittels induktiver Kopplung zur Energieversorgung des elektrotechnischen Kerns und somit zum Zünden eines physikalischen Plasmas übertragen werden.

In einer weiteren hier nicht gezeigten Ausführungsform umfasst ein Plasmaapplikator eine integrierte Energieversorgungseinheit mit einem Akkumulator oder einen Kondensator, der mittels einer ebenfalls intergierten Ladevorrichtung geladen werden kann. Wie in Bezug auf Fig. 8F beschrieben, kann mittels induktiver Kopplung elektrische Energie von einer handelsüblichen Ladestation an die Ladevorrichtung zum Laden eines Energiespeichers der integrierten Energieversorgungseinheit zur Energieversorgung des elektrotechnischen Kerns gesendet werden.

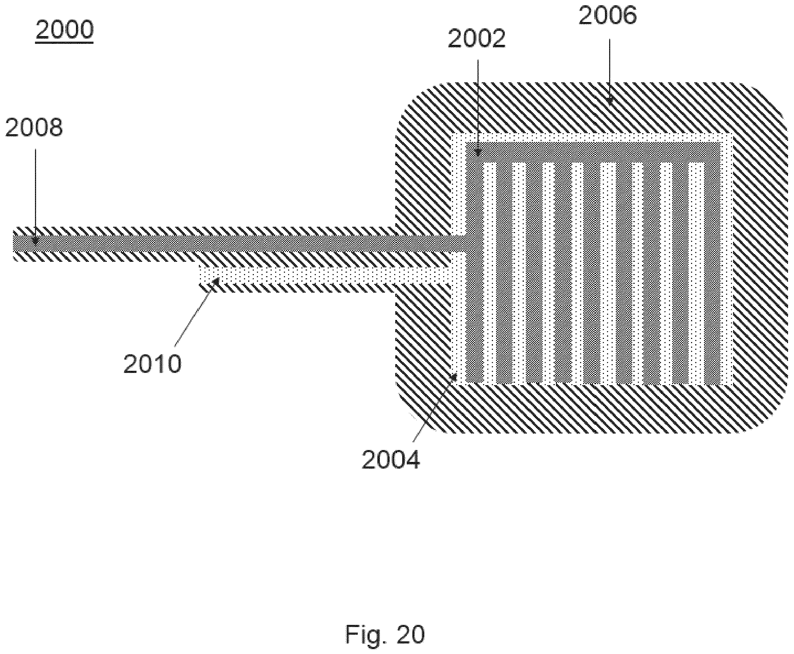

Die im Folgenden beschriebenen **Figuren 20****,** **21****,** **22** **und** **23** zeigen jeweils ausgewählte Zwischenprodukte eines Herstellungsverfahrens zum Herstellen eines wie in Fig. 19 gezeigten elektrotechnischen Kerns.

**Figur 20** zeigt eine Draufsicht auf diejenige Seite eines elektrotechnischen Kerns 2000, die während einer Plasmabehandlung von der zu behandelnden Oberfläche abgewandt ist. Der elektrotechnische Kern 2000 umfasst eine im Betrieb durch ein Spannungssignal getriebene, zweite Elektrodenstruktur 2002. Die im Anwendungsfall getriebene, zweite Elektrodenstruktur 2002 weist eine spezielle Geometrie auf, die Kamm-förmig ausgebildet ist. In Richtung der der zu behandelnden Seite zugewandten Seite folgt auf die im Anwendungsfall getriebene, zweite Elektrodenstruktur 2002 eine zweite Isolationsschicht 2006 und auf diese zweite Isolationsschicht eine geerdete, erste Elektrodenstruktur 2004, die diejenige Massenelektrode darstellt, die im Anwendungsfall der zu behandelnden Oberfläche zugewandt ist. Beide, die im Anwendungsfall getriebene, zweite Elektrodenstruktur 2002 und die erste Elektrodenstruktur 2004, weisen jeweils eine Leiterbahn 2008, 2010 auf, die von einer Längsseite des elektrotechnischen Kerns 2000 von den entsprechenden Elektrodenstrukturen 2002, 2004 senkrecht in derselben horizontalen Ebene weggeführt. Diese Leiterbahnen 2008, 2010 bilden die Leiterbahnen einer Steckvorrichtung. Entsprechende Leiterbahnen der Steckvorrichtung sind dann elektrisch leitfähig mit dem elektrotechnischen Kern 2000 verbunden. Optional kann die Steckvorrichtung eine Versteifung aufweisen.

In Richtung der zu behandelnden Oberfläche Seite folgt auf die gezeigte erste Elektrodenstruktur2004 eine erste Isolationsschicht (nicht gezeigt), die im Anwendungsfall in direktem Kontakt mit einer zu behandelnden Oberfläche kommen kann. Die erste Isolationsschicht (nicht gezeigt) ist derart ausgebildet, dass sie auch die Leiterbahnen 2008, 2010 in Richtung einer zu behandelnden Oberfläche elektrisch isoliert.

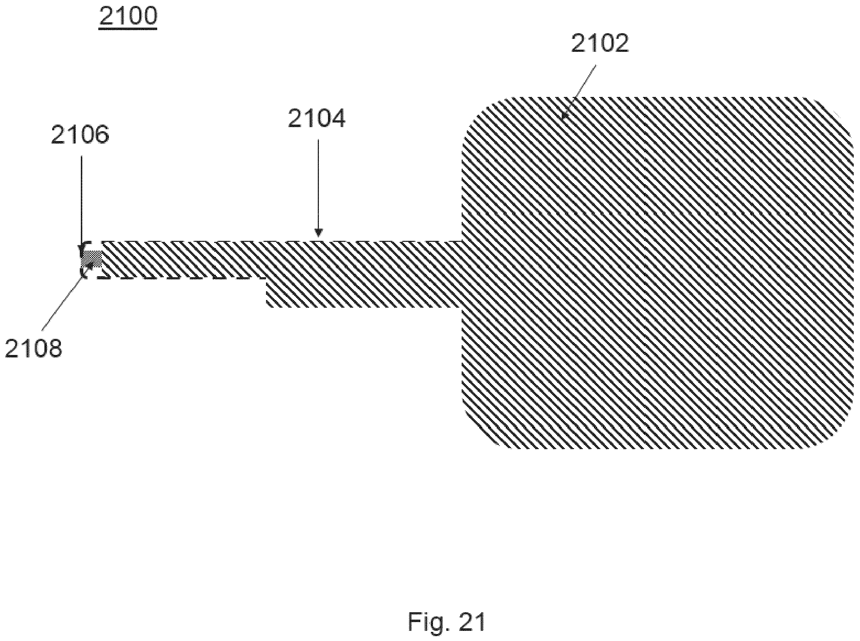

**Figur 21** zeigt eine Draufsicht auf diejenige Seite eines elektrotechnischen Kerns 2100, die während einer Plasmabehandlung von der zu behandelnden Oberfläche abgewandt ist. Von dem gezeigten elektrotechnischen Kern 2100 sieht man im Wesentlichen die dritte Isolationsschicht 2102, die auf der während einer Plasmabehandlung von der zu behandelnden Oberfläche abgewandten Seite auf der im Betrieb getriebenen, zweiten Elektrodenstruktur (nicht gezeigt) angeordnet ist. Diese dritte Isolationsschicht 2102 erfüllt die Funktion, die im Betrieb getriebene, zweite Elektrodenstruktur und eine auf der dritten Isolationsschicht angeordnete dritte Elektrodenstruktur (nicht gezeigt) galvanisch voneinander zu trennen. Die gezeigte dritte Isolationsschicht 2102 ist vollflächig ausgebildet und weist an derjenigen Seite, an der Leiterbahnen von der im Anwendungsfall getriebenen, zweiten Elektrodenstruktur und der an der zu behandelnden Oberfläche zugewandten Seite des elektrotechnischen Kerns 2100 angeordneten ersten Elektrodenstruktur wegführen, eine Lasche 2104 auf, die die beiden Leiterbahnen überdeckt. Die Lasche 2104 schließt jedoch nicht mit dem Ende 2106 der Leiterbahn der im Betrieb getriebenen Elektrodenstruktur ab, sondern endet vorher. Dadurch bleibt eine Kontaktfläche 2108 der Leiterbahn frei, die eine Kontaktierungsfläche zum zwischen Steckvorrichtung und den Kontakten der Einschubvorrichtung zum Übertragen eines Spannungssignals darstellt.

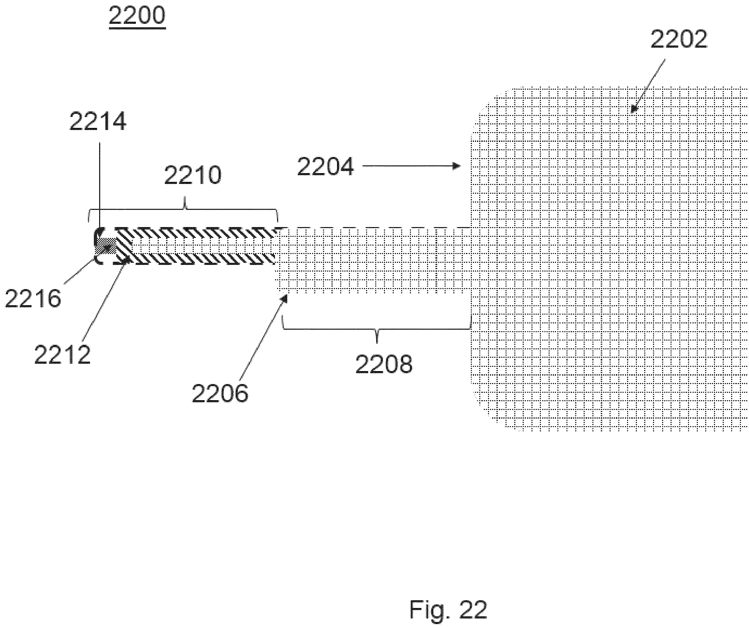

In **Figur 22** ist eine Draufsicht auf diejenige Seite eines elektrotechnischen Kerns 2200 gezeigt, die während einer Plasmabehandlung von der zu behandelnden Oberfläche abgewandt ist. Von dem gezeigten elektrotechnischen Kern 2200 sieht man im Wesentlichen die dritte Elektrodenstruktur 2202, die auf der der zu behandelnden Seite abgewandten Seite angeordnet ist und die durch eine dritte Isolationsschicht (Bezugszeichen 2102 in Fig. 21) von einer im Anwendungsfall getriebenen, zweiten Elektrodenstruktur galvanisch getrennt ist. Diese dritte Elektrodenstruktur 2202 erfüllt die Funktion eines Berührungsschutzes sowie EMV-Schutzes, sodass es im Betrieb eines Plasmaapplikators nicht zu einem elektrischen Durchschlag zwischen einer getriebenen Elektrodenstruktur des elektrotechnischen Kerns 2200 und einem direkt auf der Außenseite anliegenden Erdpotential oder einem virtuellen Erdpotential durch z.B. Berührung eines Patienten oder Anwenders kommt. Die gezeigte dritte Elektrodenstruktur 2202 ist bevorzugt als Flächenelektrode ausgebildet, d.h. sie weist keine spezielle Geometrie auf. An der Seite 2204 des elektrotechnischen Kerns 2200, an der Leiterbahn von der im Anwendungsfall getriebenen, zweiten Elektrodenstruktur und der an der zu behandelnden Oberfläche zugewandten Seite des elektrotechnischen Kerns 2200 angeordneten ersten Elektrodenstruktur wegführen, weist auch die gezeigte dritte Elektrodenstruktur 2202 eine laschenförmige Leiterbahn 2206 auf. Die laschenförmige Leiterbahn 2206 überdeckt den Bereich 2208 vollständig, in dem sich sowohl die Leiterbahn der im Betrieb getriebenen, zweiten Elektrodenstruktur als auch die Leiterbahn der ersten Elektrodenstruktur (nicht gezeigt) befinden. In demjenigen Bereich 2210, der über den Bereich 2208 mit beiden Leiterbahnen hinausgeht und nur noch die Leiterbahn der im Betrieb getriebenen, zweiten Elektrodenstruktur umfasst, hat die laschenförmige Leiterbahn 2206 der gezeigten dritten Elektrodenstruktur 2202 eine ausreichende Breite, dass eine Abschirmung der zweite Elektrodenstruktur gewährleistet wird, ein Berührungsschutz gegeben ist und gleichzeitig keine Plasmaentladung zwischen der dritten Elektrodenstruktur und der zweiten Elektrodenstruktur zünden kann. Die dazwischen angeordnete dritte Isolationsschicht 2212 weist eine größere Breite als die Leiterbahn der im Betrieb getriebenen, zweiten Elektrodenstruktur auf, um eine galvanische Trennung der beiden Elektrodenstrukturen sicherzustellen. Die laschenförmige Leiterbahn 2206 der gezeigten dritten Elektrodenstruktur 2202 endet bereits vor dem Ende 2214 der Lasche der gezeigten dritten Isolationsschicht 2212, sodass weiterhin eine Kontaktfläche 2216 der Leiterbahn der im Betrieb getriebenen, zweiten Elektrodenstruktur frei bleibt, um einen elektrischen Kontakt zwischen der gebildeten Steckvorrichtung und den Kontakten der Einschubvorrichtung zum Übertragen eines Spannungssignals herzustellen.

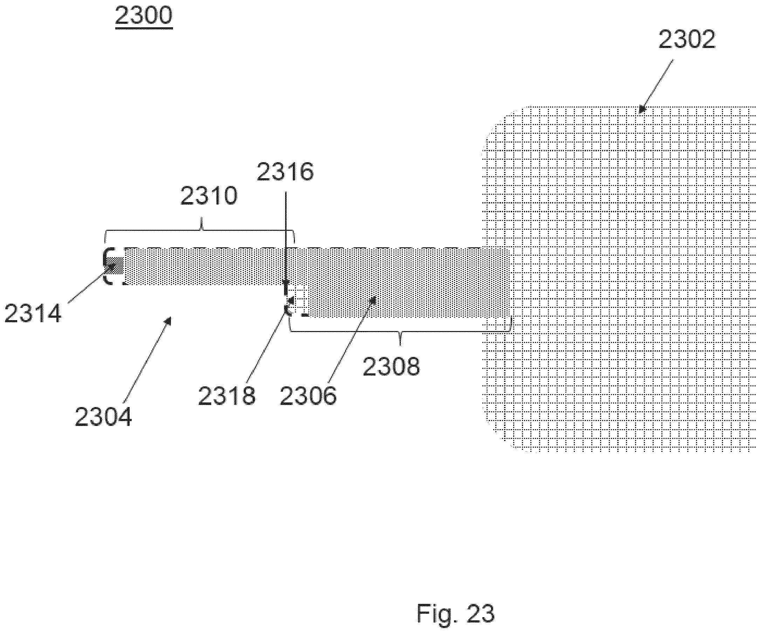

**Figur 23** zeigt eine Draufsicht auf diejenige Seite eines elektrotechnischen Kerns 2300, die während einer Plasmabehandlung von der zu behandelnden Oberfläche abgewandt ist. Von dem gezeigten elektrotechnischen Kern 2300 sieht man im Wesentlichen die dritte Elektrodenstruktur 2302 mit einer laschenförmigen Leiterbahn 2304, wie sie in Bezug auf Fig. 22 beschrieben ist. Zusätzlich zu dem in Fig. 22 gezeigten elektrotechnischen Kern 2200 weist der hier gezeigte elektrotechnische Kern 2300 eine chipkartenförmige Versteifung 2306 auf, die die laschenförmige Leiterbahn der in Bezug auf Figuren 20, 21, und 22 beschriebenen Elektrodenstrukturen und Isolationsschichten sowie laschenförmige Leiterbahnen der nicht beschriebenen aber in dem elektrotechnischen Kern 2300 zusätzlich vorhandenen Elektrodenstrukturen und Laschen der Isolationsschichten umschließt. Die chipkartenförmige Versteifung 2306 hat insbesondere die gleiche Grundform, wie die von ihr eingeschlossenen oder auf einer Seite angeordneten laschenförmigen Leiterbahnen und Laschen, d.h. in dem Bereich 2308, in dem sich sowohl die Leiterstruktur der im Betrieb getriebenen, zweiten Elektrodenstruktur als auch die Leiterbahn der auf Massepotenzial liegenden dritten Elektrodenstruktur befindet, weist auch die chipkartenförmige Struktur 2306 eine größere Breite auf als in dem Bereich 2310, in dem sich nur die Leiterbahn der im Betrieb getriebenen, zweiten Elektrodenstruktur befindet. In dem Bereich 2310, in dem sich nur die Leiterbahn der im Betrieb getriebenen, zweiten Elektrodenstruktur befindet, schließt die chipkartenförmige Versteifung 2306 mit dem Ende der in Fig. 21 gezeigten Isolationsschicht ab, sodass die Kontaktfläche 2314 der Leiterbahn der im Betrieb getriebenen, zweiten Elektrodenstruktur weiterhin frei bleibt.

In dem Bereich 2308, in dem sich sowohl die Leiterbahn der im Betrieb getriebenen, zweiten Elektrodenstruktur als auch die Leiterbahn der dritten Elektrodenstruktur befindet, ist die chipkartenförmige Struktur 2306 derart ausgebildet, dass sie auf derjenigen Seite, auf der sich die Leiterbahn der dritten Elektrodenstruktur 2302 befindet, vor dem Ende 2316 der laschenförmige Leiterbahn 2304 der dritten Elektrodenstruktur 2302 abschließt, sodass auch hier eine Kontaktfläche 2318 der Leiterbahn in Form einer laschenförmige Leiterbahn 2316 der dritten Elektrodenstruktur 2302 frei bleibt.

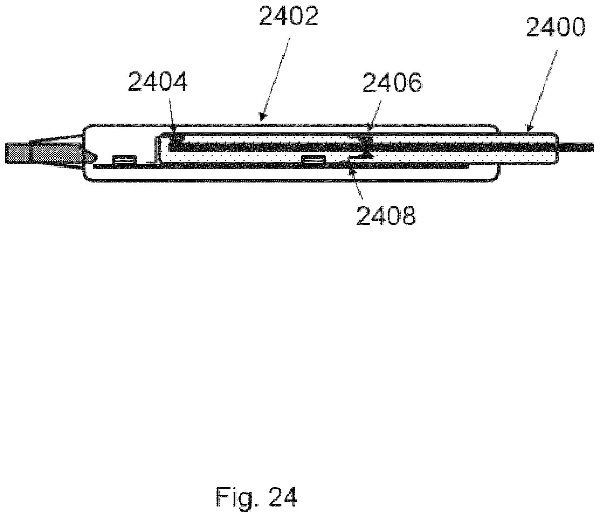

**Figur 24** zeigt eine Steckvorrichtung 2400, die mit einer komplementär ausgebildeten Einschubvorrichtung 2402 zusammengesteckt ist. Die Steckvorrichtung 2400 umfasst drei Leiterbahnen, wobei eine Leiterbahn die Leiterbahn einer ersten Elektrodenstruktur, eine zweite Leiterbahn, die Leiterbahn einer im Betrieb getriebenen, zweiten Elektrodenstruktur und eine dritte Leiterbahn, die Leiterbahn einer dritten Elektrodenstruktur ist, wobei die dritte Elektrodenstruktur im Betrieb die Funktion eines Berührungsschutzes für ein auf der zu behandelnden Seite abgewandten Seite anliegendes Erdpotential erfüllt sowie einen EMV-Schutz darstellt.

Da in der gezeigten Steckvorrichtung 2400 drei Leiterbahnen vorhanden sind, weist die als Kupplung ausgebildete Einschubvorrichtung 2402 einen Anschluss 2404 zum Übertragen eines Spannungssignals an die im Betrieb getriebene, zweite Elektrodenstruktur und zwei weitere Anschlüsse 2406, 2408 zum Kontaktieren der beiden Leiterbahnen der ersten und dritten Elektrodenstruktur, welche bevorzugt auf Massepotenzial liegen, auf. Im Vergleich zu der in Fig. 2 gezeigten Einschubvorrichtung ist also ein zusätzlicher Anschluss 2406 zum Kontaktieren der zweiten Massenelektrode vorgesehen.

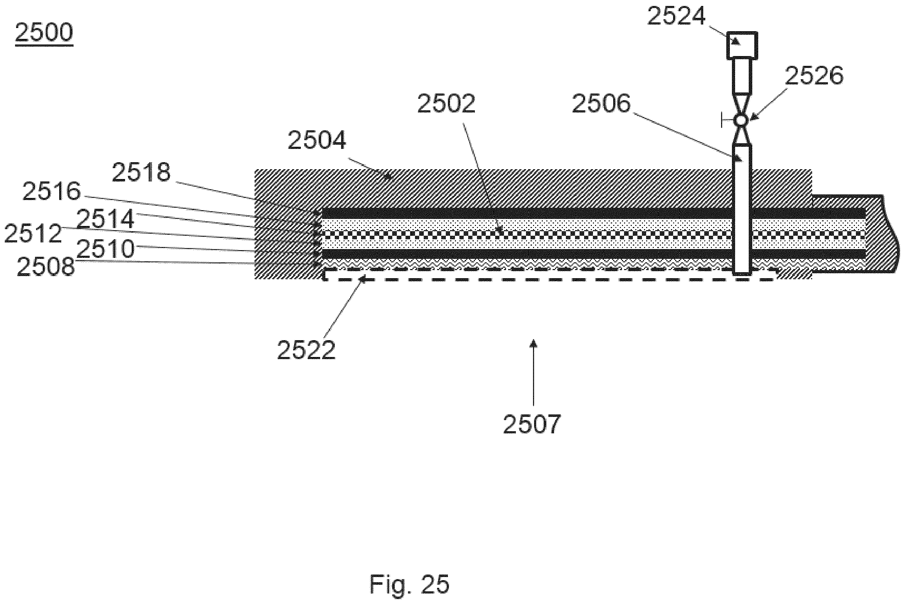

**Figur 25** zeigt einen Plasmaapplikator 2500 mit einem elektrotechnischen Kern 2502, einer Umschließung 2504 und einem Zugangsanschluss 2506. Der elektrotechnische Kern 2502 ist wie in Bezug auf Figur 19 beschrieben ausgebildet und umfasst in Schichtdickenrichtung von der einer zu behandelnden Oberfläche zugewandten Seite 2507 beginnend eine erste Isolationsschicht 2508, eine erste Elektrodenstruktur 2510, eine zweite Isolationsschicht 2512, eine zweite Elektrodenstruktur 2514, eine dritte Isolationsschicht 2516 und eine dritte Elektrodenstruktur 2518. Im Betrieb liegen die erste und die dritte Elektrodenstruktur 2510, 2518 auf Masse. Der hier gezeigte elektrotechnische Kern 2502 ist also bereits für sich berührungssicher ausgebildet. Die zweite Elektrodenstruktur 2514 wird im Betrieb mit einem zum Zünden eines Plasmas ausreichenden Spannungssignals beaufschlagt. In hier nicht gezeigten Ausführungsformen weist der Plasmaapplikator jeweils einen unterschiedlich ausgebildeten elektrotechnischen Kern auf, der beispielweise in einer Ausführungsform lediglich eine zweite Elektrodenstruktur und eine zweite Isolationsschicht umfasst.

Der Zugangsanschluss 2506 ist in der gezeigte Ausführungsform als schlauchförmige Tülle ausgebildet und senkrecht in Bezug auf eine zu behandelnde Oberfläche durch die Umschließung 2504 und den elektrotechnischen Kern 2502 geführt. Hierfür weisen der elektrotechnische Kern 2502 und die Umschließung 2504 jeweils eine Durchführung auf, welche einem Durchmesser aufweist, der dem Außendurchmesser der schlauchförmigen Tülle entspricht. Die Tülle 2506 ist innen hohl, sodass ein fluides Medium durch die Tülle durchgeführt werden kann. Das eine Ende der Tülle 2506 befindet sich im Anwendungsfall in einem zwischen dem Plasmaapplikator 2500 und einer zu behandelnden Oberfläche gebildeten, abgeschlossenen Gasraum 2522. Das andere Ende der Tülle 2506 befindet sich außerhalb des Plasmaapplikators 2500 auf der einer zu behandelnden Oberfläche abgewandten Seite, sodass, wenn ein Plasmaapplikator auf einer zu behandelnden Oberfläche angeordnet ist, durch die schlauchförmige Tülle 2506 ein oder auch mehrere fluide Medien in den abgeschlossenen Gasraum 2522 hinzugefügt oder aus dem abgeschlossenen Gasraum 2522 abtransportiert werden können.

In der gezeigten Ausführungsform weist die Tülle 2506 eine weibliche Muffe 2524 auf, um einen Schlauch (nicht gezeigt) mit einem komplementären Gewinde an der schlauchförmigen Tülle 2506 zu befestigen. Über einen an die weibliche Muffe 2524 angeschlossenen Schlauch (nicht gezeigt) kann ein fluides Medium in den bzw. aus dem abgeschlossenen Gasraum 2522 hinzugeführt und/oder abtransportiert werden. Beispielsweise kann ein Schlauch (nicht gezeigt) mit einer Vakuumpumpe (nicht gezeigt) in Verbindung stehen und durch die Vakuumpumpe ein Unterdruck in dem abgeschlossenen Gasraum 2522 erzeugt werden.

In der gezeigten Ausführungsform weist die schlauchförmige Tülle 2506 ein integriertes Ventil 2526 auf, mit dem ein Durchfluss eines fluiden Mediums durch die schlauchförmige Tülle 2506 geregelt und gestoppt werden kann. Ein solches Ventil 2526 kann manuell, maschinell oder elektronisch regelbar sein.

In einer hier nicht gezeigten Ausführungsform weist ein Plasmaapplikator eine Umschlie-βung und einen wie in Bezug auf Figur 25 beschriebenen Zugangsanschluss und einen wie in Bezug auf Figur 7 beschriebenen elektrotechnischen Kern auf. In einer weiteren hier nicht gezeigten Ausführungsform weist ein Plasmaapplikator eine Umschließung und einen wie in Bezug auf Figur 25 beschriebenen Zugangsanschluss und einen elektrotechnischen Kern mit lediglich einer Elektrodenstruktur auf, die im Betrieb mit einem Spannungssignal beaufschlagt wird. In dieser Ausführungsform erfüllt eine zu behandelnde Oberfläche im Betrieb die Funktion einer Gegenelektrode.

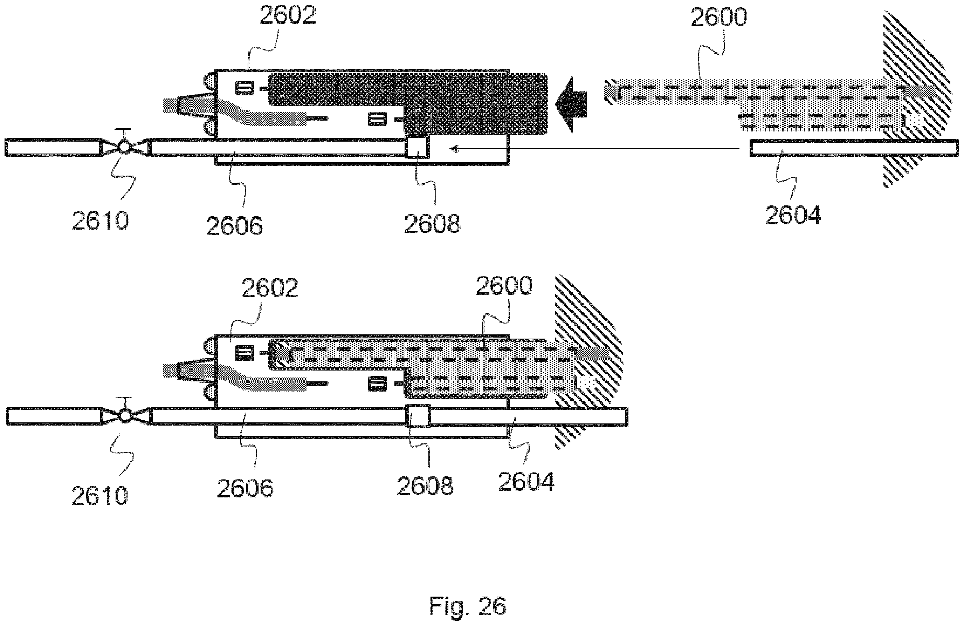

**Figur 26** zeigt eine Steckvorrichtung 2600 und eine zu der Steckvorrichtung 2600 komplementär ausgebildete Einschubvorrichtung 2602 auf. Die Steckvorrichtung 2600 ist an einem elektrotechnischen Kern eines Plasmaapplikators angeordnet, der in der gezeigten Darstellung lediglich angedeutet ist. Die Steckvorrichtung 2600 ist wie in Bezug auf Figuren 2A und 2B beschrieben ausgebildet, weist jedoch zusätzlich einen Zugangsanschluss 2604 auf. In der gezeigten Darstellung ist der Zugangsanschluss 2604 in einem Abstand neben den Laschen der Steckvorrichtung 2600 geführt, bildet jedoch eine Komponente der Steckvorrichtung.

Die Einschubvorrichtung 2602 ist ebenfalls wie in Bezug auf Figuren 2A und 2B beschrieben ausgebildet, weist jedoch zusätzlich einen Schlauch 2606 mit einer Muffe 2608 und einem Ventil 2610 auf. In einer hier nicht gezeigten Ausführungsform weist eine wie in Bezug auf Figur 24 beschrieben ausgebildete Steckvorrichtung einen Zugangsanschluss und eine wie in Bezug auf Figur 24 beschrieben ausgebildete Einschubvorrichtung einen Schlauch mit einer Muffe und einem Ventil auf.

In der gezeigten Ausführungsform ist also ein Zugangsanschluss 2604 in Form einer Tülle Teil der Steckvorrichtung 2600. In der komplementären Einschubvorrichtung 2602 befindet sich das Gegenstück zur Tülle 2604. Die Tülle 2604 kann also über die Muffe 2608 mit dem Schlauch 2606 verbunden werden, sodass ein fluides Medium über den Schlauch 2606 in der Einschubvorrichtung 2602 an die Tülle 2604 der Steckvorrichtung 2600 geleitet werden kann. Im unten dargestellten zusammengefügten Zustand von Steckvorrichtung 2600 und Einschubvorrichtung 2602 gehen Steckvorrichtung 2600 und Einschubvorrichtung 2602 eine wasser- und luftdichte Verbindung ein.

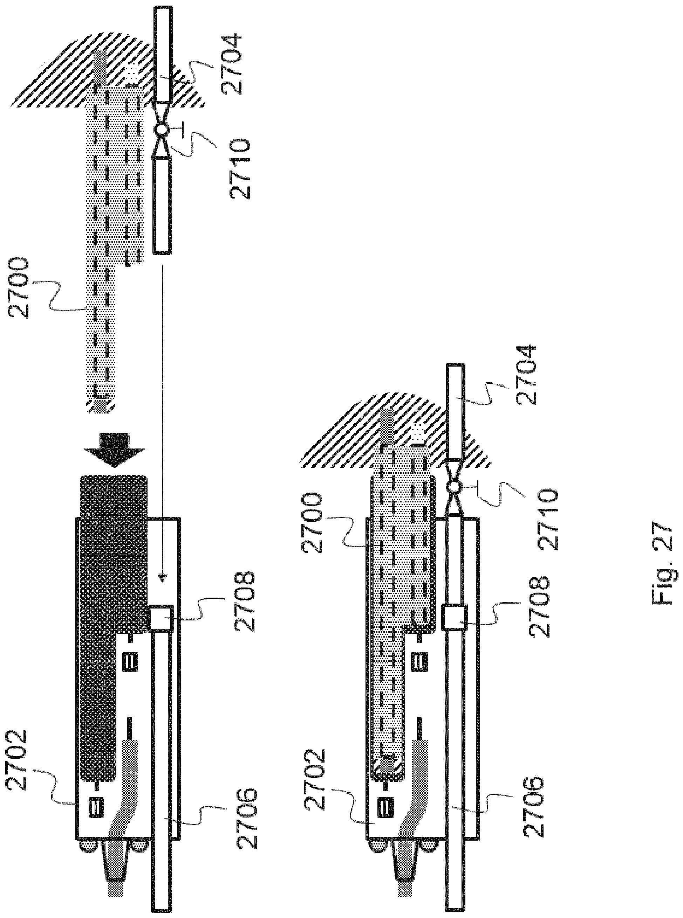

**Figur 27** zeigt eine Steckvorrichtung 2700 und eine zu der Steckvorrichtung 2700 komplementär ausgebildete Einschubvorrichtung 2702. Die Steckvorrichtung 2700 ist an einem elektrotechnischen Kern eines Plasmaapplikators angeordnet, der in der gezeigten Darstellung lediglich angedeutet ist. Die Steckvorrichtung 2700 ist wie in Bezug auf Figuren 2A und 2B beschrieben ausgebildet, weist jedoch zusätzlich einen Zugangsanschluss 2704 mit einem Ventil 2710 auf. In der gezeigten Darstellung ist der Zugangsanschluss 2704 in einem Abstand neben den Laschen der Steckvorrichtung 2700 geführt, bildet jedoch eine Komponente der Steckvorrichtung 2700.

Die Einschubvorrichtung 2702 ist ebenfalls wie in Bezug auf Figuren 2A und 2B beschrieben ausgebildet, weist jedoch zusätzlich einen Schlauch 2706 mit einer Muffe 2708 auf. In einer hier nicht gezeigten Ausführungsform weist eine wie in Bezug auf Figur 24 beschrieben ausgebildete Steckvorrichtung einen Zugangsanschluss mit Ventil und eine wie in Bezug auf Figur 24 beschrieben ausgebildete Einschubvorrichtung einen Schlauch mit einer Muffe auf.

In der gezeigten Ausführungsform ist also ein Zugangsanschluss 2704 in Form einer Tülle Teil der Steckvorrichtung 2700. In der komplementären Einschubvorrichtung 2702 befindet sich das Gegenstück zur Tülle 2704. Die Tülle 2704 kann also über die an einem entsprechenden Ende des Schlauchs angebrachte Muffe 2708 mit dem Schlauch 2706 verbunden werden, sodass ein fluides Medium über den Schlauch 2706 in der Einschubvorrichtung 2702 an die Tülle 2704 der Steckvorrichtung 2700 geleitet werden kann. Im unten gezeigten zusammengefügten Zustand von Steckvorrichtung 2700 und Einschubvorrichtung 2702 gehen Steckvorrichtung 2700 und Einschubvorrichtung 2702 eine wasser- und luftdichte Verbindung ein. Der Durchfluss eines fluiden Mediums kann durch Einstellen des Ventils des Zugangsanschlusses 2704 geregelt oder gestoppt werden.

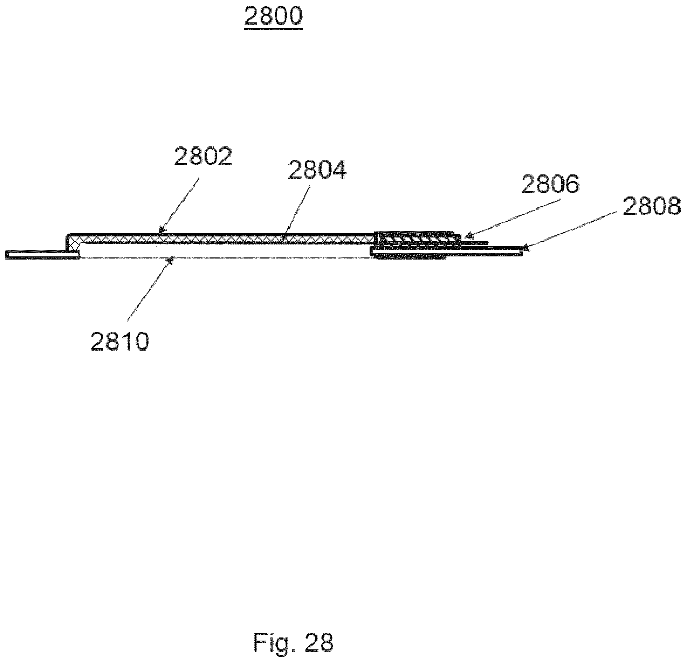

**Figur 28** zeigt einen Plasmaapplikator 2800, der eine Umschließung 2802, einen elektrotechnischen Kern 2804 und eine Steckvorrichtung 2806 mit einem Zugangsanschluss 2808 umfasst. Im Anwendungsfall ist zwischen dem Plasmaapplikator 2800 und einer zu behandelnden Oberfläche ein abgeschlossener Gasraum 2810 gebildet. Mit dem einen Ende endet der Zugangsanschluss 2808 in dem Gasraum 2810 und mit dem anderen Ende endet der Zugangsanschluss 2808 außerhalb des Plasmaapplikators 2800, sodass ein fluides Medium durch den Zugangsanschluss von außerhalb des Plasmaapplikators in den abgeschlossenen Gasraum 2810 zugeführt oder aus dem angeschlossenen Gasraum 2810 abgeführt werden kann. Insbesondere kann die Steckvorrichtung 2806 mit einer komplementären Einschubvorrichtung (nicht gezeigt) zusammengesteckt werden, die einen Schlauch aufweist, der beispielsweise über eine Muffe oder durch zusammenstecken mit dem Zugangsanschluss verbunden werden kann.

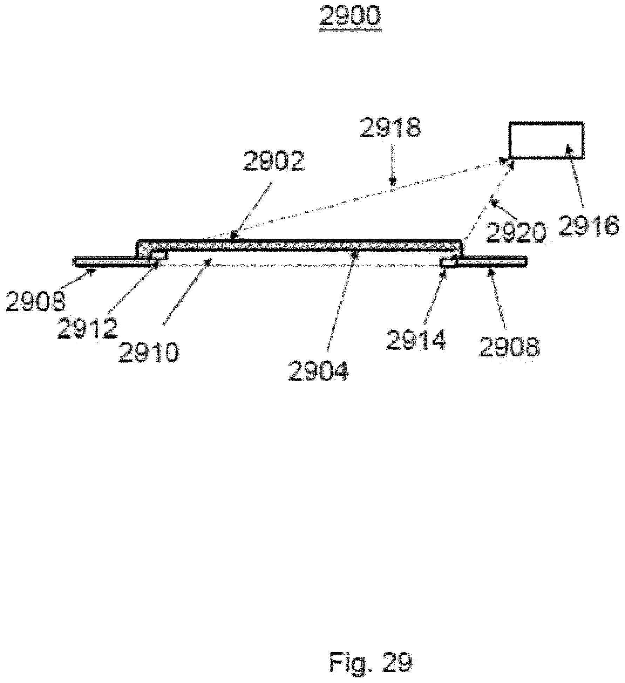

**Figur 29** zeigt einen Plasmaapplikator 2900 mit einer Sensorik. Der Plasmaapplikator 2900 umfasst weiterhin eine Umschließung 2902 und einen elektrotechnischen Kern 2904. In einem wenigstens in einem Bereich entlang eines Umfangs des Plasmaapplikators 2900, weist der Plasmaapplikator eine Adhäsionsschicht 2908 auf. Im Anwendungsfall stellt die Umschließung 2902 einen abgeschlossenen Gasraum 2910 zwischen dem Plasmaapplikator 2900 und einem zu behandelnden Körperabschnitt her. Die Sensorik umfasst einen ersten Sensor 2912 und einen zweiten Sensor 2914. Der erste Sensor 2912 ist in einem Abstand zu einem zu behandelnden Körperabschnitt an dem Plasmaapplikator angebracht und ist ausgebildet, eine für den Gasraum 2910 charakteristische Messgröße zu erfassen und ein die erfasste Messgröße repräsentierendes Datensignal 2918 an ein Datenverarbeitungsgerät 2916 zu übermitteln. Der zweite Sensor 2914 ist im Anwendungsfall in direktem Kontakt mit einem zu behandelnden Körperabschnitt an dem Plasmaapplikator angebracht und ausgebildet, im Anwendungsfall eine physiologische Messgröße eines von dem Plasmaapplikator 2900 bedeckten Körperabschnitts zu erfassen und ein die erfasste Messgröße repräsentierendes Datensignal 2920 an ein Datenverarbeitungsgerät 2916 zu übermitteln.

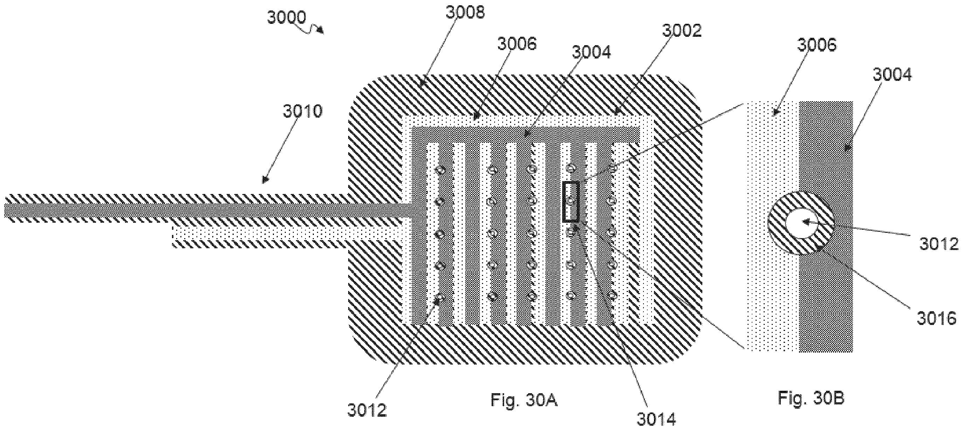

**Figur 30A** zeigt einen Plasmaapplikator 3000 mit einem elektrotechnischen Kern 3002, der eine erste Elektrodenstruktur 3004 und eine zweite Elektrodenstruktur 3006 aufweist. Der Plasmaapplikator weist weiterhin eine Umschließung 3008 und eine Steckvorrichtung 3010 auf.

Der elektrotechnische Kern 3002 weist Löcher bzw. Durchführungen 3012 auf, die über die gesamte Fläche des elektrotechnischen Kerns 3002 verteilt angeordnet sind. Durch die Löcher bzw. Durchführungen 3012 kann ein Medientransport durch den elektrotechnischen Kern 3002 von der einer zu behandelnden Oberfläche zugewandten Seite zu der einer zu behandelnden Oberfläche abgewandten Seite des Plasmaapplikators 3000 oder in umgekehrter Richtung von der einer zu behandelnden Oberfläche abgewandten Seite zu der einer zu behandelnden Oberfläche zugewandten Seite des Plasmaapplikators 3000 ermöglicht werden. Die Elektrodenstrukturen 3004, 3006 weisen einen Abstand zu den jeweiligen Löchern 3012 auf, sind also nicht Teil der eine Durchführung 3012 umschließenden Oberfläche des elektrotechnischen Kerns 3002. Die Umschließung 3008 ist von einem medientransportierenden Material gebildet.

**Figur 30B** zeigt den von dem Kasten 3014 eingerahmten Teilbereich des Plasmaapplikators 3000 in einer vergrößerten Darstellung. In diesem Teilbereich ist ein Teil eines Elektrodenabschnitts der ersten Elektrodenstruktur 3004 und ein Teil eines Elektrodenabschnitts der zweiten Elektrodenstruktur 3006 zu sehen. Weiterhin ist eine der Durchführungen 3012 zu sehen. Aus dieser Darstellung wird deutlich, dass die Elektrodenabschnitte nicht an die Durchführung heranreichen, sondern in einem Abstand zu der enden. Diejenige Oberfläche, die die Durchführung 3012 umschließt, wird ausschließlich von Isolationsschichten 3016 des elektrotechnischen Kerns 3002 gebildet.

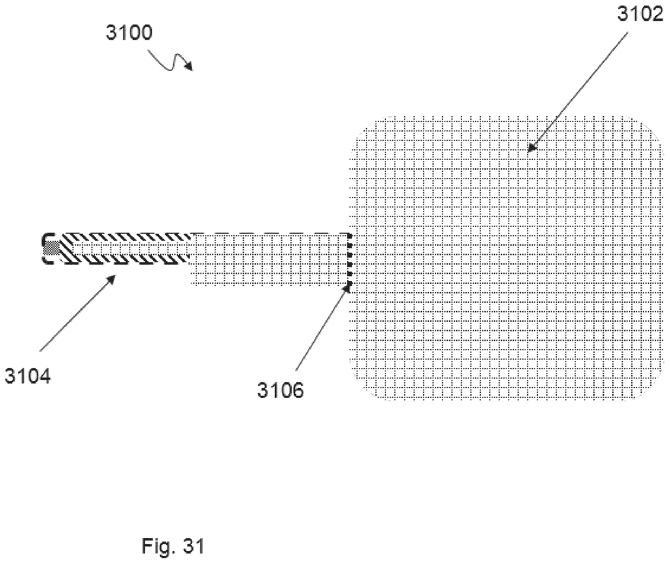

In **Figur 31** ist ein Plasmaapplikator 3100 mit einem elektrotechnischen Kern 3102 und einer Steckvorrichtung 3104 gezeigt. An dem Übergang von elektrotechnischem Kern 3102 und Steckvorrichtung 3104 weist der Plasmaapplikator 3100 eine Perforation 3106 auf. Die Funktion der Perforation 3106 ist die Reduktion der Festigkeit zwischen Steckvorrichtung 3104 und elektrotechnischem Kern 3102. Die Perforation 3106 stellt eine Sollbruchstelle dar. An dieser Perforation 3106 kann die Steckvorrichtung 3104 nach einer Plasmabehandlung von dem elektrotechnischen Kern 3102 abgerissen bzw. entfernt werden. Dadurch kann ein Plasmaapplikator 3100 weiterhin, für einen längeren Zeitraum von Tagen bis hin zu Wochen, unabhängig von einer Energieversorgungseinheit auf einer zu behandelnden Oberfläche verbleiben, da die nicht mehr benötigte Steckvorrichtung 3104 entfernt werden.

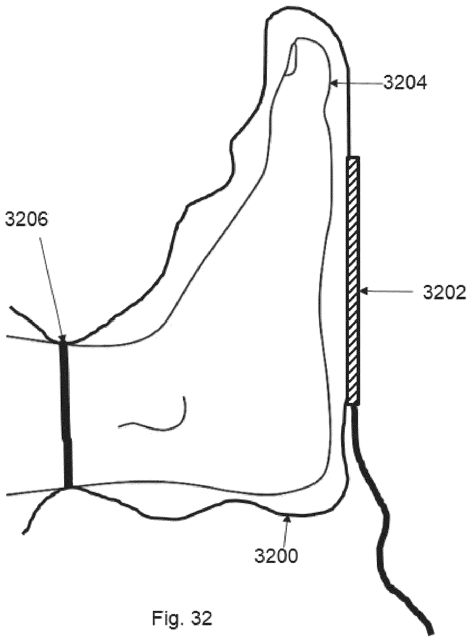

**Figur 32** zeigt einen an einem Beutel 3200 befestigten Plasmaapplikator 3202. Der Plasmaapplikator ist an dem Beutel 3200 fixiert und der Beutel 3200 umschließt einen zu behandelnden Fuß und bildet so einen angeschlossenen Gasraum 3204. Der Beutel 3200 ist aus einer dünnen Folie gebildet und oberhalb des Knöchels mittels eines Gummis oder eines Bandes 3206 fixiert.

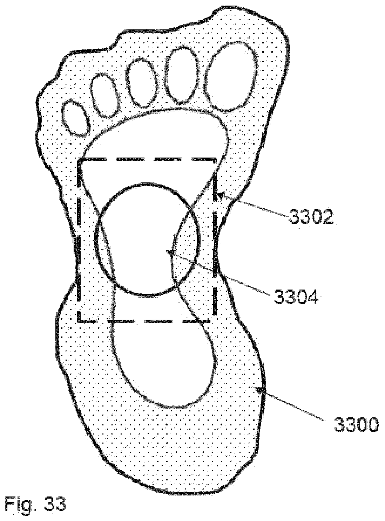

In **Figur 33** ist ein an einem Beutel 3300 befestigter Plasmaapplikator 3302 gezeigt. Der Plasmaapplikator 3302 ist lediglich als Kontur dargestellt, sodass sichtbar ist, das der Beutel 3300 ein Loch 3304 aufweist, über dem der Plasmaapplikator 3302 befestigt ist. Im Anwendungsfall kann ein gezündetes Plasma durch das Loch 3304 in den Beutel 3300 eintreten und mit der zu behandelnden Oberfläche, hier dem Fuß, wechselwirken. Somit wird eine großflächige Behandlung beispielsweise eines Fußes oder wenigstens einer Fußunterseite ermöglicht.

Aspekte des elektrotechnischen Kerns:
1. Elektrotechnischer Kern zum Erzeugen eines kalten Atmosphärendruck- oder Niederdruckplasmas für die Behandlung von menschlichen und/oder tierischen und/oder technischen Oberflächen, wobei der elektrotechnische Kern eine der zu behandelnden Oberfläche zugewandten Seite und eine der zu behandelnden Oberfläche abgewandten Seite aufweist und von der der zu behandelnden Oberfläche zugewandten Seite beginnend die folgenden übereinander angeordneten Schichten umfasst:
   - eine erste Isolationsschicht,
   - eine erste Elektrodenstruktur, die mit einer ersten Kontaktierung zum Herstellen eines elektrischen Kontakts zwischen der ersten Elektrodenstruktur und einer Energieversorgungseinheit versehen ist, und im Betrieb geerdet ist,
   - eine zweite Isolationsschicht, die ausgebildet ist, die erste Elektrodenstruktur und eine zweite Elektrodenstruktur galvanisch voneinander zu trennen,
   - eine zweite Elektrodenstruktur, die mit einer zweiten Kontaktierung zum Herstellen eines elektrischen Kontakts zwischen der zweiten Elektrodenstruktur und einer Energieversorgungseinheit versehen ist, und im Betrieb durch ein von einer Energieversorgungseinheit bereitgestellten und zum Zünden eines Plasmas ausreichendem Spannungssignal getrieben wird,
   - eine dritte Isolationsschicht, die ausgebildet ist, die zweite Elektrodenstruktur und eine dritte Elektrodenstruktur galvanisch voneinander zu trennen, und
   - eine dritte Elektrodenstruktur, die mit einer dritten Kontaktierung versehen ist, um die dritte Elektrodenstruktur im Betrieb zu erden.
2. Elektrotechnischer Kern nach Aspekt 1 mit einer Steckvorrichtung, wobei die erste und zweite Kontaktierung entsprechend eine erste und zweite Leiterbahn der Steckvorrichtung bilden, die jeweils an derselben Längsseite des elektrotechnischen Kerns jeweils von der entsprechenden Elektrodenstruktur von deren Längsseite abstehen und wobei die Steckvorrichtung weiterhin eine isolierende Lasche aufweist, die entsprechend mit der zweiten Isolationsschicht verbunden ist, wobei die erste und die zweite Leiterbahn durch die isolierende Lasche galvanisch voneinander getrennt sind.
3. Elektrotechnischer Kern nach Aspekt 1 oder 2, wobei der elektrotechnische Kern weiterhin eine Distanzstruktur aufweist, die auf der der zu behandelnden Oberfläche zugewandten Seite des elektrotechnischen Kerns an die erste Isolationsschicht angrenzend angeordnet ist, sodass sich die Distanzstruktur während einer Plasmabehandlung zwischen einer zu behandelnden Oberfläche und der ersten Isolationsschicht befindet.
4. Elektrotechnischer Kern nach wenigstens einem der Aspekte 1 bis 3, wobei eine Kontaktierung oder eine Elektrodenstruktur wenigstens ein Merkmal aufweist, welches sich infolge eines Gebrauchs derart ändert, dass kein zum Zünden eines Plasmas ausreichendes Spannungssignal mehr an die zweite Elektrodenstruktur übertragen werden kann.
5. Elektrotechnischer Kern nach Aspekt 4, wobei das Merkmal von einer Verjüngung einer Kontaktierung oder eines Elektrodenabschnitts der zweiten Elektrodenstruktur gebildet ist, die kurz vor einem Ende des erstmaligen Gebrauchs durch einen dann bereitzustellenden hohen Strompuls zerstört wird.

Aspekte des Plasmaapplikators:
1. Plasmaapplikator mit einem elektrotechnischen Kern gemäß wenigstens einem der Aspekte 1 bis 5 des elektrotechnischen Kerns, wobei der Plasmaapplikatorweiterhin eine Umschließung mit einer Tasche umfasst, die ausgebildet ist, dass ein elektrotechnischer Kern in die Tasche eingeschoben werden kann und dann wenigstens teilweise von der Umschließung umschlossen ist.
2. Plasmaapplikator nach Aspekt 6, wobei die Umschließung aus einem biokompatiblen Material, wie z.B. medizinischem Silikon, einem Lack, einem Klebstoff, einer Folie, einem Textil, einem Kompressionstextil oder organischem Material wie z.B. Mull, Zellstoff oder Baumwolle gebildet ist.
3. Plasmaapplikator nach Aspekte 1 oder 2, wobei die Umschließung wenigstens einer Lage mit flüssigkeitsabsorbierenden und/oder flüssigkeitsabführenden und/oder flüssigkeitsverteilenden Materialien umfasst.
4. Plasmaapplikator nach wenigstens einem der Aspekte 1 bis 3, wobei die Umschlie-βung Einschubschlitze umfasst, die an der der zu behandelnden Oberfläche abgewandten Seite des Plasmaapplikators angeordnet und ausgebildet sind, dass eine zu den Einschubschlitzen komplementäre Energieversorgungseinheit oder Einschubvorrichtung in die Einschubschlitze eingeschoben werden kann, um dann elektrisch mit den Kontaktierungen des elektrotechnischen Kerns elektrisch verbunden zu sein.
5. Plasmaapplikator nach wenigstens einem der Aspekte 1 bis 4, wobei der Plasmaapplikator einen Zugangsanschluss umfasst, der derart angeordnet und ausgebildet ist, dass ein fluides Medium während einer Plasmabehandlung einem zwischen dem elektrotechnischen Kern und einerzu behandelnden Oberfläche durch die Umschlie-βung gebildeten abgeschlossenen Gasraum zu- oder aus dem Gasraum abgeführt werden kann.
6. Plasmaapplikator nach wenigstens einem der Aspekte 1 bis 5, wobei der Plasmaapplikator auf einer einer zu behandelnden Oberfläche zugewandten Seite des Plasmaapplikators einen mit Wiederhaken besetzen Teil eines Klettverschlusses aufweist.
7. Plasmaapplikator nach wenigstens einem der Aspekte 1 bis 6, wobei der Plasmaapplikator eine integrierte Energieversorgungseinheit aufweist, die einen Energiespeicher umfasst, der elektrisch mit den Kontaktierungen des elektrotechnischen Kerns verbunden ist, um im Betrieb ein zum Zünden eines Plasmas ausreichendes Spannungssignal an wenigstens eine der Elektrodenstrukturen zu übertragen.
8. Plasmaapplikator nach Aspekt 7, wobei die integrierte Energieversorgungseinheit eine elektrische Schaltung aufweist, die ausgebildet ist, eine von dem Energiespeicher bereitgestellte Spannung in ein zum Zünden eines Plasmas ausreichendes Spannungssignal zu wandeln und dieses an die Kontaktierung wenigstens einer der Elektrodenstrukturen zu übertragen.
9. Plasmaapplikator nach Aspekt 7 oder 8, wobei der Plasmaapplikator eine mit wenigstens der Kontaktierung der zweiten Elektrodenstruktur elektrisch verbundene Energieaufnahmevorrichtung aufweist, die jeweils eine oder mehrere Empfangs-Spulenanordnungen enthält und wobei mittels elektromagnetischer Induktion elektrische Energie von einer Sende-Spulenanordnungen einer Energieabgabevorrichtung auf die Empfangs-Spulenanordnungen im Plasmaapplikator übertragen werden kann.

### Energieversorgungseinheit

Energieversorgungseinheit mit einer Einschubvorrichtung und einem Energiespeicher zum Verbinden mit einer Steckvorrichtung eines Plasmaapplikators und zum Bereitstellen eines zum Zünden eines Plasmas ausreichenden Spannungssignals an eine mit der der Einschubvorrichtung zusammengesteckte Steckvorrichtung, wobei die Energieversorgungseinheit eine elektrische Schaltung aufweist, die ausgebildet ist, eine von dem Energiespeicher bereitgestellte Spannung in ein zum Zünden eines Plasmas ausreichendes Spannungssignal zu wandeln und dieses an die Einschubvorrichtung zu übertragen.

### Bezugszeichenliste

- 1: Vorrichtung zur Erzeugung eines kalten Atmosphärendruckplasmas
- 2: Mehrschichtsystem
- 3: die der zu behandelnden Oberflächen zugewandte Seite
- 4: die der zu behandelnden Oberfläche abgewandten Seite
- 10: erste Elektrodenstruktur
- 10': zweite Elektrodenstruktur
- 11: erste isolierende Struktur
- 12: erste Elektrodenstruktur
- 13: Dielektrikumsschicht
- 14: zweite Elektrodenstruktur
- 15: zweite isolierende Struktur
- 16: Abstandshalterstruktur
- 17: dritte isolierende Struktur
- 20: isolierende Struktur
- 30: Behandlungsbereich
- 40: Adhäsionsschicht
- 45: Umschließung
- 50: elektrotechnischer Kern
- 60: Einschubvorrichtung
- 61: Silikontülle
- 63: Verjüngung
- 64: Einrastvorrichtung
- 65: Anschluss mit einem elektromagnetisch-verträglichen Schirm
- 66: Verschlussstopfen
- 67: Induktivitäten
- 68: Gehäuse der Einschubvorrichtung
- 69: Innenraum der Einschubvorrichtung
- 70: Steckvorrichtung
- 71: Hochspannungsanschluss (HV-Anschluss)
- 72: Masseanschluss (GND-Anschluss)
- 75: Versteifung
- 76: Bohrung mit einer Einrastfunktion
- 77: Kontakt an der Steckvorrichtung
- 78: Klemmkontakts
- 79: Leiterbahn
- 79`: zweite Leiterbahn
- 80: Kabel
- 81: Knickschutz
- 100: Plasmaapplikator
- 110: Energieversorgungseinheit
- 110': integrierte Energieversorgungseinheit
- 110": mobile Energieversorgungseinheit
- 112: Kontaktierung
- 114: zwei separate Kontakte
- 120`: integrierter Energieversorgungseinheit
- 120": integrierten Energieversorgungseinheit
- 122: Distanzstruktur
- 122`: Distanzstruktur
- 130: Einschubschlitz
- 140: Empfangs-Spulenanordnung
- 150: Sende-Spulenanordnung
- 160: induktiven Ladevorrichtung
- 200: Distanzstruktur
- 210: Wabe
- 220: Plasma
- 230: Elektrodenstruktur
- 240: Gegenelektrode
- 300: geschlossener Stromkreis
- 300`,: 300" andere geschlossene Stromkreise
- 310: Verbindungsstellen
- 910: Wundabdeckung
- 1900: Plasmaapplikator
- 1902: elektrotechnischen Kern
- 1904: Seite eines elektrotechnischen Kerns
- 1906: erste Isolationsschicht
- 1908: erste Elektrodenstruktur
- 1910: zweite Isolationsschicht
- 1912: zweite Elektrodenstruktur
- 1914: dritten Isolationsschicht
- 1916: dritte Elektrodenstruktur
- 1918: Umschließung
- 2000: elektrotechnischen Kerns
- 2002: zweite Elektrodenstruktur
- 2004: erste Elektrodenstruktur
- 2006: zweite Isolationsschicht
- 2008, 2010: Leiterbahn
- 2100: elektrotechnischer Kern
- 2102: dritte Isolationsschicht
- 2104: Lasche
- 2106: Ende einer Leiterbahn
- 2108: Kontaktfläche
- 2200: elektrotechnischen Kerns
- 2202: dritte Elektrodenstruktur
- 2204: Seite eines elektrotechnischen Kerns
- 2206: laschenförmige Leiterbahn
- 2208: Bereich
- 2210: Bereich
- 2212: dritte Isolationsschicht
- 2214: Ende einer Lasche
- 2216: Kontaktfläche einer Leiterbahn
- 2300: elektrotechnischen Kern
- 2302: dritte Elektrodenstruktur
- 2304: laschenförmigen Leiterbahn
- 2306: chipkartenförmige Versteifung
- 2308: Bereich
- 2310: Bereich
- 2314: Kontaktfläche
- 2316: Ende einer laschenförmige Leiterbahn
- 2318: Kontaktfläche einer Leiterbahn
- 2400: Steckvorrichtung
- 2402: Einschubvorrichtung
- 2404: Anschluss zum Übertragen eines Spannungssignals
- 2406, 2408: weitere Anschlüsse
- 2500: Plasmaapplikator
- 2502: elektrotechnischen Kern
- 2504: Umschließung
- 2506: Zugangsanschluss
- 2507: einer zu behandelnden Oberfläche zugewandten Seite
- 2508: eine erste Isolationsschicht
- 2510: erste Elektrodenstruktur
- 2512: zweite Isolationsschicht
- 2514: zweite Elektrodenstruktur
- 2516: dritte Isolationsschicht
- 2518: dritte Elektrodenstruktur
- 2522: Gasraum
- 2524: weibliche Muffe
- 2526: integriertes Ventil
- 2600: Steckvorrichtung
- 2602: Einschubvorrichtung
- 2604: Zugangsanschluss
- 2606: Schlauch
- 2608: Muffe
- 2610: Ventil
- 2700: Steckvorrichtung
- 2702: Einschubvorrichtung
- 2704: Zugangsanschluss
- 2706: Schlauch
- 2708: Muffe
- 2710: Ventil
- 2800: Plasmaapplikator
- 2802: Umschließung
- 2804: elektrotechnischen Kern
- 2806: Steckvorrichtung
- 2808: Zugangsanschluss
- 2810: Gasraum
- 2900: Plasmaapplikator
- 2902: Umschließung
- 2904: elektrotechnischer Kern
- 2908: Adhäsionsschicht
- 2912: erster Sensor
- 2914: zweiter Sensor
- 2916: Datenverarbeitungsgerät
- 2918: Datensignal
- 2920: Datensignal
- B1: Breite der Einschubvorrichtung
- B2: Breite der Steckvorrichtung
- B3: Breite der Steckvorrichtung
- H1: Höhe der Einschubvorrichtung
- H2: Höhe der Steckvorrichtung
- K1: minimaler Kriechweg
- L1: minimale Länge des Kriechwegs patientenseitig
- L2: Länge des zusammengesteckten Systems
- L3: Länge der Steckvorrichtung außerhalb der Einschubvorrichtung
- L4: Länge der Steckvorrichtung
- L5: Gesamtlänge der Steckvorrichtung

## Patentansprüche

1. Plasmaapplikator mit einem elektrotechnischen Kern, einer Umschließung und einem Zugangsanschluss, der derart angeordnet und ausgebildet ist, dass ein fluides Medium vor, während und/oder nach einer Plasmabehandlung einem zwischen dem elektrotechnischen Kern und einerzu behandelnden Oberfläche durch die Umschlie-βung gebildeten abgeschlossenen Gasraum zu- oder aus dem Gasraum abgeführt werden kann.

2. Plasmaapplikator nach Anspruch 1, wobei die Umschließung eine Tasche aufweist, die ausgebildet ist, dass ein elektrotechnischer Kern in die Tasche eingeschoben werden kann und dann wenigstens teilweise von der Umschließung umschlossen ist.

3. Plasmaapplikator nach Anspruch 1 oder 2, wobei die Umschließung wenigstens eine Lage mit flüssigkeitsabsorbierenden und/oder flüssigkeitsabführenden und/oder flüssigkeitsverteilenden Materialien umfasst.

4. Plasmaapplikator nach wenigstens einem der Ansprüche 1 bis 3, wobei die Umschließung aus einem biokompatiblen Material, wie medizinischem Silikon, einem Lack, einem Klebstoff, einer Folie, einem Textil, einem Kompressionstextil oder organischem Material wie Mull, Zellstoff oder Baumwolle gebildet ist.

5. Plasmaapplikator nach wenigstens einem der Ansprüche 1 bis 4, wobei die Umschließung den elektrotechnischen Kern vollständig umschließt und wenigstens in einem Teilbereich absorbierende Eigenschaften hat.

6. Plasmaapplikator nach wenigstens einem der Ansprüche 1 bis 5, wobei der elektrotechnische Kern in ein Unterdruck-Wundtherapie-System integriert ist.

7. Plasmaapplikator nach wenigstens einem der Ansprüche 1 bis 6, wobei die Umschließung durch eine Saugkompresse realisiert ist in die der elektrotechnische Kern integriert ist.

8. Plasmaapplikator nach wenigstens einem der Ansprüche 1 bis 7, wobei die Umschließung auf ihrer patientenabgewandten Seite von einer dünnen Folie gebildet ist, während sie auf der patientenzugewandten Seite aus einem oder mehreren Lagen Textil und /oder einem oder mehreren Absorbern besteht.

9. Plasmaapplikator nach wenigstens einem der Ansprüche 1 bis 8, wobei der elektrotechnische Kern Löcher oder Durchführungen aufweist, die in einem Teilbereich des elektrotechnischen Kerns oder über die gesamte Fläche des elektrotechnischen Kerns verteilt angeordnet sind.

10. Plasmaapplikator nach Anspruch 1 bis 9, wobei die Umschließung derart gestaltet ist, dass die Umschließung einen Medientransport durch den elektrotechnischen Kern hindurch von der einer zu behandelnden Oberfläche zugewandten Seite des Plasmaapplikators zu der einer zu behandelnden Oberfläche abgewandten Seite des Plasmaapplikators zulässt oder selbst ermöglicht.

11. Plasmaapplikator nach Anspruch 10, wobei die Umschließung von einem medientransportierenden Material gebildet ist oder Löcher und Durchführungen aufweist.

12. Plasmaapplikator nach wenigstens einem der Ansprüche 1 bis 11, der eine Distanzstruktur aufweist, die aus einem biokompatiblen Material wie Silikon, Textil/Silikon-Verbundmatrix, Mull, Absorber, Zellstoff, Wundgaze oder PU-Schaum und/oder aus einer Kombination der genannten Materialien besteht und vorzugsweise von einem V.A.C.-Schaum gebildet ist.

13. Plasmaapplikator nach Anspruch 12, bei dem die Distanzstruktur selbst eine Plasmaquelle ist und wenigstens eine Elektrodenstruktur und eine Reihe von Hohlräumen oder Durchführungen aufweist.

14. Plasmaapplikator nach wenigstens einem der Ansprüche 1 bis 13, bei dem die Umschließung eine V.A.C.-Folie aufweist.

15. Plasmaapplikator nach wenigstens einem der Ansprüche 1 bis 14,
- mit einem elektrotechnischen Kern, der berührungssicher ausgebildet ist, wobei die Umschließung einlagig ausgebildet ist und einen Zugangsanschluss aufweist, mit der ein fluides Medium aus dem abgeschlossenen Gasraum abtransportiert werden kann
oder
- mit einem elektrotechnischen Kern, wobei der Plasmaapplikator an einem Beutel fixiert ist, der dafür vorgesehen ist, ein bestimmtes Körperteil zu umschließen und so einen abgeschlossenen Gasraum zu bilden, wobei der Beutel ein Loch aufweist und der Plasmaapplikator so angeordnet ist, dass dieser das Loch abdeckt.

16. System mit einem Plasmaapplikator nach einem der Ansprüche 1 bis 15 und einer Vakuumpumpe, wobei der Zugangsanschluss als Tülle oder Nippel ausgebildet ist und an die Tülle oder den Nippel ein Schlauch angeschlossen ist, welcher mit einer Vakuumpumpe derart in Verbindung steht, dass ein durch die Vakuumpumpe erzeugter Unterdruck über den Schlauch an den Nippel oder die Tülle derart an den Plasmaapplikator geleitet werden kann, dass in dem abgeschlossenen Gasraum zwischen dem Plasmaapplikator und einer zu behandelnden Oberfläche ein Spülen, ein Absaugen, eine V.A.C.-Therapie sowie die Kombination der einzelnen Funktionen untereinander vor, nach und/oder während einer Plasmabehandlung erfüllbar sind.
